# FASCICULE DE BREVET EUROPEEN

(11) **EP 2 094 699 B1**
(45) Date de publication et mention de la délivrance du brevet: **20.07.2011**
(21) Numéro de dépôt: 07858418.2
(22) Date de dépôt: 10.10.2007
(51) Int. Cl.: C07D 471/04, A61K 31/437, A61P 35/00

(54) **NOUVEAUX DERIVES IMIDAZOLONES, LEUR PREPARATION A TITRE DE MEDICAMENTS, COMPOSITIONS PHARMACEUTIQUES, UTILISATION COMME INHIBITEURS DE PROTEINES KINASES NOTAMMENT CDC7**
NEUE IMIDAZOLON-DERIVATE, WIRKSTOFFPRÄPARAT DARAUS, PHARMAZEUTISCHE ZUSAMMENSETZUNGEN UND IHRE VERWENDUNG ALS HEMMER VON PROTEINKINASEN, INSBESONDERE CDC7
NEW IMIDAZOLONE DERIVATIVES, PREPARATION THEREOF AS DRUGS, PHARMACEUTICAL COMPOSITIONS, AND USE THEREOF AS PROTEIN KINASE INHIBITORS, IN PARTICULAR CDC7

(30) Priorité: 12.10.2006 FR 0608924
(43) Date de publication de la demande: 02.09.2009
(73) Titulaire: Sanofi-Aventis, 75013 Paris (FR)
(72) Inventeur: LEROY, Vincent, 75013 Paris (FR); BACQUE, Eric, 75013 Paris (FR); CONSEILLER, Emmanuel, 75013 Paris (FR); STEINMETZ, Anke, 75013 Paris (FR); RONAN, Baptiste, 75013 Paris (FR); LETALLEC, Jean-Philippe, 75013 Paris (FR)
(74) Mandataire: Le Coupanec, Pascale A.M.P.
(86) Numéro de dépôt international: PCT/FR2007/001651
(87) Numéro de publication internationale: WO 2008/046982

(56) Documents cités:
- WO-A-2006/040049
- WO-A1-2005/014572
- DATABASE CHEMCATS CHEMICAL ABSTRACTS SERVICE, COLUMBUS, OHIO, US; 1 août 2005 (2005-08-01), XP002431747 extrait de STN & "TimTec Overseas Stock" 1 août 2005 (2005-08-01), TIMTEC INC , NEWARK, DE, USA

## Description

La présente invention concerne de nouveaux dérivés d'imidazolones, leur procédé de préparation, les nouveaux intermédiaires obtenus, leur application à titre de médicaments, les compositions pharmaceutiques les renfermant et les nouvelles utilisations de tels dérivés d'imidazolones.

L'invention a ainsi pour objet de nouveaux dérivés d'imidazolones dotés d'effets inhibiteurs vis-à-vis de protéines kinases.

Les produits de la présente invention peuvent ainsi notamment être utilisés pour la prévention ou le traitement d'affections capables d'être modulées par l'inhibition de l'activité de protéines kinases.

Les produits de la présente demande comme inhibiteurs de protéines kinases peuvent tout particulièrement être utilisés pour le traitement ou la prévention des cancers. Le cancer reste une maladie pour laquelle les traitements existants sont insuffisants. Certaines protéines kinases jouent un rôle important dans de nombreux cancers. L'inhibition de telles protéines kinases est potentiellement importante dans la chimiothérapie de cancers notamment pour supprimer la croissance ou la survie des tumeurs.

La présente invention concerne donc l'identification de nouveaux produits qui inhibent de telles protéines kinases.

L'inhibition et la régulation de protéines kinases constituent notamment un nouveau puissant mécanisme d'action pour le traitement d'un grand nombre de tumeurs solides.

De telles affections que peuvent traiter les produits de la présente demande sont donc tout particulièrement les tumeurs solides.

### Protéines kinases

Les protéines kinases sont une famille d'enzymes qui catalysent la phosphorylation de groupes hydroxy de résidus spécifiques de protéines tels que des résidus tyrosine, sérine ou thréonine. De telles phosphorylations peuvent largement modifier la fonction des protéines ; ainsi, les protéines kinases jouent un rôle important dans la régulation d'une grande variété de processus cellulaires, incluant notamment le métabolisme, la prolifération cellulaire, la différentiation cellulaire ou la survie cellulaire. Parmi les différentes fonctions cellulaires dans lesquelles l'activité d'une protéine kinase est impliquée, certains processus représentent des cibles attractives pour traiter certaines maladies. Comme exemple, on peut citer notamment l'angiogénèse et le contrôle du cycle cellulaire, dans lesquels les protéines kinases peuvent jouer un rôle essentiel. Ces processus sont essentiels pour la croissance des tumeurs solides ainsi que d'autres maladies.

Les protéines kinases participent aux évènements de signalisation qui contrôlent l'activation, la croissance et la différentiation des cellules en réponse, soit à des médiateurs extracellulaires, soit à des changements de l'environnement. En général, ces kinases appartiennent à deux groupes: celles qui phosphorylent préférentiellement les résidus sérines et/ou thréonine et celles qui phosphorylent préférentiellement les résidus tyrosines [S.K.Hanks and T.Hunter, FASEB. J., 1995, 9, pages 576-596]. Les sérine/thréonine kinases sont par exemple, les isoformes des protéines kinases C [A.C.Newton, J. Biol. Chem., 1995, 270, pages 28495-28498] et un groupe de kinases dépendantes des cyclines, comme cdc2 (cdk1) [J.Pines, Trends in Biochemical Sciences, 1995, 18, pages 195-197]. Les tyrosine kinases comprennent les récepteurs aux facteurs de croissance comme le récepteur au facteur de croissance épidermal (EGF) [S.Iwashita and M.Kobayashi, Cellular Signalling, 1992, 4, pages 123-132], et des kinases cytosoliques comme p56tck, p59fYn, ZAP-70 et les kinases csk [C. Chan et. al., Ann. Rev. Immunol., 1994, 12, pages 555-592].

Des niveaux anormalement élevés d'activité protéine kinase ont été impliqués dans de nombreuses maladies, résultant en des fonctions cellulaires anormales. Ceci peut provenir soit directement soit indirectement, d'un disfonctionnement dans les mécanismes de contrôle de l'activité kinase, lié par exemple à une mutation, une sur-expression ou une activation inappropriée de l'enzyme, ou par une sur- ou sous-production de cytokines ou des facteurs de croissance, également impliqués dans la transduction des signaux en amont ou en aval des kinases. Dans tous ces cas, une inhibition sélective de l'action des kinases laisse espérer un effet bénéfique.

Parmi ces protéines kinases, on cite tout particulièrement la protéine kinase Cdc7.

Cdc7 est une kinase de serine/thréonine qui a été caractérisée au niveau moléculaire comme facteur essentiel pour le déclenchement de la réplication de l'ADN.

L'activité catalytique de Cdc7, conservée à travers les eucaryotes, est dépendante de sa sous-unité régulatrice Dbf4. Bien que l'expression de Cdc7 (au niveau ARN messager et protéique) soit constante au cours du cycle cellulaire, le niveau d'expression de Dbf4 est, quant à lui, dépendant du cycle cellulaire, qui induit une augmentation de l'activité kinase Cdc7 au cours de la transition G1-S. Pour cette raison, Cdc7 est désigné sous le nom de DDK (kinase dépendante de Dbf4).

L'activité principale du complexe Cdc7/Dbf4 a lieu à l'initiation de la réplication de l'ADN pendant la phase S. Il phosphoryle MCM2 qui active ainsi le complexe MCM (Maintenance du Mini Chromosome) qui est un constituant essentiel de l'activité ADN-hélicase

Cdc7 joue également un rôle important dans la mutagenèse induite, principalement par action au niveau des voies et des points de contrôle des dommages à l'ADN (DNA-damage checkpoints), en particulier dans le checkpoint dépendant de ATR qui empêche le déclenchement de la réplication d'ADN en réponse à des dommages dé type simple-brin provoqués par des agents chimique comme l'étoposide.

Cdc7 et Dbf4 sont sur-exprimés dans les lignées cellulaires tumorales humaines et dans beaucoup de spécimens de tumeur (poumon, sein, thyroïde, colon-rectum, oesophage, utérus, testicule, foie, (Hess et al, 1998 et données internes) en comparaison aux tissus normaux correspondant.

Des expériences d'extinction de l'expression de Cdc7 utilisant la technologie de RNA interférence (RNAi) montrent que l'inhibition de l'expression cdc7 induit un arrêt du cycle cellulaire et empêche la prolifération cellulaire des lignées cellulaires tumorales humaines HeLa et HCT116, mais produit un effet limité sur les cellules normales (fibroblastes cutanés humains normaux). Ceci se traduit par un arrêt prolongé en G1 induisant de l'apoptose dans des cellules déficientes en p53 (> 50% de tumeurs) mais réversible en cellules normales [A. Montagnoli et al., CANCER RESEARCH 64, 7110-7116, October 1, 2004].

Les inhibiteurs de l'activité de la kinase Cdc7 peuvent constituer une nouvelle classe de thérapie cytotoxique ciblée ainsi que d'inhibiteurs de la réplication de l'ADN. De tels inhibiteurs inhiberaient la réplication avant que les fourches de réplication ne soient établies, bloquant ainsi la réplication sans provoquer de dommages à l'ADN.

Des inhibiteurs de la protéine Kinase CDC7 sont décrits dans les demandes WO2005/014572 et WO2006/040049.

La présente demande concerne ainsi particulièrement de nouveaux inhibiteurs de la protéine kinase CDC7 pouvant être utilisés notamment pour le traitement de prolifération anormale de cellules et plus particulièrement en oncologie.

La présente invention a ainsi pour objet les produits de formule (I): 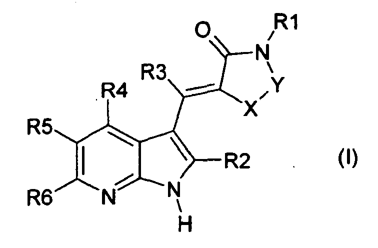 dans laquelle :
X-Y représente NH-C(S), N=C-NR7R8, N=C-SR, N=C-R ou N=COR ;
R1 représente l'atome d'hydrogène, un radical cycloalkyle ou un radical alkyle, hétérocycloalkyle, aryle ou hétéroaryle, tous ces radicaux étant éventuellement substitués;
R identique ou différent de R1 est choisi parmi les valeurs de R1 ;
R2 représente l'atome d'hydrogène , un atome d'halogène ou un radical alkyle ;
R3 représente l'atome d'hydrogène, un atome d'halogène , le radical hydroxyle ou un radical alkyle ou alcoxy ;
R4 représente l'atome d'hydrogène , un atome d'halogène ou un radical cyano, CF3 ou alkyle;
R5 représente l'atome d'hydrogène, un atome d'halogène, un radical hydroxyle, cyano, NR7R8, CONR7R8, NR11COR12 ou un radical cycloalkyle, alkyle, alcoxy, hétérocycloalkyle, aryle ou hétéroaryle, tous ces derniers radicaux étant éventuellement substitués ;
R6 représente l'atome d'hydrogène, un atome d'halogène ou un radical NR7R8, alkyle ou alcoxy ;
R7 et R8 sont tels que :
   soit R7 et R8 identiques ou différents sont tels que l'un de R7 et R8 représente l'atome d'hydrogène ou un radical alkyle éventuellement substitué; et l'autre de R7 et R8 représente un atome d'hydrogène ou un radical cycloalkyle, alkyle, hétérocycloalkyle, hétéroaryle ou aryle, tous ces radicaux étant éventuellement substitués ;
   soit R7 et R8 forment ensemble avec l'atome d'azote auquel ils sont liés un radical cyclique formé de 3 à 7 chaînons renfermant éventuellement un ou plusieurs autres hétéroatomes choisi(s) parmi O, S ou N, N étant éventuellement substitué par R11, ce radical cyclique étant lui-même éventuellement substitué;
   tous les radicaux alkyle, alcoxy, cycloalkyle hétérocycloalkyle, hétéroaryle et aryle, ainsi que le radical cyclique que peuvent former R7 et R8 ensemble avec l'atome d'azote auquel ils sont liés, indiqués comme éventuellement substitués, étant ainsi éventuellement substitués par un ou plusieurs radicaux identiques ou différents choisis parmi les atomes d'halogène et les radicaux hydroxyle, cyano, nitro, CF3, NR9R10, NHCOR11, NHCO2R11, NHCONR9R10, NHSO2R13, COOH, COOalk, CONR9R10, SO2NR9R10 alcoxy, alkylthio, haloalcoxy, haloalkylthio, alkyle, fluoroalkyle, hydroxyalkyle, cycloalkyle, hétérocycloalkyle, hétéroaryle et phényle, ces derniers radicaux hétéroaryle, aryle et phényle, étant eux-mêmes éventuellement substitués par un ou plusieurs radicaux identiques ou différents choisis parmi les atomes d'halogène et les radicaux hydroxyle, NH2, NH(alk), N(alk)2, alkyle, hydroxyalkyle et alcoxy ;
R9 et R10 sont tels que :
   soit R9 et R10 identiques ou différents sont tels que l'un de R9 et R10 représente l'atome d'hydrogène ou un radical alkyle éventuellement substitué par un ou plusieurs radicaux identiques ou différents choisis parmi les atomes d'halogène et les radicaux hydroxyle et alcoxy ; et l'autre de R9 et R10 représente un atome d'hydrogène ou un radical cycloalkyle, alkyle, hétérocycloalkyle, hétéroaryle ou aryle, tous ces radicaux étant eux-mêmes éventuellement substitués par un ou plusieurs radicaux identiques ou différents choisis parmi les atomes d'halogène et les radicaux hydroxyle, NH2, NH(alk), N(alk)2, alkyle, hydroxyalkyle et alcoxy ;
   soit R9 et R10 forment ensemble avec l'atome d'azote auquel ils sont liés un radical cyclique formé de 3 à 7 chaînons renfermant éventuellement un ou plusieurs autres hétéroatomes choisi(s) parmi O, S ou N, N étant éventuellement substitué par R12, ce radical cyclique étant lui-même éventuellement substitué par un ou plusieurs radicaux identiques ou différents choisis parmi les atomes d'halogène et les radicaux hydroxyle, NH2, NH(alk), N(alk)2, alkyle, hydroxyalkyle et alcoxy ;
R11 et R12, identiques ou différents représentent l'atome d'hydrogène ou un radical alkyle ou phényle éventuellement substitués par un ou plusieurs radicaux identiques ou différents choisis parmi les atomes d'halogène et les radicaux hydroxyle et alcoxy, le radical phényle étant lui-même éventuellement substitué par un ou plusieurs radicaux alkyle ;
R13 représente un radical alkyle ou phényle éventuellement substitués par un ou plusieurs radicaux identiques ou différents choisis parmi les atomes d'halogène et les radicaux hydroxyle et alcoxy, le radical phényle étant lui-même éventuellement substitué par un ou plusieurs radicaux alkyle ;
tous les radicaux alkyle (alk) et alcoxy ci-dessus étant linéaires ou ramifiés et renfermant au plus 12 atomes de carbone ;
lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

Parmi les produits de formule (I) tels que définis ci-dessus, on peut exclure les produits de formule (I) dans lesquels toutes les conditions ci-dessous sont remplies:
- R2 représente hydrogène ;
- R3 représente hydrogène ou alkyle;
- X-Y représente N=C-NR7R8, N=C-SR ou N=CR dans lesquels X représente N et Y représente =C-NR7R8, =C-SR ou =CR et avec R représente aryle ou hétéroaryle
- R1 représente H ou alk ;
- et R4, R5 et R6 sont tels que deux d'entre eux représentent H et l'autre représente hydrogène,NH2 ou NHalk.

La présente invention a notamment ainsi pour objet les produits de formule (I) tels que définis ci-dessus dans laquelle :
R1 représente l'atome d'hydrogène, ou un radical alkyle, tous ces radicaux étant éventuellement substitués comme indiqué ci-dessus ou ci-après;
et R5 représente, un atome d'halogène, un radical hydroxyle, cyano, NR7R8, CF3 ou un radical cycloalkyle, alkyle, alcoxy, hétérocycloalkyle, aryle ou hétéroaryle,
tous ces derniers radicaux étant éventuellement substitués comme indiqué ci-dessus ou ci-après;
les autres radicaux substituants R2,R3,R4,R6 et X-Y ayant les valeurs définies comme indiqué ci-dessus ou ci-après,
lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

La présente invention a notamment ainsi pour objet les produits de formule (I) tels que définis ci-dessus dans laquelle :
R2 représente l'atome d'hydrogène ;
et R5 représente un atome d'halogène, un radical hydroxyle, cyano, NR7R8, CF3 ou un radical cycloalkyle, alkyle, alcoxy, hétérocycloalkyle, aryle ou hétéroaryle, tous ces derniers radicaux étant éventuellement substitués comme indiqué ci-dessus ou ci-après;
les autres radicaux substituants R1,R3,R4,R6 et X-Y ayant les valeurs définies comme indiqué ci-dessus ou ci-après,
lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

La présente invention a notamment ainsi pour objet les produits de formule (I) tels que définis ci-dessus dans laquelle :
R3 représente l'atome d'hydrogène;
et R5 représente un atome d'halogène, un radical hydroxyle, cyano, CONR7R8 ou un radical cycloalkyle, alkyle, alcoxy, hétérocycloalkyle, aryle ou hétéroaryle, tous ces derniers radicaux étant éventuellement substitués comme indiqué ci-dessus ou ci-après;
les autres radicaux R2,R3,R4,R6 et X-Y ayant les valeurs définies comme indiqué ci-dessus ou ci-après,
lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

La présente invention a notamment ainsi pour objet les produits de formule (I) tels que définis ci-dessus dans laquelle :
X-Y représente NH-C(S), N=C-NR7R8 ou N=C-R;
R1 représente l'atome d'hydrogène, un radical cycloalkyle ou un radical alkyle, hétérocycloalkyle, aryle ou hétéroaryle, tous ces radicaux étant éventuellement substitués comme indiqué ci-dessus ou ci-après;
R identique ou différent de R1 est choisi parmi les valeurs de R1 telles que définies comme indiqué ci-dessus ou ci-après à l'exception de aryle et hétéroaryle;
les autres substituants R2, R3, R4, R5 et R6 ayant les valeurs définies comme indiqué ci-dessus ou ci-après;
lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

La présente invention a notamment pour objet les produits de formule (I) telle que définie ci-dessus dans laquelle
X-Y représente NH-C(S), N=C-NR7R8, N=C-SR, N=C-R ou N=COR ;
R1 représente l'atome d'hydrogène, un radical cycloalkyle ou un radical alkyle, hétérocycloalkyle, phényle ou hétéroaryle, ces derniers radicaux étant éventuellement substitués;
R identique ou différent de R1 est choisi parmi les valeurs de R1 ;
R2 représente l'atome d'hydrogène , un atome d'halogène ou un radical alkyle ;
R3 représente l'atome d'hydrogène, un atome d'halogène , le radical hydroxyle ou un radical alkyle ou alcoxy ;
R4 représente l'atome d'hydrogène , un atome d'halogène ou un radical cyano, CF3 ou alkyle;
R5 représente l'atome d'hydrogène, un atome d'halogène, un radical hydroxyle, cyano, NR7R8, CONR7R8, NR11COR12 ou un radical cycloalkyle, alkyle, alcoxy, hétérocycloalkyle, phényle ou hétéroaryle, tous ces derniers radicaux étant éventuellement substitués ;
R6 représente l'atome d'hydrogène , un atome d'halogène ou un radical NR7R8, alkyle ou alcoxy ;
R7 et R8 sont tels que :
   soit R7 et R8 identiques ou différents sont tels que l'un de R7 et R8 représente l'atome d'hydrogène ou un radical alkyle éventuellement substitué par un ou plusieurs radicaux identiques ou différents choisis parmi les atomes d'halogène et les radicaux hydroxyle et alcoxy ;
   et l'autre de R7 et R8 représente un atome d'hydrogène ou un radical cycloalkyle, alkyle, hétérocycloalkyle, hétéroaryle ou phényle, tous ces radicaux étant éventuellement substitués ;
   soit R7 et R8 forment ensemble avec l'atome d'azote auquel ils sont liés un radical cyclique formé de 3 à 7 chaînons renfermant éventuellement un ou plusieurs autres hétéroatomes choisi(s) parmi O, S ou N, N étant éventuellement substitué par R11, ce radical cyclique étant lui-même éventuellement substitué;
   tous les radicaux alkyle, alcoxy, cycloalkyle hétérocycloalkyle, hétéroaryle et aryle, ainsi que le radical cyclique que peuvent former R7 et R8 ensemble avec l'atome d'azote auquel ils sont liés, indiqués comme éventuellement substitués, étant ainsi éventuellement substitués par un ou plusieurs radicaux identiques ou différents choisis parmi les atomes d'halogène et les radicaux hydroxyle, cyano, nitro, CF3, NR9R10, NHCOR11, NHSO2R13, COOH, COOalk, CONR9R10, SO2NR9R10, alcoxy, haloalcoxy, alkyle, fluoroalkyle, hydroxyalkyle, cycloalkyle, hétérocycloalkyle, hétéroaryle et phényle, ces derniers radicaux hétéroaryle et phényle, étant eux-mêmes éventuellement substitués par un ou plusieurs radicaux identiques ou différents choisis parmi les atomes d'halogène et les radicaux hydroxyle, NH2, NH(alk), N(alk)2, alkyle, hydroxyalkyle et alcoxy ;
R9 et R10 sont tels que :
   soit R9 et R10 identiques ou différents sont tels que l'un de R9 et R10 représente l'atome d'hydrogène ou un radical alkyle et l'autre de R9 et R10 représente un atome d'hydrogène ou un radical alkyle, phényle ou phénylalkyle eux-mêmes éventuellement substitués par un ou plusieurs radicaux identiques ou différents choisis parmi les atomes d'halogène et les radicaux hydroxyle, NH2, NH(alk), N(alk)2, alkyle, hydroxyalkyle et alcoxy ;
   soit R9 et R10 forment ensemble avec l'atome d'azote auquel ils sont liés un radical pyrrolidinyle, morpholine, pipéridyle, azépinyle ou pipérazinyle éventuellement substitué par un radical alkyle ou phényle lui-même éventuellement substitué par un ou plusieurs radicaux identiques ou différents choisis parmi les atomes d'halogène et les radicaux alkyles, hydroxyle, NH2, NH(alk), N(alk)2, alkyle, hydroxyalkyle et alcoxy;
R11 et R12, identiques ou différents représentent l'atome d'hydrogène ou un radical alkyle ou phényle;
R13 représente un radical alkyle ou phényle;
tous les radicaux alkyle (alk) et alcoxy ci-dessus étant linéaires ou ramifiés et renfermant au plus 6 atomes de carbone ;
lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

Lorsque R9 et R10 forment ensemble avec l'atome d'azote auquel ils sont liés un radical cyclique, notamment R9 et R10 forment ensemble avec l'atome d'azote auquel ils sont liés un radical pyrrolidinyle, morpholine, pipéridyle ou pipérazinyle éventuellement substitué par un radical alkyle.

La présente invention a particulièrement pour objet les produits de formule (I) telle que définie ci-dessus dans laquelle:
X-Y représente NH-C(S), N=C-NR7R8, N=C-SR, N=C-R ou N=COR;
R1 représente l'atome d'hydrogène ou un radical alkyle ou phényle éventuellement substitués ;
R représente l'atome d'hydrogène ; un radical cycloalkyle; un radical alkyle ; hétérocycloalkyle ; phényle ; ou hétéroaryle ; tous ces radicaux étant éventuellement substitués;
R2 représente l'atome d'hydrogène , un atome d'halogène ou un radical alkyle ;
R3 représente l'atome d'hydrogène, un atome d'halogène , le radical hydroxyle ou un radical alkyle;
R4 représente l'atome d'hydrogène , un atome d'halogène ou un radical alkyle ;
R5 représente l'atome d'hydrogène ; un atome d'halogène ; le radical hydroxyle ; cyano ; NR7R8 ; alkyle; alcoxy ; hétérocycloalkyle; phényle; ou hétéroaryle; tous ces derniers radicaux ainsi que le reste phényle de NHphényle et NH(phénylalk), étant éventuellement substitués ;
R6 représente l'atome d'hydrogène , un atome d'halogène, ou un radical NH2, NHalk, N(alk)2, alkyle ou alcoxy ;
R7 et R8 sont tels que :
   soit R7 et R8 identiques ou différents sont tels que l'un de R7 et R8 représente l'atome d'hydrogène ou un radical alkyle ; et l'autre de R7 et R8 représente un atome d'hydrogène, ou un radical alkyle ou cycloalkyle, éventuellement substitués ; soit R7 et R8 forment ensemble avec l'atome d'azote auquel ils sont liés un radical cyclique choisis parmi les radicaux azétidyle ; pipéridyle ; azépanyle ; morpholinyle ; thiomorpholinyle ; pyrrolidinyle ; imidazolidinyle ; pipérazinyle éventuellement substitué sur son second atome d'azote par un radical alkyle ou phényle eux-mêmes éventuellement substitués; et homopipérazinyle, tous ces radicaux cycliques étant éventuellement substitués ;
   tous les radicaux alkyle, alcoxy, hétérocycloalkyle, hétéroaryle et phényle, ainsi que le radical cyclique que peuvent former R7 et R8 ensemble avec l'atome d'azote auquel ils sont liés étant éventuellement substitués par un ou plusieurs radicaux identiques ou différents choisis parmi les atomes d'halogène et les radicaux hydroxyle, NH2, NH(alk), N(alk)2, NHphényle, NH(phénylalk), alkyle, CF3, alcoxy, OCF3, cycloalkyle, hétérocycloalkyle, hétéroaryle et phényle ; ces derniers radicaux hétéroaryle et phényle , ainsi que le reste phényle des radicaux NHphényle et NH(phénylalk), étant eux-mêmes éventuellement substitués par un ou plusieurs radicaux identiques ou différents choisis parmi les atomes d'halogène et les radicaux hydroxyle, NH2, NHAlk, N(Alk)2, alkyle, hydroxyalkyle et alcoxy;
   tous les radicaux alkyle (alk) et alcoxy ci-dessus étant linéaires ou ramifiés et renfermant au plus 6 atomes de carbone ;
   lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

Dans les produits de formule (I) et dans ce qui suit:
- le terme radical alkyle ou alk désigne les radicaux, linéaires et ramifiés renfermant au plus 12 atomes de carbone, tels que par exemple les radicaux méthyle, éthyle, propyle, isopropyle, butyle, isobutyle, sec-butyle, tert-butyle, pentyle, isopentyle, hexyle, isohexyle et également heptyle, octyle, nonyle et décyle ainsi que leurs isomères de position linéaires ou ramifiés ; le terme représente notamment les radicaux linéaires et ramifiés renfermant au plus 6 atomes de carbone choisis parmi ceux définis ci-dessus et également les radicaux linéaires et ramifiés renfermant au plus 4 atomes de carbone choisi parmi ceux définis ci-dessus ;
- le terme radical alkylthio désigne les radicaux -S-alkyle dans lesquels le radical alkyle a la signification indiquée ci-dessus ;
- les termes haloalkyle et haloalkylthio représentent les radicaux alkyle et alkylthio tels que définis ci-dessus susbtitués par un ou plusieurs atomes d'halogène ;
- le terme radical hydroxyalkyle désigne les radicaux alkyle indiqués ci-dessus substitués par au moins un radical hydroxyle ;
- le terme radical alcoxy désigne les radicaux linéaires et ramifiés renfermant au plus 12 atomes de carbone, tels que par exemple les radicaux méthoxy, éthoxy, propoxy, isopropoxy, butoxy linéaire, secondaire ou tertiaire, pentoxy ou hexoxy ainsi que leurs isomères de position linéaires ou ramifiés ; le terme représente notamment les radicaux linéaires et ramifiés renfermant au plus 6 atomes de carbone choisis parmi ceux définis ci-dessus et également les radicaux linéaires et ramifiés renfermant au plus 4 atomes de carbone choisi parmi ceux définis ci-dessus ;
- les termes NH(alk) et N(alk)2 désigne des radicaux amino substitués respectivement par un ou deux radicaux alkyle, de tels radicaux alkyle étant linéaires ou ramifiés et choisis parmi les radicaux alkyle tels que définis ci-dessus, renfermant de préférence au plus 6 atomes de carbone ;
- dans les groupements ci-dessus et ci-après, NR7R8, CONR7R8, NR11COR12, NR9R10, NHCOR11, NHCO2R11, NHCONR9R10, NHSO2R13, CONR9R10 et SO2NR9R10, les radicaux R7, R8 , R9, R10, R11, R12 et R13 peuvent prendre toutes les valeurs indiquées pour ces radicaux ;
- le terme atome d'halogène désigne les atomes de chlore, de brome, d'iode ou de fluor et de préférence l'atome de chlore, de brome ou de fluor;
- le terme radical cycloalkyle désigne un radical carbocyclique saturé renfermant 3 à 10 atomes de carbone et désigne ainsi notamment les radicaux cyclopropyle, cyclobutyle, cyclopentyle et cyclohexyle et tout particulièrement les radicaux cyclopentyle et cyclohexyle ;
- le terme radical hétérocycloalkyle désigne ainsi un radical carbocyclique monocyclique ou bicyclique interrompu par un ou plusieurs hétéroatomes, identiques ou différents, choisis parmi les atomes d'oxygène, d'azote ou de soufre : on peut citer par exemple les radicaux morpholinyle, thiomorpholinyle, aziridyle, azétidyle, pipérazinyle, pipéridyle, homopipérazinyle, pyrrolidinyle, imidazolidinyle, pyrazolidinyle, tétrahydrofuryle, tétrahydrothiényle, hexahydropyranne, oxodihydropyridazinyle, tous ces radicaux étant éventuellement substitués ;
- les termes aryle et hétéroaryle désignent des radicaux insaturés ou partiellement insaturés, respectivement carbocycliques et hétérocycliques, monocycliques ou bicycliques, renfermant au plus 12 chaînons, pouvant éventuellement contenir un chaînon -C(O), les radicaux hétérocycliques contenant un ou plusieurs hétéroatomes identiques ou différents choisis parmi O, N, ou S avec N, le cas échéant, éventuellement substitué ;
- le terme radical aryle désigne ainsi des radicaux monocycliques ou bicycliques renfermant 4 à 12 chaînons tels que par exemple par exemple les radicaux phényle, naphtyle, biphényle, indényle, fluorényle et anthracényle, plus particulièrement les radicaux phényle et naphtyle et encore plus particulièrement le radical phényle. On peut noter qu'un radical carbocyclique contenant un chaînon -C(O) est par exemple le radical tétralone;
- le terme radical hétéroaryle désigne ainsi des radicaux monocycliques ou bicycliques renfermant 4 à 12 chaînons : des radicaux hétéroaryles monocycliques tels que par exemple les radicaux thiényle tel que 2-thiényle et 3-thiényle, furyle tel que 2-furyle, 3-furyle, pyrannyle, pyrrolyle, pyrrolinyle, pyrazolinyle, imidazolyle, pyrazolyle, pyridyle tel que 2-pyridyle, 3-pyridyle et 4-pyridyle, pyrazinyle, pyrimidinyle, pyridazinyle, oxazolyle, thiazolyle, isothiazolyle, diazolyle, thiadiazolyle, thiatriazolyle, oxadiazolyle, isoxazolyle tel que 3- ou 4-isoxazolyle, furazannyle, tétrazolyle libre ou salifié, tous ces radicaux étant éventuellement substitués parmi lesquels plus particulièrement les radicaux thiényle tel que 2-thiényle et 3-thiényle, furyle tel que 2-furyle, pyrrolyle, pyrrolinyle, pyrazolinyle, imidazolyle, pyrazolyle, oxazolyle, isoxazolyle, pyridyle, pyridazinyle, ces radicaux étant éventuellement substitués ; des radicaux hétéroaryles bicycliques tels que par exemple les radicaux benzothiényle tel que 3-benzothiényle, benzothiazolyle, quinolyle, isoquinolyle, dihydroquinolyle, quinolizinyle, tetrahydroquinolizinyle, quinolone, tétralone, adamentyl, benzofuryle, isobenzofuryle, dihydrobenzofuranne, éthylènedioxyphényle, thianthrényle, benzopyrrolyle, benzimidazolyle, benzoxazolyle, thionaphtyle, indolyle, azaindolyle, indazolyle, purinyle, thiénopyrazolyle, tétrahydroindazolyle, tétrahydrocyclopentapyrazolyle, dihydrofuropyrazolyle, tétrahydropyrrolopyrazolyle, oxotétrahydropyrrolopyrazolyle, tétrahydropyranopyrazolyle, tétrahydropyridinopyrazolyle ou oxodihydropyridino-pyrazolyle, tous ces radicaux étant éventuellement substitués ;

Comme exemples de radicaux hétéroaryles, on peut citer plus particulièrement les radicaux pyrimidinyle, pyridyle, pyrrolyle, azaindolyle, indazolyle ou pyrazolyle, éventuellement substitués par un ou plusieurs substituants identiques ou différents comme indiqué ci-dessus.
- le terme phénylalkyle désigne un radical alkyle tel que défini ci-dessus dans lequel le radical alkyle est linéaire ou ramifié de préférence renfermant au plus 4 atomes de carbone et le radical phényle est éventuellement substitué par un ou plusieurs radicaux tel que défini ci-avant ou ci-après ;

Le ou les radicaux carboxy des produits de formule (I) peuvent être salifiés ou estérifiés par les groupements divers connus de l'homme du métier parmi lesquels on peut citer, par exemple :
- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxycarbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxyméthyle, propionyloxyméthyle, méthylthiométhyle, diméthylaminoéthyle, benzyle ou phénéthyle.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, trifluoroacétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêtaéthanedisulfonique, les acides arylmonosulfoniques tels que l'acide benzènesulfonique et les acides aryldisulfoniques.

On peut rappeler que la stéréoisomérie peut être définie dans son sens large comme l'isomérie de composés ayant les mêmes formules développées, mais dont les différents groupes sont disposés différemment dans l'espace, tels que notamment dans des cyclohexanes monosubstitués dont le substituant peut être en position axiale ou équatoriale, et les différentes conformations rotationnelles possibles des dérivés de l'éthane. Cependant, il existe un autre type de stéréoisomérie, dû aux arrangements spatiaux différents de substituants fixés, soit sur des doubles liaisons, soit sur des cycles, que l'on appelle souvent isomérie géométrique ou isomérie cis-trans. Le terme stéréoisomères est utilisé dans la présente demande dans son sens le plus large et concerne donc l'ensemble des composés indiqués ci-dessus.

La présente invention a ainsi pour objet les produits de formule (I) telle que définie ci-dessus dans laquelle
X-Y représente NH-C(S), N=C-NR7R8, N=C-SR, N=C-R ou N=COR;
R1 représente l'atome d'hydrogène ou un radical alkyle ou phényle éventuellement substitués ;
R identique ou différent de R1 est choisi parmi les valeurs de R1 ;
R2 représente l'atome d'hydrogène , un atome d'halogène ou un radical alkyle ;
R3 représente l'atome d'hydrogène, un atome d'halogène , le radical hydroxyle ou un radical alkyle;
R4 représente l'atome d'hydrogène , un atome d'halogène ou un radical alkyle ;
R5 représente l'atome d'hydrogène , un atome d'halogène, le radical hydroxyle, un radical NH2, NHalk, N(alk)2, NR7R8, NHphényle, NH(phénylalk) ou un radical alkyle, hétérocycloalkyle, alcoxy, phényle ou hétéroaryle, ces derniers radicaux ainsi que le reste phényle de NHphényle et NH(phénylalk), étant éventuellement substitués ;
R6 représente l'atome d'hydrogène , un atome d'halogène, ou un radical NH2, NHalk, N(alk)2, alkyle ou alcoxy ;
R7 et R8 sont tels que :
   soit R7 et R8 identiques ou différents sont tels que l'un de R7 et R8 représente l'atome d'hydrogène ou un radical alkyle ;
   et l'autre de R7 et R8 représente un atome d'hydrogène, ou un radical alkyle ou cycloalkyle, éventuellement substitué ;
   soit R7 et R8 forment ensemble avec l'atome d'azote auquel ils sont liés un radical cyclique renfermant 4 à 6 chaînons choisis parmi azétidyle ; pipéridyle ; morpholinyle ; thiomorpholinyle ; pyrrolidinyle ; imidazolidinyle ; pipérazinyle ; et homopipérazinyle, ces radicaux étant éventuellement substitués ;
   tous les radicaux alkyle, alcoxy, hétéroaryle et phényle, ainsi que le radical cyclique que peuvent former R7 et R8 ensemble avec l'atome d'azote auquel ils sont liés, indiqués comme éventuellement substitués, étant ainsi éventuellement substitués par un ou plusieurs radicaux identiques ou différents choisis parmi les atomes d'halogène et les radicaux hydroxyle, NH2, NH(alk), N(alk)2, NHphényle, NH(phénylalk), alkyle, CF3, alcoxy, OCF3 , cycloalkyle, hétérocycloalkyle, hétéroaryle et phényle ; ces derniers radicaux hétéroaryle et phényle , ainsi que le reste phényle des radicaux NHphényle et NH(phénylalk), étant eux-mêmes éventuellement substitués par un ou plusieurs radicaux identiques ou différents choisis parmi les atomes d'halogène et les radicaux hydroxyle, NH2, NHAlk, N(Alk)2, alkyle, hydroxyalkyle et alcoxy;
   tous les radicaux alkyle (alk) et alcoxy ci-dessus étant linéaires ou ramifiés et renfermant au plus 6 atomes de carbone ;
   lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

Dans les produits de formule (I) selon la présente invention, les substituants X-Y, R1, R2, R3, R4, R5 et R6 peuvent notamment avoir indépendamment les uns des autres les valeurs suivantes i) à vii):
i) notamment X-Y représente NH-C(S), N=C-NR7R8 ou N=C-R avec NR7R8 et R tels que définis ci-dessus ou ci-après;
ii) notamment R1 représente l'atome d'hydrogène ou un radical alkyle ou phényle éventuellement substitués par N(alk)2, morpholinyle ou pyrrolidinyle, alcoxy ou phényle ;
iii) notamment R2 représente l'atome d'hydrogène , ou un radical alkyle ;
iv) notamment R3 représente l'atome d'hydrogène ;
v) notammnent R4 représente l'atome d'hydrogène ou un atome d'halogène;
vi) notamment R5 représente notamment l'atome d'hydrogène ; un atome d'halogène ; un radical hydroxyle, cyano, NH2, NHalk, N(alk)2, NHphényle ; NH (phénylalk))) ; CF3 ; alkyle éventuellement substitué par phényle; alcoxy ; hétérocycloalkyle tel que par exemple morpholinyle ; phényle éventuellement susbtitué par un radical hétérocyclique tel que par exemple pipérazinyle; ou hétéroaryle tel que par exemple thiényle, ces derniers radicaux ainsi que phényle et le reste phényle de NHphényle et NH(phénylalk), étant éventuellement substitués ;
vii) notamment R6 représente l'atome d'hydrogène ou un atome d'halogène;
tous les radicaux alkyle, alcoxy, hétérocycloalkyle, hétéroaryle et phényle, étant éventuellement substitués par un ou plusieurs radicaux identiques ou différents comme indiqué ci-dessus ou ci-après,
étant entendu que pour chacune des valeurs i) à vii), les autres substituants desdits produits de formule (I) peuvent avoir l'une quelconque des valeurs définies ci-dessus ou ci-dessous,
lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

Dans les produits de formule (I) selon la présente invention, dans le radical X-Y, R représente notamment l'atome d'hydrogène ; un radical cycloalkyle; alkyle ; hétérocycloalkyle tel que notammnent morpholino, tétrahydropyrane; phényle; ou hétéroaryle tel que notamment pyridine, quinoline, quinolizinyle, tetrahydroquinolizinyle, indolyle, thiényle, furannyle, pyrrolyle et pyrazolyle; tous ces radicaux étant éventuellement substitués comme indiqués ci-dessus ou ci-après.

Plus précisément R représente l'atome d'hydrogène ; ou un radical cycloalkyle tel que notamment cyclohexyle ; un radical alkyle éventuellement substitué notamment par cycloalkyle tel que par exemple cyclopropyle, cyclobutyle, cyclopentyle, par phényle ou par hétéroaryle tel que notamment pyridine, tous ces radicaux étant éventuellement substitués comme indiqué ci-dessus ou ci-après; hétérocycloalkyle tel que notammnent morpholino ou tétrahydropyrane; phényle ou hétéroaryle tel que notamment pyridine, quinolyle, thienyle, furannyle, pyrrolyle et pyrazolyle éventuellement substitués comme indiqué ci-dessus ou ci-après.

Lorsque les produits de formule (I) selon la présente invention porte un radical NR7R8, notamment R7 et R8 sont tels que soit R7 et R8 identiques ou différents sont tels que l'un de R7 et R8 représente l'atome d'hydrogène ou un radical alkyle ; et l'autre de R7 et R8 représente un atome d'hydrogène, ou un radical alkyle éventuellement substitué par cycloalkyle ; soit R7 et R8 forment ensemble avec l'atome d'azote auquel ils sont liés un radical pipéridyle ; azépinyle; morpholinyle ; pyrrolidinyle ; pipérazinyle éventuellement substitué sur son second atome d'azote par alkyle ou phényle; ces radicaux étant éventuellement substitués comme indiqué ci-dessus ou ci-après.

La présente invention a ainsi pour objet les produits de formule (I) telle que définie ci-dessus dans laquelle
X-Y, R2, R3, R4 et R6 ont les significations indiquées ci-dessus,
R1 représente l'atome d'hydrogène ou un radical alkyle éventuellement substitué ;
R5 représente l'atome d'hydrogène, un atome d'halogène, un radical hydroxyle, CF3, NH2, NHalk, N(alk)2 ou un radical alkyle, alcoxy ou phényle, éventuellement substitués ;
   le radical alkyle que peut représenter R1 ou le radical alkyle, alcoxy ou phényle que peut représenter R5, étant éventuellement substitués par un ou plusieurs radicaux identiques ou différents choisis parmi les atomes d'halogène et les radicaux hydroxyle, NHalk, N(alk)2, alcoxy, cycloalkyle, hétérocycloalkyle, hétéroaryle et phényle, ces derniers radicaux hétéroaryle et phényle étant éventuellement substitués par un ou plusieurs radicaux identiques ou différents choisis parmi les atomes d'halogène et les radicaux hydroxyle, NH2, NHAlk, N(Alk)2, alkyle, hydroxyalkyle et alcoxy;
R7 et R8 sont tels que :
   soit R7 et R8 identiques ou différents sont tels que l'un de R7 et R8 représente l'atome d'hydrogène ou un radical alkyle,
   et l'autre de R7 et R8 représente un atome d'hydrogène, un radical alkyle éventuellement substitué par un ou plusieurs radicaux identiques ou différents choisis parmi les atomes d'halogène, les radicaux hydroxyle, NH2, NHalk, N(alk)2, NH(phényle), NH(phénylalk), alcoxy, OCF3, cycloalkyle, et les radicaux pyrrolidinyle, pipérazinyle, pipéridyle, morpholinyle et phényle, tous ces derniers radicaux cycliques, ainsi que le reste phényle du radical phénylalkyle, étant eux-mêmes éventuellement substitués par un ou plusieurs radicaux identiques ou différents choisis parmi les atomes d'halogène et les radicaux hydroxyle, NH2, NHAlk, N(alk)2, alcoxy, alkyle et hydroxyalkyle;
   soit R7 et R8 forment ensemble avec l'atome d'azote auquel ils sont liés un radical choisi préférentiellement parmi les radicaux pipéridyle, morpholinyle, et les radicaux pyrrolidinyle, pipérazinyle et homopipérazinyle éventuellement substitués par un ou plusieurs radicaux identiques ou différents choisis parmi les radicaux alkyle et phényle, eux-mêmes éventuellement substitués par un ou plusieurs radicaux choisis parmi les atomes d'halogène et les radicaux hydroxyle, NH2, NHAlk, N(Alk)2, alcoxy et cycloalkyle;
   tous les radicaux alkyle (alk) et alcoxy ci-dessus étant linéaires ou ramifiés et renfermant au plus 6 atomes de carbone;
   lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

La présente invention a ainsi pour objet les produits de formule (I) telle que définie ci-dessus dans laquelle
X-Y représente NH-C(S), N=C-NR7R8 ou N=C-R,
R7, R8 et R étant choisis parmi toutes les valeurs définies ci-dessus pour R7, R8 et R et les autres substituants R1,R2,R3,R4,R5 et R6 desdits produits de formule (I) étant choisis parmi toutes les valeurs définies ci-dessus respectivement pour R1,R2,R3,R4,R5 et R6,
   lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

La présente invention a ainsi pour objet les produits de formule (I) telle que définie ci-dessus dans laquelle
X-Y représente NH-C(S), N=C-NR7R8 ou N=C-R;
R1 représente l'atome d'hydrogène ou un radical alkyle éventuellement substitué par un ou plusieurs radicaux identiques ou différents choisis parmi les atomes d'halogène et les radicaux N(alk)2 et alcoxy ;
R identique ou différent de R1 est choisi parmi les valeurs de R1 ;
R2 représente l'atome d'hydrogène, un atome d'halogène ou un radical alkyle ;
R3 représente l'atome d'hydrogène ou un radical alkyle ;
R4 représente l'atome d'hydrogène ou un atome d'halogène,
R5 représente l'atome d'hydrogène, un atome d'halogène ou un radical hydroxyle, NH2, NHalk, N(alk)2, alkyle, alcoxy ou phényle, le radical alkyle étant éventuellement substitué par un radical alcoxy, N(alk)2 ou hétérocycloalkyle et le radical phényle étant éventuellement substitué par un ou plusieurs radicaux identiques ou différents choisis parmi les atomes d'halogène et les radicaux hydroxyle, NH2, NHalk, N(alk)2, alkyle et alcoxy ;
R6 représente un atome d'hydrogène, un atome d'halogène ou un radical alkyle ;
et R7 et R8 sont tels que:
   soit R7 et R8 identiques ou différents sont tels que l'un de R7 et R8 représente l'atome d'hydrogène ou un radical alkyle, et l'autre de R7 et R8 représente un radical alkyle éventuellement substitué par un radical cycloalkyle;
   soit R7 et R8 forment ensemble avec l'atome d'azote auquel ils sont liés un radical pyrrolidinyle, morpholine, pipéridyle ou pipérazinyle éventuellement substitué par un radical alkyle ;
   lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

Dans les produits de formule (I) tels que définis ci-dessus, on note tout particulièrement les produits pour lesquels R3 représente un atome d'hydrogène, les autres subtituants R1, R2, R4, R5, R6 et X-Y desdits produits de formule (I) étant choisis parmi l'une quelconque des valeurs définies ci-dessus,
lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

La présente invention a ainsi pour objet les produits de formule (I) telle que définie ci-dessus dans laquelle
X-Y représente NH-C(S), N=C-NR7R8 ou N=C-R;
R1 et R2 identiques ou différents, représente l'atome d'hydrogène ou un radical alkyle ;
R3 représente l'atome d'hydrogène ;
R4, R5 et R6, identiques ou différents, représentent l'atome d'hydrogène ou un atome d'halogène;
R7 et R8 représentent les valeurs définies à l'une quelconque des revendications ci-dessus;
tous les radicaux alkyle (alk) ci-dessus étant linéaires ou ramifiés et renfermant au plus 4 atomes de carbone ;
lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

La présente invention concerne notamment les produits de formule (I) telle que définie ci-dessus dans laquelle
X-Y représente N=C-NR7R8 ou N=C-R,
R7, R8 et R étant choisis parmi toutes les valeurs définies ci-dessus pour R7, R8 et R et les autres substituants R1,R2,R3,R4,R5 et R6 desdits produits de formule (I) étant choisis parmi toutes les valeurs définies ci-dessus respectivement pour R1,R2,R3,R4,R5 et R6,
lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

La présente invention concerne notamment les produits de formule (I) telle que définie ci-dessus dans laquelle
X-Y représente N=C-NR7R8,
R7 et R8 étant choisis parmi toutes les valeurs définies ci-dessus pour R7 et R8 et les autres substituants R1, R2, R3, R4, R5 et R6 desdits produits de formule (I) étant choisis parmi toutes les valeurs définies ci-dessus respectivement pour R1, R2, R3, R4, R5 et R6,
lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

La présente invention concerne notamment les produits de formule (I) telle que définie ci-dessus dans laquelle
X-Y représente N=C-R,
R étant choisi parmi toutes les valeurs définies ci-dessus pour R7, R8 et R et les autres substituants R1,R2,R3,R4,R5 et R6 desdits produits de formule (I) étant choisis parmi toutes les valeurs définies ci-dessus respectivement pour R1, R2, R3, R4, R5 et R6,
lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

La présente invention concerne notamment les produits de formule (I) telle que définie ci-dessus dans laquelle
X-Y représente N=C-OR,
R étant choisi parmi toutes les valeurs définies ci-dessus pour R7, R8 et R et les autres substituants R1,R2,R3,R4,R5 et R6 desdits produits de formule (I) étant choisis parmi toutes les valeurs définies ci-dessus respectivement pour R1, R2, R3, R4, R5 et R6,
lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

La présente invention a plus particulièrement pour objet les produits de formule (I) telle que définie ci-dessus, répondant aux noms suivants :
- la (5Z)-5-(1H-pyrrolo[2,3-b]pyridin-3-ylméthylène)-2-thioxoimidazolidin-4-one
- la (5Z)-5-[(5-chloro-1H-pyrrolo[2,3-b]pyridin-3-yl)méthylène]-2-thioxoimidazolidin-4-one
- la (5Z)-2-[(cyclopropylméthyl)amino]-5-(1H-pyrrolo[2,3-b]pyridin-3-ylméthylène)-3,5-dihydro-4H-imidazol-4-one
- la (5Z)-5-[(5-chloro-1H-pyrrolo[2,3-b]pyridin-3-yl)méthylène]-2-[(cyclopropylméthyl)amino]-3,5-dihydro-4H-imidazol-4-one
- la (5Z)-5-[(6-chloro-1H-pyrrolo[2,3-b]pyridin-3-yl)méthylène]-2-[(cyclopropylméthyl)amino]-3,5-dihydro-4H-imidazol-4-one
- la (5Z)-2-[(cyclopropylméthyl)amino]-3-méthyl-5-(1H-pyrrolo[2,3-b]pyridin-3-ylméthylène)-3,5-dihydro-4H-imidazol-4-one
- la (5Z)-2-butyl-5-(1H-pyrrolo[2,3-b]pyridin-3-ylméthylène)-3,5-dihydro-4H-imidazol-4-one
- (5Z)-2-azépan-1-yl-5-[(5-chloro-1H-pyrrolo[2,3-b]pyridin-3-yl)méthylidène]-3,5-dihydro-4H-imidazol-4-one
- 5Z)-5-[(5-chloro-1H-pyrrolo[2,3-b]pyridin-3-yl)méthylidène]-2-pipéridin-1-yl-3,5-dihydro-4H-imidazol-4-one
- 5Z)-5-[(5-chloro-1H-pyrrolo[2,3-b]pyridin-3-yl)méthylidène]-2-[méthyl(2-méthylpropyl)amino]-3,5-dihydro-4H-imidazol-4-one
- (5Z)-5-[(5-bromo-1H-pyrrolo[2,3-b]pyridin-3-yl)méthylidène]-2-[(cyclopropylméthyl)amino]-3,5-dihydro-4H-imidazol-4-one
- (5Z)-2-[(cyclopropylméthyl)amino]-5-[(5-méthoxy-1H-pyrrolo[2,3-b]pyridin-3-yl)méthylidène]-3,5-dihydro-4H-imidazol-4-one
- (5Z)-2-[(cyclopropylméthyl)amino]-5-[(5-fluoro-1H-pyrrolo[2,3-b]pyridin-3-yl)méthylidène]-3,5-dihydro-4H-imidazol-4-one
- (5Z)-2-azépan-1-yl-5-[(5-fluoro-1H-pyrrolo[2,3-b]pyridin-3-yl)méthylidène]-3,5-dihydro-4H-imidazol-4-one
- 3-[(Z)-{2-[(cyclopropylméthyl)amino]-5-oxo-1,5-dihydro-4H-imidazol-4-ylidène}méthyl]-1H-pyrrolo[2,3-b]pyridine-5-carbonitrile
- (5Z)-2-[(cyclopropylméthyl)amino]-5-[(5-phényl-1H-pyrrolo[2,3-b]pyridin-3-yl)méthylidène]-3,5-dihydro-4H-imidazol-4-one
- (5Z)-2-[(cyclopropylméthyl)amino]-5-{[5-(trifluorométhyl)-1H-pyrrolo[2,3-b]pyridin-3-yl]méthylidène}-3,5-dihydro-4H-imidazol-4-one
- (5Z)-5-[(5-chloro-1H-pyrrolo[2,3-b]pyridin-3-yl)méthylidène]-2-(méthylsulfanyl)-3,5-dihydro-4H-imidazol-4-one
- (5Z)-2-butyl-5-[(5-chloro-1H-pyrrolo[2,3-b]pyridin-3-yl)méthylidène]-3,5-dihydro-4H-imidazol-4-one
- (5Z)-2-(3-méthylbutyl)-5-(1H-pyrrolo[2,3-b]pyridin-3-ylméthylidène)-3,5-dihydro-4H-imidazol-4-one
- (5Z)-2-cyclohexyl-5-(1H-pyrrolo[2,3-b]pyridin-3-ylméthylidène)-3,5-dihydro-4H-imidazol-4-one
- (5Z)-5-[(5-chloro-1H-pyrrolo[2,3-b]pyridin-3-yl)méthylidène]-3-méthyl-2-(tétrahydro-2H-pyran-4-yl)-3,5-dihydro-4H-imidazol-4-one
- (5Z)-5-[(5-chloro-1H-pyrrolo[2,3-b]pyridin-3-yl)méthylidène]-3-méthyl-2-(2-méthylpropyl)-3,5-dihydro-4H-imidazol-4-one
- (5Z)-2-(cyclopropylméthyl)-5-(5-chloro-1H-pyrrolo[2,3-b]pyridin-3-ylméthylidène)-3,5-dihydro-4H-imidazol-4-one
- (5Z)-2-(1-méthyléthyl)-5-(5-chloro-1H-pyrrolo[2,3-b]pyridin-3-ylméthylidène)-3,5-dihydro-4H-imidazol-4-one
- (5Z)-2-[benzyl(méthyl)amino]-5-[(5-chloro-1H-pyrrolo[2,3-b]pyridin-3-yl)méthylidène]-3,5-dihydro-4H-imidazol-4-one
- (5Z)-5-[(5-chloro-1H-pyrrolo[2,3-b]pyridin-3-yl)méthylidène]-2-[(2-méthoxyéthyl)(méthyl)amino]-3,5-dihydro-4H-imidazol-4-one
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

Les produits de formule (I) tels que définis ci-dessus peuvent être préparés par les méthodes générales connues de l'homme du métier et plus particulièrement peuvent être préparés en utilisant les méthodes de synthèse décrites dans les schémas ci-dessous 1 à 7.

La présente invention a ainsi encore pour objet des procédés de préparation des produits de formule (I), et notamment les procédés définis ci-dessous dans les schémas 1 à 7 qui sont utilisés pour la préparation de composés décrits dans des tableaux comme suit.

Les tableaux et schémas qui suivent sont reliés comme suit :
- les tableaux 1, 2 et 4 décrivent des produits de formule (I) qui peuvent être préparés selon le schéma 1
- le tableau 6 décrit des produits de formule (I) qui peuvent être préparés selon le schéma 2
- le tableau 3 décrit un produit de formule (I) qui peut être préparé selon le schéma 3
- le tableau 5 décrit des produits de formule (I) qui peuvent être préparés selon les schémas 3 et 5
- le tableau 7 décrit des produits de formule (I) qui peuvent être préparés selon le schéma 6 ou 7
- le tableau 8 décrit des produits de formule (I) qui peuvent être préparés selon les schémas 1 à 7

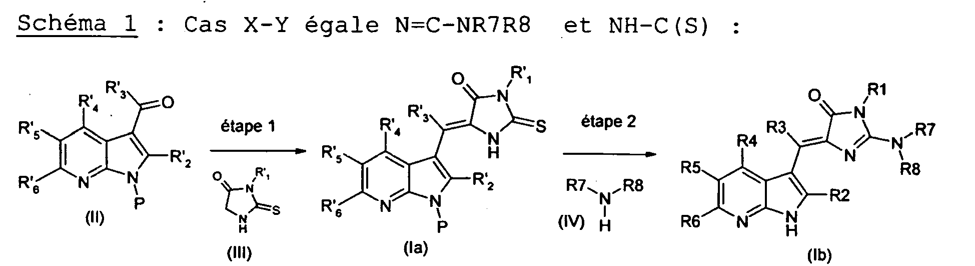

Dans une première étape, les (5Z)-5-[(1H-pyrrolo[2,3-b]pyridin-3-yl)méthylène]-2-thioxoimidazolidin-4-one (Ia) sont obtenues par réaction de Knoevenagel entre une 2-thioxoimidazolidin-4-one (III) et un aldéhyde, cétone ou ester de formule (II) dérivé de la 1H-pyrrolo[2,3-b]pyridine, en présence d'une base telle que la pipéridine dans un solvant tel que l'éthanol. La température de choix pour effectuer cette réaction est comprise entre la température ambiante et la température de reflux. Toutes autres conditions habituelles pour une réaction de type Knoevenagel peuvent être utilisées.

Le tableau 1 qui suit donne des exemples de composés (Ia) préparés suivant le Schéma 1 :

**Tableau 1**

| Exemple (Ia) | Composé (II) | Composé de formule (III) | Composé de formule (Ia) |
|---|---|---|---|
| 1 | 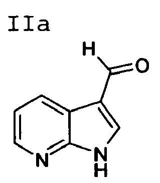 | 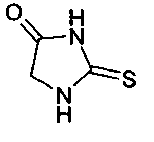 | 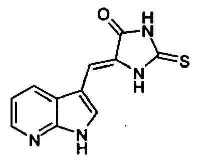 |
| 2 | 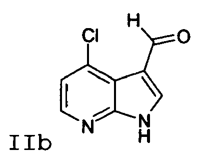 | 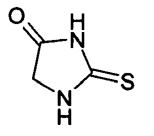 | 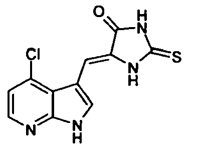 |
| 3 | 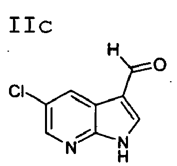 | 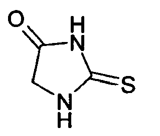 | 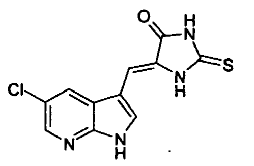 |
| 4 | 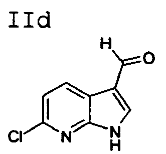 | 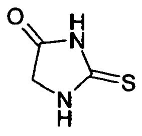 | 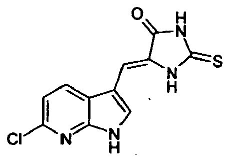 |
| 5 | 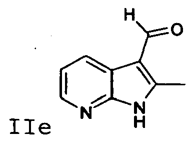 | 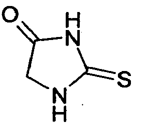 | 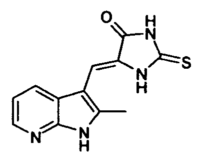 |
| 6 | 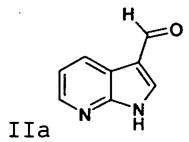 | 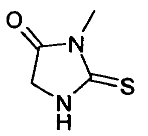 | 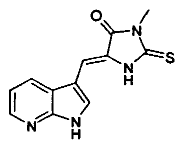 |
| 13 | 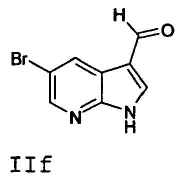 | 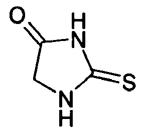 | 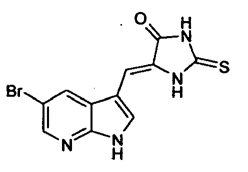 |
| 14 | 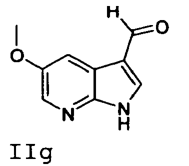 | 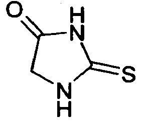 | 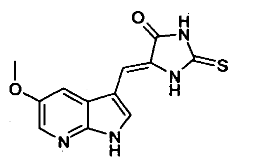 |
| 15 | 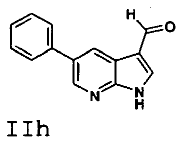 | 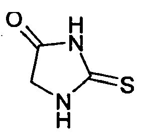 | 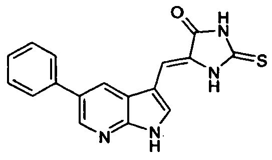 |
| 16 | 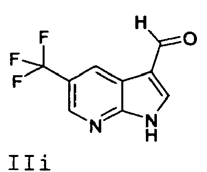 | 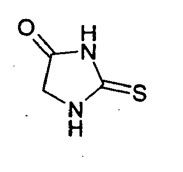 | 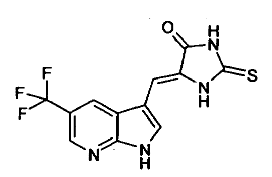 |
| 17 | 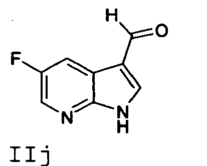 | 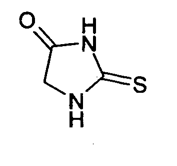 | 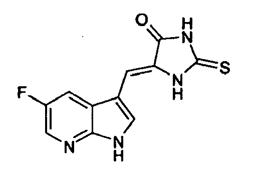 |
| 18 | 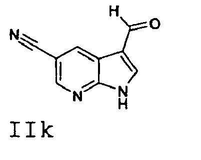 | 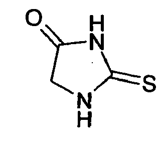 | 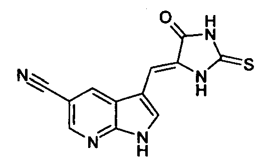 |
| 19 | 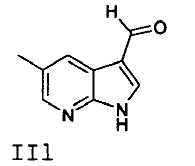 | 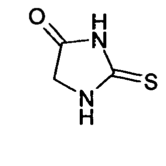 | 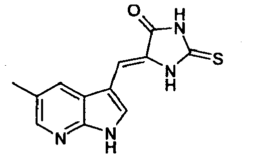 |
| 20 | 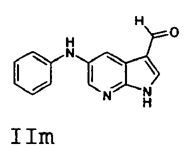 | 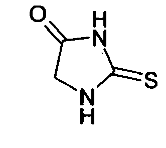 | 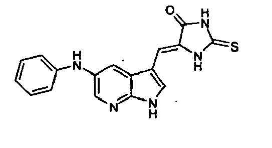 |
| 21 | 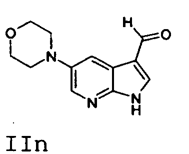 | 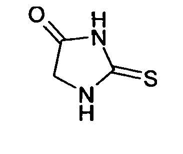 | 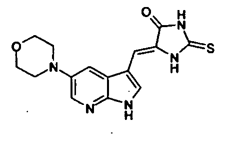 |
| 22 | 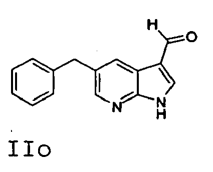 | 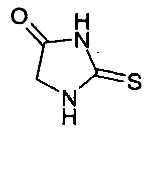 | 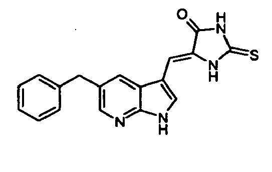 |
| 23 | 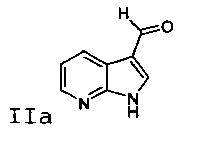 | 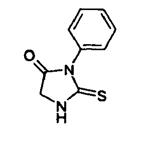 | 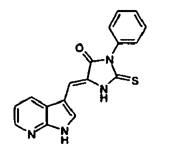 |

Dans une deuxième étape, les (5Z)-5-[(1H-pyrrolo[2,3-b]pyridin-3-yl)méthylène)-2-amino-3,5-dihydro-4H-imidazol-4-one (Ib) sont obtenues par réaction entre les (5Z)-5-[(1H-pyrrolo[2,3-b]pyridin-3-yl)méthylène]-2-thioxoimidazolidin-4-one (Ia) et une amine primaire ou secondaire de structure générale (IV (avec R7 et R8 tels que définis dans la formule générale (I) par exemple dans de l'éthanol à une température variant de 120 à 170°C en tube scellé sous irradiation micro-ondes.

Le tableau 2 donne des exemples de composés (Ib) préparés suivant le schéma 1 :

**Tableau 2**

| Exemple (Ib) | Composé (Ia) | Amine (IV) | Composé de formule (Ib) |
|---|---|---|---|
| 7 | 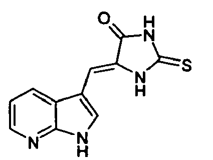 | 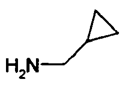 | 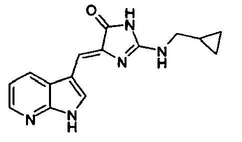 |
| 8 | 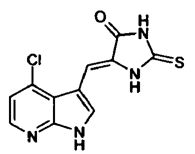 | 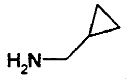 | 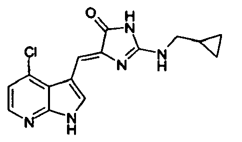 |
| 9 | 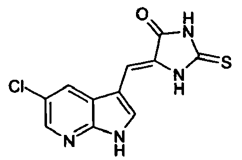 | 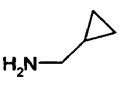 | 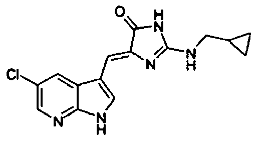 |
| 10 | 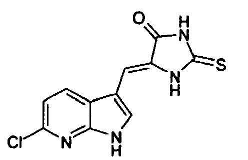 | 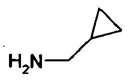 | 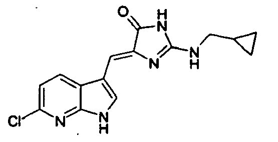 |
| 11 | 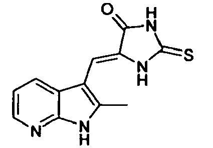 | 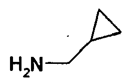 | 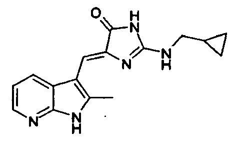 |
| 12 | 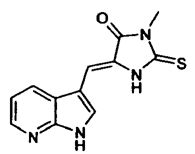 | 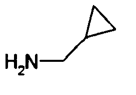 | 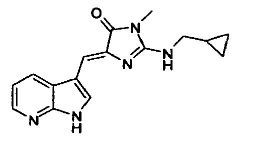 |

Le tableau 4 donne des exemples de composés I(b) préparés suivant le schéma 1: ces produits de formule (I) sont décrits ci-après en exemples dans la partie expérimentale.

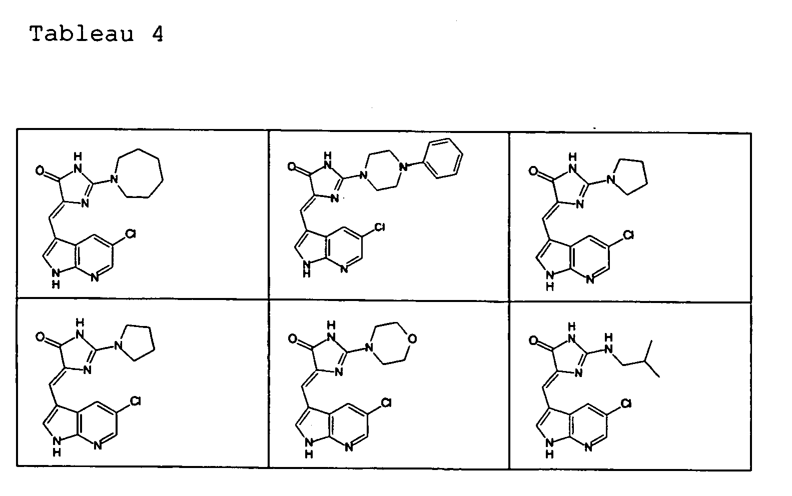

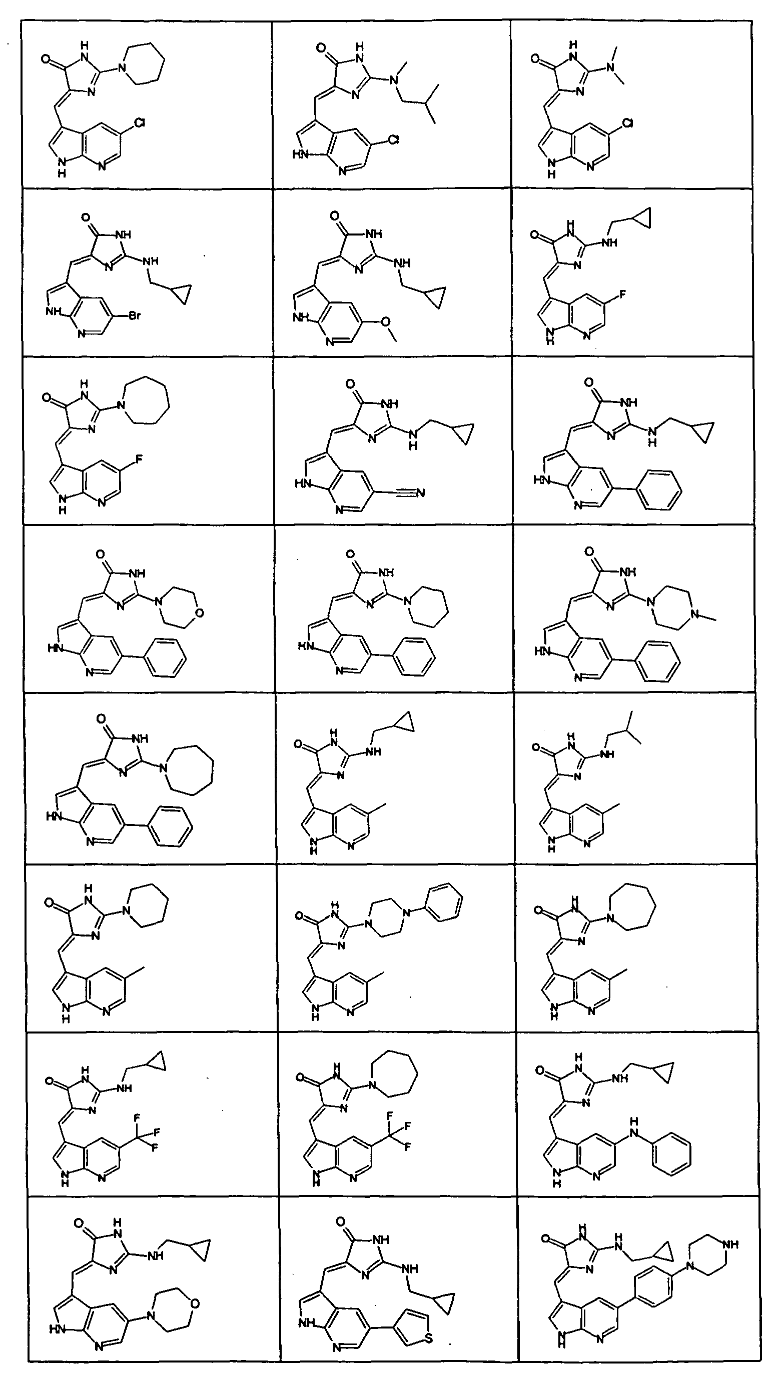

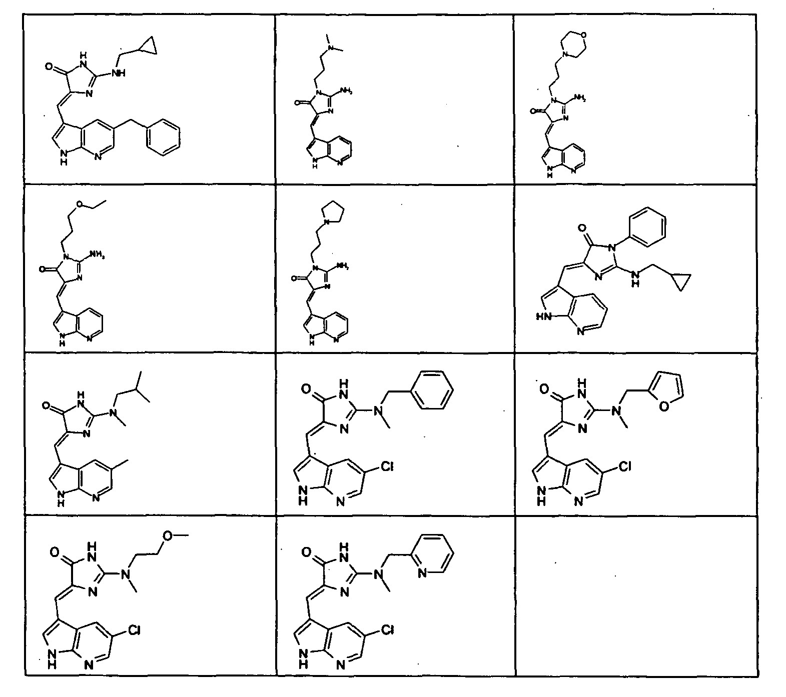

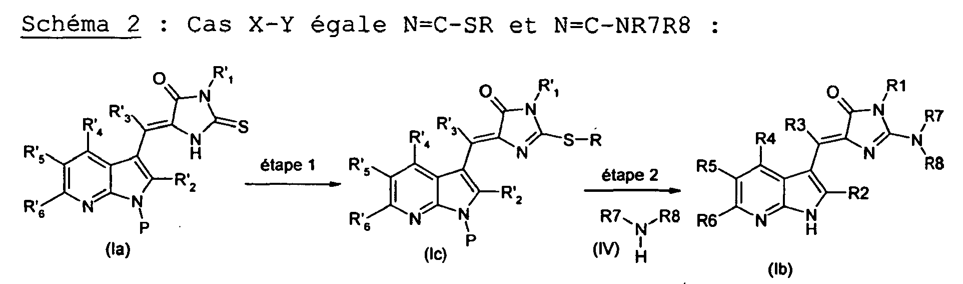

Alternativement, les (5Z)-5-[(1H-pyrrolo[2,3-b]pyridin-3-yl)méthylène]-2-amino-3,5-dihydro-4H-imidazol-4-one (Ic) peuvent être préparés suivant le schéma 2. La réaction des (5Z)-5-[(1H-pyrrolo[2,3-b]pyridin-3-yl)méthylène]-2-thioxoimidazolidin-4-one (Ia) avec un agent alkylant (de préférence par exemple l'iodure de méthyle) en présence d'une base telle que la diisopropyléthylamine ou la soude, pour donner un dérivé thioalkylé (Ic). Le groupe thioalkylée est enfin déplacé dans une deuxième étape par une amine R7R8NH, pour obtenir les dérivés (Ib). Ce déplacement se fait dans un solvant tel que l'acétonitrile ou l'éthanol, à une température comprise entre l'ambiante et le reflux, ou encore dans un tube scellé sous irradiation micro-ondes.

Le tableau 6 donne des exemples de composés I(c) préparés suivant le schéma 2 : ces produits de formule (Ic) sont décrits ci-après en exemples dans la partie expérimentale.

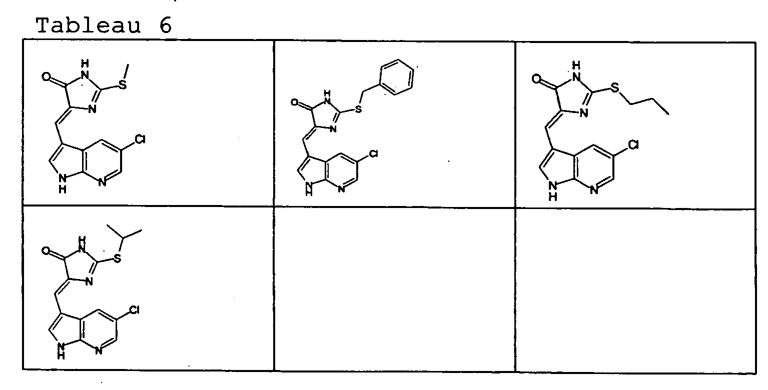

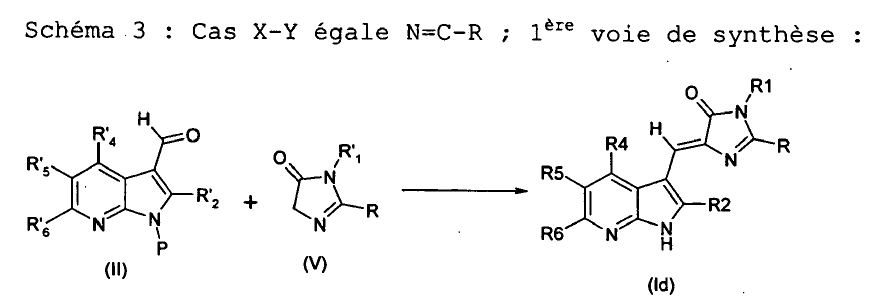

Les (5Z)-5-[(1H-pyrrolo[2,3-b]pyridin-3-yl)méthylène]-3,5-dihydro-4H-imidazolidin-4-one (Id) sont obtenues par réaction de Knoevenagel entre une imidazolidin-4-one (V) et un aldéhyde, cétone ou ester de formule (II) dérivé de la 1H-pyrrolo[2,3-b]pyridine, en présence d'une base telle que la pipéridine dans un solvant tel que l'éthanol, selon le schéma 3. La température de choix pour effectuer cette réaction est comprise entre la température ambiante et la température de reflux. Toutes autres conditions habituelles pour une réaction de type Knoevenagel, telles que des conditions acides, peuvent être utilisées. Les imidazolidin-4-ones (V) peuvent être préparées selon J.Org. Chem. 1999, 64(22), 8084.

Un exemple de préparation d'un produit de formule (I) suivant le schéma 3 est donné dans la partie expérimentale ci-après par l'exemple 13 comme indiqué dans le tableau 3.

**Tableau 3**

| Exemple (Id) | Composé (II) | Composé de formule (V) | Composé de formule (Id) |
|---|---|---|---|
| 13 | 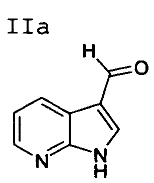 | 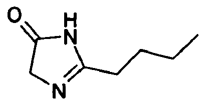 | 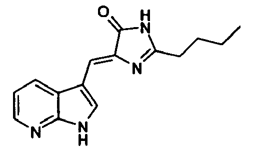 |

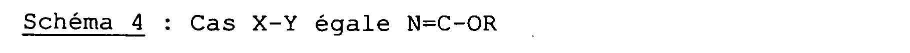

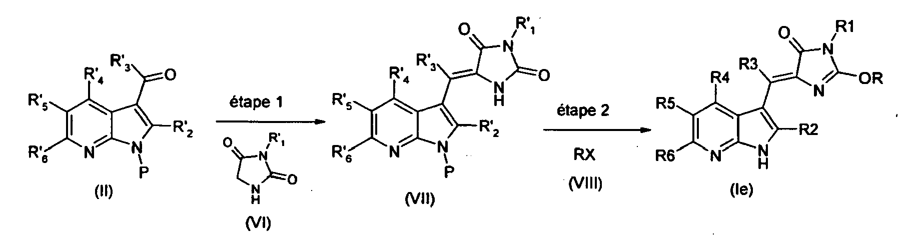

Dans une première étape, les (5Z)-5-[(1H-pyrrolo[2,3-b]pyridin-3-yl)méthylène]-2-oxoimidazolidin-4-one (VII) sont obtenues par réaction de Knoevenagel entre une 2-oxoimidazolidin-4-one (VI) et un aldéhyde, cétone ou ester de formule (II) dérivé du 1H-pyrrolo[2,3-b]pyridine, en présence d'une base telle que la pipéridine dans un solvant tel que l'éthanol, selon le schéma 4. La température de choix pour effectuer cette réaction est comprise entre température ambiante et le reflux. Toute autre condition habituelle pour une réaction de type Knoevenagel, tel que des conditions acides, peut être utilisée.

Dans une deuxième étape, les (5Z)-5-[(1H-pyrrolo[2,3-b]pyridin-3-yl)méthylène]-2-alkoxy-3,5-dihydro-4H-imidazol-4-one (Ie) sont obtenues par réaction entre les (5Z)-5-[(1H-pyrrolo[2,3-b]pyridin-3-yl)méthylène]-2-oxoimidazolidin-4-one (VII) et un agent alkylant RX (VIII) dans un solvant inerte à une température variant de l'ambiante au reflux. Les agents alkylant VIII connus pour favoriser une O-alkylation, tel que des halogénures ou des sels d'oxonium de tétrafluoroborate sont préférés.

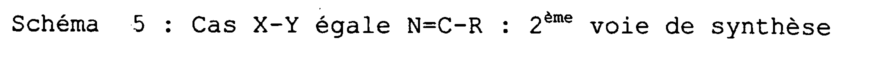

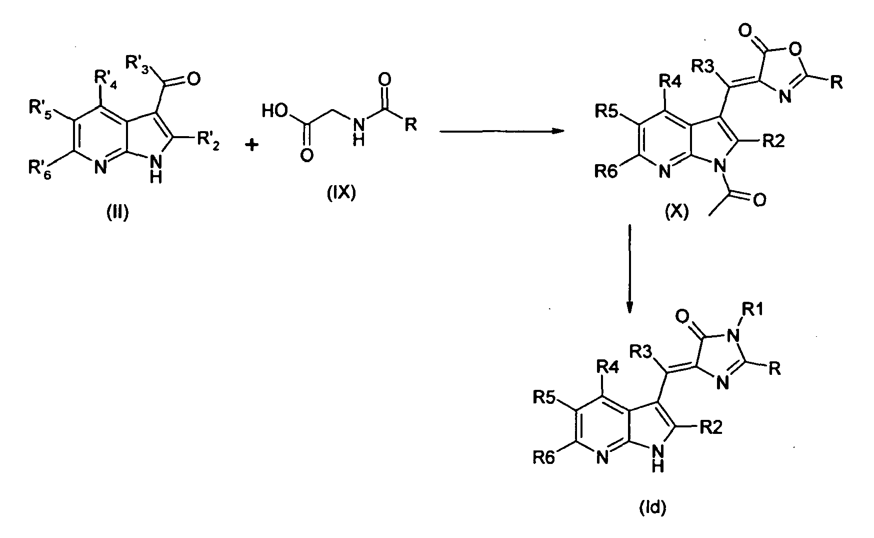

Alternativement, les (5Z)-5-[(1H-pyrrplo[2,3-b]pyridin-3-yl)méthylène]-3,5-dihydro-4H-imidazolidin-4-one (Id) sont obtenues à partir d'une oxazolone de formule (X). Dans une première étape, le cyclo-condensation d'un dérivé amide N-acylé de la glycine (IX) sur un aldéhyde, cétone ou ester de formule (II), en présence d'une base telle que l'acétate de sodium dans l'anhydrique acétique et à une température comprise entre l'ambiante et le reflux, donne une oxazolone de formule (X). Cette réaction avec des dérivés de l'indole a été décrite : J. Am. Chem. Soc. 1946, 647. Dans une deuxième étape, la réaction de l'oxazolone (X) avec un excès d'amine de formule R1NH2, ou d'ammoniaque, donne une imidazolone (Id). Cette réaction se fait dans un solvant tel que le méthanol ou l'éthanol, a une température comprise entre l'ambiante et le reflux. Alternativement, cette réaction peut se faire dans un tube scellé sous irradiation micro-ondes, à une température comprise entre 80°C et 200°C.

Tableau 5 les produits de formule (I) du tableau 5 peuvent être préparés selon le schéma 3 ou 5 ci-dessus et sont décrits en exemples dans la partie expérimentale ci-après

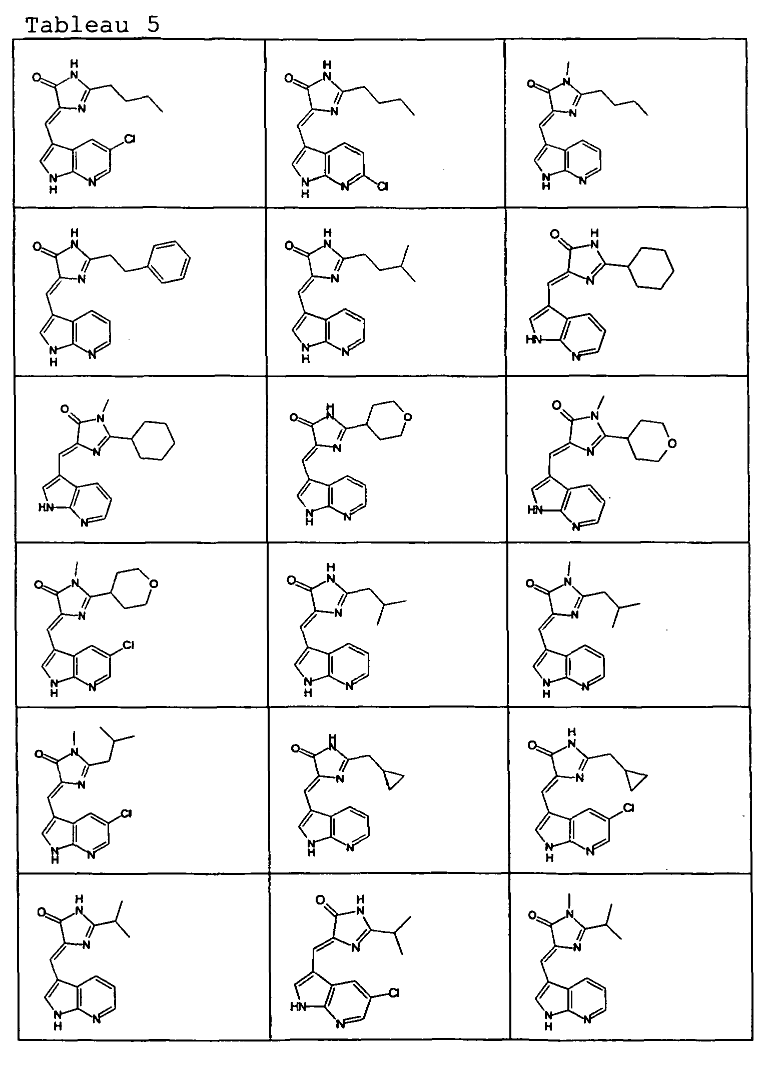

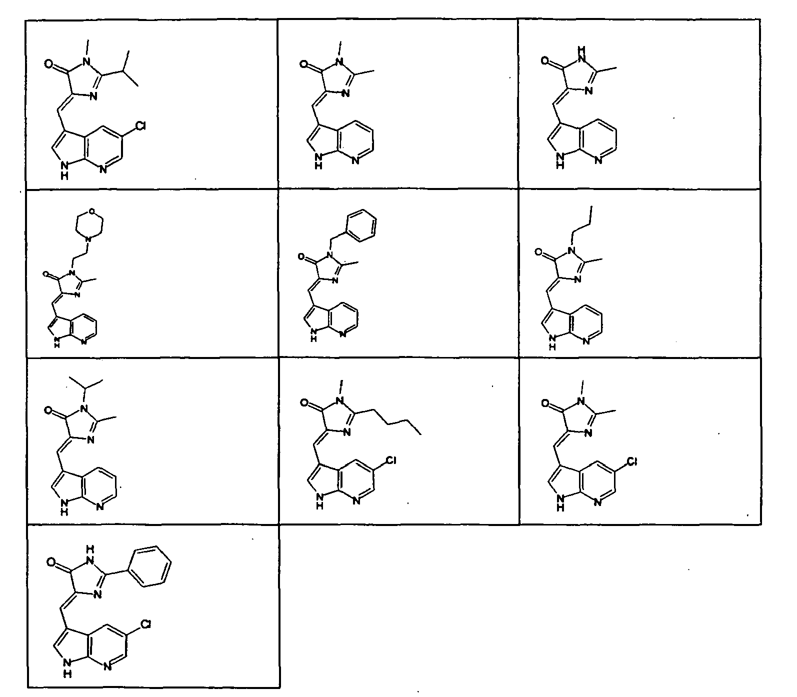

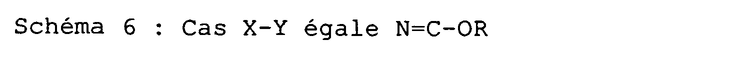

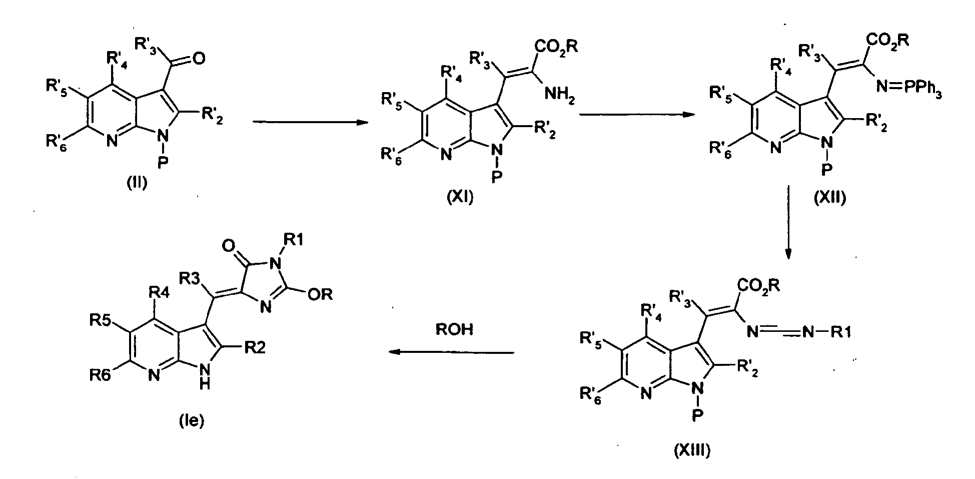

Les composés de type (Ie) peuvent être préparés par analogie à des dérivés d'imidazopyridines décrits par exemple dans J. Org.Chem 2001, 66(20), 6576-6584.

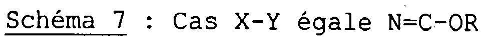

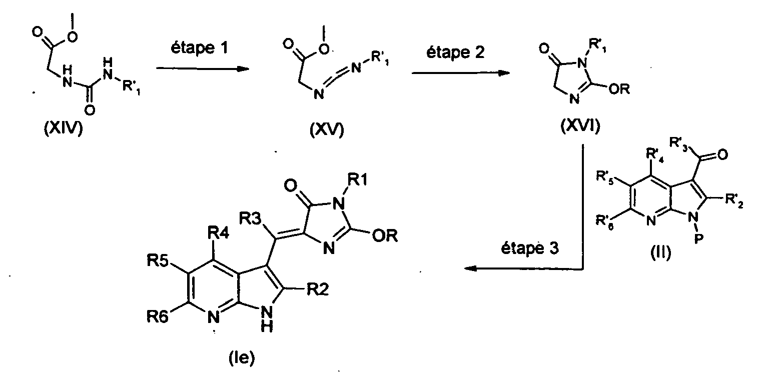

Alternativement, les molécules de type (Ie) peuvent être préparées en formant d'abord l'imidazolone selon une méthode analogue à celle décrite par exemple dans Bull. Korean Chem. Soc. 2007, 28(6), 913-914. Dans une première étape, la déhydratation d'une ureé (XIV), avec par exemple du brome et de la triphénylphosphine, donne un carbodiimide de formule (XV). Dans une deuxième étape, l'addition d'un alcool et cyclisation donne l'imidazolone de formule (XVI). Enfin, la condensation sur un aldéhyde ou cétone de formule (II) donne un composé (Ie).

Les exemples du tableau 7 peuvent être préparés selon le schéma 6 ou 7.

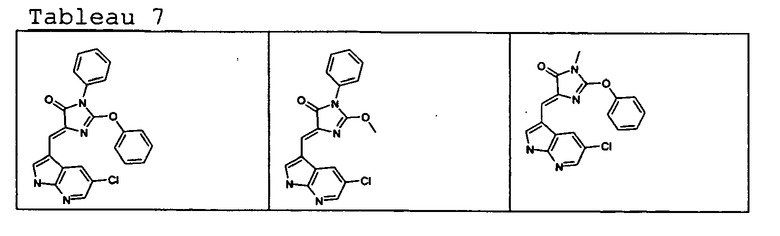

Tableau 8: les composés du tableau 8 peuvent être préparés selon les méthodes décrites ci-dessus dans les schémas 1 à 7:

**Tableau 8: exemples de composés I**

| Composé | exemple | Préparé selon le schéma | RMN (400MHz, DMSO-d6) |
|---|---|---|---|
| 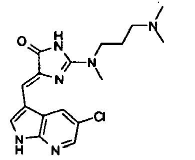 | 100 | 1 | 1.59-2.36 (m, 10H); 3.10 (b s, 1H) ; 3.48 (b s, 2H) ; 6.59 (s, 1H) ; 8.14 (b s, 1H) ; 8.23 (s, 1H) ; 9.06 (b s, 1H) ; 12.19 (b s, 1H). |
| 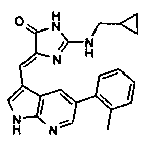 | 101 | 1 | 0.06-0.55 (m, 4H), 1.03 (m, 1H), 2.28 (s, 3H), 3.13 (m, 2H), 6.64 (s, 1H), 7.15 (m 1, 1M), 7.20-7.38 (m, 4H), 8.16 (s 1, 1H), 8.19 (s 1, 1H), 8.90 (s 1, 1H), 10. 4 (m 1, 1H), 12.01 (m 1, 1H). |
| 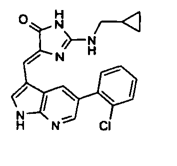 | 102 | 1 | 0.03-0.49 (m, 4H), 1.04 (m, 1H), 3.16 (m 1, 2H), 6.61 (s, 1H), 7.20 (m 1, 1H), 7.38-7.63 (m, 4H), 8.14 (s 1, 1H), 8.28 (s 1, 1H), 9.10 (m 1, 1H), 10.4 (m 1, 1H), 12.08 (m 1, 1H). |
| 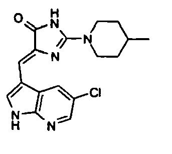 | 103 | 1 | 0.94 (d, J = 6.2 Hz, 3H), 1.16 (m, 2H), 1.55-1.77 (m, 3H), 3.03 (m, 2H), 4.13 (m, 2H), 6.62 (s, 1H), 8.19 (s, 1H), 8.23 (d, J = 2.5 Hz, 1H), 9.03 (m 1,1H), 10.4-12.5 (m 1, 2H). |
| 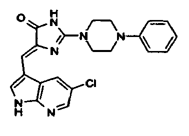 | 104 | 1 | 1.70 (m, 2H), 1.89 (m, 2H), 2.85 (tt, J = 12.2, 3.7Hz, 1H), 3.15 (m, 2H), 4.33 (m, 2H), 6.65 (s, 1H), 7.13-7.36 (m, 5H), 8.23 (m, 2H), 9.0 (s 1, 1H), 12.1 (s 1, 1H). |
| 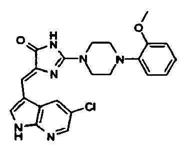 | 105 | 1 | 3.07 (m, 4H), 3.74 (m, 4H), 3.82 (s, 3H), 6.69 (s, 1H), 6.81-7.06 (m, 4H), 8.24 (d, J = 2.3 Hz, 1H), 8.26 (s, 1H), 8.93 (s 1, 1H), 11.20 (m 1, 1H), 12.15 (m 1, 1H). |
| 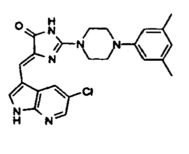 | 106 | 1 | 2.22 (s, 6H), 3.24 (m, 4H), 3. 73 (m, 4H), 6. 48 (s 1, 1H), 6.64 (s 1, 2H), 6.69 (s, 1H), 8.25 (m, 2H), 8.96 (s 1, 1H), 11.20 (m 1, 1H), 12.25 (m 1, 1H) . |
| 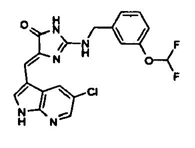 | 107 | 1 | 4.58 (d, J = 5.7Hz, 2H), 6.64 (s 1, 1H), 7.08 (m, 2H), 7.20 (t, J = 74.5 Hz, 1H), 7.22 (s 1, 1H), 7.31 (d 1, J = 7.8Hz, 1H), 7.43 (t, J = 7.8 Hz, 1H), 7.76 (m 1, 1H), 8.17 (s 1, 1H), 8.22 (d 1, J = 5.2Hz, 1H), 8.58 (d 1, J = 7.9Hz, 1H), 10.7 (m 1, 1H), 11.92 (s 1, 1H). |
| 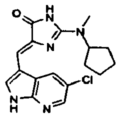 | 108 | 2 | 1.47-1.97 (m, 8H), 2.96 (s 1, 3H), 4.62 (m, 1H), 6.58 (s 1, 1H), 8.14 (s 1, 1H), 8.22 (s 1, 1H), 9.22 (s 1, 1H), 11.07 (m 1, 1H), 12.16 (s 1, 1H). |
| 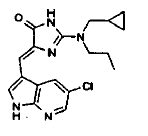 | 109 | 2 | 0.34 (m, 2H), 0.52 (m, 2H), 0.93 (t, J = 8Hz, 3H), 1.15 (m, 1H), 1.68 (q, J = 8Hz, 2H), 3.42 (d, J = 8Hz, 2H), 3.50 (m, 2H), 6.56 (s, 1H), 8.07 (s, 1H), 8.20 (s, 1H), 9.09 (s, 1H), 10. 9 (s 1, 1H), 11.9 (s 1, 1H). |
| 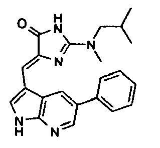 | 110 | 1 | 0.85 (d, J = 6.8 Hz, 6H), 2.01 (m, 1H), 3.10 (s, 3H), 3.26 (d, J = 6.8 Hz, 2H), 6.71 (s, 1H), 7.37 (t, J = 7.6 Hz, 1H), 7.48 (t, J = 7.6 Hz, 2H), 7.75 (d, J = 7.6 Hz, 2H), 8.20 (d, J = 2.9 Hz, 1H), 8.54 (d, J = 2 Hz, 1H), 9.08 (s 1, 1H), 11.05 (s 1, 1H), 12.0 (s 1, 1H). |
| 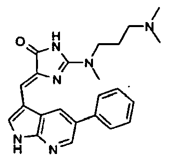 | 111 | 1 | 1.55-2.30 (m, 10H), 3.10 (s, 3H), 3.40-3.60 (m, 2H), 6.71 (s, 1H), 7.36 (t, J = 7.7Hz, 1H), 7.48 (t, J = 7.7Hz, 2H), 7.77 (d, J = 7.7Hz, 2H), 8.21 (m 1, 1H), 8. 55 (s 1, 1H), 9.09 (m 1, 1H), 11.07 (m 1, 1H), 12.05 (m 1, 1H). |
| 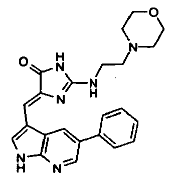 | 112 | 1 | 2.40 (m, 6H), 3.50 (m, 2H), 3.56 (m, 4H), 6.72 (s, 1H), 6.88 (s 1, 1H), 7.37 (t, J = 7.6Hz, 1H), 7.49 (t, J = 7.6Hz, 2H), 7.78 (d, J = 7.6Hz, 2H), 8.20 (s, 1H), 8.55 (s, 1H), 9.11 (s, 1H), 10.4 (s 1, 1H), 12.0 (s 1, 1H). |
| 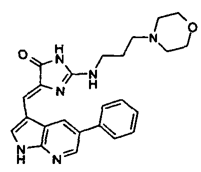 | 113 | 1 | 1.72 (m, 2H), 2.31 (m, 6H), 3.41 (m, 2H), 3.56 (m, 4H), 6.71 (s, 1H), 7.12 (s 1, 1H), 7.37 (t, J = 7. 6Hz, 1H), 7.49 (t, J = 7.6Hz, 2H), 7.78 (d, J = 7.6Hz, 2H), 8.18 (s, 1H), 8.55 (s, 1H), 9.13 (s, 1H), 10.5 (s 1, 1H), 12.0 (s 1, 1H). |
| 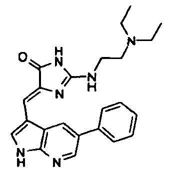 | 114 | 1 | 0.97 (t, J = 7Hz, 6H), 2.50 (m, 4H), 2.61 (t, J = 6.4Hz, 2H), 3.43 (m, 2H), 6.73 (s, 1H), 6.82 (s 1, 1H), 7.38 (t, J = 8Hz, 1H), 7.49 (t, J = 8Hz, 2H), 7.79 (d, J = 8Hz, 2H), 8.21 (s, 1H), 8.56 (s, 1H), 9.05 (s, 1H), 10.49 (s 1, 1H), 12.06 (s 1, 1H) . |
| 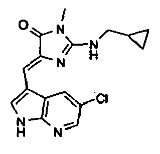 | 115 | 1 | 0.35 (m, 2H), 0.52 (m, 2H), 1.24 (m, 1H), 3.07 (s, 3H), 3.35 (t, J = 7Hz, 2H), 6.73 (s, 1H), 7.65 (t, J = 6Hz, 1H), 8.19 (d, J = 2Hz, 1H), 8.24 (d, J = 2Hz, 1H), 9.28 (d, J = 2Hz, 1H), 12.2 (s 1, 1H). |
| 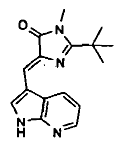 | 116 | 5 | 1.43 (s, 9H); 3.30 (s, 3H); 7.19 (dd, J = 8.1, 4.6 Hz, 1H); 7.35 (s, 1H); 8.29 (dd, J = 4.6, 1.5 Hz, 1H), 8. 38 (s, 1H), 9.07 (d 1, J = 8.1 Hz, 1H), 12.4 (b, 1H). |
| 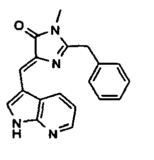 | 117 | 5 | 3.10 (s, 3H), 4.10 (s, 2H), 7.00 (dd, J = 7.9, 4.7 Hz, 1H), 7.29 (s, 1H), 7.31-7.48 (m, 5H), 8.24 (dd, J = 4.7, 1.4 Hz, 1H), 8.29 (d, J = 2.2 Hz, 1H), 8.81 (dd, J = 7.9, 1.4 Hz, 1H), 12.3 (m 1, 1H). |
| 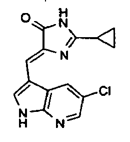 | 118 | 3 | 1.12 (d, J = 6.6Hz, 4H), 1.85 (q, J = 6.6Hz, 1H), 7.06 (s, 1H), 8.28 (d, J = 2.6Hz, 1H), 8.32 (s, 1H), 9.25 (d, J = 2.6Hz, 1H), 11.22 (m 1, 1H), 12.36 (m 1, 1H). |
| 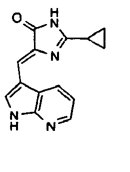 | 119 | 3 | 1.06-1.13 (m, 4H), 1.87 (m, 1H), 7.08 (s, 1H), 7.18 (dd, J = 8.1, 4.6Hz, 1H), 8.28 (dd, J = 4.6, 1.5Hz, 1H), 8.31 (s, 1H), 8.83 (dd, J = 8.1, 1.5Hz, 1H), 11.17 (m 1, 1H), 12.33 (m 1, 1H). |
| 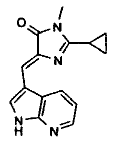 | 120 | 3 | 1.10-1.19 (m, 4H), 2.06 (m, 1H), 3.23 (s, 3H), 7.19 (dd, J = 7.8, 4.9Hz, 1H), 7.22 (s, 1H), 8.29 (dd, J = 4.9, 1.5MHz, 1H), 8.32 (s, 1H), 8.86 (dd, J = 7.8, 1.5Hz, 1H), 12.35 (s 1, 1H). |
| 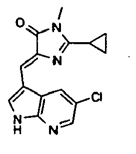 | 121 | 3 | 1.17 (d, J = 6.6Hz, 4H), 2.08 (q, J = 6.6Hz, 1H), 3.23 (s, 3H), 7.21 (s, 1H), 8.29 (d, J = 2.6Hz, 1H), 8.33 (s, 1H), 9.32 (d, J = 2.6Hz, 1H), 12.58 (s 1, 1H). |
| 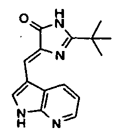 | 122 | 3 | 1.33 (s, 9H), 7.18 (m, 1H), 7.20 (s, 1H), 8.29 (m, 1H), 8.35 (s, 1H), 9.02 (d, J = 8Hz, 1H), 11.2 (s 1, 1H), 12.3 (s 1, 1H) . |
| 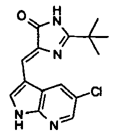 | 123 | 3 | 1.37 (s, 9H), 7.17 (s, 1H), 8.28 (d, J = 2Hz, 1H), 8.32 (s, 1H), 9.55 (s, 1H), 11.2 (s 1, 1H), 12.5 (s 1, 1H). |
| 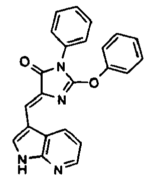 | 12.4 | 6 | 6.89 (m, 1H), 7.32 (s, 1H), 7.35-7.7 (m, 10H), 8.09 (s, 1H), 8.21 (s, 1H), 8.60 (d, J = 8.6Hz, 1H), 12.25 (s 1, 1H). |
| 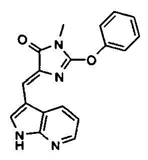 | 125 | 7 | 3.20 (s, 3H), 6.90 (s, 1H), 7.21 (s, 1H), 7.3-7.7 (m, 5H), 8.04 (s, 1H), 8.20 (s, 1H), 8. 59 (d, J = 7.6Hz, 1H), 12.2 (s 1, 1H) . |
| 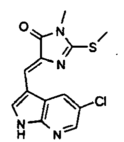 | 126 | 2 | 2.81 (s, 3H), 3.13 (s, 3H), 7.22 (s, 1H), 8.32 (d, J = 2Hz, 1H), 8.43 (s, 1H), 9.36 (d, J = 2Hz, 1H), 12.60 (s 1, 1H). |
| 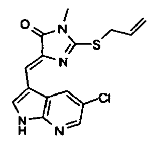 | 127 | 2 | 3.10 (s, 3H), 4.11 (d, J = 6.8Hz, 2H), 5.23 (d, J = 10Hz, 1H), 5.45 (d, J = 19Hz, 1H), 6.10 (m, 1H), 7.22 (s, 1H), 8.30 (d, J = 2Hz, 1H), 8.43 (s, 1H), 9.25 (d, J = 2Hz, 1H), 12.6 (s 1, 1H). |
| 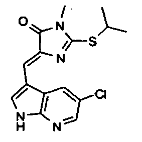 | 128 | 2 | 1.57 (d, J = 6.6Hz, 6H), 3.07 (s, 3H), 4.20 (m, 1H), 7.19 (s, 1H), 8.32 (d, J = 2Hz, 1H), 8.39 (s, 1H), 9.37 (d, J = 2Hz, 1H), 12.6 (s 1, 1H). |
| 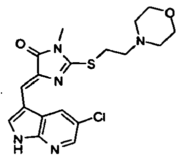 | 129 | 2 | 2.47 (m, 4H), 2.79 (t, J = 6.8Hz, 2H), 3.10 (s, 3H), 3.60 (m, 6H), 7.22 (s, 1H), 8.31 (d, J = 2Hz, 1H), 8.44 (s, 1H), 9.18 (d, J = 2Hz, 1H), 12.6 (s 1, 1H) . |

De tels produits obtenus par les schémas ci-dessus peuvent être des produits de formule (I) ou encore, pour obtenir des produits de formule (I) ou être transformés en d'autres produits de formule (I), peuvent être soumis, si désiré et si nécessaire, dans un ordre quelconque, à une ou plusieurs réactions de transformations connues de l'homme du métier telles que par exemple les réactions suivantes:
a) une réaction d'estérification ou d'amidification de fonction acide
b) le cas échéant, une réaction d'oxydation de groupement alkylthio en sulfoxyde ou sulfone correspondant,
c) une réaction de réduction de la fonction carboxy libre ou estérifié en fonction alcool,
d) une réaction de transformation de fonction alcoxy, en fonction hydroxyle, ou encore de fonction hydroxyle en fonction alcoxy,
e) une réaction d'élimination des groupements protecteurs que peuvent porter les fonctions réactives protégées,
f) une réaction de salification par un acide minéral ou organique ou par une base pour obtenir le sel correspondant,
g) une réaction de dédoublement des formes racémiques en produits dédoublés,
lesdits produits de formule (I) ainsi obtenus étant sous toutes les formes isomères possibles racémiques, énantiomères et diastéréoisomères.

On peut noter que de telles réactions de transformation de substituants en d'autres substituants peuvent également être effectuées sur les produits de départ ainsi que sur les intermédiaires tels que définis ci-dessus avant de poursuivre la synthèse selon les réactions indiquées dans le procédé décrit ci-dessus.

Les procédés décrits dans les schémas ci-dessus peut être réalisés selon les conditions usuelles connues de l'homme du métier et notamment selon les conditions réactionnelles décrites ci-après pour la préparation des exemples de la présente demande.

Parmi les produits de départ utilisés pour la préparation des produits de formule (I) selon la présente invention, certains peuvent être obtenus commercialement ou peuvent être préparés selon les méthodes usuelles connues de l'homme du métier.

On peut encore notamment préparer certains produits de départ à partir de produits connus ou commerciaux par exemple en les soumettant à une ou plusieurs réactions connues de l'homme du métier.

La partie expérimentale ci-après donne des exemples de tels produits de départ.

Pour la préparation des produits de formule (I) selon la présente invention, les diverses fonctions réactives que peuvent porter certains composés des réactions définies ci-dessus peuvent, si nécessaire, être protégées : il s'agit par exemple des radicaux hydroxyle, acyle, carboxy libres ou encore amino et monoalkylamino qui peuvent être protégés par les groupements protecteurs appropriés.

La liste suivante, non exhaustive, d'exemples de protection de fonctions réactives peut être citée :
- les groupements hydroxyle peuvent être protégés par exemple par les radicaux alkyle tels que tert-butyle, triméthylsilyle, tert-butyldiméthylsilyle, méthoxyméthyle, tétrahydropyrannyle, benzyle ou acétyle,
- les groupements amino peuvent être protégés par exemple par les radicaux acétyle, trityle, benzyle, tert-butoxycarbonyle, BOC, benzyloxycarbonyle, phtalimido ou d'autres radicaux connus dans la chimie des peptides,
- les groupements acyles tel que le groupement formyle peuvent être protégés par exemple sous forme de cétals ou de thiocétals cycliques ou non cycliques tels que le diméthyl ou diéthylcétal ou l'éthylène dioxycétal, ou le diéthylthiocétal ou l'éthylènedithiocétal,
- les fonctions acide des produits décrits ci-dessus peuvent être, si désiré, amidifiées par une amine primaire ou secondaire par exemple dans du chlorure de méthylène en présence, par exemple, de chlorhydrate de 1-éthyl-3-(diméthylaminopropyl) carbodiimide à la température ambiante
- les fonctions acide peuvent être protégées par exemple sous forme d'esters formés avec les esters facilement clivables tels que les esters benzyliques ou ter butyliques ou des esters connus dans la chimie des peptides.

Notamment on peut protéger le NH ou 1H-pyrrolo[2,3-b]pyridine si nécessaire avec du paratoluènesulfonyle, phénylsulfonyle, acétyle, triisopropylsilyle, terbutyldiméthylsilyle ou encore triméthylsilyléthoxyméthyle.

Les réactions a) à g) peuvent être réalisées, par exemple, comme indiqué ci-après.
a) Les produits décrits ci-dessus peuvent, si désiré, faire l'objet, sur les éventuelles fonctions carboxy, de réactions d'estérification ou d'amidification qui peuvent être réalisées selon les méthodes usuelles connues de l'homme du métier. Les réactions d'amidification peuvent notament être réalisées en présence d'un agent de couplage tel qu'un dérivé de carbodiimide. On cite à titre d'exemples le N-(3-diméthylaminopropyl),
   -N'-éthylcarbodiimide (EDCI), le N,N'-diisoproyl-carbodiimide (DIC)ou le N,N'-dicyclohexyl-carbodiimide.
b) Les éventuels groupements alkylthio des produits décrits ci-dessus-peuvent être, si désiré, transformés en les fonctions sulfoxyde ou sulfone correspondantes dans les conditions usuelles connues de l'homme du métier telles que par exemple par les peracides comme par exemple l'acide peracétique ou l'acide métachloroperbenzoïque ou encore par l'ozone, l'oxone, le périodate de sodium dans un solvant tel que par exemple le chlorure de méthylène ou le dioxanne à la température ambiante.

L'obtention de la fonction sulfoxyde peut être favorisée par un mélange équimolaire du produit renfermant un groupement alkylthio et du réactif tel que notamment un peracide.

L'obtention de la fonction sulfone peut être favorisée par un mélange du produit renfermant un groupement alkylthio avec un excès du réactif tel que notamment un peracide.
c) Les éventuelles fonctions carboxy libre ou estérifié des produits décrits ci-dessus peuvent être, si désiré, réduites en fonction alcool par les méthodes connues de l'homme de métier : les éventuelles fonctions carboxy estérifié peuvent être, si désiré, réduites en fonction alcool par les méthodes connues de l'homme du métier et notamment par de l'hydrure de lithium et d'aluminium dans un solvant tel que par exemple le tétrahydrofuranne ou encore le dioxane ou l'éther éthylique.

Les éventuelles fonctions carboxy libre des produits décrits ci-dessus peuvent être, si désiré, réduites en fonction alcool notamment par de l'hydrure de bore.
d) Les éventuelles fonctions alcoxy telles que notamment méthoxy des produits décrits ci-dessus peuvent être, si désiré, transformées en fonction hydroxyle dans les conditions usuelles connues de l'homme du métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide trifluoro acétique au reflux.

Il est entendu que les réactions décrites ci-dessus peuvent être effectuées comme indiqué ou encore, le cas échéant, selon d'autres méthodes usuelles connues de l'homme du métier.
e) L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou para-toluène sulfonique, formique ou trifluoroacétique ou encore par une hydrogénation catalytique.

Le groupement phtalimido peut être éliminé par l'hydrazine.

On trouvera une liste de différents groupements protecteurs utilisables par exemple dans le brevet BF 2 499 995.
f) Les produits décrits ci-dessus peuvent, si désiré, faire l'objet de réactions de salification par exemple par un acide minéral ou organique ou par une base minérale ou organique selon les méthodes usuelles connues de l'homme du métier : une telle réaction de salification peut être réalisée par exemple en présence d'acide chlorhydrique par exemple ou encore d'acide tartrique, citrique ou méthane sulfonique, dans un alcool tel que par exemple l'éthanol ou le méthanol.
g) Les éventuelles formes optiquement actives des produits décrits ci-dessus peuvent être préparées par dédoublement des racémiques selon les méthodes usuelles connues de l'homme du métier.

Les produits de formule (I) tels que définis ci-dessus ainsi que leurs sels d'addition avec les acides présentent d'intéressantes propriétés pharmacologiques notamment en raison de leurs propriétés inhibitrices de kinases ainsi qu'il est indiqué ci-dessus.

On peut indiquer que certaines protéines kinases jouant un rôle central dans l'initiation, le développement et l'achèvement des évènements du cycle cellulaire, des molécules inhibitrices de telles kinases sont susceptibles de limiter des proliférations cellulaires non désirées telles que celles observées dans les cancers, peuvent intervenir dans la prévention, la régulation ou le traitement de maladies neurodégénératives telles que la maladie d'Alzheimer ou encore l'apoptose neuronale.

Les produits de la présente invention sont notamment utiles pour la thérapie de tumeurs.

Les produits de l'invention peuvent également ainsi augmenter les effets thérapeutiques d'agents anti-tumoraux couramment utilisés.

Les produits de formule (I) de la présente invention possèdent donc tout particulièrement des propriétés anti-prolifératives.

Ces propriétés justifient leur application en thérapeutique et l'invention a particulièrement pour objet à titre de médicaments, les produits de formule (I) telle que définie ci-dessus, lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I).

L'invention a particulièrement pour objet, à titre de médicaments, les produits décrits ci-après dans les exemples et notamment les produits dont les noms suivent:
- la (5Z)-5-(1H-pyrrolo[2,3-b]pyridin-3-ylméthylène)-2-thioxoimidazolidin-4-one
- la (5Z)-5-[(5-chloro-1H-pyrrolo[2,3-b]pyridin-3-yl)méthylène]-2-thioxoimidazolidin-4-one
- la (5Z)-2-[(cyclopropylméthyl)amino]-5-(1H-pyrrolo[2,3-b]pyridin-3-ylméthylène)-3,5-dihydro-4H-imidazol-4-one
- la (5Z)-5-[(5-chloro-1H-pyrrolo[2,3-b]pyridin-3-yl)méthylène]-2-[(cyclopropylméthyl)amino]-3,5-dihydro-4H-imidazol-4-one
- la (5Z)-5-[(6-chloro-1H-pyrrolo[2,3-b]pyridin-3-yl)méthylène]-2-[(cyclopropylméthyl)amino]-3,5-dihydro-4H-imidazol-4-one
- la (5Z)-2-[(cyclopropylméthyl)amino]-3-méthyl-5-(1H-pyrrolo[2,3-b]pyridin-3-ylméthylène)-3,5-dïhydro-4H-imidazol-4-one
- la (5Z)-2-butyl-5-(1H-pyrrolo[2,3-b]pyridin-3-ylméthylène)-3,5-dihydro-4H-imidazol-4-one
- (5Z)-2-azépan-l-yl-5-[(5-chloro-1H-pyrrolo[2,3-b]pyridin-3-yl)méthylidène]-3,5-dihydro-4H-imidazol-4-one
- 5Z)-5-[(5-chloro-1H-pyrrolo[2,3-b]pyridin-3-yl)méthylidène]-2-pipéridin-1-yl-3,5-dihydro-4H-imidazol-4-one
- 5Z)-5-[(5-chloro-1H-pyrrolo[2,3-b]pyridin-3-yl)méthylidène]-2-[méthyl(2-méthylpropyl)amino]-3,5-dihydro-4H-imidazol-4-one
- (5Z)-5-[(5-bromo-1H-pyrrolo[2,3-b]pyridin-3-yl)méthylidène]-2-[(cyclopropylméthyl)amino]-3,5-dihydro-4H-imidazol-4-one
- (5Z)-2-[(cyclopropylméthyl)amino]-5-[(5-méthoxy-1H-pyrrolo[2,3-b]pyridin-3-yl)méthylidène]-3,5-dihydro-4H-imidazol-4-one
- (5Z)-2-[(cyclopropylméthyl)amino]-5-[(5-fluoro-1H-pyrrolo[2,3-b]pyridin-3-yl)méthylidène]-3,5-dihydro-4H-imidazol-4-one
- (5Z)-2-azépan-1-yl-5-[(5-fluoro-1H-pyrrolo[2,3-b]pyridin-3-yl)méthylidène]-3,5-dihydro-4H-imidazol-4-one
- 3-[(Z)-{2-[(cyclopropylméthyl)amino]-5-oxo-1,5-dihydro-4H-imidazol-4-ylidène}méthyl]-1H-pyrrolo[2,3-b]pyridine-5-carbonitrile
- (5Z)-2-[(cyclopropylméthyl)amino]-5-[(5-phényl-1H-pyrrolo[2,3-b]pyridin-3-yl)méthylidène]-3,5-dihydro-4H-imidazol-4-one
- (5Z)-2-[(cyclopropylméthyl)amino]-5-{[5-(trifluorométhyl)-1H-pyrrolo[2,3-b]pyridin-3-yl]méthylidène}-3,5-dihydro-4H-imidazol-4-one
- (5Z)-5-[(5-chloro-1H-pyrrolo[2,3-b]pyridin-3-yl)méthylidène]-2-(méthylsulfanyl)-3,5-dihydro-4H-imidazol-4-one
- (5Z)-2-butyl-5-'[(5-chloro-1H-pyrrolo[2,3-b]pyridin-3-yl)méthylidène]-3,5-dihydro-4H-imidazol-4-one
- (5Z)-2-(3-méthylbutyl)-5-(1H-pyrrolo[2,3-b]pyridin-3-ylméthylidène)-3,5-dihydro-4H-imidazol-4-one
- (5Z)-2-cyclohexyl-5-(1H-pyrrolo[2,3-b]pyridin-3-ylméthylidène)-3,5-dihydro-4H-imidazol-4-one
- (5Z)-5-[(5-chloro-1H-pyrrolo[2,3-b]pyridin-3-yl)méthylidène]-3-méthyl-2-(tétrahydro-2H-pyran-4-yl)-3,5-dihydro-4H-imidazol-4-one
- (5Z)-5-[(5-chloro-1H-pyrrolo[2,3-b]pyridin-3-yl)méthylidène]-3-méthyl-2-(2-méthylpropyl)-3,5-dihydro-4H-imidazol-4-one
- (5Z)-2-(cyclopropylméthyl)-5-(5-chloro-1H-pyrrolo[2,3-b]pyridin-3-ylméthylidène)-3,5-dihydro-4H-imidazol-4-one
- (5Z)-2-(1-méthyléthyl)-5-(5-chloro-1H-pyrrolo[2,3-b]pyridin-3-ylméthylidène)-3,5-dihydro-4H-imidazol-4-one
- (5Z)-2-[benzyl(méthyl)amino]-5-[(5-chloro-1H-pyrrolo[2,3-b]pyridin-3-yl)méthylidène]-3,5-dihydro-4H-imidazol-4-one
- (5Z)-5-[(5-chloro-1H-pyrrolo[2,3-b]pyridin-3-yl)méthylidène]-2-[(2-méthoxyéthyl)(méthyl)amino]-3,5-dihydro-4H-imidazol-4-one
- (5Z)-2-azépan-1-yl-5-[(5-chloro-1H-pyrrolo[2,3-b]pyridin-3-yl)méthylidène]-3,5-dihydro-4H-imidazol-4-one
- 5Z)-5-[(5-chloro-1H-pyrrolo[2,3-b]pyridin-3-yl)méthylidène]-2-pipéridin-1-yl-3,5-dihydro-4H-imidazol-4-one
- 5Z)-5-[(5-chloro-1H-pyrrolo[2,3-b]pyridin-3-yl)méthylidène]-2-[méthyl(2-méthylpropyl)amino]-3,5-dihydro-4H-imidazol-4-one
- (5Z)-5-[(5-bromo-1H-pyrrolo[2,3-b]pyridin-3-yl)méthylidène]-2-[(cyclopropylméthyl)amino]-3,5-dihydro-4H-imidazol-4-one
- (5Z)-2-[(cyclopropylméthyl)amino]-5-[(5-méthoxy-1H-pyrrolo[2,3-b]pyridin-3-yl)méthylidène]-3,5-dihydro-4H-imidazol-4-one
- (5Z)-2-[(cyclopropylméthyl)amino]-5-[(5-fluoro-1H-pyrrolo[2,3-b]pyridin-3-yl)méthylidène]-3,5-dihydro-4H-imidazol-4-one
- (5Z)-2-azépan-1-yl-5-[(5-fluoro-1H-pyrrolo[2,3-b]pyridin-3-yl)méthylidène]-3,5-dihydro-4H-imidazol-4-one
- 3-[(Z)-{2-[(cyclopropylméthyl)amino]-5-oxo-1,5-dihydro-4H-imidazol-4-ylidène}méthyl]-1H-pyrrolo[2,3-b]pyridine-5-carbonitrile
- (5Z)-2-[(cyclopropylméthyl)amino]-5-[(5-phényl-1H-pyrrolo[2,3-b]pyridin-3-yl)méthylidène]-3,5-dihydro-4H-imidazol-4-one
- (5Z)-2-[(cyclopropylméthyl)amino]-5-{[5-(trifluorométhyl)-1H-pyrrolo[2,3-b]pyridin-3-yl]méthylidène}-3,5-dihydro-4H-imidazol-4-one
- (5Z)-5-[(5-chloro-1H-pyrrolo[2,3-b]pyridin-3-yl)méthylidène]-2-(méthylsulfanyl)-3,5-dihydro-4H-imidazol-4-one
- (5Z)-2-butyl-5-[(5-chloro-1H-pyrrolo[2,3-b]pyridin-3-yl)méthylidène]-3,5-dihydro-4H-imidazol-4-one
- (5Z)-2-(3-méthylbutyl)-5-(1H-pyrrolo[2,3-b]pyridin-3-ylméthylidène)-3,5-dihydro-4H-imidazol-4-one
- (5Z)-2-cyclohexyl-5-(1H-pyrrolo[2,3-b]pyridin-3-ylméthylidène)-3,5-dihydro-4H-imidazol-4-one
- (5Z)-5-[(5-chloro-1H-pyrrolo[2,3-b]pyridin-3-yl)méthylidène]-3-méthyl-2-(tétrahydro-2H-pyran-4-yl)-3,5-dihydro-4H-imidazol-4-one
- (5Z)-5-[(5-chloro-1H-pyrrolo[2,3-b]pyridin-3-yl)méthylidène]-3-méthyl-2-(2-méthylpropyl)-3,5-dihydro-4H-imidazol-4-one
- (5Z)-2-(cyclopropylméthyl)-5-(5-chloro-1H-pyrrolo[2,3-b]pyridin-3-ylméthylidène)-3,5-dihydro-4H-imidazol-4-one
- (5Z)-2-(1-méthyléthyl)-5-(5-chloro-1H-pyrrolo[2,3-b]pyridin-3-ylméthylidène)-3,5-dihydro-4H-imidazol-4-one
- (5Z)-2-[benzyl(méthyl)amino]-5-[(5-chloro-1H-pyrrolo[2,3-b]pyridin-3-yl)méthylidène]-3,5-dihydro-4H-imidazol-4-one
- (5Z)-5-[(5-chloro-1H-pyrrolo[2,3-b]pyridin-3-yl)méthylidène]-2-[(2-méthoxyéthyl)(méthyl)amino]-3,5-dihydro-4H-imidazol-4-one
lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I).

L'invention concerne aussi des compositions pharmaceutiques contenant à titre de principe actif l'un au moins des produits de formule (I) tels que définis ci-dessus ou un sel pharmaceutiquement acceptable de ce produit ou un prodrug de ce produit et, le cas échéant, un support pharmaceutiquement acceptable.

L'invention s'étend ainsi aux compositions pharmaceutiques contenant à titre de principe actif l'un au moins des médicaments tels que définis ci-dessus.

De telles compositions pharmaceutiques de la présente invention peuvent également, le cas échéant, renfermer des principes actifs d'autres médicaments antimitotiques tels que notamment ceux à base de taxol, cis-platine, les agents intercalants de l'ADN et autres.

Ces compositions pharmaceutiques peuvent être administrées par voie buccale, par voie parentérale ou par voie locale en application topique sur la peau et les muqueuses ou par injection par voie intraveineuse ou intra-musculaire.

Ces compositions peuvent être solides ou liquides et se présenter sous toutes les formes pharmaceutiques couramment utilisées en médecine humaine comme, par exemple, les comprimés simples ou dragéifiés, les pilules, les tablettes, les gélules, les gouttes, les granulés, les préparations injectables, les pommades, les crèmes ou les gels ; elles sont préparées selon les méthodes, usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 0,05 à 5 g par jour chez l'adulte, ou de préférence de 0,1 à 2 g par jour.

La présente invention a également pour objet l'utilisation des produits de formule (I) tels que définis ci-dessus ou de sels pharmaceutiquement acceptables de ces produits pour la préparation d'un médicament destiné à l'inhibition de l'activité d'une protéine kinase.

La présente invention a également pour objet l'utilisation de produits de formule (I) tels que définis ci-dessus pour la préparation d'un médicament destiné au traitement ou à la prévention d'une maladie caractérisée par le dérèglement de l'activité d'une protéine kinase.

Un tel médicament peut notamment être destiné au traitement ou à la prévention d'une maladie chez un mammifère.

La présente invention a également pour objet l'utilisation définie ci-dessus dans laquelle la protéine kinase est une protéine sérine-thréonine kinase.

La présente invention a notamment pour objet l'utilisation définie ci-dessus dans laquelle la protéine kinase est CDC7.

La présente invention a également pour objet l'utilisation définie ci-dessus dans laquelle la protéine kinase est dans une culture cellulaire.

La présente invention a également pour objet l'utilisation définie ci-dessus dans laquelle la protéine kinase est dans un mammifère.

La présente invention a particulièrement pour objet l'utilisation d'un produit de formule (I) tel que défini ci-dessus pour la préparation d'un médicament destiné au traitement ou à la prévention d'une maladie choisie dans le groupe suivant : troubles de la prolifération de vaisseaux sanguins, troubles fibrotiques, troubles de la prolifération de cellules mesangiales, désordres métaboliques, allergies, asthmes, thromboses, maladies du système nerveux, rétinopathie, psoriasis, arthrite rhumatoïde, diabète, dégénérescence musculaire et cancers.

La présente invention a plus particulièrement pour objet l'utilisation d'un produit de formule (I) tel que défini ci-dessus pour la préparation d'un médicament destiné au traitement ou à la prévention d'une maladie choisie dans le groupe suivant : troubles de la prolifération de cellules mésangiales, psoriasis, arthrite rhumatoïde, diabète, dégénérescence musculaire et cancers.

La présente invention a tout particulièrement pour objet l'utilisation d'un produit de formule (I) tel que défini ci-dessus pour la préparation d'un médicament destiné à la prévention ou au traitement de maladies liées à une prolifération cellulaire non contrôlée, pour la préparation d'un médicament destiné au traitement de maladies en oncologie et notamment destiné au traitement de cancers.

Parmi ces cancers, on s'intéresse notamment au traitement de tumeurs solides, au traitement de cancers résistant à des agents cytotoxiques.

Parmi ces cancers, on s'intéresse notamment au traitement de cancers du sein, de l'estomac, des ovaires, du colon, du poumon, du cerveau, du larynx, du système lymphatique, du tractus génito-urinaire incluant vessie et prostate, de cancers des os et du pancréas, tout particulièrement au traitement de cancers du sein, du colon ou du poumon.

La présente invention a aussi pour objet l'utilisation des produits de formule (I) telle que définie ci-dessus pour la préparation de médicaments destinés à la chimiothérapie de cancers.

De tels médicaments destinés à la chimiothérapie de cancers peuvent être utilisés seuls ou en en association.

Les produits de la présente demande peuvent notamment être administrés seuls ou en association avec de la chimiothérapie ou de la radiothérapie ou encore en association par exemple avec d'autres agents thérapeutiques.

De tels agents thérapeutiques peuvent être des agents anti-tumoraux couramment utilisés.

Comme inhibiteurs de kinases, on peut citer la butyrolactone, le flavopiridol et la 2(2-hydroxyéthylamino)-6-benzylamino-9-méthylpurine appelée olomucine.

La présente invention a particulièrement pour objet les produits de formule (I) tels que définis ci-dessus comme inhibiteurs de CDC7.

La présente invention concerne tout particulièrement les produits de formule (I) qui constituent les exemples 1 à 99 de la présente invention.

Les 99 produits qui suivent illustrent ainsi plus précisément la formule (I) de la présente invention sans toutefois la limiter.

Dans la préparation des 99 produits en exemples dans la présente demande, on a utilisé les dispositifs suivants:
Spectre RMN 1H à 400 MHz sur spectromètre BRUKER AVANCE DRX-400 avec les déplacements chimiques (δ en ppm) - dans le solvant diméthylsulfoxide - d6 (DMSO-d6) référencé à 2,50 ppm à la température de 303K.
Spectre RMN 1H à 300 MHz sur spectromètre BRUKER AVANCE DPX-300 avec les déplacements chimiques (δ en ppm) - dans le solvant diméthylsulfoxide - d6 (DMSO-d6) référencé à 2,50 ppm à la température de 303K.

Analyse LC-MS-DAD-ELSD ( MS : Waters ZQ; électrospray mode +/-; Domaine de masse m/z =100-1200; LC : Agilent HP 1100; Colonne LC : X Bridge 18C Waters 3,0 x 50 mm - 2,5 µm; four LC : 60 °C; débit de phase mobile : 1,1 ml/minute.

Eluants : A : Eau + 0,1 % Acide Formique, B : Acétonitrile avec le gradient suivant :

| Time | A% | B% |
|---|---|---|
| 0.0 | 95 | 5.0 |
| 5.0 | 5.0 | 100 |
| 5.5 | 5.0 | 100 |
| 6.5 | 95.0 | 5.0 |
| 7.0 | 95.0 | 5.0. |

DAD longueur d'onde considérée λ=210-400 nm

ELSD : Sedere SEDEX 85

Analyse SM-EI-CI-Introduction directe-DCI (EI=Ionisation par impact d'électrons, CI : Ionisation chimique , DCI : dépôt de l'échantillon sur filament ) (MS : Finnigan SSQ7000); Energie des électrons : 70 eV; Domaine de masse m/z =29-900; Température de la source d'ionisation =70 °C; gaz réactant CI : ammoniac.

### (1) : Analyse LC/MS en routine :

Détecteur de masse : ZQ (Waters)

LC : colonne : X Bridge 18C Waters 3,0 x 50 mm - 2,5 µm ; débit =1,1 ml/minute.

Eluants : A : Eau + 0,1 % Acide Formique, B : Acétonitrile avec le gradient suivant :

| Time | A% | B% |
|---|---|---|
| 0.0 | 95 | 5.0 |
| 5.0 | 5.0 | 100 |
| 5.5 | 5.0 | 100 |
| 6.5 | 95.0 | 5.0 |
| 7.0 | 95.0 | 5.0 |

DAD : longueur d'onde considérée λ=200-400 nm

ELSD : Sedere SEDEX 85

### (2) : Micro-ondes : BIOTAGE INITIATOR MICROWAVE SYNTHESIZER

### Puissance maximum d'irradiation 300Watts

On peut noter que dans les exemples décrits ci-après, les composés 1H-pyrrolo[2,3-b]pyridine-3-carbaldéhydes (II) peuvent être obtenus à partir des dérivés 1H-pyrrolo[2,3-b]pyridine par réaction avec le 1,3,5,7-tétraazatricyclo[3.3.1.1∼3,7∼]décane (A. Verbiscar J. Med. Chem. 1972, 15, 149) ou par réaction de Vilsmeier (M-C Viaud et al. Heterocycles. 1999, 50, 1065).

### Exemple 1

### (5Z)-5-(1H-pyrrolo[2,3-b]pyridin-3-ylméthylène)-2-thioxoimidazolidin-4-one

La (5Z)-5-(1H-pyrrolo[2,3-b]pyridin-3-ylméthylène)-2-thioxoimidazolidin-4-one peut être préparée de la manière suivante :

A une suspension de 500 mg de 1H-pyrrolo[2,3-b]pyridine-3-carbaldéhyde dans 10 cm³ d'éthanol, sont ajoutés 0,1 cm³ de pipéridine et 400 mg de 2-thioxoimidazolidin-4-one. Le mélange réactionnel est porté au reflux et le solide passe en solution. Après trente minutes de chauffage un précipité se forme. Le chauffage est arrêté et le mélange réactionnel est filtré à une température voisine de 25°C.

On obtient 561 mg de (5Z)-5-(1H-pyrrolo[2,3-b]pyridin-3-ylméthylène)-2-thioxoimidazolidin-4-one sous forme d'une poudre orangée dont les caractéristiques sont les suivantes :
Spectre de RMN ¹H à 400MHz : 6,80 (s : 1H) ; 7,20 (dd, J = 5 et 8 Hz : 1H) ; de 8,26 à 8,34 (m : 2H) ; 8,55 (s : 1H) ; de 11,7 à 12,2 (m étalé : 2H) ; de 12,3 à 12,6 (m étalé : 1H)
Spectre de masse : LC-MS-DAD-ELSD : 243(-) = (M-H)(-) ; 245 (+) = (M+H) (+)

### Exemple 2

### (5Z)-5-[(4-chloro-1H-pyrrolo[2,3-b]pyridin-3-yl)méthylène]-2-thioxoimidazolidin-4-one

a) La (5Z)-5-[(4-chloro-1H-pyrrolo[2,3-b]pyridin-3-yl)méthylène]-2-thioxoimidazolidin-4-one peut être préparée comme dans l'exemple 1 mais à partir de 182 mg de 4-chloro-1H-pyrrolo[2,3-b]pyridine-3-carbaldéhyde dans 3 cm³ d'éthanol, de 117 mg de 2-thioxoimidazolidin-4-one et de 0,1 cm³ de pipéridine. Après deux heures dé reflux, on obtient 250mg d'un mélange contenant 60% de (5Z)-5-[(4-chloro-1H-pyrrolo[2,3-b]pyridin-3-yl)méthylène]-2-thioxoimidazolidin-4-one sous forme d'une poudre orangée. Les caractéristiques de l'isomère (5Z) sont les suivantes :
   Spectre de RMN ¹H à 400MHz : 7,26 (s : 1H) ; 7,30 (m : 1H) ; 8,24 (d, J = 5 Hz : 1H) ; 8,58 (s : 1H) ; de 11, 5 à 13,0 (m très étalé : 3H)
   Spectre de masse : LC-MS-DAD-ELSD : 243(-) = (M-H)(-) ; 245 (+) = (M+H) (+)
b) 4-chloro-1H-pyrrolo[2,3-b]pyridine-3-carbaldéhyde Le 4-chloro-1H-pyrrolo[2,3-b]pyridine-3-carbaldéhyde peut être préparé de la manière suivante :
   A une suspension de 1 g de 4-chloro-1H-pyrrolo[2,3-b]pyridine dans un mélange de 7 cm³ d'eau et 3,5 cm³ d'acide acétique, est ajouté 1,4 g de 1,3,5,7-tétraazatricyclo[3.3.1.1∼3,7∼]décane. Le mélange réactionnel est porté au reflux pendant une heure et trente minutes. Après retour à une température voisine de 25°C, sont ajoutés de l'eau et de la glace. Il y a formation d'un précipité sous forme de gomme. Celle-ci est reprise par deux fois 20 cm³ d'acétate d'éthyle. Les phases organiques sont rassemblées, lavées par de l'eau, séchées sur sulfate de magnésium, filtrées et concentrées à sec sous pression réduite. Le résidu est repris par 5 cm³ d'acétate d'éthyle. Le précipité obtenu est filtré et séché sous pression réduite. On obtient 82 mg de 4-chloro-1H-pyrrolo[2,3-b]pyridine-3-carbaldéhyde. Le filtrat est concentré à sec sous pression réduite et après flash chromatographie sur colonne de silice [éluant : dichlorométhane/méthanol (gradient de 99/1 à 90/10 en volumes)], on obtient 160 mg de 4-chloro-1H-pyrrolo[2,3-b]pyridine-3-carbaldéhyde dont les caractéristiques sont les suivantes :
      LC/MS (1) : Temps de rétention : 2,69 mn Spectre de masse (1) : (ES+) : m/z = 181 [MH+]
c) La 4-chloro-1H-pyrrolo[2,3-b]pyridine peut être préparée comme décrit dans C.Thibault et al : Org. Lett. 2003, 5 (26) ; 5023.

### Exemple 3

### (5Z)-5-[(5-chloro-1H-pyrrolo[2,3-b]pyridin-3-yl)méthylène]-2-thioxoimidazolidin-4-one

a) La (5Z)-5-[(5-chloro-1H-pyrrolo[2,3-b]pyridin-3-yl)méthylène]-2-thioxoimidazolidin-4-one peut être préparée comme dans l'exemple 1 mais à partir de 400 mg de 5-chloro-1H-pyrrolo[2,3-b]pyridine-3-carbaldéhyde dans 15 cm³ d'éthanol, de 257 mg de 2-thioxoimidazolidin-4-one et de 0,22 cm³ de pipéridine. Après deux heures de reflux, on obtient 320 mg de (5Z)-5-[(5-chloro-1H-pyrrolo[2,3-b]pyridin-3-yl)méthylène]-2-thioxoimidazolidin-4-one sous forme d'une poudre orangée ) dont les caractéristiques sont les suivantes :
   Spectre de RMN ¹H à 400MHz : 6,79 (s : 1H) ; 8,29 (d, J = 3 Hz : 1H) ; 8,54 (d, J = 3 Hz : 1H) ; 8,57 (s : 1H) ; de 7,00 à 8,30 (m très étalé : 2H) ; de 11,4 à 12,5 (m étalé : 1H)
   Spectre de masse : LC-MS-DAD-ELSD : 279(+)= (M+H)(+) (1 atome de chlore Cl présent)
b) Le 5-chloro-1H-pyrrolo[2,3-b]pyridine-3-carbaldéhyde peut être préparé comme décrit dans WO05/95400.

### Exemple 4

### (5Z)-5-[(6-chloro-1H-pyrrolo[2,3-b]pyridin-3-yl)méthylène]-2-thioxoimidazolidin-4-one

a) La (5Z)-5-[(6-chloro-1H-pyrrolo[2,3-b]pyridin-3-yl)méthylène]-2-thioxoimidazolidin-4-one peut être préparée comme dans l'exemple 1 mais à partir de 140 mg de 6-chloro-1H-pyrrolo[2,3-b]pyridine-3-carbaldéhyde dans 5 cm³ d'éthanol, de 90 mg de 2-thioxoimidazolidin-4-one et de 0,08 cm³ de pipéridine. Après deux heures de reflux, on obtient 180 mg de (5Z)-5-[(6-chloro-1H-pyrrolo[2,3-b]pyridin-3-yl)méthylène]-2-thioxoimidazolidin-4-one sous forme d'une poudre dont les caractéristiques sont les suivantes :
   Spectre de RMN ¹H à 40MHz 6,57 (s : 1H) ; 7,20 (d, J = 8 Hz : 1H) ; 8,40 (s : 1H) ; 8,49 (d, J = 8 Hz : 1H) ; de 9,40 à 10,5 (m étalé : 3H)
   Spectre de masse : LC-MS-DAD-ELSD: 279(+)= (M+H)(+) (1 atome de chlore Cl présent)
b) Le 6-chloro-1H-pyrrolo[2,3-b]pyridine-3-carbaldéhyde peut être préparé comme décrit dans WO01/98299.

### Exemple 5

### (5Z)-5-[(2-méthyl-1H-pyrrolo[2,3-b]pyridin-3-yl)méthylène]-2-thioxoimidazolidin-4-one

a) La (5Z)-5-[(2-méthyl-1H-pyrrolo[2,3-b]pyridin-3-yl)méthylène]-2-thioxoimidazolidin-4-one peut être préparée comme dans l'exemple 1 mais à partir de 400 mg de 2-méthyl-1H-pyrrolo[2,3-b]pyridine-3-carbaldéhyde dans 15 cm³ d'éthanol, de 290 mg de 2-thioxoimidazolidin-4-one et de 0,25 cm³ de pipéridine. Après quatre heures de reflux, on obtient 463 mg d'un mélange contenant 65% de (5Z)-5-[(2-méthyl-1H-pyrrolo[2,3-b]pyridin-3-yl)méthylène]-2-thioxoimidazolidin-4-one sous forme d'une poudre orangée qui sera utilisée tel que pour l'étape suivante et dont les caractéristiques sont les suivantes :
   Spectre de RMN ¹H à 400MHz : 2,45 (s : 3H) ; 6,70 (s large : 1H) ; 7; 12 (dd, J = 5 et 8 Hz : 1H) ; 7,88 (m : 1H) ; 8,20 (dd, J = 1,5 et 5 Hz : 1H) ; 11,55 (s large : 1H); de 12,0 à 12,2 (m étalé : 2H)
   Spectre de masse : LC-MS-DAD-ELSD : mélange contenant la structure attendue 259(+) = (M+H)(+)
b) 2-méthyl-1H-pyrrolo[2,3-b]pyridine-3-carbaldéhyde
   Le 2-méthyl-1H-pyrrolo[2,3-b]pyridine-3-carbaldéhyde peut être préparé comme dans l'exemple 2 mais à partir de 835 mg de 2-méthyl-1H-pyrrolo[2,3-b]pyridine dans un mélange de 5,3 cm³ d'eau et 1,6 cm³ d'acide acétique et 1,32 g de 1,3,5,7-tétraazatricyclo[3.3.1.1∼3,7∼]décane. On obtient 580 mg d'un mélange contenant 60% de 2-méthyl-1H-pyrrolo[2,3-b]pyridine-3-carbaldéhyde sous forme d'un solide beige. Les caractéristiques pour le 2-méthyl-1H-pyrrolo[2,3-b]pyridine-3-carbaldéhyde sont les suivantes :
   LC/MS (1) : Temps de rétention : 1,7 mn
   Spectre de masse (1) : (ES+) : m/z = 161 [MH+]
c) 2-méthyl-1H-pyrrolo[2,3-b]pyridine
   La 2-méthyl-1H-pyrrolo[2,3-b]pyridine peut être préparée de la manière suivante :
   A une solution de 2,2 g de 2-méthyl-1-[(4-méthylphényl)sulfonyl]-1H-pyrrolo[2,3-b]pyridine dans 70 cm³ de méthanol, sont ajoutés 8,2 g d'hydroxyde de potassium. Le mélange réactionnel est laissé sous agitation à une température voisine de 20°C durant cinq heures puis porté au reflux durant cinq heures. Le milieu 1 (ou mélange) est alors concentré de moitié sous pression réduite : un précipité se forme. Le solide est filtré et le filtrat est concentré à sec sous pression réduite, repris par 100 cm³ d'eau, puis par une fois 100 cm³ et deux fois 50 cm³ d'acétate d'éthyle. Les phases organiques réunies sont séchées sur sulfate de magnésium, filtrées et concentrées à sec sous pression réduite. Après flash chromatographie sur colonne de silice [éluant : heptane/acétate d'éthyle (gradient de 80/20 à 40/60 en volumes)], on obtient 835 mg de 2-méthyl-1H-pyrrolo[2,3-b]pyridine sous forme d'un solide beige dont les caractéristiques sont les suivantes :
   Rf CCM silice = 0,49 [éluant : heptane/acétate d'éthyle (60/40 en volumes)]
   Spectre de masse : LC-MS-DAD-ELSD : 133(+) = (M+H)(+)
   SM-EI : 132 (+) - M(+)
d) 2-méthyl-1-[(4-méthylphényl)sulfonyl]-1H-pyrrolo[2,3-b]pyridine.
   La 2-méthyl-1-[(4-méthylphényl)sulfonyl]-1H-pyrrolo[2,3-b]pyridine peut être préparée de la manière suivante :
   Une solution de 2 g de 1-[(4-méthylphényl)sulfonyl]-1H-pyrrolo[2,3-b]pyridine dans 20 cm³ de tétrahydrofuranne est refroidi à une température voisine de -70°C à l'aide d'un bain carboglace/acétone. A cette température et sous argon, sont introduits goutte à goutte 9,2 cm³ d'une solution dans l'hexanne de n-butyllithium 1,6M. Le milieu (ou mélange) réactionnel est laissé sous agitation à cette température durant trente minutes puis l'on ajoute 1,37 cm³ d'iodure de méthyle. On laisse le mélange sous argon, sous agitation et à une température voisine de - 70°C durant trois heures. Après réchauffement du milieu à une température voisine de 0°C, on additionne 20 cm³ d'eau, puis on laisse remonter à une température voisine de 20°C et on extrait par trois fois 50 cm³ d'acétate d'éthyle. Les phases organiques rassemblées sont lavées par une solution aqueuse saturée en chlorure de sodium, séchées sur sulfate de magnésium, filtrées et concentrées à sec sous pression réduite. On obtient 2,2 g de 2-méthyl-1-[(4-méthylphényl)sulfonyl]-1H-pyrrolo[2,3-b]pyridine sous forme d'une poudre beige dont les caractéristiques sont les suivantes :
      Spectre de RMN ¹H à 40MHz 2,30 (s : 3H) ; 2,32 (s : 3H) ; 2,69 (s large : 3H) ; 6,59 (s large : 1H) ; 7,15 (s large : 1H) ; 7,17 (dd, J = 5 et 8 Hz : 1H) ; 7,26 (d large, J = 8 Hz : 1H) ; 7,90 (dd, J = 2 et 8 Hz : 1H) ; 7,97 (d, J = 8 Hz : 1H) ; 8,08 (dd, J = 2 et 5 Hz : 1H)
      Spectre de masse : LC-MS-DAD-ELSD : 301(+) - (M+H)(+) ; 299(-) = (M-H) (-)
e) 1-[(4-méthylphényl)sulfonyl]-1H-pyrrolo[2,3-b]pyridine
   La 1-[(4-méthylphényl)sulfonyl]-1H-pyrrolo[2,3-b]pyridine peut être préparée de la manière suivante :
   A une solution de 4,72 g de 1H-pyrrolo[2,3-b]pyridine dans 110 cm³ de toluène on introduit càd ajoute 100 mg d'hydrogène sulfate de N,N,N-tributylbutan-1-amonium et 8,75 g de chlorure de 4-méthylbenzènesulfonyle. Au mélange refroidi à 0°C, on additionne une solution de 20,8 g d'hydroxyde de sodium dans 110 cm³ d'eau. Le mélange réactionnel est laissé sous agitation, sous argon et à une température voisine de 20°C durant quatre heures puis 100 cm³ d'eau sont ajoutés. Le mélange résultant est extrait par 200 cm³ puis 100 cm³ d'acétate d'éthyle. Les phases organiques rassemblées sont lavées par une solution aqueuse saturée en chlorure de sodium, séchées sur sulfate de magnésium, filtrées et concentrées à sec sous pression réduite. On obtient 10,9g de 1-[(4-méthylphényl)sulfonyl]-1H-pyrrolo[2,3-b]pyridine sous forme d'une poudre blanche dont les caractéristiques sont les suivantes :
      Rf CCM silice = 0,34 [éluant : heptane/acétate d'éthyle (70/30 en volumes)]
      Spectre de masse : LC-MS-DAD-ELSD : 273 (+) = (M+H)(+) 295 (+) = (M+Na)(+)

### Exemple 6 (5Z)-3-méthyl-5-(1H-pyrrolo[2,3-b]pyridin-3-ylméthylène)-2-thioxoimidazolidin-4-one

La (5Z)-3-méthyl-5-(1H-pyrrolo[2,3-b]pyridin-3-ylméthylène)-2-thioxoimidazolidin-4-one peut être préparée comme dans l'exemple 1 mais à partir de 225 mg de 1H-pyrrolo[2,3-b]pyridine-3-carbaldéhyde dans 10 cm³ d'éthanol, de 200 mg de 3-méthyl-2-thioxoimidazolidin-4-one et de 0,061 cm³ de pipéridine. Après cinq heures de reflux, on obtient 271 mg d'un mélange contenant 85% de l'isomère (5Z)-3-méthyl-5-(1H-pyrrolo[2,3-b]pyridin-3-ylméthylène)-2-thioxoimidazolidin-4-one et 15% de l'isomère (5E) sous forme d'une poudre jaune-orangée. Les caractéristiques de l'isomère 5Z sont les suivantes :
Spectre de RMN ¹H à 400MHz : 3,21 (s : 3H) ; 6,96 (s : 1H) ; 7,20 (dd, J = 5 et 8 Hz : 1H) ; de 8,27 à 8,37 (m : 2H) ; 8,59 (d, J = 3 Hz : 1H) ; 12,05 (s large : 1H) ; 12,5 (s large : 1H)
Spectre de masse : LC-MS-DAD-ELSD : 257(-) - (M-H)(-) ; 259 (+) = (M+H)(+)

### Exemple 7

### (5Z)-2-[(cyclopropylméthyl)amino]-5-(1H-pyrrolo[2,3-b]pyridin-3-ylméthylène)-3,5-dihydro-4H-imidazol-4-one

La (5Z)-2-[(cyclopropylméthyl)amino]-5-(1H-pyrrolo[2,3-b]pyridin-3-ylméthylène)-3,5-dihydro-4H-imidazol-4-one peut être préparée de la manière suivante :

Dans un réacteur pour four micro-ondes, sont introduits 100 mg de (5Z)-5-(1H-pyrrolo[2,3-b]pyridin-3-ylméthylène)-2-thioxoimidazolidin-4-one, 1 cm³ d'éthanol et 290 mg de 1-cyclopropylméthanamine. Le réacteur est fermé hermétiquement et le milieu est mis sous irradiation micro-ondes pendant vingt minutes à une température de 140°C, puis trente minutes à une température de 150°C. Après retour à une température voisine de 20°C, le précipité formé est filtré, lavé par de l'éthanol et séché sous pression réduite. On obtient 40 mg de (5Z)-2-[(cyclopropylméthyl)amino]-5-(1H-pyrrolo[2,3-b]pyridin-3-ylméthylène)-3,5-dihydro-4H-imidazol-4-one sous forme d'une poudre jaune dont les caractéristiques sont les suivantes :
Spectre de RMN ¹H à 400MHz : 0,30 (m : 2H) ; 0,48 (m : 2H) ; 1,13 (m : 1H); 3,23 (t large, J = 6,5 Hz : 2H) ; 6,61 (s large : 1H) ; 7,11 (d, J = 5 Hz : 1H) ; 7,24 (m : 1H) ; de 8,15 à 8,30 (m : 2H) ; 8,55 (d large, J = 7 Hz : 1H) ; de 9,70 à 10,7 (m étalé : 1H) ; 11,6 (m étalé : 1H)
Spectre de masse : SM-EI : 281(+) = M(+)

### Exemple 8

### (5Z)-5-[(4-chloro-1H-pyrrolo[2,3-b]pyridin-3-yl)méthylène]-2-[(cyclopropylméthyl)amino]-3,5-dihydro-4H-imidazol-4-one

La (5Z)-5-[(4-chloro-1H-pyrrolo[2,3-b]pyridin-3-yl)méthylène]-2-[(cyclopropylméthyl)amino]-3,5-dihydro-4H-imidazol-4-one peut être préparée comme dans l'exemple 7 mais à partir de 190 mg de (5Z)-5-[(9-chloro-1H-pyrrolo[2,3-b]pyridin-3-yl)méthylène]-2-thioxoimidazolidin-4-one, de 4 cm³ d'éthanol et de 485 mg de 1-cyclopropylméthanamine. Après une heure à une température de 145°C sous irradiation micro-ondes et trente minutes à une température de 150°C, on obtient 100 mg de (5Z)-5-[(4-chloro-1H-pyrrolo[2,3-b]pyridin-3-yl)méthylène]-2-[(cyclopropylméthyl)amino]-3,5-dihydro-4H-imidazol-4-one sous forme d'une poudre orangée dont les caractéristiques sont les suivantes :
Spectre de RMN ¹H à 400MHz : 0,28 (m : 2H) ; 0,48 (m : 2H) ; 1,12 (m : 1H) ; 3,22 (m partiellement masqué : 2H) ; 7,10 (s large : 1H) ; 7,22 (d, J = 5 Hz : 1H) ; 7,37 (m : 1H) ; 8,18 (d, J = 5 Hz : 1H) ; 8,58 (s large : 1H) ; de 10,25 à 10,8 (m étalé : 1H) ; de 12,0 à 12,6 (m étalé : 1H)
Spectre de masse : LC-MS-DAD-ELSD : 314(-)= (M-H)(-) ; 316(+)= (M+H)(+) (1 atome de chlore Cl présent)

### Exemple 9

### (5Z)-5-[(5-chloro-1H-pyrrolo[2,3-b]pyridin-3-yl)méthylène]-2-[(cyclopropylméthyl)amino]-3,5-dihydro-4H-imidazol-4-one

La (5Z)-5-[(5-chloro-1H-pyrrolo[2,3-b]pyridin-3-yl)méthylène]-2-[(cyclopropylméthyl)amino]-3,5-dihydro-4H-imidazol-4-one peut être préparée comme dans l'exemple 7 mais à partir de 200 mg de (5Z)-5-[(5-chloro-1H-pyrrolo[2,3-b]pyridin-3-yl)méthylène]-2-thioxoimidazolidin-4-one, de 4 cm³ d'éthanol et de 511 mg de 1-cyclopropylméthanamine, après trente minutes à une température de 145°C sous irradiation micro-ondes. Le milieu (ou mélange) est ensuite concentré puis purifié par flash chromatographie sur colonne de silice [éluant : dichlorométhane /méthanol (gradient de 95/5 à 70/30 en volumes)]. On obtient 20 mg de (5Z)-5-[(5-chloro-1H-pyrrolo[2,3-b]pyridin-3-yl)méthylène]-2-[(cyclopropylméthyl)amino]-3,5-dihydro-4H-imidazol-4-one sous forme d'une poudre jaune pâle dont les caractéristiques sont les suivantes :
Spectre de RMN ¹H à 400MHz : 0,30 (m : 2H) ; 0,48 (m : 2H) ; 1,16 (m : 1H) ; 3,24 (m partiellement masqué : 2H) ; 6,58 (s : 1H) ; 7,25 (m : 1H) ; 8,16 (s large : 1H) ; 8,22 (s large : 1H) ; 9,15 (s large : 1H) ; de 10,25 à 10,7 (m étalé : 1H) ; de 11,9 à 12,4 (m étalé : 1H)
Spectre de masse : LC-MS-DAD-ELSD : 314(-) = (M-H)(-): 316(+)= (M+H)(+) (1 atome de chlore Cl présent)

### Exemple 10

### (5Z)-5-[(6-chloro-1H-pyrrolo[2,3-b]pyridin-3-yl)méthylène]-2-[(cyclopropylméthyl)amino]-3,5-dihydro-4H-imidazol-4-one

La (5Z)-5-[(6-chloro-1H-pyrrolo[2,3-b]pyridin-3-yl)méthylène]-2-[(cyclopropylméthyl)amino]-3,5-dihydro-4H-imidazol-4-one peut être préparée comme dans l'exemple 7, mais à partir de 150 mg de (5Z)-5-[(6-chloro-1H-pyrrolo[2,3-b]pyridin-3-yl)méthylène]-2-thioxoimidazolidin-4-one, de 3,75 cm³ d'éthanol et de 383 mg de 1-cyclopropylméthanamine. Après quinze minutes à une température de 140°C, puis trente minutes à une température de 150°C sous irradiation micro-ondes, on obtient 55 mg de (5Z)-5-[(6-chloro-1H-pyrrolo[2,3-b]pyridin-3-yl)méthylène]-2-[(cyclopropylméthyl)amino]-3,5-dihydro-4H-imidazol-4-one sous forme d'une poudre jaune pâle dont les caractéristiques sont les suivantes : Spectre de RMN ¹H à 40MHz 0,30 (m : 2H) ; 0,48 (m : 2H) ; 1,12 (m : 1H) ; 3,24 (t, J = 6, 5 Hz : 2H) ; 6,57 (s : 1H) ; 7,16 (d, J = 8 Hz : 1H) ; de 7,20 à 7,35 (m étalé : 1H) ; 8,18 (m : 1H) ; 8,73 (m : 1H) ; de 9,80 à 10,70 (m très étalé : 1H) ; de 11,5 à 12,3 (m très étalé : 1H) Spectre de masse : LC-MS-DAD-ELSD : 316(+)= (M+H)(+) (1 atome de chlore Cl présent)

### Exemple 11

### (5Z)-2-[(cyclopropylméthyl)amino]-5-[(2-méthyl-1H-pyrrolo[2,3-b]pyridin-3-yl)méthylène]-3,5-dihydro-4H-imidazol-4-one

La (5Z)-2-[(cyclopropylméthyl)amino]-5-[(2-méthyl-1H-pyrrolo[2,3-b]pyridin-3-yl)méthylène]-3,5-dihydro-4H-imidazol-4-one peut être préparée comme dans l'exemple 7, mais à partir de 200 mg d'un mélange contenant 65% de (5Z)-5-[(2-méthyl-1H-pyrrolo[2,3-b]pyridin-3-yl)méthylène]-2-thioxoimidazolidin-4-one, de 2 cm³ d'éthanol et de 551 mg de 1-cyclopropylméthanamine, après quinze minutes à une température de 140°C et quinze minutes à 145°C sous irradiation micro-ondes. Le mélange obtenu est ensuite purifié par flash chromatographie sur colonne de silice [éluant : dichlorométhane /méthanol (gradient de 98/2 à 90/10 en volumes)]. On obtient 35 mg de (5Z)-2-[(cyclopropylméthyl)amino]-5-[(2-méthyl-1H-pyrrolo[2,3-b]pyridin-3-yl)méthylène]-3,5-dihydro-4H-imidazol-4-one sous forme d'une poudre jaune pâle dont les caractéristiques sont les suivantes :
Spectre de RMN ¹H à 400MHz : 0,29 (m : 2H) ; 0,47 (m : 2H) ; 1,13 (m : 1H) ; 2,50 (s partiellement masqué : 3H) ; 3,23 (t, J = 6,5 Hz : 2H) ; 6,48 (s : 1H) ; 7,00 (m : 1H) ; 7,08 (m : 1H) ; 8,10 (d large, J = 4 Hz : 1H) ; 9,42 (d, J = 8 Hz : 1H) ; de 10,3 à 10,6 (m étalé : 1H) ; 11,8 (s large : 1H)
Spectre de masse : LC-MS-DAD-ELSD : 294(-) = (M-H)(-) ; 296 (+) - (M+H) (+)

### Exemple 12

### (5Z)-2-[(cyclopropylméthyl)amino]-3-méthyl-5-(1H-pyrrolo[2,3-b]pyridin-3-ylméthylène)-3,5-dihydro-4H-imidazol-4-one

La (5Z)-2-[(cyclopropylméthyl)amino]-3-méthyl-5-(1H-pyrrolo[2,3-b]pyridin-3-ylméthylène)-3,5-dihydro-4H-imidazol-4-one peut être préparée comme dans l'exemple 7, mais à partir de 100 mg de (5Z)-3-méthyl-5-(1H-pyrrolo[2,3-b]pyridin-3-ylméthylène)-2-thioxoimidazolidin-4-one, de 4 cm³ d'éthanol et de 275 mg de 1-cyclopropylméthanamine. Après quinze minutes à une température de 140°C, quarante minutes à 150°C et quarante cinq minutes à 160°C sous irradiation micro-ondes, on obtient 39 mg de (5Z)-2-[(cyclopropylméthyl)amino]-3-méthyl-5-(1H-pyrrolo[2,3-b]pyridin-3-ylméthylène)-3,5-dihydro-4H-imidazol-4-one sous forme d'une poudre jaune dont les caractéristiques sont les suivantes :
Spectre de RMN ¹H à 40MHz 0,35 (m : 2H) ; 0,51 (m : 2H) ; 1,22 (m : 1H) ; 3,07 (s : 3H) ; 3,33 (t, J = 6,5 Hz : 2H) ; 6,74 (s : 1H) ; 7,11 (dd, J = 5 et 8 Hz : 1H) ; 7,55 (t, J = 6,5 Hz : 1H) ; de 8,20 à 8,27 (m : 2H) ; 8,61 (d, J = 8 Hz : 1H) ; 11,9 (s large : 1H)
Spectre de masse : LC-MS-DAD-ELSD : 294(-) - (M-H)(-); 296 (+) = (M+H) (+)

### Exemple 13 (5Z)-2-butyl-5-[(1H-pyrrolo[2,3-b]pyridin-3-yl)méthylidène]-3,5-dihydro-4H-imidazol-4-one

### Préparation A de l'exemple 13

### (5Z)-2-butyl-5-(1H-pyrrolo[2,3-b]pyridin-3-ylméthylène)-3,5-dihydro-4H-imidazol-4-one

La (5Z)-2-butyl-5-(1H-pyrrolo[2,3-b]pyridin-3-ylméthylène)-3,5-dihydro-4H-imidazol-4-one peut être préparée comme dans l'exemple 1, mais à partir de 198 mg de 1H-pyrrolo[2,3-b]pyridine-3-carbaldéhyde dans 15 cm3 d'éthanol, sont ajoutés 0,15 cm³ de pipéridine et 324mg de 2-butyl-3,5-dihydro-4H-imidazol-4-one (préparée par exemple selon J. Org. Chem 1999, 64, 8084-8089). Le mélange réactionnel est porté au reflux pendant trois heures et le mélange concentré sous vide. Addition d'un peu d'éthanol fait apparaître un solide jaune qui est filtré, lavé avec 3 cm3 de dichlorométhane, et séché sous vide pour obtenir 65 mg. Ce solide est combiné avec les 66 mg de solide obtenu par une réaction identique sur 245 mg de 1H-pyrrolo[2,3-b]pyridine-3-carbaldéhyde. Les solides sont repris avec 20 cm³ d'eau et extrait avec deux fois 30 cm³ d'acétate d'éthyle. Les phases organiques sont séchées sur sulfate de magnésium, filtrées et concentrées sous pression réduite pour obtenir 88 mg de (5Z)-2-butyl-5-(1H-pyrrolo[2,3-b]pyridin-3-ylméthylène)-3,5-dihydro-4H-imidazol-4-one sous forme d'une poudre jaune dont les caractéristiques sont les suivantes :
Spectre RMN 1H à 400 MHz : 0,95 (t, J = 7,5 Hz, 3H) ; 1,40 (m, 2H) ; 1,71 (m, 2H) ; 2,54 (t, J = 7,5 Hz, 2H) ; 7,16 (s, 1H) ; 7,18 (dd, J = 5,0 et 8,0 Hz, 1H) ; 8,28 (dd, J = 1,5 et 5,0 Hz, 1H) ; 8,34 (s, 1H) ; 8,92 (d large, J = 8,0 Hz, 1H) ; 11,1 (m étalé, 1H) ; 12,4 (m étalé, 1H)

### Préparation B de l'exemple 13

### (5Z)-2-butyl-5-[(1H-pyrrolo[2,3-b]pyridin-3-yl)méthylidène]-3,5-dihydro-4H-imidazol-4-one

a) La (5Z)-2-butyl-5-[(1H-pyrrolo[2,3-b]pyridin-3-yl)méthylidène]-3,5-dihydro-4H-imidazol-4-one peut être préparée de la manière suivante :
   Un mélange de 0,32 g de 2-butyl-3,5-dihydro-4H-imidazol-4-one brute, de 0,2 g de 1H-pyrrolo[2,3-b]pyridine-3-carbaldéhyde, et de 0,14 cm³ de piperidine et 15 cm³ d'éthanol est porté au reflux pendant quatre heures. Le solide jaune est filtré, lavé avec un peu de chlorure de méthylène puis séché sous vide pour obtenir 65 mg de (5Z)-2-butyl-5-[(1H-pyrrolo[2,3-b]pyridin-3-yl)méthylidène]-3,5-dihydro-4H-imidazol-4-one sous forme d'une poudre jaune dont les caractéristiques sont les suivantes :
      Spectre de RMN ¹H à 400MHz : 0,95 (t, J = 7,5 Hz, 3H) ; 1,40 (m, 2H) ; 1, 71 (m, 2H) ; 2,54 (t, J = 7, 5 Hz, 2H) ; 7,17 (s, 1H) ; 7,20 (dd, J = 5,0 et 8,0 Hz, 1H) ; 8,29 (dd, J = 1,5 et 5,0 Hz, 1H) ; 8,34 (s, 1H) ; 8,92 (d large, J = 8,0 Hz, 1H) ; 11,1 (m étalé, 1H) ; 12,4 (m étalé, 1H).
         HPLC-MS-DAD-ELSD : 269(+)=(M+H)(+); 267(-)=(M-H)(-).
b) La 2-butyl-3,5-dihydro-4H-imidazol-4-one peut être préparée de la manière suivante :
   Une solution de 63 mg de soude dans 2 cm³ d'éthanol est ajoutée lentement à 0,2 g de chlorhydrate de méthyl glycinate à -10°C. Après 5 min, une solution de 0,18 g de pentanimidoate de méthyle dans 5 cm³ de toluène est ajoutée et le mélange est agité à l'ambiante pendant 20 min. Le pH est alors ajusté à pH 7 avec une solution d'HCl 1M. Le mélange obtenu est concentré sous vide à l'ambiante. Le résidu est repris dans du dichlorométhane et le solide insoluble est filtré. L'évaporation du filtrat donne 0,32 g de 2-butyl-3,5-dihydro-4H-imidazol-4-one.
c) Le pentanimidoate de méthyle peut être préparé de la manière suivante :
   Une solution de potasse 6M (10,6 cm³) est ajoutée lentement à une solution de 0,3 g de chlorhydrate de pentanimidoate de méthyle dans 5 cm³ d'éther à -10°C. Le mélange est agité 10 min puis extrait à l'éther (2x20 cm³) . Les phases organiques sont lavées à l'eau (20 cm³), séchées sur sulfate de magnésium et concentrées sous vide pour obtenir 179 mg de pentanimidoate de méthyle.
d) Le chlorhydrate de pentanimidoate de méthyle peut être préparé de la manière suivante :
   De l'acide chlorhydrique gazeux est bullé pendant 30 min dans une solution de 5 cm³ de butane carbonitrile dans un mélange de méthanol (2,1 cm³) et d'éther (5 cm³), refroidie à -10°C. On laisse remonter la température à 0°C et l'on met le mélange réactionnel au réfrigérateur pour la nuit. La solution est concentrée pour obtenir 6,9 g de chlorhydrate de pentanimidoate de méthyle sous forme d'un solide blanc.

Analyse UPLC-MS-DAD-ELSD (MS= Quattro Premier XE Waters; électrospray +/- ; domaine de masse m/z=100-1100; UPLC Waters ; colonne Acquity UPLC BeH C18 1,7 µm

3mm.50mm; four UPLC=70°C, débit=0,7 ml/minute.

Eluants: A= Eau +0,1% Acide Formique B=Acétonitrile+0 ,1 % Acide Formique avec le gradient :

| Time | A% | B% |
|---|---|---|
| 0 | 95 | 5 |
| 5 | 0 | 100 |
| 5.5 | 95 | 5 |
| 6.0 | 95 | 5 |

DAD longueur d'onde considérée λ=210-400 nm

ELSD : Sedere SEDEX 85 température de nébulisation=35°C; pression de nébulisation=3,7bars

### Exemple 14

### (5Z)-5-[(5-bromo-1H-pyrrolo[2,3-b]pyridin-3-yl)méthylène]-2-thioxoimidazolidin-4-one

a) La (5Z)-5-[(5-bromo-1H-pyrrolo[2,3-b]pyridin-3-yl)méthylène]-2-thioxoimidazolidin-4-one peut être préparée comme dans l'exemple 1 mais à partir de 655 mg de 5-bromo-1H-pyrrolo[2,3-b]pyridine-3-carbaldéhyde dans 6 cm³ d'éthanol, de 340 mg de 2-thioxoimidazolidin-4-one et de 0,28 cm³ de pipéridine. Après deux heures de reflux, on obtient 700 mg de (5Z)-5-[(5-bromo-1H-pyrrolo[2,3-b]pyridin-3-yl)méthylène]-2-thioxoimidazolidin-4-one sous forme d'une poudre orangée dont les caractéristiques sont les suivantes :
   Spectre RMN de ¹H à 400MHz : 6,82 (s, 1H) ; 8,37 (d, J = 2,0 Hz, 1H) ; 8,57 (s, 1H) ; 8,64 (d, J = 2,0 Hz, 1H) ; de 11,6 à 12,3 (m très étalé, 2H) ; 12,6 (m étalé, 1H).
   Spectre de masse : HPLC-MS-DAD-ELSD : 323(+)=(M+H)(+) ; 321(-)=(M-H)(-) (1 atome de brome Br présent).
b) Le 5-bromo-1H-pyrrolo[2,3-b]pyridine-3-carbaldéhyde peut être préparé de la manière suivante :
   A une suspension de 3,35 g de chlorure d'aluminium dans 50 cm³ de dichlorométhane, on introduit à 0°C, 0,93 g de 5-bromo-1H-pyrrolo[2,3-b]pyridine. Le mélange réactionnel est laissé sous agitation pendant 15 minutes, puis on introduit goutte à goutte 1,4 cm³ de dichloro(méthoxy)méthane. Le mélange réactionnel est laissé sous agitation pendant trois heures à 0°C, puis dilué à 0°C par addition de 5 cm³ de méthanol. Le mélange obtenu est versé dans un mélange de 100 cm³ d'eau-glace, puis amené à pH basique par addition de soude 5M. Les phases sont séparées, et la phase dichlorométhane est lavée à l'eau, puis séchée sur sulfate de sodium. La phase aqueuse est réextraite avec deux fois 100 cm³ d'acétate d'éthyle,. Les deux extraits sont combinés puis lavés à l'eau, séchés sur sulfate de sodium et rassemblés avec la phase organique dichlorométhane ci-dessus. Après concentration sous vide on obtient 0,61 g de 5-bromo-1H-pyrrolo[2,3-b]pyridine-3-carbaldéhyde sous forme de poudre ocre dont les caractéristiques sont les suivantes:
      - Spectre RMN de ¹H à 400MHz : 8,44 (d, J = 2Hz, 1H) ; 8,51 (m, 2H) ; 9,92 (s, 1H).
   LC-MS (1) _{:} temps de rétention : 3.08 mn, LC-MS-DAD-ELSD : 223(-)=(M-H)(-) ; 225(+)=(M+H)(+) (1 Br présent).
c) La 5-bromo-1H-pyrrolo[2,3-b]pyridine peut être préparée comme décrit dans D.Mazéas et al : Heterocycles. 1999, 50(2); 1065-1080.

### Exemple 15

### (5Z)-5-[(5-méthoxy-1H-pyrrolo[2,3-b]pyridin-3-yl)méthylène]-2-thioxoimidazolidin-4-one

a) La (5Z)-5-[(5-méthoxy-1H-pyrrolo[2,3-b]pyridin-3-yl)méthylène]-2-thioxoimidazolidin-4-one peut être préparée comme dans l'exemple 1 mais à partir de 160 mg de 5-méthoxy-1H-pyrrolo[2,3-b]pyridine-3-carbaldéhyde dans 4 cm³ d'éthanol, de 110 mg de 2-thioxoimidazolidin-4-one et de 0,09 cm³ de pipéridine. Après deux heures et demi de reflux, on obtient 220 mg de (5Z)-5-[(5-méthoxy-1H-pyrrolo[2,3-b]pyridin-3-yl)méthylène]-2-thioxoimidazolidin-4-one sous forme d'une poudre orangée dont les caractéristiques sont les suivantes :
   Spectre RMN de ¹H à 400MHz : 3,87 (s, 3H) ; 6,86 (s, 1H) ; 7,95 (d, J = 2,5 Hz, 1H) ; 8,03 (d, J = 2,5 Hz, 1H) ; 8,50 (s, 1H) ; 11,84 (s large, 1H) ; 12,30 (s large, 1H).
   LC-MS (1) : temps de rétention 2.60 mn, LC-MS-DAD-ELSD : 273(-)=(M-H)(-) ; 275 (+) = (M+H)(+).
b) Le 5-méthoxy-1H-pyrrolo[2,3-b]pyridine-3-carbaldéhyde peut être préparé comme décrit dans D.Mazéas et al : Heterocycles. 1999, 50(2) ; 1065-1080.

### Exemple 16

### (5Z)-5-[(5-phényl-1H-pyrrolo[2,3-b]pyridin-3-yl)méthylène]-2-thioxoimidazolidin-4-one

a) La (5Z)-5-[(5-phényl-1H-pyrrolo[2,3-b]pyridin-3-yl)méthylidène]-2-thioxoimidazolidin-4-one peut être préparée comme dans l'exemple 1 mais à partir de 135 mg de 5-phényl-1H-pyrrolo[2,3-b]pyridine-3-carbaldéhyde dans 3 cm³ d'éthanol, de 70 mg de 2-thioxoimidazolidin-4-one et de 0,06 cm³ de pipéridine. Après deux heures et demi de reflux, on obtient 180 mg de (5Z)-5-[(5-phényl-1H-pyrrolo[2,3-b]pyridin-3-yl)méthylène]-2-thioxoimidazolidin-4-one sous forme d'une poudre orangée dont les caractéristiques physiques sont les suivantes :
   Point de fusion: 265-270°C
   Spectre RMN ¹H à 400MHz : 6,84 (s, 1H) ; 7,38 (t, J = 7,5 Hz, 1H); 7,49 (t, J = 7,5 Hz, 2H) ; 7,82 (d, J = 7,5 Hz, 2H) ; 8,52 (s, 1H) ; 8,61 (d, J = 2,0 Hz, 1H) ; 8,68 (d, J = 2,0 Hz, 1H) ; de 10,0 à 11,5 (m très étalé, 3H).
   LC-MS (1) : temps de rétention 3.49 mn ; LC-MS-DAD-ELSD : 319(-)=(M-H)(-); 321 (+) = (M+H) (+).
b) Le 5-phényl-1H-pyrrolo[2,3-b]pyridine-3-carbaldéhyde peut être préparé de la manière suivante :
   Dans un tubes micro-ondes sont introduits 500 mg de 5-bromo-1H-pyrrolo[2,3-b]pyridine-3-carbaldéhyde, 13 cm³ de dioxane, 3,7 cm³ d'eau, 2,9 g de carbonate de césium, 340 mg d'acide phénylboronique, 160 mg de 1,1'-Bis(diphénylphosphino)ferrocène-dichloropalladium(II). Le mélange réactionnel est irradié sous micro-ondes pendant trente minutes à 140°C. Après refroidissement, le mélange réactionnel est versé dans 50 cm³ d'eau. Le mélange obtenu est extrait par deux fois 50 cm³ d'acétate d'éthyle. Les phases organiques rassemblées sont -séchées sur sulfate de sodium, puis amenées à sec sous vide. Le résidu obtenu est purifié par flash chromatographie sur colonne de silice avec un éluant dichlorométhane/acétate d'éthyle (gradient dichlorométhane/acétate d'éthyle de 90/10 à 50/50 en volumes) pour donner 377 mg de 5-phényl-1H-pyrrolo[2,3-b]pyridine-3-carbaldéhyde dont les caractéristiques physiques sont les suivantes:
      Spectre RMN de ¹H à 400MHz : 7,41 (tt, J=1 et 7,4 Hz, 1H) ; 7,52 (t, J=7,4 Hz, 2H) ; 7,73(d, J=7,4 Hz, 2H) ; 8,51(s, 1H) ; 8,59 (d, J=2,3 Hz, 1H) ; 8,66 (d, J=2,3 Hz) ; 9,97 (s, 1H) ; 12,77 (sl, 1H).
      LC-MS (1): temps de rétention 3,38 mn ; LC-MS-DAD-ELSD : 221 (-)=(M-H)(-) ; 225(+)=(M+H)(+).

### Exemple 17

### (5Z)-5-[(5-méthyl-1H-pyrrolo[2,3-b]pyridin-3-yl)méthylidène]-2-thioxoimidazolidin-4-one

a) La (5Z)-5-[(5-méthyl-1H-pyrrolo[2,3-b]pyridin-3-yl)méthylidène]-2-thioxoimidazolidin-4-one peut être préparée comme dans l'exemple 1 mais à partir de 500 mg de 5-méthyl-1H-pyrrolo[2,3-b]pyridine-3-carbaldéhyde brut titré à 65%, de 362 mg de 2-thioxoimidazolidin-4-one et 106 mg de pipéridine dans 15 cm³ d'éthanol. Le mélange est chauffé au reflux de l'éthanol pendant quatre heures sous argon, puis refroidi à 0°C à l'aide d'un bain de glace pendant dix minutes. La suspension est filtrée et le filtrat est ensuite concentré sous pression réduite pour obtenir 685 mg de (5Z)-5-[(5-méthyl-1H-pyrrolo[2,3-b]pyridin-3-yl)méthylidène]-2-thioxoimidazolidin-4-one sous forme d'une poudre jaune dont les caractéristiques sont les suivantes:
   LC-MS : temps de rétention : 2,83 mn, 259(+)= (M+H)(+).
   Spectre de RMN ¹H à 400 Hz 2.42 (s : 3H); 6.73 (sl : 1H); 8.10 (d, J = 2Hz : 1H); 8.15 (d, J = 2Hz: 1H); 8.49 (s : 1H); 11.5-12.1 (massife : 2H); 12.26 (se, 1H).
b) Le 5-méthyl-1H-pyrrolo[2,3-b]pyridine-3-carbaldéhyde peut être préparé de la façon suivante :
   A une suspension de 1 g de 5-méthyl-1H-pyrrolo[2,3-b]pyridine dans un mélange de 10 cm³ d'eau et 5 cm³ d'acide acétique est ajouté 1,6 g de 1,3,5,7-tétraazatricyclo[3.3.1.1~3,7~]décane. Le mélange est porté à reflux sous argon pendant quatre heures et trente minutes, puis 30 cm³ d'eau sont ajoutés dans le mélange à chaud et on laisse revenir à température ambiante sous agitation pendant la nuit. Le mélange est alors extrait par trois fois 50 cm³ d'acétate d'éthyle. Les phases organiques sont alors rassemblées et lavées avec 3 fois 50 cm³ d'eau, séchées sur sulfate de magnésium et concentrées à sec sous pression réduite pour obtenir 610 mg d'un mélange contenant 65% (par RMN) de 5-méthyl-1H-pyrrolo[2,3-b]pyridine-3-carbaldéhyde sous forme d'une poudre jaune dont les caractéristiques du produit majoritaire sont les suivantes :
      LC-MS : temps de rétention 3,79 mn, 161(+)= (M+H)(+).
      Spectre de RMN ¹H à 400 Hz : 2.41 (s : 3H); 8.21 (d, J = 2Hz : 1H); 8.22 (d, J = 2Hz: 1H); 8.40 (s: 1H); 9.90 (s: 1H); 12.5-12.7 (se: 1H).
c) La 5-méthyl-1H-pyrrolo[2,3-b]pyridine peut être préparée de la façon suivante :
   A une suspension de 7,5 g de 5-méthyl-3-[(triméthylsilyl)éthynyl]pyridin-2-amine dans 170 cm³ de NMP sont ajoutés 7 g de tert-butanolate de potassium. Le mélange réactionnel est porté au reflux pendant six heures trente minutes sous argon puis autorisé à revenir à température ambiante pendant la nuit. Le mélange réactionnel est alors versé lentement dans 740 cm³ d'une solution aqueuse saturée en chlorure d'ammonium puis les insolubles sont filtrés sur célite. Le filtrat est alors extrait par trois fois 500 cm³ d'acétate d'éthyle puis les phases organiques combinées sont lavées par de l'eau, séchées sur sulfate de magnésium et concentrées à sec sous pression réduite. Le résidu est alors repris dans de l'acétate d'éthyle. La solution obtenue est lavée par de l'eau, puis séchée sur sulfate de magnésium et concentrée à sec sous pression réduite pour obtenir 2,6 g de 5-méthyl-1H-pyrrolo[2,3-b]pyridine sous forme d'un solide marron dont les caractéristiques sont les suivantes :
      LC-MS : temps de rétention 0,85 mn, 133(+)= (M+H)(+).
      Spectre de RMN ¹H à 400 Hz : 2.38 (s : 3H); 6.35(dd, J = 1.5, 3Hz : 1H); 7.38 (dd, J = 2.5, 3Hz : 1H), 7.73 (dl, J = 1Hz : 1H); 8.2 (d, J = 2Hz : 1H); 11.42 (se, 1H).
d) La 5-méthyl-3-[(triméthylsilyl)éthynyl]pyridin-2-amine peut être préparée de la façon suivante :
   Une suspension de 1 g de 2-amino-3-bromo-5-méthylpyridine avec 204 mg de CuI et 250 mg de chlorure de lithium dans 30 cm³ de diméthylformamide est placée sous argon et sous agitation. Sont alors successivement introduits 3,7 cm³ de triéthylamine, 195 mg de [1,1'-bis(diphénylphosphino)ferrocene]dichloropalladium(II) et 800 mg de triméthylsilylacétylène. Le mélange est chauffé à 48°C pendant vingt-deux heures. Le mélange réactionnel est alors concentré à sec sous pression réduite, puis le résidu est repris dans un mélange de 30 cm³ d'acétate d'éthyle et de 30 cm³ d'eau. Un insoluble brun est filtré sur célite. Les phases sont séparées et la phase organique est lavée avec deux fois 30 cm³ d'eau. La phase aqueuse est extraite avec 30 cm³ d'acétate d'éthyle puis toutes les phases organiques sont rassemblées, séchées sur sulfate de magnésium et concentrées à sec sous pression réduite. Le résidu est purifié par flash chromatographie sur colonne de silice [éluant : n-hexane/acétate d'éthyle (gradient de 5/95 à 40/60 en volumes)], pour donner 353 mg de 5-méthyl-3-[(triméthylsilyl)éthynyl]pyridin-2-amine sous forme d'un solide beige dont les caractéristiques sont les suivantes :
      LC-MS : temps de rétention 3,28 mn, 205(+)= (M+H)(+).
      Spectre de RMN ¹H à 400 Hz : 0.20 (m : 9H); 2.10 (s : 3H); 5.90 (se: 2H); 7.40 (s : 1H); 7.85 (se : 1H).

### Exemple 18

### (5Z)-2-thioxo-5-{[5-(trifluorométhyl)-1H-pyrrolo[2,3-b]pyridin-3-yl]méthylidène}imidazolidin-4-one

a) La (5Z)-2-thioxo-5-{[5-(trifluorométhyl)-1H-pyrrolo[2,3-b]pyridin-3-yl]méthylidène}imidazolidin-4-one peut être préparée comme dans l'exemple 1, mais à partir de 174 mg de 5-trifluorométhyl-1H-pyrrolo[2,3-b]pyridine-3-carbaldéhyde dans 6 cm³ d'éthanol, de 94 mg de 2-thioxoimidazolidin-4-one et de 0,03 cm³ de pipéridine. Après deux heures et trente minutes de reflux, on obtient 148 mg d'un mélange contenant 80% de (5Z)-2-thioxo-5-{[5-(trifluorométhyl)-1H-pyrrolo[2,3-b]pyridin-3-yl]méthylidène}imidazolidin-4-one sous forme d'une poudre orangée dont les caractéristiques du composé majoritaire sont les suivantes:
   LC-MS : temps de rétention 3,28 mn ; 313(+)= (M+H)(+).
   Spectre de RMN ¹H à 400MHz : 6,98 (s, 1H) ; 8,52 (d, J = 2 Hz, 1H) ; 8,66 (d, J = 2Hz, 1H) ; 9,07 (s, 1H) ; 12,00 (s large, 1H).
b) Le 5-trifluorométhyl-1H-pyrrolo[2,3-b]pyridine-3-carbaldéhyde peut être préparé de la manière suivante:
   A un mélange de 820 mg de 5-trifluorométhyl-1H-pyrrolo[2,3-b]pyridine dans 6 cm³ d'eau et 3 cm³ d'acide acétique, est introduit 926 mg de 1,3,5,7-tétraazatricyclo[3.3.1.1~3,7~]décane. Le mélange réactionnel est porté au reflux pendant deux heures et trente minutes. 6 cm³ d'eau sont alors ajoutés au mélange à chaud. Après retour à une température voisine de 25°C, le mélange est extrait avec de l'acétate d'éthyle (40 cm³). La phase organique est lavée avec une solution saturée de chlorure de sodium, séchée sur sulfate de magnésium et concentrée sous vide. Le résidu est purifié par chromatographie sur silice (élution avec un gradient 20-100% acétate d'éthyl dans l'heptane) pour obtenir 123 mg de 5-trifluorométhyl-1H-pyrrolo[2,3-b]pyridine-3-carbaldéhyde sous forme d'une poudre beige dont les caractéristiques sont les suivantes:
      LC-MS : temps de rétention 3,14 mn ; 215(+)= (M+H)(+).
      Spectre de RMN ¹H à 400MHz : 8,67 (d, J = 2,4 Hz, 1H) ; 8,75 (d, J = 2,4 Hz, 1H) ; 8,69 (s, 1H) ; 10,00 (s, 1H) ; 13,17 (s large, 1H).
c) La 5-trifluorométhyl-1H-pyrrolo[2,3-b]pyridine peut être préparée de la manière suivante :
   Une solution de 3,4 g de 5-trifluorométhyl-3-[(triméthylsilyl)éthynyl]pyridin-2-amine et de 2,96 g de ter-butylate de potassium dans 55 cm³ de N-méthylpyrrolidinone est chauffée à 90°C pendant trois heures. Le mélange est refroidi à température ambiante puis versé lentement sur 250 cm³ d'eau saturée en chlorure d'ammonium. Le mélange est dilué avec de l'acétate d'éthyle, filtré et les phases sont séparées. La phase aqueuse est extraite avec de l'acétate d'éthyle (2x100 cm³). Les phases organiques sont rassemblées puis séchées sur sulfate de magnésium et concentrées sous vide. Le résidu est repris lentement à l'eau et la suspension résultante est filtée. Le solide est séché sous vide pour obtenir 1,58 g de 5-trifluorométhyl-1H-pyrrolo[2,3-b]pyridine sous forme d'une poudre marron dont les caractéristiques sont les suivantes:
      LC-MS : temps de rétention 3,55 mn ; 187(+)= (M+H)(+).
      Spectre de RMN ¹H à 400MHz : 6,63 (dd, J = 1,7 et 3,5 Hz, 1H) ; 7,69 (dd, J = 2,2 et 3,5 Hz, 1H) ; 8,38 (d, J = 1,7 Hz, 1H) ; 8,55 (d, J= 2,2 Hz, 1H) ; 12,16 (s large, 1H).
d) La 5-trifluorométhyl-3-[(triméthylsilyl)éthynyl]pyridin-2-amine peut être préparée de la manière suivante :
   Un mélange de 6,68 g de 2-amino-3-iodo-5-trifluorométhyl- pyridine, 11,7 g de chlorure de lithium, 0,88 g d'iodure de cuivre, 11,7 g de triéthylamine, 0,85 g de [1,1'-bis(diphénylphosphino)ferrocene]-dichloropalladium(II) et de 3,4 g de triméthylsilylacétylène dans 160 cm³ de diméthylformamide est chauffé à 48°C pendant vingt-trois heures. Le mélange est refroidi à l'ambiante puis concentré sous vide. Le résidu est repris par 200 cm³ d'eau et 200 cm³ d'acétate d'éthyle. Les insolubles sont filtrés et la phase organique est lavée à l'eau, séchée sur sulfate de magnésium et concentrée sous vide. Le résidu est purifié par chromatographie sur colonne de silice [éludant : acétate d'éthyle /heptane (gradient de 0/100 à 30/70 en volumes)], pour obtenir 3,42 g de 5-trifluorométhyl-3-[(triméthylsilyl)éthynyl]pyridin-2-amine sous forme de poudre beige dont les caractéristiques sont les suivantes :
      LC-MS: temps de rétention: 4,94 mn ; LC-MS-DAD-ELSD : 259(+)= (M+H)(+).
e) La 2-amino-3-iodo-5-trifluorométhyl-pyridine peut être préparée de la manière suivante:
   Un mélange de 4,0 g de 2-amino-5-trifluorométhyl-pyridine et de 6,2 g de N-iodo succinimide dans 110 cm³ d'acide acétique est chauffé à 70°C pendant trois heures, puis refroidit à l'ambiante et concentré sous vide. Le résidu est repris lentement avec 60 cm³ d'eau saturée en bicarbonate de sodium, puis 60 cm³ d'eau. Le solide qui précipite est filtré puis séché sous vide pour obtenir 6,68 g de 2-amino-3-iodo-5-trifluorométhyl-pyridine sous forme de solide beige dont les caractéristiques sont les suivantes :
      LC-MS : temps de rétention : 3,72 mn ; LC-MS-DAD-ELSD : 289(+)=(M+H) (+).

### Exemple 19

### (5Z)-5-[(5-fluoro-1H-pyrrolo[2,3-b]pyridin-3-yl)méthylidène]-2-thioxoimidazolidin-4-one

a) La (5Z)-5-[(5-fluoro-1H-pyrrolo[2,3-b]pyridin-3-yl)méthylidène]-2-thioxoimidazolidin-4-one peut être préparé comme dans l'exemple 1, mais à partir de 265 mg de 5-fluoro-1H-pyrrolo[2,3-b]pyridine-3-carbaldéhyde, 13 cm³ d'éthanol, de 190 mg de 2-thioxoimidazolidin-4-one et de 0,16 cm³ de pipéridine. Après deux heures et demi de reflux, on obtient 327 mg de (5Z)-5-[(5-fluoro-1H-pyrrolo[2,3-b]pyridin-3-yl)méthylène]-2-thioxoimidazolidin-4-one sous forme de poudre orangée dont les caractéristiques physiques sont les suivantes:
   Spectre RMN ¹H à 400MHz : 6,81 (s, 1H) ; 8,30 (m, 2H) ; 8,60 (s, 1H) ; 11,93 (s large, 1H) ; 12,57 (s large, 1H).
   LC-MS (1) : temps de rétention 2.91 mn ; LC-MS-DAD-ELSD : 261 (-)-(M-H)(-) ; 263(+)-(M+H)(+).
b) Le 5-fluoro-1H-pyrrolo[2,3- b]pyridine-3-carbaldéhyde peut être préparé comme dans l'exemple 2 mais à partir de 545 mg de 5-fluoro-1H-pyrrolo[2,3-b]pyridine, dans un mélange de 3,4 cm³ d'eau et de 1,6 cm³ d'acide acétique, et 545 mg de 1,3,5,7,-tétraazatricyclo[3.3.1.1~3,7~]-décane. On obtient 265 mg de 5-fluoro-1H-pyrrolo[2,3-b]pyridine-3-carbaldéhyde sous forme d'un solide beige dont les caractéristiques physiques sont les suivantes :
   Spectre RMN de ¹H à 400MHz : 8,15 (dd, J = 3 et 9 Hz, 1H) ; 8,36 (dd, 1,8 et 3 Hz, 1H) ; 8,53 (s, 1H) ; 9,90 (s, 1H) ; 12,79 (s large, 1H).
   LC-MS (1) : temps de rétention: 2.59 mn ; LC-MS-DAD-ELSD 163 (-)=(M-H)(-); 165(+)=(M+H)(+).
c) La 5-fluoro-1H-pyrrolo[2,3-b]pyridine peut être préparée comme décrit dans WO2005/103050.

### Exemple 20

### 3-[(Z)-(5-oxo-2-thioxoimidazolidin-4-ylidène)méthyl]-1H-pyrrolo[2,3-b]pyridine-5-carbonitrile

a) Le 3-[(Z)-(5-oxo-2-thioxoimidazolidin-4-ylidène)méthyl]-1H-pyrrolo[2,3-b]pyridine-5-carbonitrile peut être préparé comme dans l'exemple 1, mais à partir de 173 mg de 3-formyl-1H-pyrrolo[2,3]pyridine-5-carbonitrile, dans 7,4 cm³ d'éthanol, de 122 mg de 2-thioxoimidazolidin-4-one, et de 0,05 cm³ de piperidine. Après trois heures de reflux, on obtient 200 mg de 3-[5-oxo-2thioxo-imidazolidin-(4Z)-ylidenméthyl]-1H-pyrrolo{[2,3-b]pyridine-5-carbonitrile sous forme de poudre orangée, dont les caractéristiques physiques sont les suivantes :
   LC-MS (1) : temps de rétention 2,86 ; LC-MS-DAD-ELSD: 270 (+) = (M+H) (+).
   Spectre de RMN 1H à 400MHz : 6,84 (s, 1H) ; 8,66 (s, 1H) ; 8,69 (s, 1H) ; 8,99 (s, 1H) ; 12,17 (s large, 1H).
b) Le 3-formyl-1H-pyrrolo[2,3]pyridine-5-carbonitrile peut être préparé comme l'exemple 2, mais à partir de 860 mg de 1H-pyrrolo[2,3]pyridine-5-carbonitrile dans un mélange de 15 cm³ d'eau et de 4,8 cm³ d'acide acétique, et de 1,26 g de tétraazatricyclo[3.3.1.1~3,7~]décane. On obtient 275 mg de 3-formyl-1H-pyrrolo[2,3]pyridine-5-carbonitrile sous forme de poudre beige dont les caractéristiques physiques sont les suivantes :
   Spectre RMN de ¹H à 400MHz: 8,66 (s, 1H) ; 8,77 (m, 2H) ; 9,98 (s, 1H).
   LC-MS (1) : temps de rétention 2.39 mn ; LC-MS-DAD-ELSD: 170(-)=(M-H)(-); 172 (+) = (M+H) (+).
c) Le 1H-pyrrolo[2,3-b]pyridine-5-carbonitrile peut être préparé comme décrit dans W02004/078756.

### Exemple 21

### (5Z)-5-[(5-phénylamino-1H-pyrrolo[2,3-b]pyridin-3-yl)méthylène]-2-thioxoimidazolidin-4-one

a) La (5Z)-5-[(5-phénylamino-1H-pyrrolo[2,3-b]pyridin-3-yl)méthylène]-2-thioxoimidazolidin-4-one peut être préparée comme dans l'exemple 1, mais à partir de 63 mg de 5-phénylamino-1H-pyrrolo[2,3-b]pyridine-3-carbaldéhyde dans 2 cm³ d'éthanol, de 32 mg de 2-thioxoimidazolidin-4-one et de 0,03 cm³ de pipéridine. Après deux heures et demi de reflux, on obtient 70 mg de (5Z)-5-[(5-phénylamino-1H-pyrrolo[2,3-b]pyridin-3-yl)méthylène]-2-thioxoimidazolidin-4-one sous forme d'une poudre orangée dont les caractéristiques physiques sont les suivantes:
   Spectre RMN de ¹H à 400MHz : 6,71 (s, 1H) ; 6,76 (t, J = 7,6 Hz, 1H) ; 7,01 (d, J = 7,6 Hz, 2H) ; 7,21 (t, J = 7,6 Hz, 2H) ; 7,98 (d, J = 2,4 Hz, 1H) ; 8,06 (s, 1H) ; 8,12 (d, J = 2,4 Hz, 1H) ; 8,47 (s, 1H) ; 11,70 (s large, 3H) ; 12,29 (s large, 1H).
   LC-MS (1): temps de rétention 3.55 mn ; LC-MS-DAD-ELSD 334 (-)=(M-H)(-); 336 (+)=(M+H)(+).
b) La 5-phénylamino-1H-pyrrolo[2,3-b]pyridine-3-carbaldéhyde peut être préparée comme dans l'exemple 14 mais à partir de 1,6 g de chlorure d'aluminium en suspension dans 25 cm³ de dichlorométhane, de 0,5 g de N-phényl-1H-pyrrolo[2,3-b]pyridin-5-amine, et de 1,1 cm³ de dichloro(méthoxy)méthane. Après une heure trente minutes de réaction et hydrolyse du mélange réactionnel, on obtient 180 mg d'un produit brut que l'on purifie par flash chromatographie sur colonne de silice avec l'éluant dichlorométhane/acétate d'éthyle (gradient de 80/20 à 50/50 en volumes). On obtient 70 mg de 5-phényl-1H-pyrrolo[2;3-b]pyridine-3-carbaldéhyde sous forme de poudre ocre dont les caractéristiques physiques sont les suivantes :
   Spectre RMN de ¹H à 400MHz : 6,80 (t, J = 7,4 Hz, 1H) ; 7,02 (d, J = 7,4 Hz, 2H) ; 7,24 (t, J = 7,4 Hz, 2H) ; 8,17 (m, 3H) ; 8,36. (s, 1H) ; 9,86 (s, 1H), 12,56 (s large, 1H).
   LC-MS-DAD-ELSD : 236(-)=(M-H)(-) ;237(+)=(M+H)(+).
c) La 5-phénylamino-1H-pyrrolo[2,3-b]pyridine peut être préparée comme dans l'exemple 5, mais à partir de 1,34 g de 1-[(4-méthylphényl)sulfonyl]-N-phényl-1H-pyrrolo[2,3-b]pyridin-5-amine dans 30 cm³ de méthanol, et de 4,15 g de potasse, pendant trois heures à température ambiante. Après traitement du mélange réactionnel, on obtient 0,63 g de 5-phénylamino-1H-pyrrolo[2,3-b]pyridine, sous forme de poudre beige dont les caractéristiques physiques sont les suivantes :
   Spectre RMN de ¹H à 400MHz : 6,35 (d, J = 3,5Hz, 1H) ; 6,69 (t, J = 7,5Hz, 1H) ; 6,89 (d, J = 7,5 Hz, 2H) ; 7,15 (t, J = 7,5 Hz, 2H) ; 7,40 (s large, 1H) ; 7,70 (d, J = 2,6 Hz, 1H) 7,83 (s, 1H) ; 8,04 (d, J = 2,6 Hz, 1H), 11,45 (s large, 1H).
   LC/MS temps de rétention 3.22 mn ; LC-MS-DAD-ELSD208(-)=(M-H)(-); 210 (+) = (M+H) (+).
d) La 1-[(4-méthylphényl)sulfonyl]-N-phényl-1H-pyrrolo[2,3-b]pyridin-5-amine peut être préparée de la manière suivante:
   Dans un réacteur pour four micro-ondes, sont introduits 750 mg de 5-bromo-1-[(4-méthylphényl)sulfonyl]-1H-pyrrolo[2,3-b]pyridine dans 19 cm³ de toluène, 54 mg de 9,9-diméthyl-4,5-bis(diphénylphosphino)xanthène, 970 mg de carbonate de césium, 280 mg d'aniline et 40 mg de tri(dibenzylideneacétone)dipalladium(0). Le réacteur est mis sous irradiation dans un four à micro-ondes, pendant quarante cinq minutes à 150°C. Après refroidissement, le mélange réactionnel est filtré, puis amené à sec sous vide au rotavapor. Le résidu marron est combiné avec deux autres résidus obtenus de façon similaire puis purifié par flash chromatographie sur colonne de silice avec l'éluant dichlorométhane pour donner 1,34 g de 1-[(4-méthylphényl)sulfonyl]-N-phényl-1H-pyrrolo[2,3-b]pyridin-5-amine sous forme d'une poudre beige dont les caractéristiques physiques sont les suivantes :
      LC/MS temps de rétention 4,58 mn ; LC-MS-DAD-ELSD ; 364 (+) = (M+H) (+).
e) La 5-bromo-1-[(4-méthylphényl)sulfonyl]-1H-pyrrolo[2,3-b]pyridine peut être préparée de la manière suivante:
   A 41 cm³ de toluène sont ajoutés, 1,63 g de 5-bromo-1H-pyrrolo[2,3- b]pyridine, 30 mg de tetrabutyl ammonium hydrogénosulfate, 1,81 g de chlorure de tosyle et 4,3 g de soude en pastille en solution dans 40 cm³ d'eau. Après quatre heures sous agitation à température ambiante, le mélange réactionnel est versé sur 50 cm³ d'eau. Le mélange obtenu est extrait avec 50 cm³ d'acétate d'éthyle. La phase organique est décantée, séchée sur sulfate de magnésium, filtrée puis amenée à sec sous vide au rotavapor. On obtient 2,4 g de 5-bromo-1-[(4-méthylphényl)sulfonyl]-1H-pyrrolo[2,3-b]pyridine sous forme de poudre blanche dont les caractéristiques physiques sont les suivantes:
      LC/MS temps de rétention 4,70 mn ; LC-MS-DAD-ELSD 353(+)=(M+H)(+) (1 atome de brome Br présent).

### Exemple 22

### (5Z)-5-[(5-morpholin-4-yl-1H-pyrrolo[2,3-b]pyridin-3-yl)méthylidène]-2-thioxoimidazolidin-4-one

a) La (5Z)-5-[(5-morpholin-4-yl-1H-pyrrolo[2,3-b]pyridin-3-yl)méthylidène]-2-thioxoimidazolidin-4-one peut être préparée comme dans l'exemple 1, mais à partir de 140 mg de 5-morpholin-4yl-1H-pyrrolo[2,3-b]pyridine-3-carbaldéhyde dans 4,5 cm³ d'éthanol, de 75 mg de 2-thioxoimidazolidin-4-one et de 0,03 cm³ de pipéridine. Après deux heures et demi de reflux, on obtient 160 mg de (5Z)-5-[(5-morpholin-4y1-1H-pyrrolo[2,3-b]pyridin-3-yl)méthylène]-2-thioxoimidazolidin-4-one sous forme d'une poudre orangée dont les caractéristiques physiques sont les suivantes:
   LC-MS (1) : temps de rétention 2,68 mn ; LC-MS-DAD-ELSD 328(-)=(M-H)(-); 230(+)=(M+H)(+).
   Spectre RMN de ¹H à 400MHz : 3,16 (m, 4H) ; 3,78 (m, 4H) ; 6,88 (s, 1H) ; 7,86 (d, J = 2,3 Hz, 1H) ; 8,15 (d, J = 2,3 Hz, 1H) ; 8,48 (s, 1H) ; 11,78 (s large, 1H) ; 12,09 (s large, 1H) ; 12,23 (s large, 1H).
b) La 5-morpholin-4-yl-1H-pyrrolo[2,3-b]pyridine-3-carbaldéhyde peut être préparée comme dans l'exemple 14 mais à partir de 2 g de chlorure d'aluminium en suspension dans 50 cm³ de dichlorométhane, de 0,59 g de 5-morpholin-4yl-1H-pyrrolo[2,3-b]pyridine et de 0,8 cm³ de dichloro(méthoxy)méthane. Après une heure trente minutes de réaction et hydrolyse du mélange réactionnel, on obtient 140 mg de 5-morpholin-4yl-1H-pyrrolo[2,3-b]pyridine-3-carbaldéhyde sous forme d'une poudre beige dont les caractéristiques physiques sont les suivantes:
   LC/MS temps de rétention 2,35 mn ; LC-MS-DAD-ELSD 230(-)=(M-H)(-); 232 (+)=(M+H) (+).
   Spectre RMN de ¹H à 400MHz : 3,12 (m, 4H) ; 3,78 (m, 4H) ; 7,88 (d, J = 2,8 Hz, 1H) ; 8,22 (d, J = 2,8 Hz, 1H) ; 8,34 (s, 1H) ; 9,87 (s, 1H) ; 12,48 (s large, 1H).
c) La 5-morpholin-4y1-1H-pyrrolo[2,3-b]pyridine peut être préparée comme l'exemple 5, mais à partir de 1,18 g de 1-[(4-méthylphényl)sulfonyl]-5-morpholin-4-yl-1H-pyrrolo[2,3-b]pyridine dans 33 cm³ de méthanol et de 3,7 g de potasse en pastille. Après traitement du mélange réactionnel, on obtient 0,59 g de 5-morpholin-4-yl-1H-pyrrolo[2,3-b]pyridine, sous forme de poudre beige dont les caractéristiques physiques sont les suivantes :
   Spectre RMN de ¹H à 400MHz : 3,05 (m, 4H) ; 3,77 (m, 4H) ; 6,32 (dd, J = 2 et 3,2 Hz, 1H) ; 7,36 (dd, J = 2 et 3,2 Hz, 1H) ; 7,48 (d, J = 2,7 Hz, 1H) ; 8,06 (d, J = 2,7 Hz, 1H) ; 11,35 (s large, 1H).
   LC/MS temps de rétention 1.27 mn; LC-MS-DAD-ELSD: 204 (+)=(M+H) (+).
d) La 1-[(4-méthylphényl)sulfonyl]-5-morpholin-4-yl-1H-pyrrolo[2,3-b]pyridine peut être préparé comme dans l'exemple 21 mais à partir de 62 cm³ de toluène, de 2,6 g de 5-bromo-1-[(4-méthylphényl)sulfonyl]-1H-pyrrolo[2,3-b]pyridine, de 3,35 g de carbonate de césium, de 170 mg de 9,9-diméthyl-4,5-bis(diphénylphosphino)xanthène, de 0,9 g de morpholine et de 130 mg de tri(dibenzylideneacétone)dipalladium(0). Le réacteur est mis sous irradiation dans un four à micro-ondes, pendant une heure à 150°C. Après refroidissement, le mélange réactionnel est filtré, amené à sec sous vide au rotavapor. Le résidu marron obtenu est purifié par flash chromatographie sur colonne de silice avec l'éluant dichlorométhane/acétate d'éthyle (gradient de 100 à 90/10 en volume) pour donner 1,18 g de 1-[(4-méthylphényl)sulfonyl]-5-morpholin-4-yl-1H-pyrrolo[2,3-b]pyridine sous forme de poudre beige dont les caractéristiques physiques sont les suivantes:
   LC/MS temps de rétention 3,96 mn ; LC-MS-DAD-ELSD : 358(+)=(M+H)(+).

### Exemple 23

### (5Z)-5-[(5-benzyl-1H-pyrrolo[2,3-b]pyridin-3-yl)méthylidène]-2-thioxoimidazolidin-4-one,

a) La (5Z)-5-[(5-benzyl-1H-pyrrolo[2,3-b]pyridin-3-yl)méthylidène]-2-thioxoimidazolidin-4-one peut être préparée comme dans l'exemple 1, mais à partir de 55 mg 5-benzyl-4y1-1H-pyrrolo[2,3-b]pyridine-3-carbaldéhyde dans 1,8 cm³ d'éthanol, de 27 mg de 2-thioxoimidazolidin-4-one et de 0,023 cm³ de pipéridine. Après deux heures et demi de reflux, on obtient 60 mg de (5Z)-5-[(5-morpholin-4yl -1H-pyrrolo[2,3-b]pyridin-3-yl)méthylène]-2-thioxoimidazolidin-4-one sous forme d'une poudre orangée dont les caractéristiques physiques sont les suivantes:
   Spectre RMN de ¹H à 400MHz : 4,06 (s, 2H) ; 6,69 (s, 1H) ; 7,17 (t, J = 6,9 Hz, 1H) ; 7,29 (m, 4H) ; 8,17 (d, J = 1,8 Hz, 1H) ; 8,23 (d, J = 1,8 Hz, 1H) ; 8,49 (s, 1H) ; 11,35 (s large, 1H) ; 12,26 (s large, 1H).
   LC-MS (1) : temps de rétention 3,66 mn ; LC-MS-DAD-ELSD : 333 (-)=(M-H)(-) ; 335 (+)=(M+H)(+).
b) Le 5-benzyl-1H-pyrrolo[2,3-b]pyridine-3-carbaldéhyde peut être préparé comme dans l'exemple 16, mais à partir de 260 mg de 5-bromo-1-[(4-méthylphényl)sulfonyl]-1H-pyrrolo[2,3-b]pyridine 3-carbaldéhyde, dans 4,7 cm³ de tétrahydrofurane et 2,5 cm³ d'eau, de 680 mg de carbonate de césium et de 55 mg de 1,1'-Bis(diphénylphosphino)ferrocènedichloropalladium (II). Après quarante cinq minutes d'irradiation à 150°C, le mélange réactionnel est versé dans un mélange eau/acétate d'éthyle. La phase organique est séchée sur sulfate de sodium et la phase aqueuse est réextraite avec 2 fois 50 cm³ d'un mélange dichlorométhane/méthanol 90/10 en volume. La phase dichlorométane est séchée sur sulfate de sodium puis combinée avec les phases organiques précédentes avant concentration sous vide au rotavapor. Le résidu marron est purifié par flash chromatographie sur colonne de silice avec un éluant dichlorométhane/acétate d'éthyle (gradient dichlorométhane/acétate d'éthyle de 90/10 à 50/50 en volumes) pour donner 55 mg de 5-benzyl-1H-pyrrolo[2,3-b]pyridine-3-carbaldéhyde sous forme de poudre beige dont les caractéristiques physiques sont les suivantes:
   Spectre RMN de ¹H à 400MHz : 4,09 (s, 2H) ; 7,19 (m, 1H) ; 7,28 (m, 4H) ; 8,22 (d, J = 2,1 Hz, 1H) ; 8,31 (d, J = 2,1 Hz, 1H) ; 8,42 (s, 1H) ; 9,87 (s, 1H) ; 12,61 (s large, 1H).
   LC-MS (1) : temps de rétention 3,61 mn ; LC-MS-DAD-ELSD : 235 (-)=(M-H)(-); 237(+)=(M+H)(+).
c) Le 5-bromo-1-[(4-méthylphényl)sulfonyl]-1H-pyrrolo[2,3-b]pyridine-3-carbaldéhyde peut être préparé de la manière suivante:
   A 10 cm³ de tetrahydrofurane anhydre, sont ajoutés à 0°C 1 g de 5-bromo-1H-pyrrolo[2,3-b]pyridine=3-carbaldéhyde, puis 0,27 g d'hydrure de sodium. Le mélange réactionnel est maintenu quinze minutes sous agitation à 0°C avant ajout de 1,87 g de chlorure de tosyle en solution dans 1,7 cm³ de tétrahydrofurane. Le mélange réactionnel est laissé pendant deux heures sous agitation revenir à température ambiante. Le mélange réactionnel est versé dans sur 100 cm³ d'eau glacée. Le mélange obtenu est extrait avec deux fois 100 cm³ d'acétate d'éthyle. Les phases organiques sont séchées sur sulfate de magnésium puis amenées à sec sous vide au rotavapor. On obtient 1,32 g de 5-bromo-1-[(4-méthylphényl)sulfonyl]-1H-pyrrolo[2,3-b]pyridine-3-carbaldéhyde sous forme d'une poudre beige dont les caractéristiques physiques sont les suivantes :
      LC-MS-DAD-ELSD: 381(+)=(M+H)(+) (1 atome de brome Br présent).

### Exemple 24

### (5E/Z)-3-phényl-5-(1H-pyrrolo[2,3-b]pyridin-3-ylméthylidène)-2-thioxoimidazolidin-4-one

La (5E/Z)-3-phényl-5-(1H-pyrrolo[2,3-b]pyridin-3-ylméthylidène)-2-thioxoimidazolidin-4-one peut être préparée comme dans l'exemple 1, mais à partir de 75 mg de 1H-pyrrolo[2,3-b]pyridine-3-carbaldéhyde dans 3,5 cm³ d'éthanol, de 98 mg de 2-thioxoimidazolidin-3-phényl-4-one (SIGMA) et de 0,02 cm³ de pipéridine. Après cinq heures de reflux, on obtient 151 mg d'un mélange 50/50 de (5Z/E)-3-phényl-5-(1H-pyrrolo[2,3-b]pyridin-3-ylméthylidène)-2-thioxoimidazolidin-4-one sous forme d'une poudre jaune dont les caractéristiques sont les suivantes:
Spectre de RMN ¹H à 400MHz : 7,01 (s, 0,5H) ; 7,11 (s, 0,5H) ; 7,21 (dd, J = 5,0 et 8,0 Hz, 0,5H) ; 7,28 (dd, J = 5,0 et 8,0 Hz, 0,5H) ; 7,39 (m, 2H) ; de 7,42 à 7,58 (m, 3H) ; 8,13 (dd, J = 1,5 et 8,0 Hz, 0,5H) ; 8,33 (m, 1,5H) ; 8,67 (d, J = 2,0 Hz, 0,5H) ; 8,99 (d, J = 2,0 Hz, 0,5H) ; de 12,2 à 12,6 (m, 2H).
HPLC-MS-DAD-ELSD : 321(+)=(M+H)(+); 319(-)=(M-H)(-) (mélange d'isomères 50/50).

### Exemple 25

### (5Z)-2-azépan-1-yl-5-[(5-chloro-1H-pyrrolo[2,3-b]pyridin-3-yl)méthylidène]-3,5-dihydro-4H-imidazol-4-one

La (5Z)-2-azépan-1-yl-5-[(5-chloro-1H-pyrrolo[2,3-b]pyridin-3-yl)méthylidène]-3,5-dihydro-4H-imidazol-4-one peut être préparée comme dans l'exemple 7, mais à partir de 278 mg de (5Z)-5-[(5-chloro-1H-pyrrolo[2,3-b]pyridin-3-yl)méthylène]-2-thioxoimidazolidin-4-one (exemple 3), de 2,5 cm³ d'éthanol et de 992 mg d'azépane. Après une heure à une température de 160°C sous irradiation micro-ondes et filtration du solide, on obtient 92 mg de (5Z)-2-azépan-1-yl-5-[(5-chloro-1H-pyrrolo[2,3-b]pyridin-3-yl)méthylidène]-3,5-dihydro-4H-imidazol-4-one sous forme d'une poudre jaune dont les caractéristiques sont les suivantes :
Point de fusion : 310-320°C.
Spectre de RMN ¹H à 400MHz : 1,53 (m, 4H) ; 1,78 (m large, 4H) ; 3,63 (m étalé, 4H) ; 6,58 (s, 1H) ; 8,13 (s, 1H) ; 8,21 (d, J = 3,0 Hz, 1H) ; 9,21 (s large, 1H) ; 11,0 (m étalé, 1H) ; 12,15 (m étalé, 1H).
HPLC-MS-DAD-ELSD : 344(+)=(M+H)(+); 342(-)=(M-H)(-).

### Exemple 26

### (5Z)-5-[(5-chloro-1H-pyrrolo[2,3-b]pyridin-3-yl)méthylidène]-2-(4-phénylpipérazin-1-yl)-3,5-dihydro-4H-imidazol-4-one

La (5Z)-5-[(5-chloro-1H-pyrrolo[2,3-b]pyridin-3-yl)méthylidène]-2-(4-phénylpipérazin-1-yl)-3,5-dihydro-4H-imidazol-4-one peut être préparée comme dans l'exemple 7, mais à partir de 167 mg de (5Z)-5-[(5-chloro-1H-pyrrolo[2,3-b]pyridin-3-yl)méthylène]-2-thioxoimidazolidin-4-one (exemple 3), de 2 cm³ d'éthanol et de 973 mg de 4-phénylpipérazine. Après une heure à une température de 160°C sous irradiation micro-ondes puis filtration du solide, on obtient 182 mg de (5Z)-5-[(5-chloro-1H-pyrrolo[2,3-b]pyridin-3-yl)méthylidène]-2-(4-phénylpipérazin-1-yl)-3,5-dihydro-4H-imidazol-4-one sous forme d'une poudre orangée dont les caractéristiques sont les suivantes:
Point de fusion : 340-350°C.
Spectre de RMN ¹H à 400MHz : 3,29 (m partiellement masqué, 4H) ; 3,75 (m, 4H) ; 6,70 (s, 1H) ; 6,82 (J = 7,5 Hz, 1H) ; 7,02 (d, J = 7,5 Hz, 2H) ; 7,24 (t, J = 7,5 Hz, 2H) ; 8,24 (d, J = 3,0 Hz, 1H) ; 8,28 (s, 1H) ; 8,98 (d, J = 3,0 Hz, 1H) ; 11,2 (s large, 1H) ; 12,2 (s large, 1H).
UPLC-MS-DAD-ELSD : 407 (+)/...=(M+H)(+)/... (1 atome de chlore Cl présent).

### Exemple 27

### (5Z)-5-[(5-chloro-1H-pyrrolo[2,3-b]pyridin-3-yl)méthylidène]-2-pyrrolidin-1-yl-3,5-dihydro-4H-imidazol-4-one

La (5Z)-5-[(5-chloro-1H-pyrrolo[2,3-b]pyridin-3-yl)méthylidène]-2-pyrrolidin-1-yl-3,5-dihydro-4H-imidazol-4-one peut être préparée comme dans l'exemple 7, mais à partir de 223 mg de (5Z)-5-[(5-chloro-1H-pyrrolo[2,3-b]pyridin-3-yl)méthylène]-2-thioxoimidazolidin-4-one (exemple 3), de 2,5 cm³ d'éthanol et de 569 mg de pyrrolidine. Après trente minutes à une température de 160°C sous irradiation micro-ondes puis filtration du solide, on obtient 186 mg de (5Z)-5-[(5-chloro-1H-pyrrolo[2,3-b]pyridin-3-yl)méthylidène]-2-pyrrolidin-1-yl-3,5-dihydro-4H-imidazol-4-one sous forme d'une poudre jaune dont les caractéristiques sont les suivantes:
Point de fusion : 350-360°C.
Spectre de RMN ¹H à 400MHz : 1,94 (m, 4H) ; 3,52 (m large, 4H) ; 6,59 (s, 1H) ; 8,19 (s, 1H) ; 8,21 (d, J = 3,0 Hz, 1H) ; 9,15 (m étalé, 1H) ; 11,1 (m étalé, 1H) ; 12,15 (m étalé, 1H).
HPLC-MS-DAD-ELSD : 316 (+)/...= (M+H) (+)/...; 314 (-) /...=(M-H) (-)/...

### Exemple 28

### (5Z)-5-[(5-chloro-1H-pyrrolo[2,3-b]pyridin-3-yl)méthylidène]-2-morpholin-4-yl-3,5-dihydro-4H-imidazol-4-one

La (5Z)-5-[(5-chloro-1H-pyrrolo[2,3-b]pyridin=3-yl)méthylidène]-2-morpholin-4-yl-3,5-dihydro-4H-imidazol-4-one peut être préparée comme dans l'exemple 7, mais à partir de 139 mg de (5Z)-5-[(5-chloro-1H-pyrrolo[2,3-b]pyridin-3-yl)méthylène]-2-thioxoimidazolidin-4-one (exemple 3), de 2,5 _{CM}³ d'éthanol et de 436 mg de morpholine. Après deux heures à une température de 160°C sous irradiation micro-ondes puis filtration du solide, on obtient 92 mg de (5Z)-5-[(5-chloro-1H-pyrrolo[2,3-b]pyridin-3-yl)méthylidène]-2-morpholin-4-yl-3,5-dihydro-4H-imidazol-4-one sous forme d'une poudre jaune dont les caractéristiques sont les suivantes :
Spectre de RMN ¹H à 400MHz: 3,59 (m, 4H) ; 3,70 (m, 4H) ; 6,70 (s, 1H) ; 8,22 (m, 2H) ; 8,94 (m étalé, 1H) ; 11,15 (m étalé, 1H) ; 12,2 (m étalé, 1H).

### Exemple 29

### (5Z)-5-[(5-chloro-1H-pyrrolo[2,3-b]pyridin-3-yl)méthylidène]-2-[(2-méthylpropyl)amino]-3,5-dihydro-4H-imidazol-4-one

La (5Z)-5-[(5-chloro-1H-pyrrolo[2,3-b]pyridin-3-yl)méthylidène]-2-[(2-méthylpropyl)amino]-3,5-dihydro-4H-imidazol-4-one peut être préparée comme dans l'exemple 7 mais à partir de 200 mg de (5Z)-5-[(5-chloro-1H-pyrrolo[2,3-b]pyridin-3-yl)méthylène]-2-thioxoimidazolidin-4-one (exemple 3), de 4 cm³ d'éthanol et de 525 mg de 2-méthylpropylamine. Après quarante-cinq minutes à une température de 160°C sous irradiation micro-ondes puis filtration du solide, on obtient 89 mg de (5Z)-5-[(5-chloro-1H-pyrrolo[2,3-b]pyridin-3-yl)méthylidène]-2-[(2-méthylpropyl)amino]-3,5-dihydro-4H-imidazol-4-one sous forme d'une poudre jaune dont les caractéristiques sont les suivantes:
Point de fusion: 325-330°C.
Spectre de RMN ¹H à 400MHz : 0,95 (d, J = 6,5 Hz, 6H) ; 1,93 (m, 1H) ; 3,19 (t, J = 6 Hz, 2H) ; 6,57 (s, 1H) ; 7,21 (s large 1H) ; 8,11 (s, 1H) ; 8,21 (s large, 1H) ; 9,25 (m étalé, 1H) ; 10,4 (m étalé, 1H) ; 12,15 (m étalé, 1H).
HPLC-MS-DAD-ELSD : 318 (+)/...=(M+H) (+)/...; 316 (-)/...=(M-H) (-)/... (1 Cl présent).

### Exemple 30

### (5Z)-5-[(5-chloro-1H-pyrrolo[2,3-b]pyridin-3-yl)méthylidène]-2-pipéridin-1-yl-3,5-dihydro-4H-imidazol-4-one

La (5Z)-S-[(5-chloro-1H-pyrrolo[2,3-b]pyridin-3-yl)méthylidène]-2-pipéridin-1-yl-3,5-dihydro-4H-imidazol-4-one peut être préparée comme dans l'exemple 7, mais à partir de 195 mg de (5Z)-5-[(5-chloro-1H-pyrrolo[2,3-b]pyridin-3-yl)méthylène]-2-thioxoimidazolidin-4-one (exemple 3), de 2,5 cm³ d'éthanol et de 596 mg de pipéridine. Après trente minutes à une température de 160°C sous irradiation micro-ondes puis filtration du solide, on obtient 152 mg de (5Z)-5-[(5-chloro-1H-pyrrolo[2,3-b]pyridin-3-yl)méthylidène]-2-pipéridin-1-yl-3,5-dihydro-4H-imidazol-4-one sous forme d'une poudre orangée dont les caractéristiques sont les suivantes :
Point de fusion : 315-320°C.
Spectre de RMN ¹H à 400MHz : De 1,51 à 1,70 (m, 6H) ; 3,59 (m, 4H) ; 6,61 (s, 1H) ; 8,19 (s, 1H) ; 8,22 (d, J = 3,0 Hz, 1H) ; 9,01 (m étalé, 1H) ; 11,0 (m étalé, 1H) ; 12,1 (m étalé, 1H).
HPLC-MS-DAD-ELSD : 330 (+)/...=(M+H) (+)/...; 328 (-)/...= (M-H) (-) /... (1 Cl présent).

### Exemple 31

### (5Z)-5-[(5-chloro-1H-pyrrolo[2,3-b]pyridin-3-yl)méthylidène]-2-[méthyl(2-méthylpropyl)amino]-3,5-dihydro-4H-imidazol-4-one

La (5Z)-5-[(5-chloro-1H-pyrrolo[2,3-b]pyridin-3-yl)méthylidène]-2-[méthyl(2-méthylpropyl)amino]-3,5-dihydro-4H-imidazol-4-one peut être préparée comme dans l'exemple 7, mais à partir de 63 mg de (5Z)-5-[(5-chloro-1H-pyrrolo[2,3-b]pyridin-3-yl)méthylène]-2-thioxoimidazolidin-4-one (exemple 3), de 1 cm³ d'éthanol et de 187 mg de N-méthyl-(2-méthyl)propylamine. Après quinze minutes à une température de 160°C, trente minutes à 170°C, et une heure trente minutes à 180°C sous irradiation micro-ondes, le mélange est concentré sous vide. Le résidu est repris avec du chlorure de méthylène et le solide est filtré pour obtenir 30 mg de (5Z)-5-[(5-chloro-1H-pyrrolo[2,3-b]pyridin-3-yl)méthylidène]-2-[méthyl(2-méthylpropyl)amino]-3,5-dihydro-4H-imidazol-4-one sous forme d'une poudre jaune dont les caractéristiques sont les suivantes :
Spectre de RMN ¹H à 400MHz : 0,91 (d, J = 6,5 Hz, 6H) ; 2,04 (m, 1H) ; 3,09 (s, 3H) ; 3,30 (m masqué, 2H) ; 6,59 (s, 1H) ; 8,13 (s large, 1H) ; 8,21 (d, J = 2,0 Hz, 1H) ; 9,20 (m étalé, 1H) ; 11,1 (m étalé, 1H) ; 12,15 (m étalé, 1H).
HPLC-MS-DAD-ELSD : 332 (+) /...= (M+H) (+) /...; 330 (-) /...= (M-H) (-) /... (1 atome de chlore Cl présent).

### Exemple 32

### (5Z)-5-[(5-chloro-1H-pyrrolo[2,3-b]pyridin-3-yl)méthylidène]-2-(diméthylamino)-3,5-dihydro-4H-imidazol-4-one

La (5Z)-5-[(5-chloro-1H-pyrrolo[2,3-b]pyridin-3-yl)méthylidène]-2-(diméthylamino)-3,5-dihydro-4H-imidazol-4-one peut être préparée comme dans l'exemple 7, mais à partir de 279 mg de (5Z)-5-[(5-chloro-1H-pyrrolo[2,3-b]pyridin-3-yl)méthylène]-2-thioxoimidazolidin-4-one (exemple 3), de 1,5 cm³ d'éthanol et de 2 cm³ de solution de diméthylamine à 33% dans l'éthanol. Après trente minutes à une température de 160°C sous irradiation micro-ondes puis filtration du solide, on obtient 220 mg de (5Z)-5-[(5-chloro-1H-pyrrolo[2,3-b]pyridin-3-yl)méthylidène]-2-(diméthylamino)-3,5-dihydro-4H-imidazol-4-one sous forme d'une poudre jaune dont les caractéristiques sont les suivantes :
Point de fusion : 350°C.
Spectre de RMN ¹H à 400MHz : 3,10 (s, 6H) ; 6,60 (s, 1H) ; 8,19 (s, 1H) ; 8,22 (d, J = 2,0 Hz, 1H) ; 9,09 (m étalé, 1H) ; 11,1 (m étalé, 1H) ; 12,15 (m étalé, 1H).
HPLC-MS-DAD-ELSD : 290 (+) /...= (M+H) (+) /... (1 atome de chlore Cl présent).

### Exemple 33

### (5Z)-5-[(5-bromo-1H-pyrrolo[2,3-b]pyridin-3-yl)méthylidène]-2-[(cyclopropylméthyl)amino]-3,5-dihydro-4H-imidazol-4-one

La (5Z)-5-[(5-bromo-1H-pyrrolo[2,3-b]pyridin-3-yl)méthylidène]-2-[(cyclopropylméthyl)amino]-3,5-dihydro-4H-imidazol-4-one peut être préparée comme dans l'exemple 7, mais à partir de 860 mg de (5Z)-5-[(5-bromo-1H-pyrrolo[2,3-b]pyridin-3-yl)méthylène]-2-thioxoimidazolidin-4-one (exemple 14), de 8 cm³ d'éthanol et de 1,89 g de 1-cyclopropylméthanamine. Après trente minutes à une température de 160°C sous irradiation micro-ondes puis filtration du solide, on obtient 500 mg de (5Z)-5-[(5-bromo-1H-pyrrolo[2,3-b]pyridin-3-yl)méthylidène]-2-[(cyclopropylméthyl)amino]-3,5-dihydro-4H-imidazol-4-one sous forme d'une poudre jaune pâle dont les caractéristiques sont les suivantes :
Spectre de RMN ¹H à 400MHz : 0,30 (m, 2H) ; 0,49 (m, 2H) ; 1,17 (m, 1H) ; 3,38 (m partiellement masqué, 2H) ; 6,59 (s, 1H) ; 7,23 (m large, 1H) ; 8,11 (s, 1H) ; 8,28 (d, J = 2,0 Hz, 1H) ; 9,32 (m large, 1H) ; 10,4 (m étalé, 1H) ; 12,15 (m étalé, 1H).
HPLC-MS-DAD-ELSD : 360 (+) = (M+H) (+) ; 358 (-) = (M-H) (-) (1 atome de brome Br présent).

### Exemple 34

### (5Z)-2-[(cyclopropylméthyl)amino]-5-[(5-méthoxy-1H-pyrrolo[2,3-b]pyridin-3-yl)méthylidène]-3,5-dihydro-4H-imidazol-4-one

La (5Z)-2-[(cyclopropylméthyl)amino]-5-[(5-méthoxy-1H-pyrrolo[2,3-b]pyridin-3-yl)méthylidène]-3,5-dihydro-4H-imidazol-4-one peut être préparée comme dans l'exemple 7, mais à partir de 200 mg de (5Z)-5-[(5-méthoxy-1H-pyrrolo[2,3-b]pyridin-3-yl)méthylidène]-2-thioxoimidazolidin-4-one (exemple 15), de 3 cm³ d'éthanol et de 519 mg de 1-cyclopropylméthanamine. Après quarante minutes à une température de 130°C et dix minutes à 140°C sous irradiation micro-ondes, le mélange est concentré sous vide. Le résidu est purifié par LC-MS préparative [Colonne Xterra RP18 30x100 ; 5µ ; avec eau tamponnée à 10 mM d'hydrogénocarbonate d'ammonium ajustée à pH 9 avec de l'ammoniaque / Acétonitrile avec gradient 70/30 à 0/100 en 8 min], pour obtenir 58 mg de (5Z)-2-[(cyclopropylméthyl)amino]-5-[(5-méthoxy-1H-pyrrolo[2,3-b]pyridin-3-yl)méthylidène]-3,5-dihydro-4H-imidazol-4-one sous forme d'un lyophilisat jaune dont les caractéristiques sont les suivantes :
Point de fusion : 280-286°C.
Spectre de RMN ¹H à 400MHz : 0,28 (m, 2H) ; 0,48 (m, 2H) ; 1,13 (m, 1H) ; de 3,15 à 3,40 (m partiellement masqué, 2H) ; 3,88 (s, 3H) ; 6,62 (s, 1H) ; 7,14 (m, 1H) ; 7,98 (m, 1H) ; 8,11 (m, 1H) ; 8,40 (m, 1H) ; 10,35 (m étalé, 1H) ; 11,8 (m étalé, 1H).
HPLC-MS-DAD-ELSD : 312 (+) = (M+H) (+) ; 310 (-) = (M-H) (-).

### Exemple 35

### (5Z)-2-[(cyclopropylméthyl)amino]-5-[(5-fluoro-1H-pyrrolo[2,3-b]pyridin-3-yl)méthylidène]-3,5-dihydro-4H-imidazol-4-one

La (5Z)-2-[(cyclopropylméthyl)amino]-5-[(5-fluoro-1H-pyrrolo[2,3-b]pyridin-3-yl)méthylidène]-3,5-dihydro-4H-imidazol-4-one peut être préparée comme dans l'exemple 7, mais à partir de 250 mg de (5Z)-5-[(5-fluoro-1H-pyrrolo[2,3-b]pyridin-3-yl)méthylidène]-2-thioxoimidazolidin-4-one (exemple 19), de 4 cm³ d'éthanol et de 678 mg de 1-cyclopropylméthanamine. Après trente minutes à une température de 160°C sous irradiation micro-ondes puis filtration du solide, on obtient 73 mg de (5Z)-2-[(cyclopropylméthyl)amino]-5-[(5-fluoro-1H-pyrrolo[2,3-b]pyridin-3-yl)méthylidène]-3,5-dihydro-4H-imidazol-4-one sous forme d'une poudre jaune dont les caractéristiques sont les suivantes :
Point de fusion : 312°C.
Spectre de RMN ¹H à 400MHz : 0,29 (m, 2H) ; 0,48 (m, 2H) ; 1,13 (m, 1H) ; 3,22 (t, J = 6,0 Hz, 1H) ; 6,59 (s, 1H) ; 7,27 (m étalé, 1H) ; 8,22 (m, 2H) ; 8,76 (d large, J = 9,0 Hz, 1H) ; 10,45 (m étalé, 1H) ; 12,05 (m étalé, 1H).
UPLC-MS-DAD-ELSD : 300(+)=(M+H)(+).

### Exemple 36

### (5Z)-2-azépan-1-yl-5-[(5-fluoro-1H-pyrrolo[2,3-b]pyridin-3-yl)méthylidène]-3,5-dihydro-4H-imidazol-4-one

La (5Z)-2-azépan-1-yl-5-[(5-fluoro-1H-pyrrolo[2,3-b]pyridin-3-yl)méthylidène]-3,5-dihydro-4H-imidazol-4-one peut être préparée comme dans l'exemple 7, mais à partir de 60 mg de (5Z)-5-[(5-fluoro-1H-pyrrolo[2,3-b]pyridin-3-yl)méthylidène]-2-thioxoimidazolidin-4-one (exemple 19), de 0,6 cm³ d'éthanol et de 227 mg d'azépane. Après trente minutes à une température de 160°C sous irradiation micro-ondes puis filtration du solide, on obtient 22 mg de (5Z)-2-azépan-1-yl-5-[(5-fluoro-1H-pyrrolo[2,3-b]pyridin-3-yl)méthylidène]-3,5-dihydro-4H-imidazol-4-one sous forme d'une poudre jaune dont les caractéristiques sont les suivantes :
Point de fusion : 299°C.
Spectre de RMN ¹H à 400MHz : De 1,44 à 1,87 (m, 8H) ; 3,62 (m étalé, 4H) ; 6,59 (s, 1H) ; 8,21 (s large, 2H) ; 8,77 (m étalé, 1H) ; 11,0 (m très étalé, 1H) ; 12,05 (m étalé, 1H).
HPLC-MS-DAD-ELSD : 328 (+) = (M+H) (+).

### Exemple 37

### 3-[(Z)-{2-[(cyclopropylméthyl)amino]-5-oxo-1,5-dihydro-4H-imidazol-4-ylidène}méthyl]-1H-pyrrolo[2,3-b]pyridine-5-carbonitrile

La 3-[(Z)-{2-[(cyclopropylméthyl)amino]-5-oxo-1,5-dihydro-4H-imidazol-4-ylidène}méthyl]-1H-pyrrolo[2,3-b]pyridine-5-carbonitrile peut être préparée comme dans l'exemple 7, mais à partir de 200 mg de 3-[(Z)-(5-oxo-2-thioxoimidazolidin-4-ylidène)méthyl]-1H-pyrrolo[2,3-b]pyridine-5-carbonitrile (exemple 20), de 2 cm³ d'éthanol et de 528 mg de 1-cyclopropylméthanamine. Après trente minutes à une température de 140°C sous irradiation micro-ondes puis filtration du solide, on obtient 58 mg de 3-[(Z)-{2-[(cyclopropylméthyl)amino]-5-oxo-1,5-dihydro-4H-imidazol-4-ylidène}méthyl]-1H-pyrrolo[2,3-b]pyridine-5-carbonitrile sous forme d'une poudre jaune dont les caractéristiques sont les suivantes :
Point de fusion : 335°C.
Spectre de RMN ¹H à 400MHz : 0,30 (m, 2H) ; 0,49 (m, 2H) ; 1,13 (m, 1H) ; de 3,15 à 3,40 (m masqué, 2H) ; 6,60 (s, 1H) ; 7,40 (s large, 1H) ; 8,25 (s large, 1H) ; 8,60 (s, 1H) ; 9,53 (s large, 1H) ; 10,7 (m étalé, 1H) ; 12,2 (m étalé, 1H).
HPLC-MS-DAD-ELSD : 307(+)=(M+H)(+); 305 (-) = (M-H) (-).

### Exemple 38

### (5Z)-2-[(cyclopropylméthyl)amino]-5-[(5-phényl-1H-pyrrolo[2,3-b]pyridin-3-yl)méthylidène]-3,5-dihydro-4H-imidazol-4-one

La (5z)-2-[(cyclopropylméthyl)amino]-5-[(5-phényl-1H-pyrrolo[2,3-b]pyridin-3-yl)méthylidène]-3,5-dihydro-4H-imidazol-4-one peut être préparée comme dans l'exemple 7, mais à partir de 140 mg de (5Z)-5-[(5-phényl-1H-pyrrolo[2,3-b]pyridin-3-yl)méthylidène]-2-thioxoimidazolidin-4-one (exemple 16), de 3 cm³ d'éthanol et de 311 mg de 1-cyclopropylméthanamine. Après quarante-cinq minutes à une température de 140°C et trente minutes à 150°C sous irradiation micro-ondes, le solide est filtré puis purifié par LC-MS préparative [Colonne Xterra RP18 30x100 ; 5µ ; avec eau tamponnée à 10 mM d'hydrogénocarbonate d'ammonium ajustée à pH 9 avec de l'ammoniaque / Acétonitrile avec gradient 70/30 à 0/100 en 8 min], pour obtenir 11 mg de (5Z)-2-[(cyclopropylméthyl)amino]-5-[(5-phényl-1H-pyrrolo[2,3-b]pyridin-3-yl)méthylidène]-3,5-dihydro-4H-imidazol-4-one sous forme d'une poudre jaune dont les caractéristiques sont les suivantes :
Spectre de RMN ¹H à 400MHz : 0,22 (m, 2H) ; 0,43 (m, 2H) ; 1,11 (m, 1H) ; 3,26 (m partiellement masqué, 2H) ; 6,70 (s, 1H) ; 7,15 (m large, 1H) ; 7,38 (t, J = 7,5 Hz, 1H) ; 7,49 (t, J = 7,5 Hz, 2H) ; 7,77 (d large, J = 7,5 Hz, 2H) ; 8,17 (s large, 1H) ; 8,53 (s large, 1H) ; 9,17 (m étalé, 1H) ; 10,4 (m étalé, 1H) ; 12,0 (m étalé, 1H).
HPLC-MS-DAD-ELSD : 358 (+) = (M+H) (+) ; 356 (-) = (M-H) (-).

### Exemple 39

### (5Z)-2-morpholin-4-yl-5-[(5-phényl-1H-pyrrolo[2,3-b]pyridin-3-yl)méthylidène]-3,5-dihydro-4H-imidazol-4-one

La (5Z)-2-morpholin-4-yl-5-[(5-phényl-1H-pyrrolo[2,3-b]pyridin-3-yl)méthylidène]-3,5-dihydro-4H-imidazol-4-one peut être préparée comme dans l'exemple 7, mais à partir de 200 mg de (5Z)-5-[(5-phényl-1H-pyrrolo[2,3-b]pyridin-3-yl)méthylène]-2-thioxoimidazolidin-4-one (exemple 16), de 2 cm³ d'éthanol et de 544 mg de morpholine. Après trente minutes à une température de 160°C sous irradiation micro-ondes puis filtration du solide, on obtient 160 mg de (5Z)-2-morpholin-4-yl-5-[(5-phényl-1H-pyrrolo[2,3-b]pyridin-3-yl)méthylidène]-3,5-dihydro-4H-imidazol-4-one sous forme d'une poudre jaune dont les caractéristiques sont les suivantes :
Point de fusion : 361°C.
Spectre de RMN ¹H à 400MHz : De 3,50 à 3,75 (m, 8H) ; 6,80 (s, 1H) ; 7,38 (t, J = 7,5 Hz, 1H) ; 7,49 (t, J = 7,5 Hz, 2H) ; 7,76 (d, J = 7,5 Hz, 2H) ; 8,21 (s, 1H) ; 8,57 (d, J = 2,0 Hz, 1H) ; 9, 12 (s large, 1H) ; 11,15 (m étalé, 1H) ; 12,1 (s large, 1H).
HPLC-MS-DAD-ELSD : 374(+)=(M+H)(+); 372(-)=(M-H)(-).

### Exemple 40

### (5Z)-5-[(5-phényl-1H-pyrrolo[2,3-b]pyridin-3-yl)méthylidène]-2-pipéridin-1-yl-3,5-dihydro-4H-imidazol-4-one

La (5Z)-5-[(5-phényl-1H-pyrrolo[2,3-b]pyridin-3-yl)méthylidène]-2-pipéridin-1-yl-3,5-dihydro-4H-imidazol-4-one peut être préparée comme dans l'exemple 7, mais à partir de 200 mg de (5Z)-5-[(5-phényl-1H-pyrrolo[2,3-b]pyridin-3-yl)méthylène]-2-thioxoimidazolidin-4-one (exemple 16), de 2 cm³ d'éthanol et de 531 mg de pipéridine. Après trente minutes à une température de 160°C sous irradiation micro-ondes puis filtration du solide, on obtient 137 mg de (5Z)-5-[(5-phényl-1H-pyrrolo[2,3-b]pyridin-3-yl)méthylidène]-2-pipéridin-1-yl-3,5-dihydro-4H-imidazol-4-one sous forme d'une poudre jaune dont les caractéristiques sont les suivantes :
Point de fusion : 307°C.
Spectre de RMN ¹H à 400MHz : De 1,50 à 1,69 (m, 6H) ; 3,59 (m, 4H) ; 6,71 (s, 1H) ; 7,38 (t, J = 7,5 Hz, 1H) ; 7,49 (t, J = 7,5 Hz, 2H) ; 7,75 (d, J = 7,5 Hz, 2H) ; 8,15 (s, 1H) ; 8,55 (d, J = 2,0 Hz, 1H) ; 9,20 (s large, 1H) ; 11,0 (m étalé, 1H) ; 12,05 (s large, 1H).
HPLC-MS-DAD-ELSD : 372 (+) = (M+H) (+); 370 (-) = (M-H) (-).

### Exemple 41

### (5Z)-2-(4-méthylpipérazin-1-yl)-5-[(5-phényl-1H-pyrrolo[2,3-b]pyridin-3-yl)méthylidène]-3,5-dihydro-4H-imidazol-4-one

La (5Z)-2-(4-méthylpipérazin-1-yl)-5-[(5-phényl-1H-pyrrolo[2,3-b]pyridin-3-yl)méthylidène]-3,5-dihydro-4H-imidazol-4-one peut être préparée comme dans l'exemple 7, mais à partir de 200 mg de (5Z)-5-[(5-phényl-1H-pyrrolo[2,3-b]pyridin-3-yl)méthylène]-2-thioxoimidazolidin-4-one (exemple 16), de 2 cm³ d'éthanol et de 625 mg de 4-méthylpipérazine. Après quarante minutes à une température de 160°C sous irradiation micro-ondes puis filtration du solide, on obtient 140 mg de (5Z)-2-(4-méthylpipérazin-1-yl)-5-[(5-phényl-1H-pyrrolo[2,3-b]pyridin-3-yl)méthylidène]-3,5-dihydro-4H-imidazol-4-one sous forme d'une poudre jaune dont les caractéristiques sont les suivantes :
Point de fusion : 304°C.
Spectre de RMN ¹H à 400MHz : 2,23 (s, 3H) ; 2,40 (m, 4H) ; 3, 59 (m, 4H) ; 6, 78 (s, 1H) ; 7,38 (t, J = 7,5 Hz, 1H) ; 7,49 (t, J = 7,5 Hz, 2H) ; 7,76 (d, J = 7,5 Hz, 2H) ; 8,19 (s, 1H) ; 8,55 (d, J = 2,0 Hz, 1H) ; 9,15 (s large, 1H) ; 11,1 (m étalé, 1H) ; 12,1 (s large, 1H).
HPLC-MS-DAD-ELSD : 387 (+) = (M+H) (+); 385 (-) = (M-H) (-).

### Exemple 42

### (5Z)-2-azépan-1-yl-5-[(5-phényl-1H-pyrrolo[2,3-b]pyridin-3-yl)méthylidène]-3,5-dihydro-4H-imidazol-4-one

La (5Z)-2-azépan-1-yl-5-[(5-phényl-1H-pyrrolo[2,3-b]pyridin-3-yl)méthylidène]-3,5-dihydro-4H-imidazol-4-one peut être préparée comme dans l'exemple 7, mais à partir de 200 mg de (5Z)-5-[(5-phényl-1H-pyrrolo[2,3-b]pyridin-3-yl)méthylène]-2-thioxoimidazolidin-4-one (exemple 16), de 2 cm³ d'éthanol et de 619 mg d'azépane. Après quarante minutes à une température de 160°C sous irradiation micro-ondes puis filtration du solide, on obtient 90 mg de (5Z)-2-azépan-1-yl-5-[(5-phényl-1H-pyrrolo[2,3-b]pyridin-3-yl)méthylidène]-3,5-dihydro-4H-imidazol-4-one sous forme d'une poudre jaune dont les caractéristiques sont les suivantes :
Point de fusion : 302°C.
Spectre de RMN ¹H à 400MHz : De 1,47 à 1,78 (m, 8H) ; de 3,53 à 3,78 (m étalé, 4H) ; 6,69 (s, 1H) ; 7,38 (t, J = 7,5 Hz, 1H) ; 7,49 (t, J = 7,5 Hz, 2H) ; 7,73 (d, J = 7,5 Hz, 2H) ; 8,14 (s, 1H) ; 8,53 (s large, 1H) ; 9,18 (s large, 1H) ; 11,0 (m étalé, 1H) ; 12,0 (s large, 1H).
HPLC-MS-DAD-ELSD : 386 (+) = (M+H) (+) ; 384 (-) = (M-H) (-).

### Exemple 43

### (5Z)-2-[(cyclopropylméthyl)amino]-5-[(5-méthyl-1H-pyrrolo[2,3-b]pyridin-3-yl)méthylidène]-3,5-dihydro-4H-imidazol-4-one

La (5Z)-2-[(cyclopropylméthyl)amino]-5-[(5-méthyl-1H-pyrrolo[2,3-b]pyridin-3-yl)méthylidène]-3,5-dihydro-4H-imidazol-4-one peut être préparée comme dans l'exemple 7, mais à partir de 240 mg de (5Z)-5-[(5-méthyl-1H-pyrrolo[2,3-b]pyridin-3-yl)méthylidène]-2-thioxoimidazolidin-4-one (exemple 17), de 4 cm³ d'éthanol et de 661 mg de 1-cyclopropylméthanamine. Après trente-cinq minutes à une température de 160°C sous irradiation micro-ondes puis filtration du solide, on obtient 61 mg de (5Z)-2-[(cyclopropylméthyl)amino]-5-[(5-méthyl-1H-pyrrolo[2,3-b]pyridin-3-yl)méthylidène]-3,5-dihydro-4H-imidazol-4-one sous forme d'une poudre jaune dont les caractéristiques sont les suivantes :
Point de fusion : 319-320°C.
Spectre de RMN ¹H à 400MHz : 0,29 (m, 2H) ; 0,48 (m, 2H) ; 1,16 (m, 1H) ; 2,40 (s, 3H) ; 3,23 (t, J = 6,0 Hz, 2H) ; 6,59 (s, 1H) ; 7,19 (m étalé, 1H) ; 8,09 (s, 1H) ; 8,12 (s, 1H) ; 8,49 (s, 1H) ; 10,25 (m étalé, 1H) ; 11,8 (m étalé, 1H).
HPLC-MS-DAD-ELSD : 296 (+) = (M+H) (+) ; 294 (-) = (M-H) (-).

### Exemple 44

### (5Z)-2-[(2-méthylpropyl)amino]-5-[(5-méthyl-1H-pyrrolo[2,3-b]pyridin-3-yl)méthylidène]-3,5-dihydro-4H-imidazol-4-one

La (5Z)-2-[(2-méthylpropyl)amino]-5-[(5-méthyl-1H-pyrrolo[2,3-b]pyridin-3-yl)méthylidène]-3,5-dihydro-4H-imidazol-4-one peut être préparée comme dans l'exemple 7, mais à partir de 240 mg de (5Z)-5-[(5-méthyl-1H-pyrrolo[2,3-b]pyridin-3-yl)méthylidène]-2-thioxoimidazolidin-4-one (exemple 17), de 4 cm³ d'éthanol et de 680 mg de 2-méthylpropylamine. Après trente-cinq minutes à une température de 160°C sous irradiation micro-ondes puis filtration du solide, on obtient 95 mg de (5Z)-2-[(2-méthylpropyl)amino]-5-[(5-méthyl-1H-pyrrolo[2,3-b]pyridin-3-yl)méthylidène]-3,5-dihydro-4H-imidazol-4-one sous forme d'une poudre jaune dont les caractéristiques sont les suivantes :
Point de fusion : 338-339°C.
Spectre de RMN ¹H à 400MHz : 0,93 (d, J = 7,0 Hz, 6H) ; 1,94 (m, 1H) ; 2,40 (s, 3H) ; 3,19 (t large, J = 6,0 Hz, 2H) ; 6,58 (s, 1H) ; 7,15 (m étalé, 1H) ; 8,09 (s, 1H) ; 8,11 (s, 1H) ; 8,51 (s large, 1H) ; 11,1 (m étalé, 1H) ; 11,75 (s large, 1H).
HPLC-MS-DAD-ELSD : 298 (+) = (M+H) (+) ; 296 (-) = (M-H) (-).

### Exemple 45

### (5Z)-5-[(5-méthyl-1H-pyrrolo[2,3-b]pyridin-3-yl)méthylidène]-2-pipéridin-1-yl-3,5-dihydro-4H-imidazol-4-one

La (5Z)-5-[(5-méthyl-1H-pyrrolo[2,3-b]pyridin-3-yl)méthylidène]-2-pipéridin-1-yl-3,5-dihydro-4H-imidazol-4-one peut être préparée comme dans l'exemple 7, mais à partir de 240 mg de (5Z)-5-[(5-méthyl-1H-pyrrolo[2,3-b]pyridin-3-yl)méthylidène]-2-thioxoimidazolidin-4-one (exemple 17), de 4 cm³ d'éthanol et de 791 mg de pipéridine. Après quarante minutes à une température de 160°C sous irradiation micro-ondes puis filtration du solide, on obtient 138 mg de (5Z)-5-[(5-méthyl-1H-pyrrolo[2,3-b]pyridin-3-yl)méthylidène]-2-pipéridin-1-yl-3,5-dihydro-4H-imidazol-4-one sous forme d'une poudre jaune dont les caractéristiques sont les suivantes :
Point de fusion : 320-321°C.
Spectre de RMN ¹H à 400MHz : De 1,53 à 1,69 (m, 6H) ; 2,39 (s, 3H) ; 3,58 (m, 4H) ; 6,61 (s, 1H) ; 8,09 (s, 1H) ; 8,14 (s large, 1H) ; 8,39 (m étalé, 1H) ; 11,0 (m très étalé, 1H) ; 11,8 (m étalé, 1H).
HPLC-MS-DAD-ELSD : 310(+)=(M+H)(+); 308(-)=(M-H)(-).

### Exemple 46

### (5Z)-5-[(5-méthyl-1H-pyrrolo[2,3-b]pyridin-3-yl)méthylidène]-2-(4-phénylpipérazin-1-yl)-3,5-dihydro-4H-imidazol-4-one

La (5Z)-5-[(5-méthyl-1H-pyrrolo[2,3-b]pyridin-3-yl)méthylidène]-2-(4-phénylpipérazin-1-yl)-3,5-dihydro-4H-imidazol-4-one peut être préparée comme dans l'exemple 7, mais à partir de 240 mg de (5Z)-5-[(5-méthyl-1H-pyrrolo[2,3-b]pyridin-3-yl)méthylidène]-2-thioxoimidazolidin-4-one (exemple 17), de 4 cm³ d'éthanol et de 1,51 g de 4-phénylpipérazine. Après trente minutes à une température de 160°C sous irradiation micro-ondes, le solide est filtré puis repris dans 50 cm³ d'éthanol, filtré et séché sous vide pour obtenir 166 mg de (5Z)-5-[(5-méthyl-1H-pyrrolo[2,3-b]pyridin-3-yl)méthylidène]-2-(4-phénylpipérazin-1-yl)-3,5-dihydro-4H-imidazol-4-one sous forme d'une poudre jaune dont les caractéristiques sont les suivantes :
Point de fusion : 331-332°C.
Spectre de RMN ¹H à 400MHz : 2,41 (s, 3H) ; 3,27 (m, 4H) ; 3,73 (m, 4H) ; 6,69 (s, 1H) ; 6,82 (t, J = 7,5 Hz, 1H) ; 7,01 (d, J = 7,5 Hz, 2H) ; 7,25 (t, J = 7,5 Hz, 2H) ; 8,10 (s, 1H) ; 8,21 (s, 1H) ; 8,39 (s, 1H) ; 11,15 (m très étalé, 1H) ; 11,9 (s, 1H).
HPLC-MS-DAD-ELSD : 387 (+) = (M+H) (+) ; 385 (-) = (M-H) (-).

### Exemple 47

### (5Z)-2-azépan-1-yl-5-[(5-méthyl-1H-pyrrolo[2,3-b]pyridin-3-yl)méthylidène]-3,5-dihydro-4H-imidazol-4-one

La (5Z)-2-azépan-1-yl-5-[(5-méthyl-1H-pyrrolo[2,3-b]pyridin-3-yl)méthylidène]-3,5-dihydro-4H-imidazol-4-one peut être préparée comme dans l'exemple 7 mais à partir de 240 mg de (5Z)-5-[(5-méthyl-1H-pyrrolo[2,3-b]pyridin-3-yl)méthylidène]-2-thioxoimidazolidin-4-one (exemple 17), de 4 cm³ d'éthanol et de 922 mg d'azépane. Après trente minutes à une température de 160°C sous irradiation micro-ondes puis filtration du solide, on obtient 34 mg de (5Z)-2-azépan-1-yl-5-[(5-méthyl-1H-pyrrolo[2,3-b]pyridin-3-yl)méthylidène]-3,5-dihydro-4H-imidazol-4-one sous forme d'une poudre marron dont les caractéristiques sont les suivantes :
Point de fusion : 324-326°C.
Spectre de RMN ¹H à 400MHz : 1,54 (m, 4H) ; 1,76 (s large, 4H) ; 2,39 (s, 3H) ; 3,62 (s large, 4H) ; 6,57 (s, 1H) ; 8,10 (m, 2H) ; 8,51 (s large, 1H) ; 10,72 (s large, 1H) ; 11,77 (s large, 1H).

### Exemple 48

### (5Z)-2-[(cyclopropylméthyl)amino]-5-{[5-(trifluorométhyl)-1H-pyrrolo[2,3-b]pyridin-3-yl]méthylidène}-3,5-dihydro-4H-imidazol-4-one

La (5Z)-2-[(cyclopropylméthyl)amino]-5-{[5-(trifluorométhyl)-1H-pyrrolo[2,3-b]pyridin-3-yl]méthylidène}-3,5-dihydro-4H-imidazol-4-one peut être préparée comme dans l'exemple 7, mais à partir de 70 mg de (5Z)-2-thioxo-5-{[5-(trifluorométhyl)-1H-pyrrolo[2,3-b]pyridin-3-yl]méthylidène}imidazolidin-4-one (exemple 18), de 3 cm³ d'éthanol et de 159 mg de 1-cyclopropylméthanamine. Après trente minutes à une température de 160°C sous irradiation micro-ondes puis filtration du solide, on obtient 32 mg de (5Z)-2-[(cyclopropylméthyl)amino]-5-{[5-(trifluorométhyl)-1H-pyrrolo[2,3-b]pyridin-3-yl]méthylidène}-3,5-dihydro-4H-imidazol-4-one sous forme d'une poudre jaune dont les caractéristiques sont les suivantes :
Point de fusion : 350°C.
Spectre de RMN ¹H à 400MHz : 0,27 (m, 2H) ; 0,48 (m, 2H) ; 1,13 (m, 1H) ; 3,25 (t, J = 6,0 Hz, 2H) ; 6,62 (s, 1H) ; 7,33 (m étalé, 1H) ; 8,19 (s, 1H) ; 8,57 (s large, 1H) ; 9,71 (m étalé, 1H) ; 10,6 (m étalé, 1H) ; 12,4 (m étalé, 1H).
HPLC-MS-DAD-ELSD : 350(+)=(M+H)(+).

### Exemple 49

### (5Z)-2-azépan-1-yl-5-{[5-(trifluorométhyl)-1H-pyrrolo[2,3-b]pyridin-3-yl]méthylidène}-3,5-dihydro-4H-imidazol-4-one

La (5Z)-2-azépan-1-yl-5-{[5-(trifluorométhyl)-1H-pyrrolo[2,3-b]pyridin-3-yl]méthylidène}-3,5-dihydro-4H-imidazol-4-one peut être préparée comme dans l'exemple 7, mais à partir de 70 mg de (5Z)-2-thioxo-5-([5-(trifluorométhyl)-1H-pyrrolo[2,3-b]pyridin-3-yl]méthylidène}imidazolidin-4-one (exemple 18), de 3 cm³ d'éthanol et de 222 mg d'azépane. Après cinquante minutes à une température de 160°C sous irradiation micro-ondes puis filtration du solide, on obtient 22 mg de (5Z)-2-azépan-1-yl-5-{[5-(trifluorométhyl)-1H-pyrrolo[2,3-b]pyridin-3-yl]méthylidène}-3,5-dihydro-4H-imidazol-4-one sous forme d'une poudre jaune dont les caractéristiques sont les suivantes :
Point de fusion : 340°C.
Spectre de RMN ¹H à 400MHz : 1,53 (m, 4H) ; 1,74 (m large, 4H) ; de 3,40 à 3,90 (m étalé, 4H) ; 6,61 (s, 1H) ; 8,18 (s, 1H) ; 8,56 (s large, 1H) ; 9,77 (m étalé, 1H) ; 11,1 (m étalé, 1H) ; 12,4 (m étalé, 1H).
HPLC-MS-DAD-ELSD : 378 (+) = (M+H) (+); 376(-)=(M-H)(-).

### Exemple 50

### (5Z)-2-[(cyclopropylméthyl)amino]-5-{[5-(phénylamino)-1H-pyrrolo[2,3-b]pyridin-3-yl]méthylidène}-3,5-dihydro-4H-imidazol-4-one

La (5Z)-2-[(cyclopropylméthyl)amino]-5-{[5-(phénylamino)-1H-pyrrolo[2,3-b]pyridin-3-yl]méthylidène}-3,5-dihydro-4H-imidazol-4-one peut être préparée comme dans l'exemple 7, mais à partir de 70 mg de (5Z)-5-[(5-phénylamino-1H-pyrrolo[2,3-b]pyridin-3-yl)méthylène]-2-thioxoimidazolidin-4-one (exemple 21), de 1 cm³ d'éthanol et de 148 mg de 1-cyclopropylméthanamine. Après trente minutes à une température de 160°C sous irradiation micro-ondes le solide jaune précipité dans le mélange réactionnel est filtré pour obtenir 18 mg de (5Z)-5-[(5-phénylamino-1H-pyrrolo[2,3-b]pyridin-3-yl)méthylène]-2-thioxoimidazolidin-4-one sous forme d'une poudre orangée dont les caractéristiques physiques sont les suivantes :
Spectre de RMN ¹H à 400MHz : 0,16 (m, 2H) ; 0,39 (m, 2H) ; 1,01 (m, 1H) ; 3,08 (t, J = 6,0 Hz, 2H) ; 6,54 (s, 1H) ; 6,71 (t, J = 7,5 Hz, 1H) ; 6,93 (d, J = 7,5 Hz, 2H) ; 7,03 (m étalé, 1H) ; 7,18 (t, J = 7,5 Hz, 2H) ; 7,91 (s, 1H) ; 8,05 (s, 1H) ; 8,11 (s, 1H) ; 8,50 (s, 1H) ; 10,3 (m étalé, 1H) ; 11,8 (m étalé, 1H).
LC-MS (1) : temps de rétention 3.55 mn ; HPLC-MS-DAD-ELSD : 371 (+) = (M+H) (+) ; 373 (-) = (M-H) (-).

### Exemple 51

### (5Z)-2-[(cyclopropylméthyl)amino]-5-[(5-morpholin-4-yl-1H-pyrrolo[2,3-b]pyridin-3-yl)méthylidène]-3,5-dihydro-4H-imidazol-4-one

La (5Z)-2-[(cyclopropylméthyl)amino]-5-[(5-morpholin-4-yl-1H-pyrrolo[2,3-b]pyridin-3-yl)méthylidène]-3,5-dihydro-4H-imidazol-4-one peut être préparée comme dans l'exemple 7, mais à partir de 160 mg de (5Z)-5-[(5-morpholin-4-yl-1H-pyrrolo[2,3-b]pyridin-3-yl)méthylidène]-2-thioxoimidazolidin-4-one (exemple 22), 2 cm³ d'éthanol et de 345 mg de 1-cyclopropylméthanamine. Après une heure à une température de 160°C sous irradiation micro-ondes le solide jaune précipité dans le mélange réactionnel est filtré pour obtenir 100 mg de (5Z)-2-[(cyclopropylméthyl)amino]-5-[(5-morpholin-4-yl-1H-pyrrolo[2,3-b]pyridin-3-yl)méthylidène]-3,5-dihydro-4H-imidazol-4-one sous forme d'une poudre jaune ont les caractéristiques physiques sont les suivantes :
Point de fusion : 331°C.
Spectre de RMN ¹H à 400MHz : 0,29 (m, 2H) ; 0,48 (m, 2H) ; 1,12 (m, 1H) ; 3,13 (m, 4H) ; 3,22 (t, J = 6,0 Hz, 2H) ; 3,79 (m, 4H) ; 6,61 (s 1H) ; 7,11 (m large, 1H) ; 8,08 (m, 2H) ; 8,38 (s large, 1H) ; 10,3 (m étalé, 1H) ; 11,7 (s large, 1H).
HPLC-MS-DAD-ELSD : 367=(M+H)(+); 365(-)=(M-H)(-).

### Exemple 52

### (5Z)-2-[(cyclopropylméthyl)amino]-5-[(5-thiophén-3-yl-1H-pyrrolo[2,3-b]pyridin-3-yl)méthylidène]-3,5-dihydro-4H-imidazol-4-one

La (5Z)-2-[(cyclopropylméthyl)amino]-5-[(5-thiophén-3-yl-1H-pyrrolo[2,3-b]pyridin-3-yl)méthylidène]-3,5-dihydro-4H-imidazol-4-one peut être préparée de la manière suivante :
Dans un réacteur pour four micro-ondes, sont introduits 150 mg de (5Z)-5-[(5-bromo-1H-pyrrolo[2,3-b]pyridin-3-. yl)méthylène]-2-[(cyclopropylméthyl)amino]-3,5-dihydro-4H-imidazol-4-one (exemple 33), 4,8 cm³ de dioxane, 1,2 cm³ d'eau, 80 mg d' acide thiophène-3-boronique, 540 mg de carbonate de césium et 15 mg de 1,1'-bis(diphénylphosphino)-ferrocènedichloropalladium(II). Le réacteur est scellé et mis sous irradiation pendant 2 fois trente minutes à 145°C. Après rajout de 80 mg d' acide thiophène-3-boronique et 14 mg de catalyseur, le mélange réactionnel est à nouveau irradié pendant 30 minutes à 150°C. Le mélange réactionnel est concentré sous vide, repris par 20 cm³ d'eau et 20 cm³ d'acétate d'éthyle. On filtre 85 mg d'un insoluble. Le filtrat est décanté et réextrait avec de l'acétate d'éthyle. Les phases organiques sont sèchées sur sulfate de magnésium puis concentrées sous vide pour donner 92 mg d'un solide marron. Les deux résidus solides isolés contenant le produit attendu sont rassemblés et purifiés par LC-MS préparative [Colonne Xterra RP18 30x100 ; 5µ ; avec eau tamponnée à 10 mM d'hydrogénocarbonate d'ammonium ajustée à pH 9 avec de l'ammoniaque / Acétonitrile avec gradient 70/30 à 0/100 en 8 min] pour obtenir 25 mg de (5Z)- (5Z)-2-[(cyclopropylméthyl)amino]-5-[(5-thiophén-3-yl-1H-pyrrolo[2,3-b]pyridin-3-yl)méthylidène]-3,5-dihydro-4H-imidazol-4-one sous forme de poudre marron dont les caractéristiques physiques sont les suivantes :
Spectre RMN ¹H à 400MHz : 0,29 (m, 2H) ; 0,49 (m, 2H) ; 1,16 (m, 1H) ; 3,27 (m partiellement masqué, 2H) ; 6,71 (s, 1H) ; 7,19 (m large, 1H) ; 7,69 (m, 2H) ; 7,91 (s large, 1H) ; 8,19 (s large, 1H) ; 8,64 (d, J = 2,0 Hz, 1H) ; 9,07 (s large, 1H) ; 10,4 (m étalé, 1H) ; 11, 95 (m étalé, 1H).
HPLC-MS-DAD-ELSD : 358 (+) = (M+H) (+); 356 (-) = (M-H) (-).

### Exemple 53

### (5Z)-2-[(cyclopropylméthyl)amino]-5-{[5-(4-pipérazin-1-ylphényl)-1H-pyrrolo[2,3-b]pyridin-3-yl]méthylidène}-3,5-dihydro-4H-imidazol-4-one

a) La (5Z)-2-[(cyclopropylméthyl)amino]-5-{[5-(4-pipérazin-1-ylphényl)-1H-pyrrolo[2,3-b]pyridin-3-yl]méthylidène}-3,5-dihydro-4H-imidazol-4-one peut être préparée de la manière suivante :
   140 mg de 4-(4-{3-[(Z)-{2-[(cyclopropylméthyl)amino]-5-oxo-1,5-dihydro-4H-imidazol-4-ylidène}méthyl]-1H-pyrrolo[2,3-b]pyridin-5-yl}phényl)pipérazine-1-carboxylate de 1,1-diméthyléthyle dans 3 cm³ d'une solution 4M d'acide chlorhydrique dans le dioxane sont agités à l'ambiante pendant deux heures. Le précipité formé est filtré puis purifié sur 5 g de colonne SCX avec une élution avec du méthanol puis une solution 2M d'ammoniac dans le méthanol, pour obtenir 22 mg de (5Z)-2-[(cyclopropylméthyl)amino]-5-{[5-(4-pipérazin-1-ylphényl)-1H-pyrrolo[2,3-b]pyridin-3-yl]méthylidène}-3,5-dihydro-4H-imidazol-4-one sous forme d'une poudre jaune dont les caractéristiques physiques sont les suivantes :
      Spectre de RMN ¹H à 400MHz : 0,25 (m, 2H) ; 0,45 (m, 2H) ; 1,12 (m, 1H) ; 2,91 (m, 4H) ; 3,13 (m, 4H) ; de 3,20 à 3,40 (m partiellement masqué, 2H) ; 6,69 (s, 1H) ; 7,01 (d, J = 8,5 Hz, 2H) ; 7,15 (m étalé, 1H) ; 7,61 (d, J = 8,5 Hz, 2H) ; 8,13 (s, 1H) ; 8,48 (d, J = 1,5 Hz, 1H) ; 9,05 (s large, 1H) ; 10,35 (m étalé, 1H) ; 11,95 (s large, 1H).
      HPLC-MS-DAD-ELSD : 442(+)=(M+H)(+).
b) Le 4-(4-{3-[(Z)-{2-[(cyclopropylméthyl)amino]-5-oxo-1,5-dihydro-4H-imidazol-4-ylidène}méthyl]-1H-pyrrolo[2,3-b]pyridin-5-yl}phényl)pipérazine-1-carboxylate de 1,1-diméthyléthyle peut être préparé comme dans l'exemple 52, mais à partir de 80 mg (5Z)-5-[(5-bromo-1H-pyrrolo [2,3-b]pyridin-3-yl)méthylène]-2-[(cyclopropylméthyl)amino]-3,5-dihydro-4H-imidazol-4-one (exemple 33) dans 2,4 cm³ de dioxane et 0,8 cm³ d'eau, de 100 mg d'acide (4-{4-[(1,1-diméthyléthoxy)carbonyl]pipérazin-1-yl}phényl)boronique, 280 mg de carbonate de césium et de 15 mg de de 1,1'-Bis(diphénylphosphino) ferrocènedichloropalladium (II). On obtient 110 mg de 4-(4-{3-[(Z)-{2-[(cyclopropylméthyl)amino]-5-oxo-1,5-dihydro-4H-imidazol-4-ylidène}méthyl]-1H-pyrrolo[2,3-b]pyridin-5-yl}phényl)pipérazine-1-carboxylate de 1,1-diméthyléthyle sous forme de poudre dont les caractéristiques physiques sont les suivantes :
   Spectre RMN de ¹H à 400MHz : 0,25 (s large, 2H) ; 0,44 (s large, 2H) ; 1,12 (s large, 1H) ; 1,45 (s, 9H) ; 3,17 (m, 4H) ; 3,27 (partiellement masque, 2H) ; 3,49 (m, 4H) ; 6,68 (s, 1H) ; 7,06 (d large , J = 7,8 Hz, 2H) ; 7,17 (s large, 1H) ; 7,64 (d large, J = 7,8 Hz, 2H) ; 8,14 (s, 1H) ; 8,49 (s, 1H) ; 9,05 (s large, 1H) ; 10,40 (s large, 1H) ; 11,94 (s large, 1H).
   Rf : 0.24 (éluant dichlorométhane/acétate d'éthyle 90/10).
   LC-MS-DAD-ELSD : 540 (-) = (M-H) (-) ; 542 (+) = (M+H) (+).

### Exemple 54

### (5Z)-5-[(5-benzyl-1H-pyrrolo[2,3-b]pyridin-3-yl)méthylidène]-2-[(cyclopropylméthyl)amino]-3,5-dihydro-4H-imidazol-4-one

La (5Z)-5-[(5-benzyl-1H-pyrrolo[2,3-b]pyridin-3-yl)méthylidène]-2-[(cyclopropylméthyl)amino]-3,5-dihydro-4H-imidazol-4-one peut être préparée comme dans l'exemple 7, mais à partir de 60 mg de (5Z)-5-[(5-benzyl-1H-pyrrolo[2,3-b]pyridin-3-yl)méthylidène]-2-thioxoimidazolidin-4-one (exemple 23), de 1 cm³ d'éthanol et de 13 mg de 1-cyclopropylméthanamine. Après trente minutes à une température de 160°C sous irradiation micro-ondes puis filtration du solide, on obtient 31 mg de (5Z)-5-[(5-benzyl-1H-pyrrolo[2,3-b]pyridin-3-yl)méthylidène]-2-[(cyclopropylméthyl)amino]-3,5-dihydro-4H-imidazol-4-one sous forme d'une poudre jaune dont les caractéristiques sont les suivantes :
Point de fusion : 315-318°C.
Spectre de RMN ¹H à 400MHz : 0,28 (m, 2H) ; 0,47 (m, 2H) ; 1,14 (m, 1H) ; 3,21 (m, 2H) ; 4,03' (s, 2H) ; 6,56 (s, 1H) ; de 7,11 à 7,33 (m, 5H) ; 8,11 (s, 1H) ; 8,18 (s, 1H) ; 8,58 (s, 1H) ; 10,1 (m étalé, 1H) ; 11,85 (m étalé, 1H).
UPLC-MS-DAD-ELSD : 372(+)=(M+H)(+); 370(-)=(M-H)(-).

### Exemple 55

### (5Z)-2-amino-3-[3-(diméthylamino)propyl]-5-(1H-pyrrolo[2,3-b]pyridin-3-ylméthylidène)-3,5-dihydro-4H-imidazol-4-one

La (5Z)-2-amino-3-[3-(diméthylamino)propyl]-5-(1H-pyrrolo[2,3-b]pyridin-3-ylméthylidène)-3,5-dihydro-4H-imidazol-4-one peut être préparée comme dans l'exemple 7, mais à partir de 351 mg de (5Z)-5-[(1H-pyrrolo[2,3-b]pyridin-3-yl)méthylène]-2-thioxoimidazolidin-4-one (exemple 1), de 2,5 cm³ d'éthanol et de 1,47 g de 3-diméthylamino-propylamine. Après trente minutes à une température de 150°C sous irradiation micro-ondes puis concentration sous vide et reprise du residu dans le chlorure de méthylène, la filtration du solide donne 8 mg de (5Z)-2-amino-3-[3-(diméthylamino)propyl]-5-(1H-pyrrolo[2,3-b]pyridin-3-ylméthylidène)-3,5-dihydro-4H-imidazol-4-one sous forme d'une poudre jaune dont les caractéristiques sont les suivantes :
Spectre de RMN ¹H à 400MHz : 1,67 (m, 2H) ; 2,12 (s, 6H) ; 2,20 (t, J = 7,0 Hz, 2H) ; 3,56 (t, J = 7,0 Hz, 2H) ; 6,71 (s, 1H) ; 7,11 (dd, J = 5,0 et 8,0 Hz, 1H) ; 7,33 (s large, 2H) ; 8,24 (m, 2H) ; 8,59 (dd, J = 1,5 et 8,0 Hz, 1H) ; 12,0 (s large, 1H).
UPLC-MS-DAD-ELSD : 313(+)=(M+H)(+); 311(-)=(M-H)(-).

### Exemple 56

### (5Z)-2-amino-3-(3-morpholin-4-ylpropyl)-5-(1H-pyrrolo[2,3-b]pyridin-3-ylméthylidène)-3,5-dihydro-4H-imidazol-4-one

La (5Z)-2-amino-3-(3-morpholin-4-ylpropyl)-5-(1H-pyrrolo[2,3-b]pyridin-3-ylméthylidène)-3,5-dihydro-4H-imidazol-4-one peut être préparée comme dans l'exemple 7, mais à partir de 310 mg de (5Z)-5-[(1H-pyrrolo[2,3-b]pyridin-3-yl)méthylène]-2-thioxoimidazolidin-4-one (exemple 1), de 2,5 cm³ d'éthanol et de 310 mg de 3-morpholinopropylamine. Après cinquante minutes à une température de 160°C sous irradiation micro-ondes puis filtration du solide, on obtient 86 mg de (5Z)-2-amino-3-(3-morpholin-4-ylpropyl)-5-(1H-pyrrolo[2,3-b]pyridin-3-ylméthylidène)-3,5-dihydro-4H-imidazol-4-one sous forme d'une poudre jaune dont les caractéristiques sont les suivantes :
Spectre de RMN ¹H à 400MHz : 1,67 (m, 2H) ; 2.2-2.4 (m, 6H) ; 3,6 (m, 6H) ; 6,7 (s, 1H) ; 7,15 (dd, J = 5,0 et 8,0 Hz, 1H) ; 7,35 (s large, 2H) ; 8,25 (m, 2H) ; 8,6 (dd, J = 1,5 et 8,0 Hz, 1H) ; 12,0 (s large, 1H).
HPLC-MS-DAD-ELSD : 355(+)=(M+H)(+).

### Exemple 57

### (5Z)-2-amino-3-(3-éthoxypropyl)-5-(1H-pyrrolo[2,3-b]pyridin-3-ylméthylidène)-3,5-dihydro-4H-imidazol-4-one

La (5Z)-2-amino-3-(3-éthoxypropyl)-5-(1H-pyrrolo[2,3-b]pyridin-3-ylméthylidène)-3,5-dihydro-4H-imidazol-4-one peut être préparée comme dans l'exemple 7, mais à partir de 350 mg de (5Z)-5-[(1H-pyrrolo[2,3-b]pyridin-3-yl)méthylène]-2-thioxoimidazolidin-4-one (exemple 1), de 2,5 cm³ d'éthanol et de 350 mg de 3-éthoxypropylamine. Après cinquante minutes à une température de 160°C sous irradiation micro-ondes puis filtration du solide, on obtient 28 mg de (5Z)-2-amino-3-(3-éthoxypropyl)-5-(1H-pyrrolo[2,3-b]pyridin-3-ylméthylidène)-3,5-dihydro-4H-imidazol-4-one sous forme d'une poudre jaune dont les caractéristiques sont les suivantes :
Spectre de RMN ¹H à 400MHz : 1.12 (t, J = 7 Hz, 3H) ; 1,67 (m, 2H) ; 3.3-3.5 (m, 4H) ; 3,65 (t, J = 7 Hz, 2H) ; 6,72 (s, 1H) ; 7,15 (dd, J = 5,0 et 8,0 Hz, 1H) ; 7,25 (s large, 2H) ; 8,25 (s large, 2H) ; 8,6 (dd, J = 1,5 et 8,0 Hz, 1H) ; 12,0 (s large, 1H).
HPLC-MS-DAD-ELSD : 314 (+) = (M+H) (+); 312 (-) = (M-H) (-).

### Exemple 58

### (5Z)-2-amino-3-(3-pyrrolidin-1-ylpropyl)-5-(1H-pyrrolo[2,3-b]pyridin-3-ylméthylidène)-3,5-dihydro-4H-imidazol-4-one

La (5Z)-2-amino-3-(3-pyrrolidin-1-ylpropyl)-5-(1H-pyrrolo[2,3-b]pyridin-3-ylméthylidène)-3,5-dihydro-4H-imidazol-4-one peut être préparée comme dans l'exemple 7, mais à partir de 366 mg de (5Z)-5-[(1H-pyrrolo[2,3-b]pyridin-3-yl)méthylène]-2-thioxoimidazolidin-4-one (exemple 1), de 2,5 cm³ d'éthanol et de 1,92 g de 3-(1-pyrrolidyl)propylamine. Après cinquante minutes à une température de 160°C sous irradiation micro-ondes puis filtration du solide, on obtient 58 mg de (5Z)-2-amino-3-(3-pyrrolidin-1-ylpropyl)-5-(1H-pyrrolo[2,3-b]pyridin-3-ylméthylidène)-3,5-dihydro-4H-imidazol-4-one sous forme d'une poudre jaune dont les caractéristiques sont les suivantes :
Spectre de RMN ¹H à 400MHz : 1,69 (m, 6H) ; 2,39 (t, J = 6,5 Hz, 2H) ; 2,42 (m, 4H) ; 3,59 (t, J = 6,5 Hz, 2H) ; 6,71 (s, 1H) ; 7,11 (dd, J = 5,0 et 8,0 Hz, 1H) ; 7,40 (s large, 2H) ; 8,23 (m, 2H) ; 8,59 (d large, J = 8,0 Hz, 1H) ; 12,0 (m étalé, 1H).
HPLC-MS-DAD-ELSD : 339 (+) = (M+H) (+) ; 337 (-) = (M-H) (-).

### Exemple 59

### (5Z)-2-[(cyclopropylméthyl)amino]-3-phényl-5-(1H-pyrrolo[2,3-b]pyridin-3-ylméthylidène)-3,5-dihydro-4H-imidazol-4-one

La (5Z)-2-[(cyclopropylméthyl)amino]-3-phényl-5-(1H-pyrrolo[2,3-b]pyridin-3-ylméthylidène)-3,5-dihydro-4H-imidazol-4-one peut être préparée comme dans l'exemple 7, mais à partir de 128 mg de (5Z/E)-3-phényl-5-(1H-pyrrolo[2,3-b]pyridin-3-ylméthylidène)-2-thioxoimidazolidin-4-one (exemple 24), de 3,5 cm³ d'éthanol et de 284 mg de 1-cyclopropylméthanamine. Après trente minutes à une température de 160°C sous irradiation micro-ondes et purification par LC-MS préparative [Colonne Xterra RP18 30x100 ; 5µ ; avec eau tamponnée à 10 mM d'hydrogénocarbonate d'ammonium ajustée à pH 9 avec de l'ammoniaque / Acétonitrile avec gradient 70/30 à 0/100 en 8 min], on obtient 34 mg de (5Z)-2-[(cyclopropylméthyl)amino]-3-phényl-5-(1H-pyrrolo[2,3-b]pyridin-3-ylméthylidène)-3,5-dihydro-4H-imidazol-4-one sous forme de lyophilisat jaune dont les caractéristiques sont les suivantes :
Spectre de RMN ¹H à 400MHz : 0,31 (m, 2H) ; 0,47 (m, 2H) ; 1,23 (m, 1H) ; 3,30 (m partiellement masqué, 2H) ; 6,84 (s, 1H) ; 7,00 (t, J = 6, 0 Hz, 1H) ; 7, 13 (dd, J = 5,0 et 8,0 Hz, 1H) ; 7,35 (d, J = 7,5 Hz, 2H) ; 7,50 (t, J = 7,5 Hz, 1H) ; 7,58 (t, J = 7,5 Hz, 2H) ; 8,27 (dd, J = 1,5 et 5,0 Hz, 1H) ; 8,31 (d, J = 2,0 Hz, 1H) ; 8,67 (d large, J = 8,0 Hz, 1H) ; 12,0 (s large, 1H).
HPLC-MS-DAD-ELSD : 358(+)=(M+H)(+); 356(-)=(M-H)(-).

### Exemple 60

### (5Z)-5-[(5-chloro-1H-pyrrolo[2,3-b]pyridin-3-yl)méthylidène]-2-(méthylsulfanyl)-3,5-dihydro-4H-imidazol-4-one

La (5Z)-5-[(5-chloro-1H-pyrrolo[2,3-b]pyridin-3-yl)méthylidène]-2-(méthylsulfanyl)-3,5-dihydro-4H-imidazol-4-one peut être préparée de la manière suivante :
A un mélange de 200 mg de (5Z)-5-[(5-chloro-1H-pyrrolo[2,3-b]pyridin-3-yl)méthylène]-2-thioxoimidazolidin-4-one (exemple 3) et de 122 mg d'iodure de méthyle dans 7 cm³ de méthanol à 0°C est ajouté lentement 0,86 cm³ d'une solution molaire de soude. Le mélange est ensuite agité à l'ambiante pendant deux heures. Le solide formé est filtré puis séché sous vide pour obtenir 175 mg de (5Z)-5-[(5-chloro-1H-pyrrolo[2,3-b]pyridin-3-yl)méthylidène]-2-(méthylsulfanyl)-3,5-dihydro-4H-imidazol-4-one sous forme de poudre jaune dont les caractéristiques sont les suivantes :
   Point de fusion : >410°C
   Spectre de RMN ¹H à 400MHz : 2,71 (s, 3H) ; 7,03 (s, 1H) ; 8,29 (d, J = 2,0 Hz, 1H) ; 8,37 (s, 1H) ; 9,31 (s large, 1H) ; de 11,4 à 12,7 (m très étalé, 2H).
   HPLC-MS-DAD-ELSD : 293 (+) /...= (M+H) (+) /...; 291 (-) /...= (M-H) (-) /... (1 atome de chlore Cl présent).

### Exemple 61

### (5Z)-2-(benzylsulfanyl)-5-[(5-chloro-1H-pyrrolo[2,3-b]pyridin-3-yl)méthylidène]-3,5-dihydro-4H-imidazol-4-one

La (5Z)-2-(benzylsulfanyl)-5-[(5-chloro-1H-pyrrolo[2,3-b]pyridin-3-yl)méthylidène]-3,5-dihydro-4H-imidazol-4-one peut être préparée comme dans l'exemple 60, mais à partir de 400 mg de (5Z)-5-[(5-chloro-1H-pyrrolo[2,3-b]pyridin-3-yl)méthylène]-2-thioxoimidazolidin-4-one (exemple 3), de 270 mg de bromure de benzyle, de 14 cm³ de méthanol à 0°C et de 1,8 cm³ d'une solution molaire de soude. Après deux heures à l'ambiante, le solide formé est filtré pour obtenir 452 mg de (5Z)-2-(benzylsulfanyl)-5-[(5-chloro-1H-pyrrolo[2,3-b]pyridin-3-yl)méthylidène]-3,5-dihydro-4H-imidazol-4-one sous forme de poudre jaune dont les caractéristiques sont les suivantes :
Point de fusion : 313-315°C.
Spectre de RMN ¹H à 400MHz : 4,63 (s, 2H) ; 7,10 (s, 1H) ; de 7,23 à 7,40 (m, 3H) ; 7,51 (d large, J = 7,5 Hz, 2H) ; 8,29 (d, J = 2,5 Hz, 1H) ; 8,43 (s, 1H) ; 9,20 (d, J = 2,5 Hz, 1H) ; de 11,6 à 12,7 (m très étalé, 2H).
HPLC-MS-DAD-ELSD : 369 (+)/ ... = (M+H) (+) / ... (1 atome de chlore Cl présent).

### Exemple 62

### (5Z)-5-[(5-chloro-1H-pyrrolo[2,3-b]pyridin-3-yl)méthylidène]-2-(propylsulfanyl)-3,5-dihydro-4H-imidazol-4-one

La (5Z)-5-[(5-chloro-1H-pyrrolo[2,3-b]pyridin-3-yl)méthylidène]-2-(propylsulfanyl)-3,5-dihydro-4H-imidazol-4-one peut être comme dans l'exemple 60, mais à partir de 200 mg de (5Z)-5-[(5-chloro-1H-pyrrolo[2,3-b]pyridin-3-yl)méthylène]-2-thioxoimidazolidin-4-one (exemple 3), de 146 mg d'iodo-1-propane, de 7 cm³ de méthanol à 0°C et de 0,86 cm³ d'une solution molaire de soude. Après agitation à l'ambiante pendant une nuit, le solide formé est filtré pour obtenir 172 mg de (5Z)-5-[(5-chloro-1H-pyrrolo[2,3-b]pyridin-3-yl)méthylidène]-2-(propylsulfanyl)-3,5-dihydro-4H-imidazol-4-one sous forme de poudre jaune dont les caractéristiques sont les suivantes :
Point de fusion : 316-318°C.
Spectre de RMN ¹H à 400MHz : 1,05 (t, J = 7,6 Hz, 3H) ; 1,83 (sext, J = 7,6 Hz, 2H) ; 3,33 (masqués, 2H) ; 7,03 (s, 1H) ; 8,29 (d, J = 2,4 Hz, 1H) ; 8,33 (s, 1H) ; 9,28 (d, J = 2,4 Hz, 1H) ; 12,10 (s large, 1H).

### Exemple 63

### (5Z)-5-[(5-chloro-1H-pyrrolo[2,3-b]pyridin-3-yl)méthylidène]-2-((1-méthyl)éthylsulfanyl)-3,5-dihydro-4H-imidazol-4-one

La (5Z)-5-[(5-chloro-1H-pyrrolo[2,3-b]pyridin-3-yl)méthylidène]-2-((1-méthyl)éthylsulfanyl)-3,5-dihydro-4H-imidazol-4-one peut être préparée comme dans l'exemple 60, mais à partir de 200 mg de (5Z)-5-[(5-chloro-1H-pyrrolo[2,3-b]pyridin-3-yl)méthylène]-2-thioxoimidazolidin-4-one (exemple 3), de 146 mg d'iodo-2-propane, de 7 cm³ de méthanol à 0°C et de 0,86 cm³ d'une solution molaire de soude. Le mélange est agité à 50°C pendant la nuit puis à 70°C pendant un jour suplémentaire. Le solide formé est alors filtré pour obtenir 90 mg de (5Z)-5-[(5-chloro-1H-pyrrolo[2,3-b]pyridin-3-yl)méthylidène]-2-((1-méthyl)éthylsulfanyl)-3,5-dihydro-4H-imidazol-4-one sous forme de poudre jaune dont les caractéristiques sont les suivantes :
Point de fusion : 291-293°C.
Spectre de RMN ¹H à 400MHz : 1,52 (d, J = 6,9 Hz, 6H) ; 4,13 (m, 1H) ; 7,03 (s, 1H) ; 8,29 (d, J = 2,4 Hz, 1H) ; 8,32 (s, 1H) ; 9,33 (d, J = 2,4 Hz, 1H) ; 12,05 (s large, 1H).

### Exemple 64

### (5Z)-2-butyl-5-[(5-chloro-1H-pyrrolo[2,3-b]pyridin-3-yl)méthylidène]-3,5-dihydro-4H-imidazol-4-one

La (5Z)-2-butyl-5-[(5-chloro-1H-pyrrolo[2,3-b]pyridin-3-yl)méthylidène]-3,5-dihydro-4H-imidazol-4-one peut être préparée comme dans l'exemple 13, mais à partir de 131 mg de 2-butyl-imidazolidin-4-one brut et de 135 mg de 5-chloro-1H-pyrrolo[2,3-b]pyridine-3-carbaldéhyde pour donner 164 mg de (5Z)-2-butyl-5-[(5-chloro-1H-pyrrolo[2,3-b]pyridin-3-yl)méthylidène]-3,5-dihydro-4H-imidazol-4-one sous forme d'une poudre jaune dont les caractéristiques sont les suivantes :
Point de fusion : 288-290°C.
Spectre de RMN ¹H à 400MHz : 0,97 (t, J = 7,5 Hz, 3H) ; 1,42 (m, 2H) ; 1,78 (m, 2H) ; 2,57 (t, J = 7,5 Hz, 2H) ; 7,13 (s, 1H) ; 8,29 (d, J = 2,5 Hz, 1H) ; 8,33 (s, 1H) ; 9,38 (d, J = 2,5 Hz, 1H) ; 11,1 (s large, 1H) ; 12,5 (m étalé, 1H).
HPLC-MS-DAD-ELSD : 303(+)=(M+H)(+).

### Exemple 65

### (5Z)-2-butyl-5-[(6-chloro-1H-pyrrolo[2,3-b]pyridin-3-yl)méthylidène]-3,5-dihydro-4H-imidazol-4-one

La (5Z)-2-butyl-5-[(6-chloro-1H-pyrrolo[2,3-b]pyridin-3-yl)méthylidène]-3,5-dihydro-4H-imidazol-4-one peut être préparée comme dans l'exemple 13, mais à partir de 87 mg de 2-butyl-imidazolidin-4-one brut et de 90 mg de 6-chloro-1H-pyrrolo[2,3-b]pyridine-3-carbaldéhyde pour donner 85 mg de (5Z)-2-butyl-5-[(6-chloro-1H-pyrrolo[2,3-b]pyridin-3-yl)méthylidène]-3,5-dihydro-4H-imidazol-4-one sous forme d'une poudre jaune dont les caractéristiques sont les suivantes :
Point de fusion : 318-321°C.
Spectre de RMN ¹H à 400MHz : 0,94 (t, J = 7,5 Hz, 3H) ; 1,40 (m, 2H) ; 1,71 (m, 2H) ; 2,54 (t, J = 7,5 Hz, 2H) ; 7,13 (s, 1H) ; 7,24 (d, J = 8,0 Hz, 1H) ; 8,31 (s, 1H) ; 9,07 (d, J = 8,0 Hz, 1H) ; 11,1 (s large, 1H) ; 12,5 (m étalé, 1H).
SM-EI: 302(+.)=(M)(+.); 260 (+) = (M-C3H6) (+).

### Exemple 66

### (5Z)-2-butyl-3-méthyl-5-(1H-pyrrolo[2,3-b]pyridin-3-ylméthylidène)-3,5-dihydro-4H-imidazol-4-one

a) La (5Z)-2-butyl-3-méthyl-5-(1H-pyrrolo[2,3-b]pyridin-3-ylméthylidène)-3,5-dihydro-4H-imidazol-4-one peut être préparée comme dans l'exemple 13, mais à partir de 0,63 g de 2-butyl-3-méthyl-3,5-dihydro-4H-imidazol-4-one, de 200 mg de 1H-pyrrolo[2,3-b]pyridine-3-carbaldéhyde et de 0,14 cm³ de piperidine dans 10 cm³ d'éthanol. Après une heure au reflux, le mélange est refroidi à l'ambiante et le solide jaune est filtré, lavé avec un peu d'éthanol puis séché sous vide pour donner 323 mg de (5Z)-2-butyl-3-méthyl-5-(1H-pyrrolo[2,3-b]pyridin-3-ylméthylidène)-3,5-dihydro-4H-imidazol-4-one sous forme d'une poudre jaune dont les caractéristiques sont les suivantes :
   Point de fusion : 220-222°C.
   Spectre de RMN ¹H à 400MHz : 0,99 (t, J = 7,0 Hz, 3H) ; 1,50 (m, 2H) ; 1,80 (m, 2H) ; 2,68 (t, J = 7,0 Hz, 2H) ; 3,10 (s, 3H) ; 7,19 (dd, J = 5,0 et 8,0 Hz, 1H) ; 7,29 (s, 1H) ; 8,29 (dd, J = 1,5 et 5,0 Hz, 1H) ; 8,38 (s, 1H) ; 9,01 (d large, J = 8,0 Hz, 1H) ; 12,4 (m étalé, 1H).
   HPLC-MS-DAD-ELSD : 283(+)=(M+H)(+); 281(-)=(M-H)(-).
b) La 2-butyl-3-méthyl-3,5-dihydro-4H-imidazol-4-one peut être préparée de la manière suivante :
   Une solution 2M de méthylamine dans le THF (12 cm³) est ajoutée lentement à une solution de 1,99 g de N-(1-méthoxypentylidène)glycinate de méthyle dans 20 cm³ de méthanol. Le mélange est agité à l'ambiante pendant trois heures, puis concentré sous vide pour obtenir 1,63 g de 2-butyl-3-méthyl-3,5-dihydro-4H-imidazol-4-one brute qui est utilisée telle quelle dans l'étape suivante.
c) Le N-(1-méthoxypentylidène)glycinate de méthyle peut être préparée de la manière suivante :
   Une suspension de 2 g de chlorhydrate de pentanimidoate de méthyle et de 1,67 g de chlorhydrate de méthyl glycinate dans 20 cm³ de chlorure de méthylène est agitée à 0°C pendant cinq heures, puis 1,8 cm³ de triéthylamine est ajouté. Le mélange résultant est agité à l'ambiante pendant une heure, dilué avec 10 cm³ de tampon phosphate pH 7, puis extrait avec du chlorure de méthylène (3x20 cm³). Les phases organiques combinées sont séchées sur sulfate de magnésium et concentrées sous vide pour obtenir 1,99 g de N-(1-méthoxypentylidène)glycinate de méthyle.

### Exemple 67

### (5Z)-2-(2-phényléthyl)-5-(1H-pyrrolo[2,3-b]pyridin-3-ylméthylidène)-3,5-dihydro-4H-imidazol-4-one

a) La (5Z)-2-(2-phényléthyl)-5-(1H-pyrrolo[2,3-b]pyridin-3-ylméthylidène)-3,5-dihydro-4H-imidazol-4-one peut être préparée comme dans l'exemple 13, mais à partir de 1 g de 2-(2-phényléthyl)-3,5-dihydro-4H-imidazol-4-one brut et 0,2 g de 1H-pyrrolo[2,3-b]pyridine-3-carbaldéhyde pour donner 244 mg de (5Z)-2-(2-phényléthyl)-5-(1H-pyrrolo[2,3-b]pyridin-3-ylméthylidène)-3,5-dihydro-4H-imidazol-4-one sous forme d'une poudre jaune dont les caractéristiques sont les suivantes :
   Point de fusion : 270-272°C.
   Spectre de RMN ¹H à 400MHz : 2,88 (t, J = 7,5 Hz, 2H) ; 3,09 (t, J = 7,5 Hz, 2H) ; 7,18 (s, 1H) ; 7,20 (m, 2H) ; 7,31 (m, 4H) ; 8,29 (dd, J = 1,5 et 5,0 Hz, 1H) ; 8,35 (s, 1H) ; 8,89 (d large, J = 8,0 Hz, 1H) ; 11,2 (m étalé, 1H) ; 12,4 (m très étalé, 1H).
   HPLC-MS-DAD-ELSD : 317(+)=(M+H)(+); 315(-)=(M-H)(-).
b) La 2-(2-phényléthyl)-3,5-dihydro-4H-imidazol-4-one peut être préparée comme dans l'exemple 13, mais à partir de 2 g de chlorhydrate de 3-phénylpropanimidoate de méthyle pour obtenir 2,85 g de 2-(2-phényléthyl)-3,5-dihydro-4H-imidazol-4-one brut.
c) Le chlorhydrate de 3-phénylpropanimidoate de méthyle peut être préparé comme dans l'exemple 13, mais à partir de 5 cm³ de 2-phényl-ethyl carbonitrile, de 1,7 cm³ de méthanol et de 4 cm³ d'éther. On obtient 7,5 g de chlorhydrate de 3-phénylpropanimidoate de méthyle sous forme de solide blanc.

### Exemple 68

### (5Z)-2-(3-méthylbutyl)-5-(1H-pyrrolo[2,3-b]pyridin-3-ylméthylidène)-3,5-dihydro-4H-imidazol-4-one

a) La (5Z)-2-(3-méthylbutyl)-5-(1H-pyrrolo[2,3-b]pyridin-3-ylméthylidène)-3,5-dihydro-4H-imidazol-4-one peut être préparée comme dans l'exemple 13, mais à partir de 105 mg de 2-(3-méthylbutyl)-3,5-dihydro-4H-imidazol-4-one brut et de 0,2 g de 1H-pyrrolo[2,3-b]pyridine-3-carbaldéhyde pour donner 64 mg de (5Z)-2-(3-méthylbutyl)-5-(1H-pyrrolo[2,3-b]pyridin-3-ylméthylidène)-3,5-dihydro-4H-imidazol-4-one sous forme d'une poudre jaune dont les caractéristiques sont les suivantes :
   Point de fusion : 274°C.
   Spectre de RMN ¹H à 400MHz : 0,93 (d, J = 7,5 Hz, 6H) ; 1,63 (m, 3H) ; 2,54 (t, J = 7,5 Hz, 2H) ; 7,15 (s, 1H) ; 7,19 (dd, J = 5,0 et 8,0 Hz, 1H) ; 8,29 (dd, J = 1,5 et 5,0 Hz, 1H) ; 8,33 (s, 1H) ; 8,91 (d large, J = 8,0 Hz, 1H) ; 11,05 (s large, 1H) ; 12,3 (s large, 1H).
   HPLC-MS-DAD-ELSD : 283(+)=(M+H)(+) ; 281(-)=(M-H)(-).
b) La 2-(3-méthylbutyl)-3,5-dihydro-4H-imidazol-4-one peut être préparée comme dans l'exemple 13, mais à partir de 3 g de chlorhydrate de 4-méthylpentanimidoate de méthyle pour obtenir 2,0 g de 2-(3-méthylbutyl)-3,5-dihydro-4H-imidazol-4-one brut.
c) Le chlorhydrate de 4-méthylpentanimidoate de méthyle peut être préparé comme dans l'exemple 13, mais à partir de 12,5 cm³ de 3-méthyl-butyl carbonitrile, de 4,6 cm³ de méthanol et de 5 cm³ d'éther. On obtient 20,7 g de chlorhydrate de 4-méthylpentanimidoate de méthyle sous forme d'un solide blanc.

### Exemple 69

### (5Z)-2-cyclohexyl-5-(1H-pyrrolo[2,3-b]pyridin-3-ylméthylidène)-3,5-dihydro-4H-imidazol-4-one

a) La (5Z)-2-cyclohexyl-5-(1H-pyrrolo[2,3-b]pyridin-3-ylméthylidène)-3,5-dihydro-4H-imidazol-4-one peut être préparée comme dans l'exemple 13, mais à partir de 296 mg de 2-cyclohexyl-3,5-dihydro-4H-imidazol-4-one brut et de 0,13 g de 1H-pyrrolo[2,3-b]pyridine-3-carbaldéhyde pour donner 135 mg de 5Z)-2-cyclohexyl-5-(1H-pyrrolo[2,3-b]pyridin-3-ylméthylidène)-3,5-dihydro-4H-imidazol-4-one sous forme d'une poudre jaune dont les caractéristiques sont les suivantes :
   Point de fusion : 322°C.
   Spectre de RMN ¹H à 400MHz : De 1,20 à 1,42 (m, 3H) ; 1,53 (m, 2H) ; 1,69 (m, 1H) ; 1,80 (m, 2H) ; 1,99 (m, 2H) ; 2,54 (m partiellement masqué, 1H) ; 7,18 (s, 1H) ; 7,20 (dd, J = 5,0 et 8,0 Hz, 1H) ; 8,29 (dd, J = 1,5 et 5,0 Hz, 1H) ; 8,34 (s, 1H) ; 8,93 (d large, J = 8,0 Hz, 1H) ; 11,1 (m étalé, 1H) ; 12,3 (m étalé, 1H).
   HPLC-MS-DAD-ELSD : 364(+)=(M+H)(+); 362(-)=(M-H)(-).
b) La 2-cyclohexyl-3,5-dihydro-4H-imidazol-4-one peut être préparée comme dans l'exemple 13, mais à partir de 704 mg de chlorhydrate de cyclohexanecarboximidoate de méthyle pour obtenir 1,2 g de 2-cyclohexyl-3,5-dihydro-4H-imidazol-4-one brut.
c) Le chlorhydrate de cyclohexanecarboximidoate de méthyle est préparé selon Synlett 2001, 11, 1707-1710. De l'acide chlorhydrique gazeux est bullé pendant 120 min dans une solution de 10 cm³ de cyclohexane carbonitrile dans 5,4 cm³ de méthanol et 30 cm³ d'heptane, refroidie à 0°C. Le mélange est agité une heure à 0°C puis mis au congélateur. Après quarante-huit heures, le solide blanc formé est filtré, lavé à l'heptane puis séché sous vide pour obtenir 12,0 g de chlorhydrate de cyclohexanecarboximidoate de méthyle sous forme d'un solide blanc.

### Exemple 70

### (5Z)-2-cyclohexyl-3-méthyl-5-(1H-pyrrolo[2,3-b]pyridin-3-ylméthylidène)-3,5-dihydro-4H-imidazol-4-one

a) La (5Z)-2-cyclohexyl-3-méthyl-5-(1H-pyrrolo[2,3-b]pyridin-3-ylméthylidène)-3,5-dihydro-4H-imidazol-4-one peut être préparée comme dans l'exemple 13, mais à partir de 493 mg de 2-cyclohexyl-3-méthyl-3,5-dihydro-4H-imidazol-4-one brut et de 0,16 g de 1H-pyrrolo[2,3-b]pyridine-3-carbaldéhyde pour donner 144 mg de (5Z)-2-cyclohexyl-3-méthyl-5-(1H-pyrrolo[2,3-b]pyridin ylméthylidène)-3,5-dihydro-4H-imidazol-4-one sous forme d'une poudre jaune dont les caractéristiques sont les suivantes
   Point de fusion : 261-262°C.
   Spectre de RMN ¹H à 400MHz : De 1,22 à 1,49 (m, 3H) ; 1,58 (m, 2H) ; 1,72 (m, 1H) ; 1,83 (m, 2H) ; 1,99 (m, 2H) ; 2,71 (tt, J = 3,5 et 11,0 Hz, 1H) ; 3,13 (s, 3H) ; 7,21 (dd, J = 5,0 et 8,0 Hz, 1H) ; 7,30 (s, 1H) ; 8,30 (dd, J = 1, 5 et 5,0 Hz, 1H) ; 8,39 (s, 1H) ; 8,99 (d large, J = 8,0 Hz, 1H) ; 12,4 (m étalé, 1H).
   UPLC-MS-DAD-ELSD : 309(+)=(M+H) (+).
b) La 2-cyclohexyl-3-méthyl-3,5-dihydro-4H-imidazol-4-one peut être préparée comme dans l'exemple 66, mais à partir de 1 g de N-[cyclohexyl(méthoxy)méthylidène]glycinate de méthyle pour obtenir 1,07 g de 2-cyclohexyl-3-méthyl-3,5-dihydro-4H-imidazol-4-one.
c) Le N-[cyclohexyl(méthoxy)méthylidène]glycinate de méthyle peut être préparé comme dans l'exemple 66, mais à partir de 1 g de chlorydrate de cyclohexanecarboximidoate de méthyle et de 0,71 g de chlorhydrate de méthyl glycinate pour obtenir 1,09 g de N-[cyclohexyl(méthoxy)méthylidène]glycinate de méthyle.

### Exemple 71

### (5Z)-5-(1H-pyrrolo[2,3-b]pyridin-3-ylméthylidène)-2-(tétrahydro-2H-pyran-4-yl)-3,5-dihydro-4H-imidazol-4-one

a) La (5Z)-5-(1H-pyrrolo[2,3-b]pyridin-3-ylméthylidène)-2-(tétrahydro-2H-pyran-4-yl)-3,5-dihydro-4H-imidazol-4-one peut être préparée comme dans l'exemple 13, mais à partir de 690 mg de 2-(tétrahydro-2H-pyran-4-yl)-3,5-dihydro-4H-imidazol-4-one brut et de 0,2g de 1H-pyrrolo[2,3-b]pyridine-3-carbaldéhyde pour donner 283 mg de (5Z)-5-(1H-pyrrolo[2,3-b]pyridin-3-ylméthylidène)-2-(tétrahydro-2H-pyran-4-yl)-3,5-dihydro-4H-imidazol-4-one sous forme d'une poudre jaune dont les caractéristiques sont les suivantes :
   Point de fusion : 317-319°C.
   Spectre de RMN ¹H à 400MHz : 1,79 (m, 2H) ; 1,91 (m, 2H) ; 2,82 (tt, J = 3,5 et 11,0 Hz, 1H) ; 3,45 (m, 2H) ; 3,94 (m, 2H) ; 7,20 (m, 2H) ; 8,29 (dd, J = 1,5 et 5,0 Hz, 1H) ; 8,36 (s, 1H) ; 8,95 (d large, J = 8,0 Hz, 1H) ; 11, 2 (s large, 1H) ; 12,4 (s large, 1H).
   HPLC-MS-DAD-ELSD : 297(+)=(M+H)(+); 295(-)=(M-H)(-).
b) La 2-(tétrahydro-2H-pyran-4-yl)-3,5-dihydro-4H-imidazol-4-one peut être préparée comme dans l'exemple 13, mais à partir de 2 g de chlorhydrate de tétrahydro-2H-pyran-4-carboximidoate de méthyle pour obtenir 3,2 g de 2-(tétrahydro-2H-pyran-4-yl)-3,5-dihydro-4H-imidazol-4-one brut.
c) Le tétrahydro-2H-pyran-4-carboximidoate de méthyle peut être préparé comme dans l'exemple 13, mais à partir de 3 g de tétrahydropyran-4-carbonitrile, de 1,2 cm³ de méthanol et de 6 cm³ d'éther. On obtient 4,4 g de chlorhydrate de tétrahydro-2H-pyran-4-carboximidoate de méthyle sous forme d'un solide blanc.
d) Le tétrahydropyran-4-carbonitrile peut être préparé de la manière suivante :
   A 3 g de tétrahydropyran-4-carboxamide refroidi dans un bain de glace, on ajoute lentement 10 cm³ de chlorure de thionyle. Le mélange est alors chauffé à 80°C pendant deux heures, puis concentré sous vide. Le résidu est repris par 20 cm³ d'eau et le pH de la solution est ajusté à pH 7 avec de la potasse. La phase aqueuse est extraite avec de l'acétate d'éthyle (4x50 cm³). Les phases organiques rassemblées sont lavées à l'eau (2x50 cm³), séchées sur sulfate de magnésium puis concentrées sous vide pour obtenir 2,47 g de tétrahydropyran-4-carbonitrile.
e) Le tétrahydropyran-4-carboxamide est préparé selon J. Chem. Soc. 1930, 2525-2530.

### Exemple 72

### (5Z)-5-[(1H-pyrrolo[2,3-b]pyridin-3-yl)méthylidène]-3-méthyl-2-(tétrahydro-2H-pyran-4-yl)-3,5-dihydro-4H-imidazol-4-one

a) La (5Z)-5-[(1H-pyrrolo[2,3-b]pyridin-3-yl)méthylidène]-3-méthyl-2-(tétrahydro-2H-pyran-4-yl)-3,5-dihydro-4H-imidazol-4-one peut être préparée comme dans l'exemple 13, mais à partir de 748 mg de 2-(tétrahydro-2H-pyran-4-yl)-3-méthyl-imidazolidin-4-one brut et de 0,2 g de 1H-pyrrolo[2,3-b]pyridine-3-carbaldéhyde pour donner 193 mg de (5Z)-5-[(1H-pyrrolo[2,3-b]pyridin-3-yl)méthylidène]-3-méthyl-2-(tétrahydro-2H-pyran-4-yl)-3,5-dihydro-4H-imidazol-4-one sous forme d'une poudre jaune dont les caractéristiques sont les suivantes :
   Point de fusion : 283-285°C.
   Spectre de RMN ¹H à 400MHz : De 1,53 à 1,93 (m, 4H) ; 3,03 (m, 1H) ; 3,16 (s, 3H) ; 3,51 (m, 2H) ; 3,99 (m, 2H) ; 7,21 (dd, J = 5,0 et 8,0 Hz, 1H) ; 7,32 (s, 1H) ; 8,29 (dd, J = 1,5 et 5,0 Hz, 1H) ; 8,39 (s, 1H) ; 9,01 (d large, J = 8,0 Hz, 1H) ; 12, 3 (m étalé, 1H).
   HPLC-MS-DAD-ELSD : 311(+)=(M+H)(+); 309(-)=(M-H)(-).
b) La 2-(tétrahydro-2H-pyran-4-yl)-3-méthyl-imidazolidin-4-one peut être préparée comme dans l'exemple 66, mais à partir de 2,55 g de N-[méthoxy(tétrahydro-2H-pyran-4-yl)méthylidène]glycinate de méthyle pour obtenir 2,03 g de 2-(tétrahydro-2H-pyran-4-yl)-3-méthyl-imidazolidin-4-one.
c) Le N-[méthoxy(tétrahydro-2H-pyran-4-yl)méthylidène]glycinate de méthyle peut être préparé comme dans l'exemple 66, mais à partir de 2 g de chlorhydrate de tétrahydro-2H-pyran-4-carboximidoate de méthyle et de 1,41 g de chlorhydrate de méthyl glycinate pour obtenir 2,55 g de N-[méthoxy(tétrahydro-2H-pyran-4-yl)méthylidène]glycinate de méthyle.

### Exemple 73

### (5Z)-5-[(5-chloro-1H-pyrrolo[2,3-b]pyridin-3-yl)méthylidène]-3-méthyl-2-(tétrahydro-2H-pyran-4-yl)-3,5-dihydro-4H-imidazol-4-one

La (5Z)-5-[(5-chloro-1H-pyrrolo[2,3-b]pyridin-3-yl)méthylidène]-3-méthyl-2-(tétrahydro-2H-pyran-4-yl)-3,5-dihydro-4H-imidazol-4-one peut être préparée comme dans l'exemple 13, mais à partir de 303 mg de 2-(tétrahydro-2H-pyran-4-yl)-3-méthyl-imidazolidin-4-one brut (exemple 72) et de 0,1 g de 5-chloro-1H-pyrrolo[2,3-b]pyridine-3-carbaldéhyde pour donner 130 mg de (5Z)-5-[(5-chloro-1H-pyrrolo[2,3-b]pyridin-3-yl)méthylidène]-3-méthyl-2-(tétrahydro-2H-pyran-4-yl)-3,5-dihydro-4H-imidazol-4-one sous forme d'une poudre jaune dont les caractéristiques sont les suivantes :
Point de fusion : 338°C.
Spectre de RMN ¹H à 400MHz : De 1,75 à 1,97 (m, 4H) ; 3,06 (m, 1H) ; 3,17 (s, 3H) ; 3,52 (m, 2H) ; 3,99 (m, 2H) ; 7,31 (s, 1H) ; 8,29 (d, J = 2,0 Hz, 1H) ; 8,39 (s, 1H) ; 9,48 (s large, 1H) ; 12,65 (m étalé, 1H).
HPLC-MS-DAD-ELSD : 345 (+)/...=(M+H) (+) /...; 343(-)/...=(M-H) (-) /... (1 atome de chlore CI présent).

### Exemple 74

### (5Z)-2-(2-méthylpropyl)-5-(1H-pyrrolo[2,3-b]pyridin-3-ylméthylidène)-3,5-dihydro-4H-imidazol-4-one

a) La (5Z)-2-(2-méthylpropyl)-5-(1H-pyrrolo[2,3-b]pyridin-3-ylméthylidène)-3,5-dihydro-4H-imidazol-4-one peut être préparée comme dans l'exemple 13, mais à partir de 575 mg de 2-(2-méthylpropyl)-3,5-dihydro-4H-imidazol-4-one brut et de 0,2 g de 1H-pyrrolo[2,3-b]pyridine-3-carbaldéhyde pour donner 99 mg de (5Z)-2-(2-méthylpropyl)-5-(1H-pyrrolo[2,3-b]pyridin-3-ylméthylidène)-3,5-dihydro-4H-imidazol-4-one sous forme d'une poudre jaune dont les caractéristiques sont les suivantes :
   Point de fusion : 292-293°C.
   Spectre de RMN ¹H à 400MHz : 1,00 (d, J = 7,0 Hz, 6H) ; 2,18 (m, 1H) ; 2,41 (d, J = 7,0 Hz, 2H) ; 7,17 (s, 1H) ; 7,19 (dd, J = 5,0 et 8,0 Hz, 1H) ; 8,29 (dd, J = 1,5 et 5,0 Hz, 1H) ; 8,34 (s, 1H) ; 8,89 (d large, J = 8,0 Hz, 1H) ; 11,1 (s large, 1H) ; 12,35 (m étalé, 1H).
   HPLC-MS-DAD-ELSD : 269(+)=(M+H)(+); 267(-)=(M-H)(-).
b) La 2-(2-méthylpropyl)-3,5-dihydro-4H-imidazol-4-one peut être préparée comme dans l'exemple 13, mais à partir de 2 g de chlorhydrate de 3-méthylbutanimidoate de méthyle pour obtenir 1,5 g de 2-(2-méthylpropyl)-3,5-dihydro-4H-imidazol-4-one brut.
c) Le chlorhydrate de 3-méthylbutanimidoate de méthyle peut être préparé comme dans l'exemple 13, mais à partir de 3,1 cm³ de 2-méthyl-propyl carbonitrile, de 1,3 cm³ de méthanol et de 4 cm³ d'éther. On obtient 4,0 g de chlorhydrate de 3-méthylbutanimidoate de méthyle sous forme de solide blanc.

### Exemple 75

### (5Z)-3-méthyl-2-(2-méthylpropyl)-5-(1H-pyrrolo[2,3-b)pyridin-3-ylméthylidène)-3,5-dihydro-4H-imidazol-4-one

a) La (5Z)-3-méthyl-2-(2-méthylpropyl)-5-(1H-pyrrolo[2,3-b]pyridin-3-ylméthylidène)-3,5-dihydro-4H-imidazol-4-one peut être préparée comme dans l'exemple 13, mais à partir de 466 mg de 3-méthyl-2-(2-méthylpropyl)-3,5-dihydro-4H-imidazol-4-one brut et de 0,17 g de 1H-pyrrolo[2,3-b]pyridine-3-carbaldéhyde pour donner 82 mg de (5Z)-3-méthyl-2-(2-méthylpropyl)-5-(1H-pyrrolo[2,3-b]pyridin-3-ylméthylidène)-3,5-dihydro-4H-imidazol-4-one sous forme d'une poudre jaune dont les caractéristiques sont les suivantes :
   Point de fusion : 201°C.
   Spectre de RMN ¹H à 400MHz : 1,07 (d, J = 7,5 Hz, 6H) ; 2,27 (m, 1H) ; 2,56 (d, J = 7,5 Hz, 2H) ; 3,10 (s, 3H) ; 7,19 (dd, J = 5,0 et 8,0 Hz, 1H) ; 7,29 (s, 1H) ; 8,29 (dd, J = 1,5 et 5,0 Hz, 1H) ; 8,38 (s, 1H) ; 8,98 (d large, J = 8,0 Hz, 1H) ; 12,4 (m étalé, 1H).
   UPLC-MS-DAD-ELSD : 283(+)=(M+H)(+); 281(-)=(M-H)(-).
b) La 3-méthyl-2-(2-méthylpropyl)-3,5-dihydro-4H-imidazol-4-one peut être préparée comme dans l'exemple 66, mais à partir de 1,16 g de N-(1-méthoxy-3-méthylbutylidène)glycinate de méthyle pour obtenir 901 mg de 3-méthyl-2-(2-méthylpropyl)-3,5-dihydro-4H-imidazol-4-one.
c) Le N-(1-méthoxy-3-méthylbutylidène)glycinate de méthyle peut être préparé comme dans l'exemple 66, mais à partir de 959 mg de chlorhydrate de 3-méthylbutanimidoate de méthyle et de 802 mg de chlorhydrate de méthyl glycinate pour obtenir 1,18 g de N-(1-méthoxy-3-méthylbutylidène)glycinate de méthyle.

### Exemple 76

### (5Z)-5-[(5-chloro-1H-pyrrolo[2,3-b]pyridin-3-yl)méthylidène]-3-méthyl-2-(2-méthylpropyl)-3,5-dihydro-4H-imidazol-4-one

La (5Z)-5-[(5-chloro-1H-pyrrolo[2,3-b]pyridin-3-yl)méthylidène]-3-méthyl-2-(2-méthylpropyl)-3,5-dihydro-4H-imidazol-4-one peut être préparée comme dans l'exemple 13, mais à partir de 420 mg de 3-méthyl-2-(2-méthylpropyl)-3,5-dihydro-4H-imidazol-4-one brut (exemple 75) et de 0,2 g de 5-chloro-1H-pyrrolo[2,3-b]pyridine-3-carbaldéhyde pour donner 220 mg de (5Z)-5-[(5-chloro-1H-pyrrolo[2,3-b]pyridin-3-yl)méthylidène]-3-méthyl-2-(2-méthylpropyl)-3,5-dihydro-4H-imidazol-4-one sous forme d'une poudre jaune dont les caractéristiques sont les suivantes :
Point de fusion : 282°C.
Spectre de RMN ¹H à 400MHz : 1,11 (d, J = 7,5 Hz, 6H) ; 2,33 (m, 1H) ; 2,59 (d, J = 7,0 Hz, 2H) ; 3,10 (s, 3H) ; 7,29 (s, 1H) ; 8,30 (d, J = 2,0 Hz, 1H) ; 8,38 (s, 1H) ; 9,46 (d large, J = 2,0 Hz, 1H) ; 12,6 (m étalé, 1H).
HPLC-MS-DAD-ELSD : 317 (+) /...=(M+H) (+) /...; 315 (-) /...=(M-H) (-) /... (1 atome de chlore CI présent).

### Exemple 77

### (5Z)-2-(cyclopropylméthyl)-5-(1H-pyrrolo[2,3-b]pyridin-3-ylméthylidène)-3,5-dihydro-4H-imidazol-4-one

a) La (5Z)-2-(cyclopropylméthyl)-5-(1H-pyrrolo[2,3-b]pyridin-3-ylméthylidène)-3,5-dihydro-4H-imidazol-4-one peut être préparée comme dans l'exemple 13, mais à partir de 354 mg de 2-(cyclopropylméthyl)-3,5-dihydro-4H-imidazol-4-one brut et de 0,2 g de 1H-pyrrolo[2,3-b]pyridine-3-carbaldéhyde pour donner 142 mg de (5Z)-2-(cyclopropylméthyl)-5-(1H-pyrrolo[2,3-b]pyridin-3-ylméthylidène)-3,5-dihydro-4H-imidazol-4-one sous forme d'une poudre jaune dont les caractéristiques sont les suivantes :
   Point de fusion : 289°C.
   Spectre de RMN ¹H à 400MHz : 0,29 (m, 2H) ; 0,55 (m, 2H) ; 1,14 (m, 1H) ; 2,45 (d, J = 7,5 Hz, 2H) ; 7,19 (m, 2H) ; 8,29 (dd ; J = 1,5 et 5,5 Hz, 1H) ; 8,34 (s, 1H) ; 8,98 (dd, J = 1,5 et 8,0 Hz, 1H) ; 11,1 (s large, 1H) ; 12,35 (m étalé, 1H).
   HPLC-MS-DAD-ELSD : 267(+)=(M+H)(+); 265(-)=(M-H)(-).
b) La 2-(cyclopropylméthyl)-3,5-dihydro-4H-imidazol-4-one peut être préparée comme dans l'exemple 13, mais à partir de 2 g de chlorhydrate de 2-cyclopropyléthanimidoate de méthyle pour obtenir 2,5 g de 2-(cyclopropylméthyl)-3,5-dihydro-4H-imidazol-4-one brut.
c) Le chlorhydrate de 2-cyclopropyléthanimidoate de méthyle peut être préparé comme dans l'exemple 13, mais à partir de 10 cm³ de cyclopropylméthyl carbonitrile, de 4,7 cm³ de méthanol et de 10 cm³ d'éther. On obtient 18,2 g de chlorhydrate de 2-cyclopropyléthanimidoate de méthyle sous forme d'un solide blanc.

### Exemple 78

### (5Z)-2-(cyclopropylméthyl)-5-(5-chloro-1H-pyrrolo[2,3-b]pyridin-3-ylméthylidène)-3,5-dihydro-4H-imidazol-4-one

La (5Z)-2-(cycl.opropylméthyl)-5-(5-chloro-1H-pyrrolo[2,3-b]pyridin-3-ylméthylidène)-3,5-dihydro-4H-imidazol-4-one peut être préparée comme dans l'exemple 13, mais à partir de 144 mg de 2-(cyclopropylméthyl)-3,5-dihydro-4H-imidazol-4-one brut (exemple 77) et de 0,1 g de 5-chloro-1H-pyrrolo[2,3-b]pyridine-3-carbaldéhyde pour donner 47 mg de (5Z)-2-(cyclopropylméthyl)-5-(5-chloro-1H-pyrrolo[2,3-b]pyridin-3-ylméthylidène)-3,5-dihydro-4H-imidazol-4-one sous forme d'une poudre jaune dont les caractéristiques sont les suivantes :
Point de fusion : 295-296°C.
Spectre de RMN ¹H à 400MHz : 0,30 (m, 2H) ; 0,60 (m, 2H) ; 1,17 (m, 1H) ; 2,48 (m partiellement masqué, 2H) ; 7,17 (s, 1H) ; 8,29 (d, J = 2,0 Hz, 1H) ; 8,35 (s, 1H) ; 9,42 (s large, 1H) ; 11, 15 (s large, 1H) ; 12,5 (m étalé, 1H).
HPLC-MS-DAD-ELSD : 301 (+) /...= (M+H) (+) /...; 299(-) /...= (M-H) (-) /... (1 atome de chlore CI présent).

### Exemple 79

### (5Z)-2-(1-méthyléthyl)-5-(1H-pyrrolo[2,3-b]pyridin-3-ylméthylidène)-3,5-dihydro-4H-imidazol-4-one

a) La (5Z)-2-(1-méthyléthyl)-5-(1H-pyrrolo[2,3-b]pyridin-3-ylméthylidène)-3,5-dihydro-4H-imidazol-4-one peut être préparée comme dans l'exemple 13, mais à partir de 324 mg de 2-(1-méthyléthyl)-3,5-dihydro-4H-imidazol-4-one brut et de 0,2 g de 1H-pyrrolo[2,3-b]pyridine-3-carbaldéhyde pour donner 140 mg de (5Z)-2-(1-méthyléthyl)-5-(1H-pyrrolo[2,3-b]pyridin-3-ylméthylidène)-3,5-dihydro-4H-imidazol-4-one sous forme d'une poudre jaune dont les caractéristiques sont les suivantes :
   Point de fusion : 309-310°C.
   Spectre de RMN ¹H à 400MHz : 1,29 (d, J = 7,0 Hz, 6H) ; 2,81 (m, 1H) ; 7,18 (s, 1H) ; 7,20 (dd, J = 5,0 et 8,0 Hz, 1H) ; 8,29 (dd, J = 1,5 et 5,0 Hz, 1H) ; 8,34 (s, 1H) ; 8,98 (d large, J = 8,0 Hz, 1H) ; 11,15 (m étalé, 1H) ; 12,35 (m étalé, 1H).
   HPLC-MS-DAD-ELSD : 255(+)=(M+H)(+); 253(-)=(M-H)(-).
b) La 2-(1-méthyléthyl)-3,5-dihydro-4H-imidazol-4-one peut être préparée comme dans l'exemple 13, mais à partir de 2 g de chlorhydrate de 2-méthylpropanimidoate de méthyle pour obtenir 1,5 g de 2-(1-méthyléthyl)-3,5-dihydro-4H-imidazol-4-one brut.
c) Le chlorhydrate de 2-méthylpropanimidoate de méthyle peut être préparé comme dans l'exemple 13, mais à partir de 10 cm³ d'isopropyl carbonitrile, de 5 cm³ de méthanol et de 10 cm³ d'éther. On obtient 17 g de chlorhydrate de 2-méthylpropanimidoate de méthyle sous forme d'un solide blanc.

### Exemple 80

### (5Z)-2-(1-méthyléthyl)-5-(5-chloro-1H-pyrrolo[2,3-b]pyridin-3-ylméthylidène)-3,5-dihydro-4H-imidazol-4-one

La (5Z)-2-(1-méthyléthyl)-5-(5-chloro-1H-pyrrolo[2,3-b]pyridin-3-ylméthylidène)-3,5-dihydro-4H-imidazol-4-one peut être préparée comme dans l'exemple 13, mais à partir de 131 mg de 2-(1-méthyléthyl)-3,5-dihydro-4H-imidazol-4-one brut (exemple 79) et de 0,1 g de 5-chloro-1H-pyrrolo[2,3-b]pyridine-3-carbaldéhyde pour donner 116 mg de (5Z)-2-(1-méthyléthyl)-5-(5-chloro-1H-pyrrolo[2,3-b]pyridin-3-ylméthylidène)-3,5-dihydro-4H-imidazol-4-one sous forme d'une poudre jaune dont les caractéristiques sont les suivantes :
Point de fusion : 277-280°C.
Spectre de RMN ¹H à 400MHz : 1,30 (d, J = 7,5 Hz, 6H) ; 2,81 (m, 1H) ; 7,15 (s, 1H) ; 8,29 (d, J = 2,0 Hz, 1H) ; 8,32 (s, 1H) ; 9,47 (d large, J = 2,0 Hz, 1H) ; 11,15 (s large, 1H) ; 12,55 (m étalé, 1H).
HPLC-MS-DAD-ELSD : 289 (+) /...=(M+H) (+)/...; 287 (-) /...= (M-H) (-)/... (1 atome de chlore CI présent).

### Exemple 81

### (5Z)-3-méthyl-2-(1-méthyléthyl)-5-(1H-pyrrolo[2,3-b]pyridin-3-ylméthylidène)-3,5-dihydro-4H-imidazol-4-one

a) La (5Z)-3-méthyl-2-(1-méthyléthyl)-5-(1H-pyrrolo[2,3-b]pyridin-3-ylméthylidène)-3,5-dihydro-4H-imidazol-4-one peut être préparée comme dans l'exemple 13, mais à partir de 437 mg de 3-méthyl-2-(1-méthyléthyl)-3,5-dihydro-4H-imidazol-4-one brut et de 0,19 g de 1H-pyrrolo[2,3-b]pyridine-3-carbaldéhyde pour donner 163 mg de (5Z)-3-méthyl-2-(1-méthyléthyl)-5-(1H-pyrrolo[2,3-b]pyridin-3-ylméthylidène)-3,5-dihydro-4H-imidazol-4-one sous forme d'une poudre jaune dont les caractéristiques sont les suivantes :
   Point de fusion : 222-224°C.
   Spectre de RMN ¹H à 400MHz : 1,31 (d, J = 7,0 Hz, 6H) ; 3,02 (m, 1H) ; 3,15 (s, 3H) ; 7,20 (dd, J = 5,0 et 8,0 Hz, 1H) ; 7,31 (s, 1H) ; 8,29 (dd, J = 1,5 et 5,0 Hz, 1H) ; 8,39 (s, 1H) ; 9,05 (d large, J = 8,0 Hz, 1H) ; 12,4 (m étalé, 1H).
   HPLC-MS-DAD-ELSD : 269(+)=(M+H)(+); 267(-)=(M-H)(-).
b) La 3-méthyl-2-(1-méthyléthyl)-3,5-dihydro-4H-imidazol-4-one peut être préparée comme dans l'exemple 66, mais à partir de 1 g de N-(1-méthoxy-2-méthylpropylidène)glycinate de méthyle pour obtenir 789 mg de 3-méthyl-2-(1-méthyléthyl)-3,5-dihydro-4H-imidazol-4-one.
c) Le N-(1-méthoxy-2-méthylpropylidène)glycinate de méthyle peut être préparé comme dans l'exemple 66, mais à partir de 870 mg de chlorhydrate de 2-méthylpropanimidoate de méthyle et de 802 mg de chlorhydrate de méthyl glycinate pour obtenir 1 g de N-(1-méthoxy-2-méthylpropylidène)glycinate de méthyle.

### Exemple 82

### (5Z)-5-[(5-chloro-1H-pyrrolo[2,3-b]pyridin-3-yl)méthylidène]-3-méthyl-2-(1-méthyléthyl)-3,5-dihydro-4H-imidazol-4-one

La (5Z)-5-[(5-chloro-1H-pyrrolo[2,3-b]pyridin-3-yl)méthylidène]-3-méthyl-2-(1-méthyléthyl)-3,5-dihydro-4H-imidazol-4-one peut être préparée comme dans l'exemple 13, mais à partir de 350 mg de 3-méthyl-2-(1-méthyléthyl)-3,5-dihydro-4H-imidazol-4-one brut (exemple 81) et de 0,2 g de 5-chloro-1H-pyrrolo[2,3-b]pyridine-3-carbaldéhyde pour donner 235 mg de (5Z)-5-[(5-chloro-1H-pyrrolo[2,3-b]pyridin-3-yl)méthylidène]-3-méthyl-2-(1-méthyléthyl)-3,5-dihydro-4H-imidazol-4-one sous forme d'une poudre jaune dont les caractéristiques sont les suivantes :
Point de fusion : 282°C.
Spectre de RMN ¹H à 400MHz : 1,32 (d, J = 7,5 Hz, 6H) ; 3,04 (m, 1H) ; 3,15 (s, 3H) ; 7,29 (s, 1H) ; 8,30 (d, J = 2,0 Hz, 1H) ; 8,37 (s, 1H) ; 9,55 (s large, 1H) ; 12,6 (m étalé, 1H).
HPLC-MS-DAD-ELSD : 303 (+)/...=(M+H) (+) /...; 301 (-) /...= (M-H) (-) /... (1 CI présent).

### Exemple 83

### (5Z)-2,3-diméthyl-5-(1H-pyrrolo[2,3-b]pyridin-3-ylméthylidène)-3,5-dihydro-4H-imidazol-4-one

a) La (5Z)-2,3-diméthyl-5-(1H-pyrrolo[2,3-b]pyridin-3-ylméthylidène)-3,5-dihydro-4H-imidazol-4-one peut être préparée de la manière suivante :
   Dans un réacteur pour four micro-ondes, on mélange 0,8 cm³ d'une solution 8M de méthylamine dans l'éthanol à et une suspension de 200 mg de (4Z)-4-[(1-acétyl-1H-pyrrolo[2,3-b]pyridin-3-yl)méthylidène]-2-méthyl-1,3-oxazol-5(4H)-one dans 4 cm³ d'éthanol. Le réacteur est fermé hermétiquement puis placé durant treize minutes à 170°C sous irradiation micro-ondes. Après filtration du solide, on obtient 127 mg de (5Z)-2,3-diméthyl-5-(1H-pyrrolo[2,3-b]pyridin-3-ylméthylidène)-3,5-dihydro-4H-imidazol-4-one sous forme d'une poudre orangée dont les caractéristiques sont les suivantes :
      Spectre de RMN ¹H à 400MHz : 2,37 (s, 3H) ; 3,10 (s, 3H) ; 7,20 (dd, J = 5,0 et 8,0 Hz, 1H) ; 7,29 (s, 1H) ; 8,29 (dd, J = 1,5 et 5,0 Hz, 1H) ; 8,39 (s, 1H) ; 8,91 (d large, J = 8,0 Hz, 1H) ; 12,35 (m étalé, 1H).
      HPLC-MS-DAD-ELSD : 241(+)=(M+H)(+); 239(-)=(M-H)(-).
b) La (4Z)-4-[(1-acétyl-1H-pyrrolo[2,3-b]pyridin-3-yl)méthylidène]-2-méthyl-1,3-oxazol-5(4H)-one peut être préparée de la manière suivante :
   Dans un tricol sous argon, sont introduits 1,92 g de N-acétylglycine, et 1,23 g d'acétate de sodium dans 25 cm³ d'anhydride acétique. Le mélange réactionnel est porté à 80°C sous agitation durant une heure. Sont ajoutés 2 g de 1H-pyrrolo[2,3-b]pyridine-3-carbaldéhyde et après deux heures à 140°C, le mélange réactionnel est ramené à 25°C puis filtré. Le solide est rincé à l'eau puis à l'éthanol avant séchage à sec sous pression réduite. On obtient 1,68 g de (4Z)-2-méthyl-4-(1H-pyrrolo[2,3-b]pyridin-3-ylméthylidène)-1,3-oxazol-5(4H)-one sous forme d'une poudre jaune dont les caractéristiques sont les suivantes :
      LC/MS (1) : Temps de rétention : 3,3 mn ; 270(+)=[MH+].

### Exemple 84

### (5Z)-2-méthyl-5-(1H-pyrrolo[2,3-b]pyridin-3-ylméthylidène)-3,5-dihydro-4H-imidazol-4-one

La (5Z)-2-méthyl-5-(1H-pyrrolo[2,3-b]pyridin-3-ylméthylidène)-3,5-dihydro-4H-imidazol-4-one peut être préparée comme dans l'exemple 83, mais à partir de 2,2 cm³ d'ammoniac à 40% dans l'éthanol et de 200 mg de (4Z)-4-[(1-acétyl-1H-pyrrolo[2,3-b]pyridin-3-yl)méthylidène]-2-méthyl-1,3-oxazol-5(4H)-one dans 2 cm³ d'éthanol. Après dix-huit minutes à 170°C sous irradiation micro-ondes et filtration du solide, on obtient 108 mg de (5Z)-2-méthyl-5-(1H-pyrrolo[2,3-b]pyridin-3-ylméthylidène)-3,5-dihydro-4H-imidazol-4-one sous forme d'une poudre orangée dont les caractéristiques sont les suivantes :
Point de fusion : 277-281°C.
Spectre de RMN ¹H à 400MHz : 2,25 (s, 3H) ; 7,15 (s, 1H) ; 7,19 (dd, J =5,0 et 8,0 Hz, 1H) ; 8,29 (d large, J = 5,0 Hz, 1H) 8,35 (s, 1H) ; 8,87 (d large, J = 8,0 Hz, 1H) ; 11,1 (m étalé, 1H) ; 12,35 (m étalé, 1H).
UPLC-MS-DAD-ELSD : 225(+)=(M+H)(+); 227(-)=(M-H)(-).

### Exemple 85

### (5Z)-2-méthyl-3-(2-morpholin-4-yléthyl)-5-(1H-pyrrolo[2,3-b]pyridin-3-ylméthylidène)-3,5-dihydro-4H-imidazol-4-one

La (5Z)-2-méthyl-3-(2-morpholin-4-yléthyl)-5-(1H-pyrrolo[2,3-b]pyridin-3-ylméthylidène)-3,5-dihydro-4H-imidazol-4-one peut être préparée comme dans l'exemple 83, mais à partir de 0,98 cm³ de 2-morpholin-4-yléthanamine et de 200 mg de (4Z)-4-[(1-acétyl-1H-pyrrolo[2,3-b]pyridin-3-yl)méthylidène]-2-méthyl-1,3-oxazol-5(4H)-one dans 2 cm³ d'éthanol. Après dix-huit minutes à 170°C sous irradiation micro-ondes et filtration du solide, on obtient 105 mg de (5Z)-2-méthyl-3-(2-morpholin-4-yléthyl)-5-(1H-pyrrolo[2,3-b]pyridin-3-ylméthylidène)-3,5-dihydro-4H-imidazol-4-one sous forme d'une poudre orangée dont les caractéristiques sont les suivantes :
Point de fusion : 235-240°C.
Spectre de RMN ¹H à 400MHz : De 2,30 à 2,55 (m partiellement masqué, 9H) ; 3,55 (m, 9H) ; 3,69 (t, J = 6,0 Hz, 2H) ; 7,21 (dd, J = 5,0 et 8,0 Hz, 1H) ; 7,29 (s, 1H) ; 8,30 (dd, J = 1,5 et 5,0 Hz, 1H) ; 8,39 (s, 1H) ; 8,92 (d large, J = 8,0 Hz, 1H) ; 10,0 (m très étalé, 1H).

### Exemple 86

### (5Z)-3-benzyl-2-méthyl-5-(1H-pyrrolo[2,3-b]pyridin-3-ylméthylidène)-3,5-dihydro-4H-imidazol-4-one

La (5Z)-3-benzyl-2-méthyl-5-(1H-pyrrolo[2,3-b]pyridin-3-ylméthylidène)-3,5-dihydro-4H-imidazol-4-one peut être préparée comme dans l'exemple 83, mais à partir de 0,8 cm³ de 1-phénylméthanamine et de 200 mg de (4Z)-4-[(1-acétyl-1H-pyrrolo[2,3-b]pyridin-3-yl)méthylidène]-2-méthyl-1,3-oxazol-5(4H)-one dans 2 cm³ d'éthanol. Après quinze minutes à 170°C sous irradiation micro-ondes et filtration du solide, on obtient 105 mg de (5Z)-3-benzyl-2-méthyl-5-(1H-pyrrolo[2,3-b]pyridin-3-ylméthylidène)-3,5-dihydro-4H-imidazol-4-one sous forme d'une poudre jaune dont les caractéristiques sont les suivantes :
Point de fusion : 274-275°C.
Spectre de RMN ¹H à 400MHz : 2,28 (s, 3H) ; 4,83 (s, 2H) ; 7,20 (dd, J = 5,0 et 8,0 Hz, 1H) ; 7,24 (d, J = 7,5 Hz, 2H) ; 7,30 (t, J = 7,5 Hz, 1H) ; 7,38 (m, 3H) ; 8,29 (dd, J = 1,5 et 5,0 Hz, 1H) ; 8,41 (s, 1H) ; 8,92 (d large, J = 8,0 Hz, 1H) ; 12,45 (m étalé, 1H).

### Exemple 87

### (5Z)-2-méthyl-3-propyl-5-(1H-pyrrolo[2,3-b]pyridin-3-ylméthylidène)-3,5-dihydro-4H-imidazol-4-one

La (5Z)-2-méthyl-3-propyl-5-(1H-pyrrolo[2,3-b]pyridin-3-ylméthylidène)-3,5-dihydro-4H-imidazol-4-one peut être préparée comme dans l'exemple 83, mais à partir de 0,6 cm³ de propan-1-amine et de 200 mg de (4Z)-4-[(1-acétyl-1H-pyrrolo[2,3-b]pyridin-3-yl)méthylidène]-2-méthyl-1,3-oxazol-5(4H)-one dans 2 cm³ d'éthanol. Après seize minutes à 170°C sous irradiation micro-ondes et filtration du solide, on obtient 73 mg de (5Z)-2-méthyl-3-propyl-5-(1H-pyrrolo[2,3-b]pyridin-3-ylméthylidène)-3,5-dihydro-4H-imidazol-4-one sous forme de cristaux jaunes dont les caractéristiques sont les suivantes :
Point de fusion : 206°C.
Spectre de RMN ¹H à 400MHz : 0,88 (t, J = 7,5 Hz, 3H) ; 1,58 (m, 2H) ; 2,38 (s, 3H) ; 3,53 (t, J = 7,5 Hz, 2H) ; 7,19 (m, 1H) ; 7,28 (s, 1H) ; 8,29 (m, 1H) ; 8,39 (s, 1H) ; 8,90 (d large, J = 8,0 Hz, 1H) ; 12,35 (m étalé, 1H).
HPLC-MS-DAD-ELSD : 269 (+) = (M+H) (+); 267(-)=(M-H)(-).

### Exemple 88

### (5Z)-2-méthyl-3-(1-méthyléthyl)-5-(1H-pyrrolo[2,3-b]pyridin-3-ylméthylidène)-3,5-dihydro-4H-imidazol-4-one

La (5Z)-2-méthyl-3-(1-méthyléthyl)-5-(1H-pyrrolo[2,3-b]pyridin-3-ylméthylidène)-3,5-dihydro-4H-imidazol-4-one peut être préparée comme dans l'exemple 83, mais à partir de 0,63 cm³ de propan-2-amine et de 200 mg de (4Z)-4-[(1-acétyl-1H-pyrrolo[2,3-b]pyridin-3-yl)méthylidène]-2-méthyl-1,3-oxazol-5(4H)-one dans 2 cm³ d'éthanol. Après trente-huit minutes à 170°C sous irradiation micro-ondes et filtration du solide, le filtrat est purifié par LC-MS préparative [Colonne Xterra RP18 30x100 ; 5µ ; avec eau tamponnée à 10 mM d'hydrogénocarbonate d'ammonium ajustée à pH 9 avec de l'ammoniaque / Acétonitrile avec gradient 70/30 à 0/100 en 8 min]. On obtient 39 mg de (5Z)-2-méthyl-3-(1-méthyléthyl)-5-(1H-pyrrolo[2,3-b]pyridin-3-ylméthylidène)-3,5-dihydro-4H-imidazol-4-one sous forme de lyophilisat jaune dont les caractéristiques sont les suivantes :
Point de fusion : 207°C.
Spectre de RMN ¹H à 400MHz : 1,41 (d, J = 7,0 Hz, 6H) ; 2,40 (s, 3H) ; 4,21 (m, 1H) ; 7,21 (m, 2H) ; 8,29 (dd, J = 1,5 et 5,0 Hz, 1H) ; 8,37 (s, 1H) ; 8,90 (dd, J = 1,5 et 8,0 Hz, 1H).

### Exemple 89

### (5Z)-2-butyl-5-[(5-chloro-1H-pyrrolo[2,3-b]pyridin-3-yl)méthylidène]-3-méthyl-3,5-dihydro-4H-imidazol-4-one

a) La (5Z)-2-butyl-5-[(5-chloro-1H-pyrrolo[2,3-b]pyridin-3-yl)méthylidène]-3-méthyl-3,5-dihydro-4H-imidazol-4-one peut être préparée comme dans l'exemple 83, mais à partir de 0,14 cm³ de méthylamine à 33% dans l'éthanol et de 67 mg de (4Z)-4-[(1-acétyl-5-chloro-1H-pyrrolo[2,3-b]pyridin-3-yl)méthylidène]-2-butyl-1,3-oxazol-5(4H)-one dans 4 cm³ d'éthanol. Après quinze minutes à 170°C sous irradiation micro-ondes et filtration du solide, on obtient 26 mg de (5Z)-2-butyl-5-[(5-chloro-1H-pyrrolo[2,3-b]pyridin-3-yl)méthylidène]-3-méthyl-3,5-dihydro-4H-imidazol-4-one sous forme d'une poudre jaune dont les caractéristiques sont les suivantes :
   Point de fusion : 295-300°C.
   Spectre de RMN ¹H à 400MHz : 1,00 (t, J = 7,5 Hz, 3H) ; 1,50 (m, 2H) ; 1,85 (m, 2H) ; 2,69 (t, J = 7,5 Hz, 2H) ; 3,10 (s, 3H) ; 7,27 (s, 1H) ; 8,29 (s large, 1H) ; 8,35 (s, 1H) ; 9,50 (s large, 1H) ; 12,55 (m étalé, 1H).
   UPLC-MS-DAD-ELSD : 317 (+) /...=(M+H) (+) /...; 315 (-) /...= (M-H) (-) /... (1 atome de chlore CI présent).
b) La (4Z)-4-[(1-acétyl-5-chloro-1H-pyrrolo[2,3-b]pyridin-3-yl)méthylidène]-2-butyl-1,3-oxazol-5(4H)-one peut être préparée comme dans l'exemple 83, mais à partir de 0,32 g de N-pentanoylglycine, de 0,25 g d'acétate de potassium et de 0,3 g de 5-chloro-1H-pyrrolo[2,3-b]pyridine-3-carbaldéhyde dans 1 cm³ d'anhydride acétique. Après quatre heures à 90°C, le mélange réactionnel est ramené à 25°C puis dilué par de l'eau. Le solide formé est filtré et purifié par chromatographie sur silice (élution avec du chlorure de méthylène) pour obtenir 69 mg de (4Z)-4-[(1-acétyl-5-chloro-1H-pyrrolo[2,3-b]pyridin-3-yl)méthylidène]-2-butyl-1,3-oxazol-5(4H)-one sous forme d'une poudre jaune dont les caractéristiques sont les suivantes :
   LC/MS temps de rétention 5,75 mn; LC-MS-DAD-ELSD : 346(+)=(M+H) (+).

Lors de la purification précédente, on isole aussi 88 mg de (4Z)-4-[(1-acétyl-5-chloro-1H-pyrrolo[2,3-b]pyridin-3-yl)méthylidène]-2-méthyl-1,3-oxazol-5(4H)-one.

### Exemple 90

### (5Z)-5-[(5-chloro-1H-pyrrolo[2,3-b]pyridin-3-yl)méthylidène]-2,3-diméthyl-3,5-dihydro-4H-imidazol-4-one

(5Z)-5-[(5-chloro-1H-pyrrolo[2,3-b]pyridin-3-yl)méthylidène]-2,3-diméthyl-3,5-dihydro-4H-imidazol-4-one peut être préparée comme dans l'exemple 83, mais à partir de 0.21 cm³ de méthylamine à 33% dans l'éthanol et de 85 mg de (4Z)-4-[(1-acétyl-5-chloro-1H-pyrrolo[2,3-b]pyridin-3-yl)méthylidène]-2-méthyl-1,3-oxazol-5(4H)-one (voir exemple 89) dans 4 cm³ d'éthanol. Après quinze minutes à 170°C sous irradiation micro-ondes et filtration du solide, on obtient 41 mg de (5Z)-2-méthyl-5-[(5-chloro-1H-pyrrolo[2,3-b]pyridin-3-yl)méthylidène]-3-méthyl-3,5-dihydro-4H-imidazol-4-one sous forme d'une poudre jaune dont les caractéristiques sont les suivantes :
Point de fusion : 295-300°C.
Spectre de RMN ¹H à 400MHz : 2,37 (s, 3H) ; 3,11 (s, 3H) ; 7,29 (s, 1H) ; 8,30 (d, J = 2,0 Hz, 1H) ; 8,41 (s, 1H) ; 9,20 (s large, 1H) ; 12,6 (m étalé, 1H).
UPLC-MS-DAD-ELSD : 275 (+)/...=(M+H) (+) /...; 274 (-)/...= (M-H) (-) /... (1 atome de chlore CI présent).

### Exemple 91

### (5Z)-2-[méthyl(2-méthylpropyl)amino]-5-[(5-méthyl-1H-pyrrolo[2,3-b]pyridin-3-yl)méthylidène]-3,5-dihydro-4H-imidazol-4-one

La (5Z)-2-[méthyl(2-méthylpropyl)amino]-5-[(5-méthyl-1H-pyrrolo[2,3-b]pyridin-3-yl)méthylidène]-3,5-dihydro-4H-imidazol-4-one peut être préparée comme dans l'exemple 7, mais à partir de 240 mg de (5Z)-5-[(5-méthyl-1H-pyrrolo[2,3-b]pyridin-3-yl)méthylidène]-2-thioxoimidazolidin-4-one (exemple 17), de 4 cm³ d'éthanol et de 680 mg de méthyl-(2-méthyl)propylamine. Après vingt minutes à une température de 160°C et une heure à 180°C sous irradiation micro-ondes puis filtration du solide, on obtient 55 mg de (5Z)-2-[méthyl(2-méthylpropyl)amino]-5-[(5-méthyl-1H-pyrrolo[2,3-b]pyridin-3-yl)méthylidène]-3,5-dihydro-4H-imidazol-4-one sous forme d'une poudre marron dont les caractéristiques sont les suivantes :
Point de fusion : 287-289°C.
Spectre de RMN ¹H à 400MHz : 0,90 (d, J = 6,6 Hz, 6H) ; 2,05 (m, 1H) ; 2,40 (s, 3H) ; 3,10 (s, 3H) ; 3,30 (m, masqués, 2H) ; 6,58 (s, 1H) ; 8,08 (s large, 1H) ; 8,13 (s large, 1H) ; 8,49 (s large, 1H) ; 11,00 (s large, 1H) ; 11,80 (s large, 1H).

### Exemple 92

### (5Z)-5-[(5-chloro-1H-pyrrolo[2,3-b]pyridin-3-yl)méthylidène]-2-phényl-3,5-dihydro-4H-imidazol-4-one

a) La (5Z)-5-[(5-chloro-1H-pyrrolo[2,3-b]pyridin-3-yl)méthylidène]-2-phényl-3,5-dihydro-4H-imidazol-4-one peut être préparée comme dans l'exemple 83, mais à partir de 2,7 cm³ d'ammoniac 2M dans l'éthanol et de 96 mg de (4Z)-4-[(1-acétyl-5-chloro-1H-pyrrolo[2,3-b]pyridin-3-yl)méthylidène]-2-phényl-1,3-oxazol-5(4H)-one dans 4 cm³ d'éthanol. Après quatre heures à 170°C sous irradiation micro-ondes et filtration du solide, on obtient 30 mg de (5Z)-5-[(5-chloro-1H-pyrrolo[2,3-b]pyridin-3-yl)méthylidène]-2-phényl-3,5-dihydro-4H-imidazol-4-one sous forme d'une poudre jaune dont les caractéristiques sont les suivantes :
   Point de fusion : 250°C.
   Spectre de RMN ¹H à 400MHz : 7,36 (s, 1H) ; 7,61 (m, 2H) ; 8,16 (m, 2H) ; 8,35 (d, J = 2,4 Hz, 1H) ; 8,54 (s, 1H) ; 9,43 (s, 1H) ; 11,93 (s large, 1H) ; 12,68 (s large, 1H).
b) La (4Z)-4-[(1-acétyl-5-chloro-1H-pyrrolo[2,3-b]pyridin-3-yl)méthylidène]-2-phényl-1,3-oxazol-5(4H)-one peut être préparée comme dans l'exemple 83, mais à partir de 208 mg d'acide hippurique, de 98 mg d'acétate de potassium et de 0,2 g de 5-chloro-1H-pyrrolo[2,3-b]pyridine-3-carbaldéhyde dans 5 cm³ d'anhydride acétique. Après 4 heures à 100°C, le mélange réactionnel est ramené à 25°C puis dilué par de l'eau. Le solide est filtré pour obtenir 222 mg de (4Z)-4-[(1-acétyl-5-chloro-1H-pyrrolo[2,3-b]pyridin-3-yl)méthylidène]-2-phényl-1,3-oxazol-5(4H)-one sous forme d'une poudre orange dont les caractéristiques sont les suivantes :
   Point de fusion : 242°C.

### Exemple 93

### (5Z)-2-[benzyl(méthyl)amino]-5-[(5-chloro-1H-pyrrolo[2,3-b]pyridin-3-yl)méthylidène]-3,5-dihydro-4H-imidazol-4-one

La 5Z)-2-[benzyl(méthyl)amino]-5-[(5-chloro-1H-pyrrolo[2,3-b]pyridin-3-yl)méthylidène]-3,5-dihydro-4H-imidazol-4-one peut être préparée comme dans l'exemple 7, mais à partir de 100 mg de (5Z)-5-[(5-chloro-1H-pyrrolo[2,3-b]pyridin-3-yl)méthylène]-2-thioxoimidazolidin-4-one (exemple 3), de 1,5 cm³ d'éthanol et de 435 mg de N-méthyl-1-phénylméthanamine. Après quatrevingt-dix minutes à une température de 180°C sous irradiation micro-ondes puis filtration du solide, on obtient 39 mg de 5Z)-2-[benzyl(méthyl)amino]-5-[(5-chloro-1H-pyrrolo[2,3-b]pyridin-3-yl)méthylidène]-3,5-dihydro-4H-imidazol-4-one sous forme d'une poudre jaune dont les caractéristiques sont les suivantes :
Point de fusion : 310°C.
Spectre de RMN ¹H à 400MHz : 3.05 (s, 3H) ; 4.73 (br s, 2H) ; 6.66 (s, 1H) ; 7.20-7.47 (m, 5H) ; 8.21 (s, 2H) ; 9.06 (s, 1H) ; 11.22 (br s, 1H) ; 12.17 (b s, 1H).
Spectre de masse: UPLC-MS-DAD-ELSD : 364(-)=(M-H)(-) ; 366 (+) = (M+H) (+).

### Exemple 94

### (5Z)-5-[(5-chloro-1H-pyrrolo[2,3-b]pyridin-3-yl)méthylidène]-2-[(furan-2-ylméthyl)(méthyl)amino]-3,5-dihydro-4H-imidazol-4-one

La 5Z)-5-[(5-chloro-1H-pyrrolo[2,3-b]pyridin-3-yl)méthylidène]-2-[(furan-2-ylméthyl)(méthyl)amino]-3,5-dihydro-4H-imidazol-4-one peut être préparée comme dans l'exemple 7, mais à partir de 100 mg de (5Z)-5-[(5-chloro-1H-pyrrolo[2,3-b]pyridin-3-yl)méthylène]-2-thioxoimidazolidin-4-one (exemple 3), de 1,5 cm³ d'éthanol et de 399 mg de 1-furan-2-yl-N-méthylméthanamine. Après une heure à une température de 180°C sous irradiation micro-ondes puis filtration du solide, on obtient 49 mg de 5Z)-5-[(5-chloro-1H-pyrrolo[2,3-b]pyridin-3-yl)méthylidène]-2-[(furan-2-ylméthyl)(méthyl)amino]-3,5-dihydro-4H-imidazol-4-one sous forme d'une poudre brune dont les caractéristiques sont les suivantes :
Point de fusion : 315°C.
Spectre de RMN ¹H à 400MHz : 3.07 (s, 3H); 4.71 (b s, 2H); 6.44 (s, 2H); 6.67 (s, 1H); 7.64 (s, 1H); 8.23 (s, 2H); 9.09 (b s, 1H); 11.11 (b s, 1H), 12.15 (b s, 1H). Spectre de masse: UPLC-MS-DAD-ELSD : 354(-)=(M-H)(-) ; 356(+)=(M+H)(+).

### Exemple 95

### (5Z)-5-[(5-chloro-1H-pyrrolo[2,3-b]pyridin-3-yl)méthylidène]-2-[(2-méthoxyéthyl)(méthyl)amino]-3,5-dihydro-4H-imidazol-4-one

La (5Z)-5-[(5-chloro-1H-pyrrolo[2,3-b]pyridin-3-yl)méthylidène]-2-[(2-méthoxyéthyl)(méthyl)amino]-3,5-dihydro-4H-imidazol-4-one peut être préparée comme dans l'exemple 7, mais à partir de 100 mg de (5Z)-5-[(5-chloro-1H-pyrrolo[2,3-b]pyridin-3-yl)méthylène]-2-thioxoimidazolidin-4-one (exemple 3), de 1,5 cm³ d'éthanol et de 320 mg de 2-méthoxy-N-méthyléthanamine. Après quinze minutes sous irradiation micro-ondes à chacune des températures de 160, 165, 170, 175 et 180°C, puis filtration du solide, on obtient 50 mg de (5Z)-5-[(5-chloro-1H-pyrrolo[2,3-b]pyridin-3-yl)méthylidène]-2-[(2-méthoxyéthyl)(méthyl)amino]-3,5-dihydro-4H-imidazol-4-one sous forme d'une poudre jaune dont les caractéristiques sont les suivantes :
Point de fusion : 281°C.
Spectre de RMN ¹H à 400MHz : 3.13 (b s, 3H) ; 3.28-3.30 (m, 2H) ; 3.58 (b s, 3H) ; 3.65 (b s, 2H) ; 6.61 (s, 1H) ; 8.18 (s, 1H) ; 8.22 (d, J = 2 Hz, 1H); 9.10 (b s, 1H) ; 11.07 (b s, 1H) ; 12.18 (b s, 1H).
Spectre de masse: UPLC-MS-DAD-ELSD : 332(-)=(M-H)(-) 334 4 (+) = (M+H) (+).

### Exemple 96

### (5Z)-5-[(5-chloro-1H-pyrrolo[2,3-b]pyridin-3-yl)méthylidène]-2-[méthyl(pyridin-2-ylméthyl)amino]-3,5-dihydro-4H-imidazol-4-one

La (5Z)-5-[(5-chloro-1H-pyrrolo[2,3-b]pyridin-3-yl)méthylidène]-2-[méthyl(pyridin-2-ylméthyl)amino]-3,5-dihydro-4H-imidazol-4-one peut être préparée comme dans l'exemple 7, mais à partir de 100 mg de (5Z)-5-[(5-chloro-1H-pyrrolo[2,3-b]pyridin-3-yl)méthylène]-2-thioxoimidazolidin-4-one (exemple 3), de 1,5 cm³ d'éthanol et de 439 mg de N-méthyl-1-pyridin-2-ylméthanamine. Après deux heures à une température de 180°C sous irradiation micro-ondes puis filtration du solide, on obtient 18 mg de (5Z)-5-[(5-chloro-1H-pyrrolo[2,3-b]pyridin-3-yl)méthylidène]-2-[méthyl(pyridin-2-ylméthyl)amino]-3,5-dihydro-4H-imidazol-4-one sous forme d'une poudre jaune dont les caractéristiques sont les suivantes :
Point de fusion : 350°C.
Spectre de RMN ¹H à 400MHz : 3.07 (s, 3H) ; 4.78 (s, 2H) ; 6.68 (s, 1H) ; 7.40 (dd, J = 7.3, 4.9 Hz, 1H) ; 7.77 (d, J = 8.3 Hz, 1H) ; 8.11-8.27 (m, 2H) ; 8.51 (d, J = 4.4 Hz, 1H) ; 8.60 (s, 1H) ; 9.05 (b s, 1H) ; 11.25 (b s, 1H) ;12.20 (b s, 1H).
Spectre de masse: UPLC-MS-DAD-ELSD : 365(-)=(M-H)(-) ; 367 (+) = (M+H) (+) .

### Exemple 97

### (5Z)-5-[(5-chloro-1H-pyrrolo[2,3-b]pyridin-3-yl)méthylidène]-2-phénoxy-3-phényl-3,5-dihydro-4H-imidazol-4-one

a) La (5Z)-5-[(5-chloro-1H-pyrrolo[2,3-b]pyridin-3-yl)méthylidène]-2-phénoxy-3-phényl-3,5-dihydro-4H-imidazol-4-one peut être préparée comme dans l'exemple 1, mais à partir de mais à partir de 42 mg de 5-chloro-1H-pyrrolo[2,3-b]pyridine-3-carbaldéhyde dans 4 cm³ d'éthanol, de 59 mg de 2-phénoxy-3-phényl-3,5-dihydro-4H-imidazol-4-one et de 0,023 cm³ de pipéridine. Après cinq heures de reflux, on obtient 60 mg de (5Z)-5-[(5-chloro-1H-pyrrolo[2,3-b]pyridin-3-yl)méthylidène]-2-phénoxy-3-phényl-3,5-dihydro-4H-imidazol-4-one sous forme d'un solide jaune pâle dont les caractéristiques sont les suivantes :
   Point de fusion : 287°C.
   Spectre de RMN ¹H à 400MHz : 6,96-7,77 (m, 11H), 8,18 (s, 1H), 8,20 (s, 1H), 8,80 (s, 1H), 12,5 (m étalé, 1H).
   Spectre de masse: m/z = 414 (M⁺˙)
b) La 2-phénoxy-3-phényl-3,5-dihydro-4H-imidazol-4-one peut être préparée de la manière suivante : à 0,2 g de N-[(phénylimino)méthylidène]glycinate de méthyle en solution dans 5 cm³ d'acétonitrile, on ajoute à une température voisine de 20°C, 0,1 g de phénol et 7 mg de carbonate de potassium. Après 24 heures d'agitation à une température voisine de 55°C, le mélange réactionnel est concentré à sec sous pression réduite pour donner un résidu qui est purifié par chromatographie-flash sur cartouche SVL D26 Merck SI60 25 g, 15-40µM, débit 20ml/min, vf 4,3 ml [éluant : acétate d'éthyle / cyclohexane (1 / 3 en volumes)]. Après concentration des fractions sous pression réduite, on obtient un résidu jaune qui est agité dans 5 cm³ d'éther de pétrole puis filtré et séché sous pression réduite pour donner 68 mg de 2-phénoxy-3-phényl-3,5-dihydro-4H-imidazol-4-one, sous forme d'un solide jaune pâle dont le spèctre de masse est le suivant : m/z = 252 (M⁺˙).
c) Le N-[(phénylimino)méthylidène]glycinate de méthyle peut être préparé de la manière suivante : à 3,17 g de N-(phénylcarbamoyl)glycinate de méthyle en suspension dans 100 cm³ de dichlorométhane, on ajoute à une température voisine de 0°C, sous atmosphère d'argon, 9,64 g de dibromo-triphénylphosphine et 6,35 cm³ de triéthylamine. On laisse remonter lentement la température à l'ambiante. Après 20 heures d'agitation à une température voisine de 20°C, le mélange réactionnel est lavé successivement avec 50 cm³ d'eau et 50 cm³ d'une solution aqueuse saturée en hydrogénocarbonate de potassium, séché sur sulfate de magnésium anhydre, filtré et concentré à sec sous pression réduite pour donner 9,5 g d'un solide brun qui est purifié par chromatographie-flash sur cartouche EVP D57 Merck SI60 200 g, 40-63µM, débit 30ml/min, vf 17,5 ml [éluant : acétate d'éthyle / cyclohexane (1 / 7 en volumes)]. Après concentration des fractions sous pression réduite, on obtient 1,47 g de N-[(phénylimino)méthylidène]glycinate de méthyle, sous forme d'une huile jaune pâle dont le spèctre de masse est le suivant : m/z = 190 (M⁺˙).
d) Le N-(phénylcarbamoyl)glycinate de méthyle peut être préparé de la manière suivante : à 2,51 g de chlorhydrate de glycinate de méthyle en suspension dans 50 cm³ de dichlorométhane, on ajoute à une température voisine de 20°C, sous atmosphère d'argon, 2,39 cm³ de isocyanatobenzène et 3,34 cm³ de triéthylamine. Après 5 heures d'agitation à une température voisine de 20°C, le mélange réactionnel est lavé avec 50 cm³ d'eau. La phase aqueuse est extraite avec 2 fois 50 cm³ de dichlorométhane. Les phases organiques sont réunies, séchées sur sulfate de magnésium anhydre, filtrées et concentrées à sec sous pression réduite pour donner un résidu qui est lavé successivement avec 5 fois 50 cm³ d'éther de pétrole et 3 fois 100 cm³ d'un mélange [éther de pétrole / diisopropyl éther (1 / 1 en volumes)]. Après séchage sous pression réduite à une température voisine de 20°C, on obtient 3,17 g de N-(phénylcarbamoyl)glycinate de méthyle, sous forme d'un solide blanc dont le spèctre de masse est le suivant : m/z = 208 (M⁺˙)

### Exemple 98

### (5Z)-5-[1-(5-Chloro-1H-pyrrolo[2,3-b]pyridin-3-yl)-méthylidène]-2-méthoxy-3-phényl-3,5-dihydro-4H-imidazol-4-one

a) (5Z)-5-[1-(5-Chloro-1H-pyrrolo[2,3-b]pyridin-3-yl)-méthylidène]-2-méthoxy-3-phényl-3,5-dihydro-4H-imidazol-4-one peut être préparée comme dans l'exemple 1, mais à partir de 34 mg de 5-chloro-1H-pyrrolo[2,3-b]pyridine-3-carbaldéhyde dans 4 cm³ d'éthanol, de 36 mg de 2-phénoxy-3-phényl-3,5-dihydro-4H-imidazol-4-one et de 0,02 cm³ de pipéridine. Après cinq heures de reflux, on obtient 43 mg de (5Z)-5-[1-(5-Chloro-1H-pyrrolo[2,3-b]pyridin-3-yl)-méthylidène]-2-méthoxy-3-phényl-3,5-dihydro-4H-imidazol-4-one sous forme d'un solide jaune pâle dont les caractéristiques sont les suivantes :
   Point de fusion : 227,5°C.
   Spectre de RMN ¹H à 400MHz : 4,18 (s, 3H), 7,29 (s, 1H), 7,42 (m, 3H), 7,52 (t, *J* = 7,6 Hz, 2H), 8,31 (s large, 1H), 8,41 (s, 1H), 9,18 (s large, 1H), 12,55 (m étalé, 1H).
   Spectre de masse: m/z = 352 (M⁺˙)
b) La 2-méthoxy-3-phényl-3,5-dihydro-4H-imidazol-4-one peut être préparée de la manière suivante : à 0,2 g de N-[(phénylimino)méthylidène]glycinate de méthyle (préparé comme décrit à l'exemple 97) en solution dans 5 cm³ de méthanol, on ajoute à une température voisine de 20°C, 9 mg de carbonate de potassium. Après six heures trente minutes d'agitation à une température voisine de 55°C, le mélange réactionnel est concentré à sec sous pression réduite (2,7 kPa) pour donner un résidu qui est purifié par chromatographie-flash sur cartouche SVL D26 Merck SI60 25 g, 15-40µM, débit 20ml/min, vf 4,3 ml [éluant : acétate d'éthyle / cyclohexane (1 / 1 en volumes)]. Après concentration des fractions sous pression réduite, on obtient un résidu jaune qui est agité dans 5 cm³ d'éther de pétrole puis filtré et séché sous pression réduite (2,7 kPa) pour donner 41 mg de 2-méthoxy-3-phényl-3,5-dihydro-4H-imidazol-4-one, sous forme d'un solide jaune pâle dont le spèctre de masse est le suivant : m/z = 190 (M⁺˙)

### Exemple 99

### (5Z)-5-[(5-chloro-1H-pyrrolo[2,3-b]pyridin-3-yl)méthylidène]-3-méthyl-2-phénoxy-3,5-dihydro-4H-imidazol-4-one

a) La (5Z)-5-[(5-chloro-1H-pyrrolo[2,3-b]pyridin-3-yl)méthylidène]-3-méthyl-2-phénoxy-3,5-dihydro-4H-imidazol-4-one peut être préparée comme dans l'exemple 1, mais à partir de mais à partir de 74 mg de 5-chloro-1H-pyrrolo[2,3-b]pyridine-3-carbaldéhyde dans 7 cm³ d'éthanol, de 70 mg de 3-méthyl-2-phénoxy-3,5-dihydro-4H-imidazol-4-one et de 0,038 cm³ de pipéridine. Après six heures de reflux, on obtient 48 mg de (5Z)-5-[(5-chloro-1H-pyrrolo[2,3-b]pyridin-3-yl)méthylidène]-3-méthyl-2-phénoxy-3,5-dihydro-4H-imidazol-4-one sous forme d'un solide jaune pâle dont les caractéristiques sont les suivantes :
   Spectre de RMN ¹H à 400MHz : 3,21 (s, 3H), 7,19 (s, 1H), 7,33-7,62 (m, 5H)_{,} 8,12 (s, 1H), 8,19 (s, 1H), 8,79 (s, 1H), 12,45 (m étalé, 1H)
   Spectre de masse: m/z = 352 (M⁺˙)
b) La 3-méthyl-2-phénoxy-3,5-dihydro-4H-imidazol-4-one peut être préparée de la manière suivante : à 0,151 g de N-[(méthylimino)méthylidène]glycinate de méthyle en solution dans 5 cm³ d'acétonitrile, on ajoute à une température voisine de 20°C, 0,1 g de phénol et 12 mg de carbonate de potassium. Après 6,5 heures d'agitation à une température voisine de 55°C, le mélange réactionnel est concentré à sec sous pression réduite (2,7 kPa) pour donner un résidu qui est purifié par chromatographie-flash sur cartouche SVL D26 Merck SI60 25 g, 15-40µM, débit 20ml/min, vf 4,3 ml [éluant : acétate d'éthyle / cyclohexane (1 / 1 en volumes)]. Après concentration des fractions sous pression réduite, on obtient un résidu jaune qui est agité dans 5 cm³ d'éther de pétrole puis filtré et séché sous pression réduite (2,7 kPa) pour donner 149 mg de 3-méthyl-2-phénoxy-3,5-dihydro-4H-imidazol-4-one, sous forme d'un solide blanc dont le spèctre de masse est le suivant : m/z = 190 (M⁺˙).
c) Le N-[(méthylimino)méthylidène]glycinate de méthyle peut être préparé de la manière suivante : à 1,37 g de N-(méthylcarbamoyl)glycinate de méthyle en suspension dans 50 cm³ de dichlorométhane, on ajoute à une température voisine de 0°C, sous atmosphère d'argon, 5,42 g de dibromo-triphénylphosphine et 3,6 cm³ (25,66 mmol) de triéthylamine. On laisse remonter lentement la température à l'ambiante. Après 24 heures d'agitation à une température voisine de 20°C, le mélange réactionnel est filtré plusieurs fois sur verre fritté N°4. Le filtrat est repris avec 4 fois 25 cm³ d'éther de pétrole et filtré à chaque fois pour éliminer le maximum d'oxyde de triphénylphosphine. Le filtrat est concentré à sec sous pression réduite (2,7 kPa) pour donner 0,74 g d'une huile jaune qui est purifiée par chromatographie sur 74 g d'oxyde d'alumine (Fluka type 507c neutral) activé avec 4,4 cm³ (6%) d'eau, Patm, vf 20 ml (éluant : dichlorométhane). Après concentration des fractions sous pression réduite, on obtient 0,48 g de N-[(méthylimino)méthylidène]glycinate de méthyle, sous forme d'une huile jaune pâle dont le spèctre de masse est le suivant : m/z = 142 (M⁺˙).
d) Le N-(méthylcarbamoyl)glycinate de méthyle peut être préparé de la manière suivante : à 1,0 g N-(oxométhylidène)glycinate de méthyle en solution dans 8 cm³ de tétrahydrofurane, on ajoute à une température voisine de 20°C, sous atmosphère d'argon, 3,9 cm³ d'une solution 2M de méthyl amine dans le tétrahydrofurane. Après 4 heures d'agitation à une température voisine de 20°C, le mélange réactionnel est filtré sur verre fritté N°4 puis le filtrat est concentré à sec sous pression réduite (2,7 kPa) pour donner un résidu blanc qui est agité avec 3 fois 25 cm³ d'éther de pétrole puis filtré et séché sous pression réduite (2,7 kPa) pour donner 1,37 g de N-(méthylcarbamoyl)glycinate de méthyle, sous forme d'un solide blanc dont le spèctre de masse est le suivant : m/z = 160 (M⁺˙)

### Exemple 100: Composition pharmaceutique

On a préparé des comprimés répondant à la formule suivante :

| | |
|---|---|
| Produit de l'exemple 1 | 0,2 g |
| Excipient pour un comprimé terminé à | 1 g |

(détail de l'excipient : lactose, talc, amidon, stéarate de magnésium).

### Exemple 101: Composition pharmaceutique

On a préparé des comprimés répondant à la formule suivante :

| | |
|---|---|
| Produit de l'exemple 10 | 0,2 g |
| Excipient pour un comprimé terminé à | 1 g |

(détail de l'excipient : lactose, talc, amidon, stéarate de magnésium).

Les exemples 1 et 10 sont pris à titre d'exemples de préparation pharmaceutique, cette préparation pouvant être réalisée si désiré avec d'autres produits en exemples dans la présente demande.

## Revendications

1. Produits de formule (I): 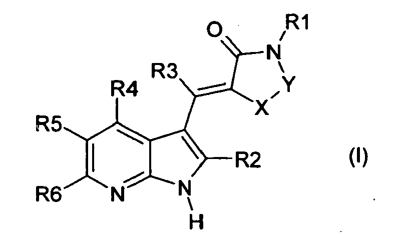 dans laquelle :
X-Y représente NH-C(S), N=C-NR7R8, N=C-SR, N=C-R
ou N=C-OR ;
R1 représente l'atome d'hydrogène, un radical cycloalkyle ou un radical alkyle, hétérocycloalkyle, aryle ou hétéroaryle, tous ces radicaux étant éventuellement substitués;
R identique ou différent de R1 est choisi parmi les valeurs de R1 ;
R2 représente l'atome d'hydrogène , un atome d'halogène ou un radical alkyle ;
R3 représente l'atome d'hydrogène, un atome d'halogène , le radical hydroxyle ou un radical alkyle ou alcoxy ;
R4 représente l'atome d'hydrogène , un atome d'halogène ou un radical cyano, CF3 ou alkyle;
R5 représente l'atome d'hydrogène, un atome d'halogène, un radical hydroxyle, cyano, NR7R8, CONR7R8, NR11COR12 ou un radical cycloalkyle, alkyle, alcoxy, hétérocycloalkyle, aryle ou hétéroaryle, tous ces derniers radicaux étant éventuellement substitués ;
R6 représente l'atome d'hydrogène , un atome d'halogène ou un radical NR7R8, alkyle ou alcoxy ;
R7 et R8 sont tels que :
soit R7 et R8 identiques ou différents sont tels que l'un de R7 et R8 représente l'atome d'hydrogène ou un radical alkyle éventuellement substitué;
et l'autre de R7 et R8 représente un atome d'hydrogène ou un radical cycloalkyle, alkyle, hétérocycloalkyle, hétéroaryle ou aryle, tous ces radicaux étant éventuellement substitués ;
soit R7 et R8 forment ensemble avec l'atome d'azote auquel ils sont liés un radical cyclique formé de 3 à 7 chaînons renfermant éventuellement un ou plusieurs autres hétéroatomes choisi(s) parmi O, S ou N, N étant éventuellement substitué par R11, ce radical cyclique étant lui-même éventuellement substitué;
tous les radicaux alkyle, alcoxy, cycloalkyle hétérocycloalkyle, hétéroaryle et aryle, ainsi que le radical cyclique que peuvent former R7 et R8 ensemble avec l'atome d'azote auquel ils sont liés, indiqués comme éventuellement substitués, étant ainsi éventuellement substitués par un ou plusieurs radicaux identiques ou différents choisis parmi les atomes d'halogène et les radicaux hydroxyle, cyano, nitro, CF3, NR9R10, NHCOR11, NHCO2R11, NHCONR9R10, NHSO2R13, COOH, COOalk, CONR9R10, SO2NR9R10 alcoxy, alkylthio, haloalcoxy, haloalkylthio, alkyle, fluoroalkyle, hydroxyalkyle, cycloalkyle, hétérocycloalkyle, hétéroaryle et phényle, ces derniers radicaux hétéroaryle, aryle et phényle, étant eux-mêmes éventuellement substitués par un ou plusieurs radicaux identiques ou différents choisis parmi les atomes d'halogène et les radicaux hydroxyle, NH2, NH(alk), N(alk)2, alkyle, hydroxyalkyle et alcoxy ;
R9 et R10 sont tels que :
soit R9 et R10 identiques ou différents sont tels que l'un de R9 et R10 représente l'atome d'hydrogène ou un radical alkyle éventuellement substitué par un ou plusieurs radicaux identiques ou différents choisis parmi les atomes d'halogène et les radicaux hydroxyle et alcoxy ;
et l'autre de R9 et R10 représente un atome d'hydrogène ou un radical cycloalkyle, alkyle, hétérocycloalkyle, hétéroaryle ou aryle, tous ces radicaux étant eux-mêmes éventuellement substitués par un ou plusieurs radicaux identiques ou différents choisis parmi les atomes d'halogène et les radicaux hydroxyle, NH2, NH(alk), N(alk)2, alkyle, hydroxyalkyle et alcoxy ;
soit R9 et R10 forment ensemble avec l'atome d'azote auquel ils sont liés un radical cyclique formé de 3 à 7 chaînons renfermant éventuellement un ou plusieurs autres hétéroatomes choisi(s) parmi O, S ou N, N étant éventuellement substitué par R12, ce radical cyclique étant lui-même éventuellement substitué par un ou plusieurs radicaux identiques ou différents choisis parmi les atomes d'halogène et les radicaux hydroxyle, NH2, NH(alk), N(alk)2, alkyle, hydroxyalkyle et alcoxy ;
R11 et R12, identiques ou différents représentent l'atome d'hydrogène ou un radical alkyle ou phényle éventuellement substitués par un ou plusieurs radicaux identiques ou différents choisis parmi les atomes d'halogène et les radicaux hydroxyle et alcoxy, le radical phényle étant lui-même éventuellement substitué par un ou plusieurs radicaux alkyle ;
R13 représente un radical alkyle ou phényle éventuellement substitués par un ou plusieurs radicaux identiques ou différents choisis parmi les atomes d'halogène et les radicaux hydroxyle et alcoxy, le radical phényle étant lui-même éventuellement substitué par un ou plusieurs radicaux alkyle ;
tous les radicaux alkyle (alk) et alcoxy ci-dessus étant linéaires ou ramifiés et renfermant au plus 12 atomes de carbone ;
étant entendu que sont exclus les produits de formule (I) dans lesquels toutes les conditions ci-dessous sont remplies:
- R2 représente hydrogène ;
- R3 représente hydrogène ou alkyle ;
- X-Y représente N=C-NR7R8, N=C-SR ou N=CR dans lesquels X représente N et Y représente =C-NR7R8, =C-SR ou =CR et avec R représente aryle ou hétéroaryle
- R1 représente H ou alk ;
- et R4, R5 et R6 sont tels que deux d'entre eux représentent H et l'autre représente hydrogène, NH2 ou NHalk,
lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

2. Produits de formule (I) tels que définis à la revendication 1 dans laquelle :
R1 représente l'atome d'hydrogène, ou un radical alkyle, tous ces radicaux étant éventuellement substitués comme indiqué à la revendication 1 ou à l'une quelconque des autres revendications;
et R5 représente, un atome d'halogène, un radical hydroxyle, cyano, NR7R8, CF3 ou un radical cycloalkyle, alkyle, alcoxy, hétérocycloalkyle, aryle ou hétéroaryle,
tous ces derniers radicaux étant éventuellement substitués comme indiqué à la revendication 1 ou à l'une quelconque des autres revendications ;
les autres radicaux substituants R2,R3,R4,R6 et X-Y ayant les valeurs définies à la revendication 1 ou à l'une quelconque des autres revendications,
lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

3. Produits de formule (I) tels que définis à la revendication 1 dans laquelle :
R2 représente l'atome d'hydrogène ;
et R5 représente un atome d'halogène, un radical hydroxyle, cyano, NR7R8, CF3 ou un radical cycloalkyle, alkyle, alcoxy, hétérocycloalkyle, aryle ou hétéroaryle, tous ces derniers radicaux étant éventuellement substitués comme indiqué à la revendication 1 ou à l'une quelconque des autres revendications;
les autres radicaux substituants R1,R3,R4,R6, et X-Y ayant les valeurs définies à la revendication 1 ou à l'une quelconque des autres revendications,
lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

4. Produits de formule (I) tels que définis à la revendication 1 dans laquelle :
R3 représente l'atome d'hydrogène ;
et R5 représente un atome d'halogène, un radical hydroxyle, cyano, CONR7R8 ou un radical cycloalkyle, alkyle, alcoxy, hétérocycloalkyle, aryle ou hétéroaryle, tous ces derniers radicaux étant éventuellement substitués comme indiqué à la revendication 1 ou à l'une quelconque des autres revendications ;
les autres radicaux R2, R3, R4, R6 et X-Y ayant les valeurs définies à la revendication 1 ou à l'une quelconque des autres revendications,
lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

5. Produits de formule (I) tels que définis à la revendication 1 dans laquelle :
X-Y représente NH-C(S), N=C-NR7R8 ou N=C-R;
R1 représente l'atome d'hydrogène, un radical cycloalkyle ou un radical alkyle, hétérocycloalkyle, aryle ou hétéroaryle, tous ces radicaux étant éventuellement substitués comme indiqué à la revendication 1 ou à l'une quelconque des autres revendications;
R identique ou différent de R1 est choisi parmi les valeurs de R1 telles que définies à la revendication 1 comme indiqué à la revendication 1 ou à l'une quelconque des autres revendications à l'exception de aryle et hétéroaryle;
les autres substituants R2, R3, R4, R5 et R6 ayant les valeurs définies à la revendication 1 ou à l'une quelconque des autres revendications ;
lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

6. Produits de formule (I) tels que définis à la revendication 1 dans laquelle :
X-Y représente NH-C(S), N=C-NR7R8; N=C-SR, N=C-R ou N=COR ;
R1 représente l'atome d'hydrogène, un radical cycloalkyle ou un radical alkyle, hétérocycloalkyle, phényle ou hétéroaryle, ces derniers radicaux étant éventuellement substitués;
R identique ou différent de R1 est choisi parmi les valeurs de R1 ;
R2 représente l'atome d'hydrogène , un atome d'halogène ou un radical alkyle ;
R3 représente l'atome d'hydrogène, un atome d'halogène , le radical hydroxyle ou un radical alkyle ou alcoxy ;
R4 représente l'atome d'hydrogène , un atome d'halogène ou un radical cyano, CF3 ou alkyle;
R5 représente l'atome d'hydrogène, un atome d'halogène, un radical hydroxyle, cyano, NR7R8, CONR7R8, NR11COR12 ou un radical cycloalkyle, alkyle, alcoxy, hétérocycloalkyle, phényle ou hétéroaryle, tous ces derniers radicaux étant éventuellement substitués ;
R6 représente l'atome d'hydrogène , un atome d'halogène ou un radical NR7R8, alkyle ou alcoxy ;
R7 et R8 sont tels que :
soit R7 et R8 identiques ou différents sont tels que l'un de R7 et R8 représente l'atome d'hydrogène ou un radical alkyle éventuellement substitué par un ou plusieurs radicaux identiques ou différents choisis parmi les atomes d'halogène et les radicaux hydroxyle et alcoxy ;
et l'autre de R7 et R8 représente un atome d'hydrogène ou un radical cycloalkyle, alkyle, hétérocycloalkyle, hétéroaryle ou phényle, tous ces radicaux étant éventuellement substitués ;
soit R7 et R8 forment ensemble avec l'atome d'azote auquel ils sont liés un radical cyclique formé de 3 à 7 chaînons renfermant éventuellement un ou plusieurs autres hétéroatomes choisi(s) parmi O, S ou N, N étant éventuellement substitué par R11, ce radical cyclique étant lui-même éventuellement substitué;
tous les radicaux alkyle, alcoxy, cycloalkyle hétérocycloalkyle, hétéroaryle et aryle, ainsi que le radical cyclique que peuvent former R7 et R8 ensemble avec l'atome d'azote auquel ils sont liés, indiqués comme éventuellement substitués, étant ainsi éventuellement substitués par un ou plusieurs radicaux identiques ou différents choisis parmi les atomes d'halogène et les radicaux hydroxyle, cyano, nitro, CF3, NR9R10, NHCOR11, NHSO2R13, COOH, COOalk, CONR9R10, SO2NR9R10, alcoxy, haloalcoxy, alkyle, fluoroalkyle, hydroxyalkyle, cycloalkyle, hétérocycloalkyle, hétéroaryle et phényle, ces derniers radicaux hétéroaryle et phényle, étant eux-mêmes éventuellement substitués par un ou plusieurs radicaux identiques ou différents choisis parmi les atomes d'halogène et les radicaux hydroxyle, NH2, NH(alk), N(alk)2, alkyle, hydroxyalkyle et alcoxy ;
R9 et R10 sont tels que :
soit R9 et R10 identiques ou différents sont tels que l'un de R9 et R10 représente l'atome d'hydrogène ou un radical alkyle et l'autre de R9 et R10 représente un atome d'hydrogène ou un radical alkyle, phényle ou phénylalkyle eux-mêmes éventuellement substitués par un ou plusieurs radicaux identiques ou différents choisis parmi les atomes d'halogène et les radicaux hydroxyle, NH2, NH(alk), N(alk)2, alkyle, hydroxyalkyle et alcoxy ;
soit R9 et R10 forment ensemble avec l'atome d'azote auquel ils sont liés un radical pyrrolidinyle, morpholine, pipéridyle, azépinyle ou pipérazinyle éventuellement substitué par un radical alkyle ou phényle lui-même éventuellement substitué par un ou plusieurs radicaux identiques ou différents choisis parmi les atomes d'halogène et les radicaux alkyles, hydroxyle, NH2, NH(alk), N(alk)2, alkyle, hydroxyalkyle et alcoxy;
R11 et R12, identiques ou différents représentent l'atome d'hydrogène ou un radical alkyle ou phényle;
R13 représente un radical alkyle ou phényle;
tous les radicaux alkyle (alk) et alcoxy ci-dessus étant linéaires ou ramifiés et renfermant au plus 6 atomes de carbone ;
lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

7. Produits de formule (I) tels que définis à l'une quelconque des autres revendications dans laquelle:
X-Y représente NH-C(S), N=C-NR7R8, N=C-SR, N=C-R ou N=COR;
R1 représente l'atome d'hydrogène ou un radical alkyle ou phényle éventuellement substitués ;
R représente l'atome d'hydrogène ; un radical cycloalkyle; un radical alkyle ; hétérocycloalkyle ; phényle ; ou hétéroaryle ; tous ces radicaux étant éventuellement substitués;
R2 représente l'atome d'hydrogène , un atome d'halogène ou un radical alkyle ;
R3 représente l'atome d'hydrogène, un atome d'halogène , le radical hydroxyle ou un radical alkyle;
R4 représente l'atome d'hydrogène , un atome d'halogène ou un radical alkyle ;
R5 représente l'atome d'hydrogène ; un atome d'halogène ; le radical hydroxyle ; cyano ; NR7R8 ; alkyle; alcoxy ; hétérocycloalkyle; phényle; ou hétéroaryle; ces derniers radicaux ainsi que le reste phényle de NHphényle et NH(phénylalk), étant éventuellement substitués ;
R6 représente l'atome d'hydrogène , un atome d'halogène, ou un radical NH2, NHalk, N(alk)2, alkyle ou alcoxy ;
R7 et R8 sont tels que :
soit R7 et R8 identiques ou différents sont tels que l'un de R7 et R8 représente l'atome d'hydrogène ou un radical alkyle ;
et l'autre de R7 et R8 représente un atome d'hydrogène, ou un radical alkyle ou cycloalkyle, éventuellement substitués ;
soit R7 et R8 forment ensemble avec l'atome d'azote auquel ils sont liés un radical cyclique choisis parmi les radicaux azétidyle ; pipéridyle ; azépanyle; morpholinyle ; thiomorpholinyle ; pyrrolidinyle ; imidazolidinyle ; pipérazinyle éventuellement substitué sur son second atome d'azote par un radical alkyle ou phényle eux-mêmes éventuellement substitués ; et homopipérazinyle, ces radicaux étant éventuellement substitués ;
tous les radicaux alkyle, alcoxy, hétérocycloalkyle, hétéroaryle et phényle, ainsi que le radical cyclique que peuvent former R7 et R8 ensemble avec l'atome d'azote auquel ils sont liés, indiqués comme éventuellement substitués, étant ainsi éventuellement substitués par un ou plusieurs radicaux identiques ou différents choisis parmi les atomes d'halogène et les radicaux hydroxyle, NH2, NH(alk), N(alk)2, NHphényle, NH(phénylalk), alkyle, CF3, alcoxy, OCF3 , cycloalkyle, hétérocycloalkyle, hétéroaryle et phényle ; ces derniers radicaux hétéroaryle et phényle , ainsi que le reste phényle des radicaux NHphényle et NH(phénylalk), étant eux-mêmes éventuellement substitués par un ou plusieurs radicaux identiques ou différents choisis parmi les atomes d'halogène et les radicaux hydroxyle, NH2, NHAlk, N(Alk)2, alkyle, hydroxyalkyle et alcoxy;
tous les radicaux alkyle (alk) et alcoxy ci-dessus étant linéaires ou ramifiés et renfermant au plus 6 atomes de carbone ;
lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

8. Produits de formule (I) tels que définis à l'une quelconque des autres revendications dans laquelle:
X-Y représente NH-C(S), N=C-NR7R8, N=C-SR, N=C-R ou N=COR;
R1 représente l'atome d'hydrogène ou un radical alkyle ou phényle éventuellement substitués ;
R identique ou différent de R1 est choisi parmi les valeurs de R1 ;
R2 représente l'atome d'hydrogène , un atome d'halogène ou un radical alkyle ;
R3 représente l'atome d'hydrogène, un atome d'halogène , le radical hydroxyle ou un radical alkyle;
R4 représente l'atome d'hydrogène , un atome d'halogène ou un radical alkyle ;
R5 représente l'atome d'hydrogène , un atome d'halogène, le radical hydroxyle, un radical NH2, NHalk, N(alk)2, NR7R8, NHphényle, NH(phénylalk) ou un radical alkyle, hétérocycloalkyle, alcoxy, phényle ou hétéroaryle, ces derniers radicaux ainsi que le reste phényle de NHphényle et NH(phénylalk), étant éventuellement substitués ;
R6 représente l'atome d'hydrogène , un atome d'halogène, ou un radical NH2, NHalk, N(alk)2, alkyle ou alcoxy ;
R7 et R8 sont tels que :
soit R7 et R8 identiques ou différents sont tels que l'un de R7 et R8 représente l'atome d'hydrogène ou un radical alkyle ;
et l'autre de R7 et R8 représente un atome d'hydrogène, ou un radical alkyle ou cycloalkyle, éventuellement substitué ;
soit R7 et R8 forment ensemble avec l'atome d'azote auquel ils sont liés un radical cyclique renfermant 4 à 6 chaînons choisis parmi azétidyle ; pipéridyle ; morpholinyle ; thiomorpholinyle ; pyrrolidinyle ; imidazolidinyle ; pipérazinyle ; et homopipérazinyle, ces radicaux étant éventuellement substitués ;
tous les radicaux alkyle, alcoxy, hétéroaryle et phényle, ainsi que le radical cyclique que peuvent former R7 et R8 ensemble avec l'atome d'azote auquel ils sont liés, indiqués comme éventuellement substitués, étant ainsi éventuellement substitués par un ou plusieurs radicaux identiques ou différents choisis parmi les atomes d'halogène et les radicaux hydroxyle, NH2, NH(alk), N(alk)2, NHphényle, NH(phénylalk), alkyle, CF3, alcoxy, OCF3 , cycloalkyle, hétérocycloalkyle, hétéroaryle et phényle ; ces derniers radicaux hétéroaryle et phényle , ainsi que le reste phényle des radicaux NHphényle et NH(phénylalk), étant eux-mêmes éventuellement substitués par un ou plusieurs radicaux identiques ou différents choisis parmi les atomes d'halogène et les radicaux hydroxyle, NH2, NHAlk, N(Alk)2, alkyle, hydroxyalkyle et alcoxy;
tous les radicaux alkyle (alk) et alcoxy ci-dessus étant linéaires ou ramifiés et renfermant au plus 6 atomes de carbone ;
lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

9. Produits de formule (I) telle que définie à l'une quelconque des autres revendications dans laquelle X-Y, R2, R3, R4 et R6 ont les significations indiquées à l'une quelconque des autres revendications,
R1 représente l'atome d'hydrogène ou un radical alkyle éventuellement substitué ;
R5 représente l'atome d'hydrogène, un atome d'halogène, un radical hydroxyle, CF3, NH2, NHalk, N(alk)2 ou un radical alkyle, alcoxy ou phényle, éventuellement substitués ;
le radical alkyle que peut représenter R1 ou le radical alkyle, alcoxy ou phényle que peut représenter R5, étant éventuellement substitués par un ou plusieurs radicaux identiques ou différents choisis parmi les atomes d'halogène et les radicaux hydroxyle, NHalk, N(alk)2, alcoxy, cycloalkyle, hétérocycloalkyle, hétéroaryle et phényle, ces derniers radicaux hétéroaryle et phényle étant éventuellement substitués par un ou plusieurs radicaux identiques ou différents choisis parmi les atomes d'halogène et les radicaux hydroxyle, NH2, NHAlk, N(Alk)2, alkyle, hydroxyalkyle et alcoxy;
R7 et R8 sont tels que :
soit R7 et R8 identiques ou différents sont tels que l'un de R7 et R8 représente l'atome d'hydrogène ou un radical alkyle,
et l'autre de R7 et R8 représente un atome d'hydrogène, un radical alkyle éventuellement substitué par un ou plusieurs radicaux identiques ou différents choisis parmi les atomes d'halogène, les radicaux hydroxyle, NH2, NHalk, N(alk)2, NH(phényle), NH(phénylalk), alcoxy, OCF3, cycloalkyle, et les radicaux pyrrolidinyle, pipérazinyle, pipéridyle, morpholinyle et phényle, tous ces derniers radicaux cycliques, ainsi que le reste phényle du radical phénylalkyle, étant eux-mêmes éventuellement substitués par un ou plusieurs radicaux identiques ou différents choisis parmi les atomes d'halogène et les radicaux hydroxyle, NH2, NHAlk, N(alk)2, alcoxy, alkyle et hydroxyalkyle;
soit R7 et R8 forment ensemble avec l'atome d'azote auquel ils sont liés un radical choisi préférentiellement parmi les radicaux pipéridyle, morpholinyle, et les radicaux pyrrolidinyle, pipérazinyle et homopipérazinyle éventuellement substitués par un ou plusieurs radicaux identiques ou différents choisis parmi les radicaux alkyle et phényle, eux-mêmes éventuellement substitués par un ou plusieurs radicaux choisis parmi les atomes d'halogène et les radicaux hydroxyle, NH2, NHAlk, N(Alk)2, alcoxy et cycloalkyle;
tous les radicaux alkyle (alk) et alcoxy ci-dessus étant linéaires ou ramifiés et renfermant au plus 6 atomes de carbone;
lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

10. Produits de formule (I) telle que définie à l'une quelconque des autres revendications dans laquelle
X-Y représente NH-C(S), N=C-NR7R8 ou N=C-R,
R7, R8 et R étant choisis parmi toutes les valeurs définies à l'une quelconque des autres revendications pour R7, R8 et R et les autres substituants R1, R2, R3, R4, R5 et R6 desdits produits de formule (I) étant choisis parmi toutes les valeurs définies à l'une quelconque des autres revendications respectivement pour R1, R2, R3, R4, R5 et R6,
lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

11. Produits de formule (I) telle que définie à l'une quelconque des autres revendications dans laquelle
X-Y représente NH-C(S), N=C-NR7R8 ou N=C-R;
R1 représente l'atome d'hydrogène ou un radical alkyle éventuellement substitué par un ou plusieurs radicaux identiques ou différents choisis parmi les atomes d'halogène et les radicaux N(alk)2 et alcoxy ;
R identique ou différent de R1 est choisi parmi les valeurs de R1 ;
R2 représente l'atome d'hydrogène, un atome d'halogène ou un radical alkyle ;
R3 représente l'atome d'hydrogène ou un radical alkyle ;
R4 représente l'atome d'hydrogène ou un atome d'halogène,
R5 représente l'atome d'hydrogène, un atome d'halogène ou un radical hydroxyle, NH2, NHalk, N(alk)2, alkyle, alcoxy ou phényle, le radical alkyle étant éventuellement substitué par un radical alcoxy, N(alk)2 ou hétérocycloalkyle et le radical phényle étant éventuellement substitué par un ou plusieurs radicaux identiques ou différents choisis parmi les atomes d'halogène et les radicaux hydroxyle, NH2, NHalk, N(alk)2, alkyle et alcoxy ;
R6 représente un atome d'hydrogène, un atome d'halogène ou un radical alkyle ;
et R7 et R8 sont tels que :
soit R7 et R8 identiques ou différents sont tels que l'un de R7 et R8 représente l'atome d'hydrogène ou un radical alkyle, et l'autre de R7 et R8 représente un radical alkyle éventuellement substitué par un radical cycloalkyle;
soit R7 et R8 forment ensemble avec l'atome d'azote auquel ils sont liés un radical pyrrolidinyle, morpholine, pipéridyle ou pipérazinyle éventuellement substitué par un radical alkyle ;
lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

12. Produits de formule (I) tels que définis à l'une quelconque des revendications précédentes dans laquelle
X-Y représente NH-C(S) ou N=C-NR7R8;
R1 et R2 identiques ou différents, représente l'atome d'hydrogène ou un radical alkyle ;
R3 représente l'atome d'hydrogène :
R4, R5 et R6, identiques ou différents, représentent l'atome d'hydrogène ou un atome d'halogène;
R7 et R8 représentent les valeurs définies à l'une quelconque des revendications 1 à 4;
tous les radicaux alkyle (alk) ci-dessus étant linéaires ou ramifiés et renfermant au plus 6 atomes de carbone ;
lesdits produits de formule (I) étant sous toutes les formes tautomères et isomères (racémiques, énantiomères et diastéréoisomères) possibles, ainsi que les sels d'addition avec les acides minéraux et organiques ou avec les bases minérales et organiques desdits produits de formule (I).

13. Produits de formule (I) telle que définie à l'une quelconque des autres revendications répondant aux noms suivants :
- la (5Z)-5-(1H-pyrrolo[2,3-b]pyridin-3-ylméthylène)-2-thioxoimidazolidin-4-one
- la (5Z)-5-[(5-chloro-1H-pyrrolo[2,3-b]pyridin-3-yl)méthylène]-2-thioxoimidazolidin-4-one
- la (5Z)-2-[(cyclopropylméthyl)amino]-5-(1H-pyrrolo[2,3-b]pyridin-3-ylméthylène)-3,5-dihydro-4H-imidazol-4-one
- la (5Z)-5-[(5-chloro-1H-pyrrolo[2,3-b]pyridin-3-yl)méthylène]-2-[(cyclopropylméthyl)amino]-3,5-dihydro-4H-imidazol-4-one
- la (5Z)-5-[(6-chloro-1H-pyrrolo[2,3-b]pyridin-3-yl)méthylène]-2-[(cyclopropylméthyl)amino]-3,5-dihydro-4H-imidazol-4-one
- la (5Z)-2-[(cyclopropylméthyl)amino]-3-méthyl-5-(1H-pyrrolo[2,3-b]pyridin-3-ylméthylène)-3,5-dihydro-4H-imidazol-4-one
- la (5Z)-2-butyl-5-(1H-pyrrolo[2,3-b]pyridin-3-ylméthylène)-3,5-dihydro-4H-imidazol-4-one
- (5Z)-2-azépan-1-yl-5-[(5-chloro-1H-pyrrolo[2,3-b]pyridin-3-yl)méthylidène]-3,5-dihydro-4H-imidazol-4-one
- 5Z)-5-[(5-chloro-1H-pyrrolo[2,3-b]pyridin-3-yl)méthylidène]-2-pipéridin-1-yl-3,5-dihydro-4H-imidazol-4-one
- 5Z)-5-[(5-chloro-1H-pyrrolo[2,3-b]pyridin-3-yl)méthylidène]-2-[méthyl(2-méthylpropyl)amino]-3,5-dihydro-4H-imidazol-4-one
- (5Z)-5-[(5-bromo-1H-pyrrolo[2,3-b]pyridin-3-yl)méthylidène]-2-[(cyclopropylméthyl)amino]-3,5-dihydro-4H-imidazol-4-one
- (5Z)-2-[(cyclopropylméthyl)amino]-5-[(5-méthoxy-1H-pyrrolo[2,3-b]pyridin-3-yl)méthylidène]-3,5-dihydro-4H-imidazol-4-one
- (5Z)-2-[(cyclopropylméthyl)amino]-5-[(5-fluoro-1H-pyrrolo[2,3-b]pyridin-3-yl)méthylidène]-3,5-dihydro-4H-imidazol-4-one
- (5Z)-2-azépan-1-yl-5-[(5-fluoro-1H-pyrrolo[2,3-b]pyridin-3-yl)méthylidène]-3,5-dihydro-4H-imidazol-4-one
- 3-[(Z)-{2-[(cyclopropylméthyl)amino]-5-oxo-1,5-dihydro-4H-imidazol-4-ylidène}méthyl]-1H-pyrrolo[2,3-b]pyridine-5-carbonitrile
- (5Z)-2-[(cyclopropylméthyl)amino]-5-[(5-phényl-1H-pyrrolo[2,3-b]pyridin-3-yl)méthylidène]-3,5-dihydro-4H-imidazol-4-one
- (5Z)-2-[(cyclopropylméthyl)amino]-5-{[5-(trifluorométhyl)-1H-pyrrolo[2,3-b]pyridin-3-yl]méthylidène}-3,5-dihydro-4H-imidazol-4-one
- (5Z)-5-[(5-chloro-1H-pyrrolo[2,3-b]pyridin-3-yl)méthylidène]-2-(méthylsulfanyl)-3,5-dihydro-4H-imidazol-4-one
- (5Z)-2-butyl-5-[(5-chloro-1H-pyrrolo[2,3-b]pyridin-3-yl)méthylidène]-3,5-dihydro-4H-imidazol-4-one
- (5Z)-2-(3-méthylbutyl)-5-(1H-pyrrolo[2,3-b]pyridin-3-ylméthylidène)-3,5-dihydro-4H-imidazol-4-one
- (5Z)-2-cyclohexyl-5-(1H-pyrrolo[2,3-b]pyridin-3-ylméthylidène)-3,5-dihydro-4H-imidazol-4-one
- (5Z)-5-[(5-chloro-1H-pyrrolo[2,3-b]pyridin-3-yl)méthylidène]-3-méthyl-2-(tétrahydro-2H-pyran-4-yl)-3,5-dihydro-4H-imidazol-4-one
- (5Z)-5-[(5-chloro-1H-pyrrolo[2,3-b]pyridin-3-yl)méthylidène]-3-méthyl-2-(2-méthylpropyl)-3,5-dihydro-4H-imidazol-4-one
- (5Z)-2-(cyclopropylméthyl)-5-(5-chloro-1H-pyrrolo[2,3-b]pyridin-3-ylméthylidène)-3,5-dihydro-4H-imidazol-4-one
- (5Z)-2-(1-méthyléthyl)-5-(5-chloro-1H-pyrrolo[2,3-b]pyridin-3-ylméthylidène)-3,5-dihydro-4H-imidazol-4-one
- (5Z)-2-[benzyl(méthyl)amino]-5-[(5-chloro-1H-pyrrolo[2,3-b]pyridin-3-yl)méthylidène]-3,5-dihydro-4H-imidazol-4-one
- (5Z)-5-[(5-chloro-1H-pyrrolo[2,3-b]pyridin-3-yl)méthylidène]-2-[(2-méthoxyéthyl)(méthyl)amino]-3,5-dihydro-4H-imidazol-4-one
lesdits produits de formule (I) étant sous toutes les formes isomères racémiques, énantiomères et diastéréoisomères) possibles, ainsi que les sels d'addition avec les acides minéraux et organiques ou avec les bases minérales et organiques desdits produits de formule (I).

14. A titre de médicaments, les produits de formule (I) telle que définie à l'une quelconque des revendications 1 à 13 ainsi que les sels d'addition avec les acides minéraux et organiques ou avec les bases minérales et organiques pharmaceutiquement acceptables desdits produits de formule (I).

15. A titre de médicaments, les produits de formule (I) telle que définie à la revendication 13 ainsi que les sels d'addition avec les acides minéraux et organiques ou avec les bases minérales et organiques pharmaceutiquement acceptables desdits produits de formule (I).

16. Compositions pharmaceutiques contenant à titre de principe actif l'un au moins des produits de formule (I) tels que définis aux revendications 14 et 15 ou un sel pharmaceutiquement acceptable de ce produit et un support pharmaceutiquement acceptable.

17. Utilisation d'un produit de formule (I) tel que défini à l'une quelconque des revendications 1 à 13 pour la préparation d'un médicament destiné au traitement ou à la prévention d'une maladie choisie dans le groupe suivant : troubles de la prolifération de vaisseaux sanguins, troubles fibrotiques, troubles de la prolifération de cellules mésangiales, désordres métaboliques, allergies, asthmes, thromboses, maladies du système nerveux, rétinopathie, psoriasis, arthrite rhumatoïde, diabète, dégénérescence musculaire et cancers.

18. Utilisation d'un produit de formule (I) tel que défini à l'une quelconque des revendications 1 à 13 pour la préparation d'un médicament destiné au traitement ou à la prévention d'une maladie choisie dans le groupe suivant : troubles de la prolifération de cellules mésangiales, psoriasis, arthrite rhumatoïde, diabète, dégénérescence musculaire et cancers.

19. Utilisation d'un produit de formule (I) tel que défini à l'une quelconque des revendications 1 à 13 pour la préparation d'un médicament destiné au traitement de cancers.

20. Utilisation selon la revendication 19 destinée au traitement de tumeurs solides.

21. Utilisation selon la revendication 19 ou 20 destinée au traitement de cancers résistant à des agents cytotoxigues.

22. Utilisation selon l'une quelconque des revendications 19 à 21 destinée au traitement de cancers du sein, de l'estomac, des ovaires, du colon, du poumon, du cerveau, du larynx, du système lymphatique, du tractus génito-urinaire incluant vessie et prostate, de cancers des os et du pancréas

23. Utilisation selon l'une quelconque des revendications 19 à 21 destinée au traitement de cancers du sein, du colon ou du poumon.

24. Utilisation d'un produit de formule (I) telle que définie tel que défini à l'une quelconque des revendications 1 à 13 pour la préparation de médicaments destinés à la chimiothérapie de cancers.

25. Utilisation d'un produit de formule (I) telle que définie tel que défini à l'une quelconque des revendications 1 à 13 pour la préparation de médicaments destinés à la chimiothérapie de cancers seul ou en en association.

## Claims

1. Products of formula (I): 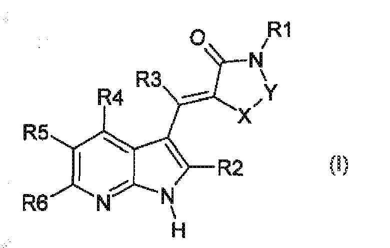 in which:
X-Y represents NH-C(S), N=C-NR7R8, N=C-SR, N=C-R
or N=C-OR;
R1 represents a hydrogen atom, a cycloalkyl radical or an alkyl, heterocycloalkyl, aryl or heteroaryl radical, all these radicals being optionally substituted;
R, which may be identical to or different from R1, is selected from the values of R1;
R2 represents a hydrogen atom, a halogen atom or an alkyl radical;
R3 represents a hydrogen atom, a halogen atom, a hydroxyl radical or an alkyl or alkoxy radical;
R4 represents a hydrogen atom, a halogen atom or a cyano, CF3 or alkyl radical;
R5 represents a hydrogen atom, a halogen atom, a hydroxyl, cyano, NR7R8, CONR7R8, NR11COR12 radical or a cycloalkyl, alkyl, alkoxy, heterocycloalkyl, aryl or heteroaryl radical, all these last-mentioned radicals being optionally substituted;
R6 represents a hydrogen atom, a halogen atom or an NR7R8, alkyl or alkoxy radical;
R7 and R8 are such that:
either R7 and R8, which may be identical or different, are such that one of R7 and R8 represents a hydrogen atom or an alkyl radical, optionally substituted;
and the other one of R7 and R8 represents a hydrogen atom or a cycloalkyl, alkyl, heterocycloalkyl, heteroaryl or aryl radical, all these radicals being optionally substituted;
or R7 and R8 form, together with the nitrogen atom to which they are attached, a cyclic radical formed from 3 to 7 ring members optionally containing one or more other heteroatoms selected from O, S or N, N being optionally substituted with R11, said cyclic radical itself being optionally substituted;
all the alkyl, alkoxy, cycloalkyl, heterocycloalkyl, heteroaryl and aryl radicals, as well as the cyclic radical that R7 and R8 can form together with the nitrogen atom to which they are attached, indicated as optionally substituted, thus being optionally substituted with one or more radicals, which may be identical or different, selected from the halogen atoms and the hydroxyl, cyano, nitro, CF3, NR9R10, NHCOR11, NHCO2R11, NHCONR9R10, NHSO2R13, COOH, COOalk, CONR9R10, SO2NR9R10, alkoxy, alkylthio, haloalkoxy, haloalkylthio, alkyl, fluoroalkyl, hydroxyalkyl, cycloalkyl, heterocycloalkyl, heteroaryl and phenyl radicals, these last-mentioned heteroaryl, aryl and phenyl radicals themselves being optionally substituted with one or more radicals, which may be identical or different, selected from the halogen atoms and the hydroxyl, NH2, NH(alk), N(alk)2, alkyl, hydroxyalkyl and alkoxy radicals;
R9 and R10 are such that:
either R9 and R10, which may be identical or different, are such that one of R9 and R10 represents a hydrogen atom or an alkyl radical optionally substituted with one or more radicals, which may be identical or different, selected from the halogen atoms and the hydroxyl and alkoxy radicals;
and the other one of R9 and R10 represents a hydrogen atom or a cycloalkyl, alkyl, heterocycloalkyl, heteroaryl or aryl radical, all these radicals themselves being optionally substituted with one or more radicals, which may be identical or different, selected from the halogen atoms and the hydroxyl, NH2, NH(alk), N(alk)2, alkyl, hydroxyalkyl and alkoxy radicals;
or R9 and R10 form, together with the nitrogen atom to which they are attached, a cyclic radical formed from 3 to 7 ring members optionally containing one or more other heteroatoms selected from O, S or N, N being optionally substituted with R12, said cyclic radical itself being optionally substituted with one or more radicals, which may be identical or different, selected from the halogen atoms and the hydroxyl, NH2, NH(alk), N(alk)2, alkyl, hydroxyalkyl and alkoxy radicals;
R11 and R12, which may be identical or different, represent a hydrogen atom or an alkyl or phenyl radical, optionally substituted with one or more radicals, which may be identical or different, selected from the halogen atoms and the hydroxyl and alkoxy radicals, the phenyl radical itself being optionally substituted with one or more alkyl radicals;
R13 represents an alkyl or phenyl radical, optionally substituted with one or more radicals, which may be identical or different, selected from the halogen atoms and the hydroxyl and alkoxy radicals, the phenyl radical itself being optionally substituted with one or more alkyl radicals;
all the above alkyl (alk) and alkoxy radicals being linear or branched and containing at most 12 carbon atoms;
it being understood that the products of formula (I) in which all of the following conditions are fulfilled, are excluded:
- R2 represents hydrogen;
- R3 represents hydrogen or alkyl;
- X-Y represents N=C-NR7R8, N=C-SR or N=CR in which X represents N and Y represents =C-NR7R8, =C-SR or =CR and R represents aryl or heteroaryl
- R1 represents H or alk;
and R4, R5 and R6 are such that two of them represent H and the other one represents hydrogen, NH2 or NHalk,
said products of formula (I) being in all possible tautomeric and isomeric forms (racemates, enantiomers and diastereoisomers), as well as salts of addition of said products of formula (I) with organic and inorganic acids or with organic and inorganic bases,

2. Products of formula (I) as defined in Claim 1 in which:
R1 represents a hydrogen atom, or an alkyl radical, all these radicals being optionally substituted as stated in Claim 1 or in any one of the other claims;
and R5 represents a halogen atom; a hydroxyl, cyano, NR7R8, CF3 radical or a cycloalkyl, alkyl, alkoxy, heterocycloalkyl, aryl or heteroaryl radical,
all these last-mentioned radicals being optionally substituted as stated in Claim 1 or in any one of the other claims;
the other substituent radicals R2, R3, R4, R6 and X-Y having the values defined in Claim 1 or in any one of the other claims,
said products of formula (I) being in all possible tautomeric and isomeric forms (racemates, enantiomers and diastereoisomers), as well as salts of addition of said products of formula (I) with organic and inorganic acids or with organic and inorganic bases.

3. Products of formula (I) as defined in Claim 1 in which:
R2 represents a hydrogen atom;
and R5 represents a halogen atom, a hydroxyl, cyano, NR7R8, CF3 radical or a cycloalkyl, alkyl; alkoxy, heterocycloalkyl, aryl or heteroaryl radical, all these last-mentioned radicals being optionally substituted as stated in Claim 1 or in any one of the other claims;
the other substituent radicals R1, R3, R4, R6 and X-Y having the values defined in Claim 1 or in any one of the other claims,
said products of formula (I) being in all possible tautomeric and isomeric forms (racemates, enantiomers and diastereoisomers), as well as salts of addition of said products of formula (I) with organic and inorganic acids or with organic and inorganic bases.

4. Products of formula (I) as defined in Claim 1 in which:
R3 represents a hydrogen atom;
and R5 represents a halogen atom, a hydroxyl, cyano, CONR7R8 radical or a cycloalkyl, alkyl, alkoxy, heterocycloalkyl, aryl or heteroaryl radical, all these last-mentioned radicals being optionally substituted as stated in Claim 1 or in any one of the other claims;
the other radicals R2, R3, R4, R6 and X-Y having the values defined in Claim 1 or in any one of the other claims,
said products of formula (I) being in all possible tautomeric and isomeric forms (racemates, enantiomers and diastereoisomers), as well as salts of addition of said products of formula (I) with organic and inorganic acids or with organic and inorganic bases.

5. Products of formula (I) as def ined in Claim 1 in which:
X-Y represents NH-C(S), N=C-NR7R8 or N=C-R;
R1 represents a hydrogen atom, a cycloalkyl radical or an alkyl, heterocycloalkyl, aryl or heteroaryl radical, all these radicals being optionally substituted as stated in Claim 1 or in any one of the other claims;
R, which may be identical to or different from R1, is selected from the values of R1 as defined in Claim 1 as stated in Claim 1 or in any one of the other claims with the exception of aryl and heteroaryl;
the other substituents R2, R3, R4, R5 and R6 having the values defined in Claim 1 or in any one of the other claims;
said products of formula (I) being in all possible tautomeric and isomeric forms (racemates, enantiomers and diastereoisomers), as well as salts of addition of said products of formula (I) with organic and inorganic acids or with organic and inorganic bases.

6. Products of formula (I) as defined in Claim 1 in which:
X-Y represents NH-C(S), N=C-NR7R8, N=C-SR, N=C-R or N=C-OR;
R1 represents a hydrogen atom, a cycloalkyl radical or an alkyl, heterocycloalkyl, phenyl or heteroaryl radical, these last-mentioned radicals being optionally substituted;
R, which may be identical to or different from R1, is selected from the values of R1;
R2 represents a hydrogen atom, a halogen atom or an alkyl radical;
R3 represents a hydrogen atom, a halogen atom, a hydroxyl radical or an alkyl or alkoxy radical;
R4 represents a hydrogen atom, a halogen atom or a cyano, CF3 or alkyl radical;
R5 represents a hydrogen atom, a halogen atom, a hydroxyl, cyano, NR7R8, CONR7R8, NR11COR12 radical or a cycloalkyl, alkyl, alkoxy, heterocycloalkyl, phenyl or heteroaryl radical, all these last-mentioned radicals being optionally substituted;
R6 represents a hydrogen atom, a halogen atom or an NR7R8, alkyl or alkoxy radical;
R7 and R8 are such that:
either R7 and R8, which may be identical or different, are such that one of R7 and R8 represents a hydrogen atom or an alkyl radical optionally substituted with one or more radicals, which may be identical or different, selected from the halogen atoms and the hydroxyl and alkoxy radicals;
and the other one of R7 and R8 represents a hydrogen atom or a cycloalkyl, alkyl, heterocycloalkyl, heteroaryl or phenyl radical, all these radicals being optionally substituted;
or R7 and R8 form, together with the nitrogen atom to which they are attached, a cyclic radical formed from 3 to 7 ring members optionally containing one or more other heteroatoms selected from O, S or N, N being optionally substituted with R11, said cyclic radical itself being optionally substituted;
all the alkyl, alkoxy, cycloalkyl, heterocycloalkyl, heteroaryl and aryl radicals, as well as the cyclic radical that R7 and R8 can form together with the nitrogen atom to which they are attached, indicated as optionally substituted, thus being optionally substituted with one or more radicals, which may be identical or different, selected from the halogen atoms and the hydroxyl, cyano, nitro, CF3, NR9R10, NHCOR11, NHSO2R13, COOH, COOalk, CONR9R10, SO2NR9R10, alkoxy, haloalkoxy, alkyl, fluoroalkyl, hydroxyalkyl, cycloalkyl, heterocycloalkyl, heteroaryl and phenyl radicals, these last-mentioned heteroaryl and phenyl radicals themselves being optionally substituted with one or more radicals, which may be identical or different, selected from the halogen atoms and the hydroxyl, NH2, NH(alk), N(alk)2, alkyl, hydroxyalkyl and alkoxy radicals;
R9 and R10 are such that:
either R9 and R10, which may be identical or different, are such that one of R9 and R10 represents a hydrogen atom or an alkyl radical and the other one of R9 and R10 represents a hydrogen atom or an alkyl, phenyl or phenylalkyl radical, themselves optionally substituted with one or more radicals, which may be identical or different, selected from the halogen atoms and the hydroxyl, NH2, NH (alk), N (alk) 2, alkyl, hydroxyalkyl and alkoxy radicals;
or R9 and R10 form, together with the nitrogen atom to which they are attached, a pyrrolidinyl, morpholine, piperidyl, azepinyl or piperazinyl radical optionally substituted with an alkyl or phenyl radical, itself optionally substituted with one or more radicals, which may be identical or different, selected from the halogen atoms and the alkyl, hydroxyl, NH2, NH(alk), N(alk)2, alkyl, hydroxyalkyl and alkoxy radicals;
R11 and R12, which may be identical or different, represent a hydrogen atom or an alkyl or phenyl radical;
R13 represents an alkyl or phenyl radical;
all the above alkyl (alk) and alkoxy radicals being linear or branched and containing at most 6 carbon atoms;
said products of formula (I) being in all possible tautomeric and isomeric forms (racemates, enantiomers and diastereoisomers), as well as salts of addition of said products of formula (I) with organic and inorganic acids or with organic and inorganic bases.

7. Products of formula (I) as defined in any one of the other claims in which:
X-Y represents NH-C(S), N=C-NR7R8, N=C-SR, N=C-R or N=C-OR;
R1 represents a hydrogen atom or an alkyl or phenyl radical, optionally substituted;
R represents a hydrogen atom; a cycloalkyl radical; an alkyl, heterocycloalkyl, phenyl, or heteroaryl radical, all these radicals being optionally substituted;
R2 represents a hydrogen atom, a halogen atom or an alkyl radical;
R3 represents a hydrogen atom, a halogen atom, a hydroxyl radical or an alkyl radical;
R4 represents a hydrogen atom, a halogen atom or an alkyl radical;
R5 represents a hydrogen atom; a halogen atom; a hydroxyl, cyano, NR7R8, alkyl, alkoxy, heterocycloalkyl, phenyl, or heteroaryl radical, these last-mentioned radicals as well as the phenyl residue in NHphenyl and NH(phenylalk) being optionally substituted;
R6 represents a hydrogen atom, a halogen atom, or an NH2, NHalk, N(alk)2, alkyl or alkoxy radical;
R7 and R8 are such that:
either R7 and R8, which may be identical or different, are such that one of R7 and R8 represents a hydrogen atom or an alkyl radical;
and the other one of R7 and R8 represents a hydrogen atom, or an alkyl or cycloalkyl radical, optionally substituted;
or R7 and R8 form, together with the nitrogen atom to which they are attached, a cyclic radical selected from the azetidyl, piperidyl, azepanyl, morpholinyl, thiomorpholinyl, pyrrolidinyl, imidazolidinyl, piperazinyl radicals optionally substituted on its second nitrogen atom with an alkyl or phenyl radical, themselves optionally substituted; and homopiperazinyl, these radicals being optionally substituted;
all the alkyl, alkoxy, heterocycloalkyl, heteroaryl and phenyl radicals, as well as the cyclic radical that R7 and R8 can form together with the nitrogen atom to which they are attached, indicated as optionally substituted, thus being optionally substituted with one or more radicals, which may be identical or different, selected from the halogen atoms and the hydroxyl, NH2, NH(alk), N(alk)2, NHphenyl, NH(phenylalk), alkyl, CF3, alkoxy, OCF3, cycloalkyl, heterocycloalkyl, heteroaryl and phenyl radicals; these last-mentioned heteroaryl and phenyl radicals, as well as the phenyl residue in the NHphenyl and NH(phenylalk) radicals, themselves being optionally substituted with one or more radicals, which may be identical or different, selected from the halogen atoms and the hydroxyl, NH2, NHAlk, N(Alk)2, alkyl, hydroxyalkyl and alkoxy radicals;
all the above alkyl (alk) and alkoxy radicals being linear or branched and containing at most 6 carbon atoms;
said products of formula (I) being in all possible tautomeric and isomeric forms (racemates, enantiomers and diastereoisomers), as well as salts of addition of said products of formula (I) with organic and inorganic acids or with organic and inorganic bases.

8. Products of formula (I) as defined in any one of the other claims in which:
X-Y represents NH-C(S), N=C-NR7R8, N=C-SR, N=C-R or N=C-OR;
R1 represents a hydrogen atom or an alkyl or phenyl radical, optionally substituted;
R, which may be identical to or different from R1, is selected from the values of R1
R2 represents a hydrogen atom, a halogen atom or an alkyl radical;
R3 represents a hydrogen atom, a halogen atom, a hydroxyl radical or an alkyl radical;
R4 represents a hydrogen atom, a halogen atom or an alkyl radical;
R5 represents a hydrogen atom, a halogen atom, a hydroxyl radical, an NH2, NHalk, N(alk)2, NR7R8. NHphenyl, NH(phenylalk) radical or an alkyl, heterocycloalkyl, alkoxy, phenyl or heteroaryl radical, these last-mentioned radicals as well as the phenyl residue in NHphenyl and NH(phenylalk) being optionally substituted;
R6 represents a hydrogen atom, a halogen atom, or an NH2, NHalk, N(alk)2, alkyl or alkoxy radical;
R7 and R8 are such that:
either R7 and R8, which may be identical or different, are such that one of R7 and R8 represents a hydrogen atom or an alkyl radical;
and the other one of R7 and R8 represents a hydrogen atom, or an alkyl or cycloalkyl radical, optionally substituted;
or R7 and R8 form, together with the nitrogen atom to which they are attached, a cyclic radical containing 4 to 6 ring members selected from azetidyl; piperidyl; morpholinyl; thiomorpholinyl; pyrrolidinyl; imidazolidinyl; piperazinyl; and homopiperazinyl, these radicals being optionally substituted;
all the alkyl, alkoxy, heteroaryl and phenyl radicals, as well as the cyclic radical that R7 and R8 can form together with the nitrogen atom to which they are attached, indicated as optionally substituted, thus being optionally substituted with one or more radicals, which may be identical or different, selected from the halogen atoms and the hydroxyl, NH2, NH(alk), N(alk)2, NHphenyl, NH(phenylalk), alkyl, CF3, alkoxy, OCF3, cycloalkyl, heterocycloalkyl, heteroaryl and phenyl radicals; these last-mentioned heteroaryl and phenyl radicals, as well as the phenyl residue in the NHphenyl and NH(phenylalk) radicals, themselves being optionally substituted with one or more radicals, which may be identical or different, selected from the halogen atoms and the hydroxyl, NH2, NHAlk, N(Alk)2, alkyl, hydroxyalkyl and alkoxy radicals;
all the above alkyl (alk) and alkoxy radicals being linear or branched and containing at most 6 carbon atoms;
said products of formula (I) being in all possible tautomeric and isomeric forms (racemates, enantiomers and diastereoisomers), as well as salts of addition of said products of formula (I) with organic and inorganic acids or with organic and inorganic bases.

9. Products of formula (I) as defined in any one of the other claims, in which X-Y, R2, R3, R4 and R6 have the meanings stated in any one of the other claims,
R1 represents a hydrogen atom or an alkyl radical, optionally substituted;
R5 represents a hydrogen atom, a halogen atom, a hydroxyl radical, CF3, NH2, NHalk, N(alk)2 or an alkyl, alkoxy or phenyl radical, optionally substituted;
the alkyl radical that can be represented by R1 or the alkyl, alkoxy or phenyl radical that can be represented by R5, being optionally substituted with one or more radicals, which may be identical or different, selected from the halogen atoms and the hydroxyl, NHalk, N(alk)2, alkoxy, cycloalkyl, heterocycloalkyl, heteroaryl and phenyl radicals, these last-mentioned heteroaryl and phenyl radicals being optionally substituted with one or more radicals, which may be identical or different, selected from the halogen atoms and the hydroxyl, NH2, NHAlk, N(Alk)2, alkyl, hydroxyalkyl and alkoxy radicals;
R7 and R8 are such that:
either R7 and R8, which may be identical or different, are such that one of R7 and R8 represents a hydrogen atom or an alkyl radical,
and the other one of R7 and R8 represents a hydrogen atom, an alkyl radical optionally substituted with one or more radicals, which may be identical or different, selected from the halogen atoms, the hydroxyl, NH2, NHalk, N(alk)2, NH(phenyl), NH(phenylalk), alkoxy, OCF3, cycloalkyl radicals, and the pyrrolidinyl, piperazinyl, piperidyl, morpholinyl and phenyl radicals, all these last-mentioned cyclic radicals, as well as the phenyl residue in the phenylalkyl radical, themselves being optionally substituted with one or more radicals, which may be identical or different, selected from the halogen atoms and the hydroxyl, NH2, NHAlk, N(alk)2, alkoxy, alkyl and hydroxyalkyl radicals;
or R7 and R8 form, together with the nitrogen atom to which they are attached, a radical preferably selected from the piperidyl, morpholinyl radicals, and the pyrrolidinyl, piperazinyl and homopiperazinyl radicals, optionally substituted with one or more radicals, which may be identical or different, selected from the alkyl and phenyl radicals, themselves optionally substituted with one or more radicals selected from the halogen atoms and the hydroxyl, NH2, NHAlk, N(Alk)2, alkoxy and cycloalkyl radicals;
all the above alkyl (alk) and alkoxy radicals being linear or branched and containing at most 6 carbon atoms;
said products of formula (I) being in all possible tautomeric and isomeric forms (racemates, enantiomers and diastereoisomers), as well as salts of addition of said products of formula (I) with organic and inorganic acids or with organic and inorganic bases.

10. Products of formula (I) as defined in any one of the other claims in which
X-Y represents NH-C(S), N=C-NR7R8 or N=C-R,
R7, R8 and R being selected from all the values defined in any one of the other claims for R7, R8 and R and the other substituents R1, R2, R3, R4, R5 and R6 of said products of formula (I) being selected from all the values defined in any one of the other claims respectively for R1, R2, R3, R4, R5 and R6,
said products of formula (I) being in all possible tautomeric and isomeric forms (racemates, enantiomers and diastereoisomers), as well as salts of addition of said products of formula (I) with organic and inorganic acids or with organic and inorganic bases,

11. Products of formula (I) as defined in any one of the other claims in which
X-Y represents NH-C(S), N=C-NR7R8 or N=C-R;
R1 represents a hydrogen atom or an alkyl radical optionally substituted with one or more radicals, which may be identical or different, selected from the halogen atoms and the N(alk)2 and alkoxy radicals;
R, which may be identical to or different from R1, is selected from the values of R1;
R2 represents a hydrogen atom, a halogen atom or an alkyl radical;
R3 represents a hydrogen atom or an alkyl radical;
R4 represents a hydrogen atom or a halogen atom,
R5 represents a hydrogen atom, a halogen atom or a hydroxyl, NH2, NHalk, N(alk)2, alkyl, alkoxy or phenyl radical, the alkyl radical being optionally substituted with an alkoxy, N(alk)2 or heterocycloalkyl radical and the phenyl radical being optionally substituted with one or more radicals, which may be identical or different, selected from the halogen atoms and the hydroxyl, NH2, NHalk, N(alk)2, alkyl and alkoxy radicals;
R6 represents a hydrogen atom, a halogen atom or an alkyl radical;
and R7 and R8 are such that:
either R7 and R8, which may be identical or different, are such that one of R7 and R8 represents a hydrogen atom or an alkyl radical, and the other one of R7 and R8 represents an alkyl radical optionally substituted with a cycloalkyl radical;
or R7 and R8 form, together with the nitrogen atom to which they are attached, a pyrrolidinyl, morpholine, piperidyl or piperazinyl radical optionally substituted with an alkyl radical;
said products of formula (I) being in all possible tautomeric and isomeric forms (racemates, enantiomers and diastereoisomers), as well as salts of addition of said products of formula (I) with organic and inorganic acids or with organic and inorganic bases.

12. Products of formula (I) as defined in any one of the preceding claims in which
X-Y represents NH-C(S) or N=C-NR7R8;
R1 and R2 which may be identical or different, represents a hydrogen atom or an alkyl radical;
R3 represents a hydrogen atom;
R4, R5 and R6, which may be identical or different, represent a hydrogen atom or a halogen atom;
R7 and R8 represent the values defined in any one of Claims 1 to 4;
all the above alkyl (alk) radicals being linear or branched and containing at most 6 carbon atoms;
said products of formula (I) being in all possible tautomeric and isomeric forms (racemates, enantiomers and diastereoisomers), as well as salts of addition of said products of formula (I) with organic and inorganic acids or with organic and inorganic bases.

13. Products of formula (I) as defined in any one of the other claims, having the following names:
- (5Z)-5-(1H-pyrrolo[2,3-b]pyridin-3-ylmethylene)-2-thioxoimidazolidin-4-one
- (5Z)-5-[(5-chloro-1H-pyrrolo[2,3-b]pyridin-3-yl)methylene]-2-thioxoimidazolidin-4-one
- (5Z)-2-[(cyclopropylmethyl)aminol-5-(1H-pyrrolo[2,3-b]pyridin-3-ylmethylene)-3,5-dihydro-4H-imidazol-4-one
- (5Z)-5-[(5-chloro-1H-pyrrolo[2,3-b]pyridin-3-yl)methylene]-2-[(cyclopropylmethyl)amino]-3,5-dihydro-4H-imidazol-4-one
- (5Z)-5-[(6-chloro-1H-pyrrolo[2,3-b]pyridin-3-yl)methylenel-2-[(cyclopropylmethyl)aminol-3,5-dihydro-4H-imidazol-4-one
- (5Z)-2-[(cyclopropylmethyl)amino]-3-methyl-5-(1H-pyrrolo[2,3-b]pyridin-3-ylmethylene)-3,5-dihydro-4H-imidazol-4-one
- (5Z)-2-butyl-5-(1H-pyrrolo[2,3-b]pyridin-3-ylmethylene)-3,5-dihydro-4H-imidazol-4-one
- (5Z)-2-azepan-1-yl-5-[(5-chloro-1H-pyrrolo[2,3-b]pyridin-3-yl)methylidene]-3,5-dihydro-4H-imidazol-4-one
- 5Z)-5-[(5-chloro-1H-pyrrolo[2,3-b]pyridin-3-yl)methylidene]-2-piperidin-1-yl-3,5-dihydro-4H-imidazol-4-one
- 5Z)-5-[(5-chloro-1H-pyrrolo[2,3-b]pyridin-3-yl)methylidene]-2-[methyl(2-methylpropyl)amino]-3,5-dihydro-4H-imidazol-4-one
- (5Z)-5-[(5-bromo-1H-pyrrolo[2,3-b]pyridin-3-yl)methylidene]-2-[(cyclopropylmethyl)amino]-3,5-dihydro-4H-imidazol-4-one
- (5Z)-2-[(cyclopropylmethyl)amino]-5-[(5-methoxy-1H-pyrrolo[2,3-b]pyridin-3-yl)methylidene]-3,5-dihydro-4H-imidazol-4-one
- (5Z)-2-[(cyclopropylmethyl)amino]-5-[(5-fluoro-1H-pyrrolo[2,3-b]pyridin-3-yl)methylidene]-3,5-dihydro-4H-imidazol-4-one
- (5Z)-2-azepan-1-yl-5-[(5-fluoro-1H-pyrrolo-[2,3-b]pyridin-3-yl)methylidene]-3,5-dihydro-4H-imidazol-4-one
- 3-[(Z)-{2-[(cyclopropylmethyl)amino]-5-oxo-1,5-dihydro-4H-imidazol-4-ylidene}methyl]-1H-pyrrolo[2,3-b]pyridine-5-carbonitrile
- (5Z)-2-[(cyclopropylmethyl)amino]-5-[(5-phenyl-1H-pyrrolo[2,3-b]pyridin-3-yl)methylidene]-3,5-dihydro-4H-imidazol-4-one
- (5Z)-2-[(cyclopropylmethyl)amino]-5-{[5-(trifluoromethyl)-1H-pyrrolo[2,3-b]pyridin-3-yl]methylidene}-3,5-dihydro-4H-imidazol-4-one
- (5Z)-5-[(5-chloro-1H-pyrrolo[2,3-b]pyridin-3-yl)methylidene]-2-(methylsulphanyl)-3,5-dihydro-4H-imidazol-4-one
- (5Z)-2-butyl-5-[(5-chloro-1H-pyrrolo[2,3-b]pyridin-3-yl)methylidene]-3,5-dihydro-4H-imidazol-4-one
- (5Z)-2-(3-methylbutyl)-5-(1H-pyrrolo[2,3-b]pyridin-3-ylmethylidene)-3,5-dihydro-4H-imidazol-4-one
- (5Z)-2-cyclohexyl-5-(1H-pyrrolo[2,3-b]pyridin-3-ylmethylidene)-3,5-dihydro-4H-imidazol-4-one
- (5Z)-5-[(5-chloro-1H-pyrrolo[2,3-b]pyridin-3-yl)methylidene]-3-methyl-2-(tetrahydro-2H-pyran-4-yl)-3,5-dihydro-4H-imidazol-4-one
- (5Z)-5-[(5-chloro-1H-pyrrolo[2,3-b]pyridin-3-yl)methylidene]-3-methyl-2-(2-methylpropyl)-3,5-dihydro-4H-imidazol-4-one
- (5Z)-2-(cyclopropylmethyl)-5-(5-chloro-1H-pyrrolo[2,3-b]pyridin-3-ylmethylidene)-3,5-dihydro-4H-imidazol-4-one
- (5Z)-2-(1-methylethyl)-5-(5-chloro-1H-pyrrolo[2,3-b]pyridin-3-ylmethylidene)-3,5-dihydro-4H-imidazol-4-one
- (5Z)-2-[benzyl(methyl)amino]-5-[(5-chloro-1H-pyrrolo[2,3-b]pyridin-3-yl)methylidene]-3,5-dihydro-4H-imidazol-4-one
- (5Z)-5-[(5-chloro-1H-pyrrolo[2,3-b]pyridin-3-yl)methylidene]-2-[(2-methoxyethyl) (methyl)amino]-3,5-dihydro-4H-imidazol-4-one
said products of formula (I) being in all possible isomeric forms (racemates, enantiomers and diastereoisomers), as well as salts of addition of said products of formula (I) with organic and inorganic acids or with organic and inorganic bases.

14. As medicinal products, the products of formula (I) as defined in any one of Claims 1 to 13, as well as salts of addition of said products of formula (I) with pharmaceutically acceptable inorganic and organic acids or inorganic and organic bases.

15. As medicinal products, the products of formula (I) as defined in Claim 13, as well as salts of addition of said products of formula (I) with pharmaceutically acceptable inorganic and organic acids or inorganic and organic bases.

16. Pharmaceutical compositions containing, as active principle, at least one of the products of formula (I) as defined in Claims 14 and 15 or a pharmaceutically acceptable salt of this product and a pharmaceutically acceptable carrier.

17. Use of a product of formula (I) as defined in any one of Claims 1 to 13 for the preparation of a medicinal product intended for the treatment or prevention of a disease selected from the following group: disorders of proliferation of blood vessels, fibrotic disorders, disorders of proliferation of mesangial cells, metabolic disorders, allergies, asthmas, thromboses, diseases of the nervous system, retinopathy, psoriasis, rheumatoid arthritis, diabetes, muscular degeneration and cancers.

18. Use of a product of formula (I) as defined in any one of Claims 1 to 13 for the preparation of a medicinal product intended for the treatment or prevention of a disease selected from the following group: disorders of proliferation of mesangial cells, psoriasis, rheumatoid arthritis, diabetes, muscular degeneration and cancers.

19. Use of a product of formula (I) as defined in any one of Claims 1 to 13, for the preparation of a medicinal product intended for the treatment of cancers.

20. Use according to Claim 19, intended for the treatment of solid tumours.

21. Use according to Claim 19 or 20, intended for the treatment of cancers that are resistant to cytotoxic agents.

22. Use according to any one of Claims 19 to 21, intended for the treatment of cancers of the breast, stomach, ovaries, colon, lung, brain, larynx, lymphatic system, urogenital tract including bladder and prostate, and treatment of cancers of the bones and of the pancreas.

23. Use according to any one of Claims 19 to 21, intended for the treatment of cancers of the breast, of the colon or of the lung.

24. Use of a product of formula (I) as defined in any one of Claims 1 to 13, for the preparation of medicinal products intended for cancer chemotherapy.

25. Use of a product of formula (I) as defined in any one of Claims 1 to 13, for the preparation of medicinal products intended for cancer chemotherapy alone or in combination.

## Patentansprüche

1. Produkte der Formel (I): 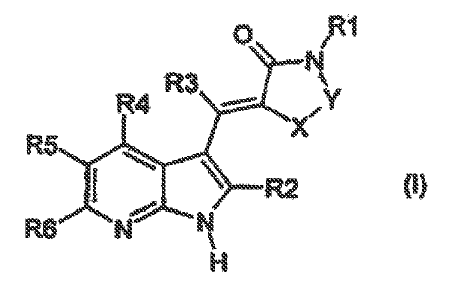 worin:
X-Y für NH-C(S), N=C-NR7R8, N=C-SR, N=C-R oder N=C-OR steht;
R1 für ein Wasserstoffatom, einen Cycloalkylrest oder einen Alkyl-, Heterocycloalkyl-, Aryl- oder Heteroarylrest steht, wobei alle diese Reste gegebenenfalls substituiert sind;
R mit R1 identisch oder davon verschieden ist und unter den Werten von R1 ausgewählt ist;
R2 für ein wasserstoffatom, ein Halogenatom oder einen Alkylrest steht;
R3 für ein Wasserstoffatom, ein Halogenatom, einen Hydroxylrest oder einen Alkyl- oder Alkoxyrest steht;
R4 für ein wasserstoffatom, ein Halogenatom oder einen Cyano-, CF3- oder Alkylrest steht;
R5 für ein Wasserstoffatom, ein Halogenatom, einen Hydroxyl-, Cyano-, NR7R8-, CONR7R8- oder NR11COR12-Rest oder einen Cycloalkyl-, Alkyl-, Alkoxy-, Heterocycloalkyl-, Aryl- oder Heteroarylrest steht, wobei alle diese letztgenannten Reste gegebenenfalls substituiert sind;
R6 für ein Wasserstoffatom, ein Halogenatom oder einen NR7R8-, Alkyl- oder Alkoxyrest steht;
R7 und R8 so beschaffen sind, dass:
entweder R7 und R8 gleich oder verschieden sind und so beschaffen sind, dass eine der Gruppen R7 und R8 für ein Wasserstoffatom oder einen gegebenenfalls substituierten Alkylrest steht;
und die andere der Gruppen R7 und R8 für ein Wasserstoffatom oder einen Cycloalkyl-, Alkyl-, Heterocycloalkyl-, Heteroaryl- oder Arylrest steht, wobei alle diese Reste gegebenenfalls substituiert sind;
oder R7 und R8 zusammen mit dem Stickstoffatom, an das sie gebunden sind, einen 3- bis 7-gliedrigen cyclischen Rest bilden, der gegebenenfalls ein oder mehrere andere Heteroatome enthält, die unter O, S oder N ausgewählt sind, wobei N gegebenenfalls durch R11 substituiert sein kann, wobei dieser cyclische Rest gegebenenfalls selbst substituiert ist;
wobei alle Alkyl-, Alkoxy-, Cycloalkyl-, Heterocycloalkyl-, Heteroaryl- und Arylreste sowie der cyclische Rest, den R7 und R8 zusammen mit dem Stickstoffatom, an das sie gebunden sind, bilden können, die als gegebenenfalls substituiert angegeben sind, somit gegebenenfalls durch einen oder mehrere gleiche oder verschiedene Reste substituiert sind, die unter Halogenatomen und Hydroxyl-, Cyano-, Nitro-, CF3-, NR9R10-, NHCOR11-, NHCO2R11-, NHCONR9R10-, NHSO2R13-, COOH-, COOalk-, CONR9R10-, SO2NR9R10-, Alkoxy-, Alkylthio-, Halogenalkoxy-, Halogenalkylthio-, Alkyl-, Fluoralkyl-, Hydroxyalkyl-, Cycloalkyl-, Heterocycloalkyl-, Heteroaryl- und Phenylresten ausgewählt sind, wobei diese letztgenannten Heteroaryl-, Aryl- und Phenylreste gegebenenfalls selbst durch einen oder mehrere gleiche oder verschiedene Reste substituiert sein können, die unter Halogenatomen und Hydroxyl-, NH2-, NH(alk)-, N(alk)2-, Alkyl-, Hydroxyalkyl- und Alkoxyresten ausgewählt sind;
R9 und R10 so beschaffen sind, dass:
entweder R9 und R10 gleich oder verschieden sind und so beschaffen sind, dass eine der Gruppen R9 und R10 für ein Wasserstoffatom oder einen Alkylrest, der gegebenenfalls durch ein oder mehrere gleiche oder verschiedene Reste, die unter Halogenatomen und Hydroxyl- und Alkoxyresten ausgewählt sind, substituiert ist, steht
und die andere der Gruppen R9 und R10 für ein Wasserstoffatom oder einen Cycloalkyl-, Alkyl-, Heterocycloalkyl- Heteroaryl- oder Arylrest stehen, wobei alle diese Reste gegebenenfalls selbst durch ein oder mehrere gleiche oder verschiedene Reste substituiert sind, die unter Halogenatomen und Hydroxyl-, NH2-, NH(alk)-, N(alk)2-, Alkyl-, Hydroxyalkyl- und Alkoxyresten ausgewählt sind;
oder R9 und R10 zusammen mit dem Stickstoffatom, an das sie gebunden sind, einen 3- bis 7-gliedrigen Rest bilden, der gegebenenfalls ein oder mehrere andere Heteroatome enthält, die unter O, S oder N ausgewählt sind, wobei N gegebenenfalls durch R12 substituiert sein kann, wobei dieser cyclische Rest gegebenenfalls selbst durch einen oder mehrere gleiche oder verschiedene Reste substituiert ist, die unter Halogenatomen und Hydroxyl-, NH2-, NH(alk)-, N(alk)2-, Alkyl-, Hydroxyalkyl- und Alkoxyresten ausgewählt sind;
R11 und R12 gleich oder verschieden sind und für ein Wasserstoffatom oder einen Alkyl- oder Phenylrest, die gegebenenfalls durch einen oder mehrere gleiche oder verschiedene Reste, die unter Halogenatomen und Hydroxyl- und Alkoxyresten ausgewählt sind, substituiert sind, stehen, wobei der Phenylrest gegebenenfalls selbst durch einen oder mehrere Alkylreste substituiert ist;
R13 für einen Alkyl- oder Phenylrest steht, der gegebenenfalls durch einen oder mehrere gleiche oder verschiedene Reste substituiert ist, die unter Halogenatomen und Hydroxyl- und Alkoxyresten ausgewählt sind, wobei der Phenylrest gegebenenfalls selbst durch einen oder mehrere Alkylreste substituiert ist;
wobei alle obigen Alkyl-(alk-) und Alkoxyreste linear oder verzweigt sind und höchstens 12 Kohlenstoffatome enthalten;
mit der Maßgabe, dass die Produkte der Formel (I), in denen alle nachstehenden Bedingungen erfüllt sind, ausgeschlossen sind:
- r2 steht für Wasserstoff;
- R3 steht für Wasserstoff oder Alkyl;
- X-Y steht für N=C-NR7R8, N=C-SR oder N=CR, worin X für N steht und Y für -C-NR7R8, =C-SR oder =CR steht, wobei R für Aryl oder Heteroaryl steht;
- R1 steht für H oder alk;
- und R4, R5 und R6 sind so beschaffen, dass zwei dieser Gruppen für H stehen und die andere Gruppe für Wasserstoff, NH2 oder NHalk steht,
wobei die Produkte der Formel (I) in allen möglichen tautomeren und isomeren Formen (Racemate, Enantiomere und Diastereoisomere) vorliegen, sowie die Additionssalze der Produkte der Formel (I) mit anorganischen und organischen Säuren oder mit anorganischen und organischen Basen.

2. Produkte der Formel (I) nach Anspruch 1, worin:
R1 für ein Wasserstoffatom oder einen Alkylrest steht, wobei alle diese Reste gegebenenfalls wie in Anspruch 1 oder einem der anderen Ansprüche angegeben substituiert sind;
und R5 für ein Halogenatom, einen Hydroxyl-, Cyano-, NR7R8- oder CF3-Rest oder einen Cycloalkyl-, Alkyl-, Alkoxy-, Heterocycloalkyl-, Aryl- oder Heteroarylrest steht,
wobei alle diese letztgenannten Reste gegebenenfalls wie in Anspruch 1 oder einem der anderen Ansprüche angegeben substituiert sind;
wobei die anderen Substituentenreste R2, R3, R4, R6 und X-Y die in Anspruch 1 oder einem der anderen Ansprüche definierten Werte aufweisen,
wobei die Produkte der Formel (I) in allen möglichen tautomeren und isomeren Formen (Racemate, Enantiomere und Diastereoisomere) vorliegen, sowie die Additionssalze der Produkte der Formel (I) mit anorganischen und organischen Säuren oder mit anorganischen und organischen Basen.

3. Produkte der Formel (I) nach Anspruch 1, worin:
R2 für ein Wasserstoffatom steht;
und R5 für ein Halogenatom, einen Hydroxyl-, Cyano-, NR7R8- oder CF3-Rest oder einen Cycloalkyl-, Alkyl-, Alkoxy-, Heterocycloalkyl-, Aryl- oder Heteroarylrest steht, wobei alle diese letztgenannten Reste gegebenenfalls wie in Anspruch 1 oder einem der anderen Ansprüche angegeben substituiert sind;
wobei die anderen Substituentenreste R1, R3, R4, R6 und X-Y die in Anspruch 1 oder einem der anderen Ansprüche definierten Werte aufweisen,
wobei die Produkte der Formel (I) in allen möglichen tautomeren und isomeren Formen (Racemate, Enantiomere und Diastereoisomere) vorliegen, sowie die Additionssalze der Produkte der Formel (I) mit anorganischen und organischen Säuren oder mit anorganischen und organischen Basen.

4. Produkte der Formel (I) nach Anspruch 1, worin:
R3 für ein Wasserstoffatom steht;
und R5 für ein Halogenatom, einen Hydroxyl-, Cyano- oder CONR7R8-Rest oder einen Cycloalkyl-, Alkyl-, Alkoxy-, Heterocycloalkyl-, Aryl- oder Heteroarylrest steht, wobei alle diese letztgenannten Reste gegebenenfalls wie in Anspruch 1 oder einem der anderen Ansprüche angegeben substituiert sind;
wobei die anderen Substituentenreste R2, R3, R4, R6 und X-Y die in Anspruch 1 oder einem der anderen Ansprüche definierten Werte aufweisen,
wobei die Produkte der Formel (I) in allen möglichen tautomeren und isomeren Formen (Racemate, Enantiomere und Diastereoisomere) vorliegen, sowie die Additionssalze der Produkte der Formel (I) mit anorganischen und organischen Säuren oder mit anorganischen und organischen Basen.

5. Produkte der Formel (I) nach Anspruch 1, worin:
X-Y für NH-C(S), N=C-NR7R8 oder N=C-R steht;
R1 für ein Wasserstoffatom, einen Cycloalkylrest oder einen Alkyl-, Heterocycloalkyl-, Aryl- oder Heteroarylrest steht, wobei alle diese Reste gegebenenfalls wie in Anspruch 1 oder einem der anderen Ansprüche angegeben substituiert sind;
R mit R1 identisch oder davon verschieden ist und unter den Werten von R1 gemäß der Definition in Anspruch 1 wie in Anspruch 1 oder einem der anderen Ansprüche angegeben mit Ausnahme von Aryl und Heteroaryl ausgewählt ist;
wobei die anderen Substituenten R2, R3, R4, R5 und R6 die in Anspruch 1 oder einem der anderen Ansprüche definierten Werte aufweisen;
wobei die Produkte der Formel (I) in allen möglichen tautomeren und isomeren Formen (Racemate, Enantiomere und Diastereoisomere) vorliegen, sowie die Additionssalze der Produkte der Formel (I) mit anorganischen und organischen Säuren oder mit anorganischen und organischen Basen.

6. Produkte der Formel (I) nach Anspruch 1, worin:
X-Y für NH-C(S), N=C-NR7R8, N=C-SR, N=C-R oder N=C-OR steht;
R1 für ein Wasserstoffatom, einen Cycloalkylrest oder einen Alkyl-, Heterocycloalkyl-, Phenyl- oder Heteroarylrest steht, wobei alle diese Reste gegebenenfalls substituiert sind;
R mit R1 identisch oder davon verschieden ist und unter den Werten von R1 ausgewählt ist;
R2 für ein Wasserstoffatom, ein Halogenatom oder einen Alkylrest steht;
R3 für ein Wasserstoffatom, ein Halogenatom, einen Hydroxylrest oder einen Alkyl- oder Alkoxyrest steht;
R4 für ein Wasserstoffatom, ein Halogenatom oder einen Cyano-, CF3- oder Alkylrest steht;
R5 für ein Wasserstoffatom, ein Halogenatom, einen Hydroxyl-, Cyano-, NR7R8-, CONR7R8- oder NR11COR12-Rest oder einen Cycloalkyl-, Alkyl-, Alkoxy-, Heterocycloalkyl-, Phenyl- oder Heteroarylrest steht, wobei alle diese letztgenannten Reste gegebenenfalls substituiert sind;
R6 für ein Wasserstoffatom, ein Halogenatom oder einen NR7R8-, Alkyl- oder Alkoxyrest steht;
R7 und R8 so beschaffen sind, dass:
entweder R7 und R8 gleich oder verschieden sind und so beschaffen sind, dass eine der Gruppen R7 und R8 für ein Wasserstoffatom oder einen gegebenenfalls durch einen oder mehrere gleiche oder verschiedene Reste, die unter Halogenatomen und Hydroxyl- und Alkoxyresten ausgewählt sind, substituierten Alkylrest steht;
und die andere der Gruppen R7 und R8 für ein Wasserstoffatom oder einen Cycloalkyl-, Alkyl-, Heterocycloalkyl-, Heteroaryl- oder Phenylrest steht, wobei alle diese Reste gegebenenfalls substituiert sind;
oder R7 und R8 zusammen mit dem Stickstoffatom, an das sie gebunden sind, einen 3- bis 7-gliedrigen cyclischen Rest bilden, der gegebenenfalls ein oder mehrere andere Heteroatome enthält, die unter O, S oder N ausgewählt sind, wobei N gegebenenfalls durch R11 substituiert sein kann, wobei dieser cyclische Rest gegebenenfalls selbst substituiert ist;
wobei alle Alkyl-, Alkoxy-, Cycloalkyl-, Heterocycloalkyl-, Heteroaryl- und Arylreste sowie der cyclische Rest, den R7 und R8 zusammen mit dem Stickstoffatom, an das sie gebunden sind, bilden können, die als gegebenenfalls substituiert angegeben sind, somit gegebenenfalls durch einen oder mehrere gleiche oder verschiedene Reste substituiert sind, die unter Halogenatomen und Hydroxyl-, Cyano-, Nitro-, CF3-, NR9R10-, NHCOR11-, NHSO2R13-, COOH-, COOalk-, CONR9R10-, SO2NR9R10-, Alkoxy-, Halogenalkoxy-, Alkyl-, Fluoralkyl-, Hydroxyalkyl-, Cycloalkyl-, Heterocycloalkyl-, Heteroaryl- und Phenylresten ausgewählt sind, wobei diese letztgenannten Heteroaryl- und Phenylreste gegebenenfalls selbst durch einen oder mehrere gleiche oder verschiedene Reste substituiert sein können, die unter Halogenatomen und Hydroxyl-, NH2-, NH(alk)-, N(alk)2-, Alkyl-, Hydroxyalkyl- und Alkoxyresten ausgewählt sind;
R9 und R10 so beschaffen sind, dass:
entweder R9 und R10 gleich oder verschieden sind und so beschaffen sind, dass eine der Gruppen R9 und R10 für ein Wasserstoffatom oder einen Alkylrest steht und die andere der Gruppen R9 und R10 für ein Wasserstoffatom oder einen Alkyl-, Phenyl- oder Phenylalkylrest steht, wobei diese Reste gegebenenfalls selbst durch einen oder mehrere gleiche oder verschiedene Reste, substituiert sind, die unter Halogenatomen und Hydroxyl-, NH2-, NH(alk)-, N(alk)2-, Alkyl-, Hydroxyalkyl- und Alkoxyresten ausgewählt sind;
oder R9 und R10 zusammen mit dem Stickstoffatom, an das sie gebunden sind, einen Pyrrolidinyl-, Morpholinyl-, Piperidyl-, Azepinyl- oder Piperazinylrest bilden, der gegebenenfalls durch einen Alkyl- oder Phenylrest substituiert ist, der gegebenenfalls selbst durch einen oder mehrere gleiche oder verschiedene Reste substituiert ist, die unter Halogenatomen und Alkyl-, Hydroxyl-, NH2-, NH(alk)-, N(alk)2-, Alkyl-, Hydroxyalkyl- und Alkoxyresten ausgewählt ist;
R11 und R12 gleich oder verschieden sind und für ein Wasserstoffatom oder einen Alkyl- oder Phenylrest stehen;
R13 für einen Alkyl- oder Phenylrest steht;
wobei alle obigen Alkyl-(alk-) und Alkoxyreste linear oder verzweigt sind und höchstens 6 Kohlenstoffatome enthalten;
wobei die Produkte der Formel (I) in allen möglichen tautomeren und isomeren Formen (Racemate, Enantiomere und Diastereoisomere) vorliegen, sowie die Additionssalze der Produkte der Formel (I) mit anorganischen und organischen Säuren oder mit anorganischen und organischen Basen.

7. Produkte der Formel (I) nach einem der anderen Ansprüche, worin:
X-Y für NH-C(S), N=C-NR7R8, N=C-SR, N=C-R oder N=C-OR steht;
R1 für ein Wasserstoffatom oder einen gegebenenfalls substituierten Alkyl- oder Phenylrest steht;
R für ein Wasserstoffatom; einen Cycloalkylrest; einen Alkyl-; Heterocycloalkyl-; Phenyl- oder Heteroarylrest steht; wobei diese Reste gegebenenfalls substituiert sind;
R2 für ein Wasserstoffatom, ein Halogenatom oder einen Alkylrest steht;
R3 für ein Wasserstoffatom, ein Halogenatom, einen Hydroxylrest oder einen Alkylrest steht;
R4 für ein Wasserstoffatom, ein Halogenatom oder einen Alkylrest steht;
R5 für ein Wasserstoffatom; ein Halogenatom; einen Hydroxyl-; Cyano-; NR7R8-; Alkyl-; Alkoxy-; Heterocycloalkyl-; Phenyl- oder Heteroarylrest steht; wobei diese letztgenannten Reste sowie der Phenylrest von NH-Phenyl und NH(phenylalk) gegebenenfalls substituiert sind;
R6 für ein Wasserstoffatom, ein Halogenatom oder einen NH2-, NHalk-, N(alk)2-, Alkyl- oder Alkoxyrest steht;
R7 und R8 so beschaffen sind, dass:
entweder R7 und R8 gleich oder verschieden sind und so beschaffen sind, dass eine der Gruppen R7 und R8 für ein Wasserstoffatom oder einen Alkylrest steht;
und die andere der Gruppen R7 und R8 für ein Wasserstoffatom oder einen gegebenenfalls substituierten Alkyl- oder Cycloalkylrest steht;
oder R7 und R8 zusammen mit dem Stickstoffatom, an das sie gebunden sind, einen unter Azetidyl-, Piperidyl-, Azepanyl-, Morpholinyl-, Thiomorpholinyl-, Pyrrolidinyl-, Imidazolidinyl- und Piperazinylresten, die gegebenenfalls am zweiten Stickstoffatom durch einen Alkyl- oder Phenylrest, der gegebenenfalls selbst substituiert sein kann, substituiert sind, und Homopiperazinyl ausgewählten cyclischen Rest bilden, wobei diese Reste gegebenenfalls substituiert sind;
wobei alle Alkyl-, Alkoxy-, Heterocycloalkyl-Heteroaryl- und Phenylreste sowie der cyclische Rest, den R7 und R8 zusammen mit dem Stickstoffatom, an das sie gebunden sind, bilden können, die als gegebenenfalls substituiert angegeben sind, somit gegebenenfalls durch einen oder mehrere gleiche oder verschiedene Reste substituiert sind, die unter Halogenatomen und Hydroxyl-, NH2-, NH(alk)-, N(alk)2-, NH-Phenyl-, NH(phenylalk)-, Alkyl-, CF3-, Alkoxy-, OCF3-, Cycloalkyl-, Heterocycloalkyl-, Heteroaryl- und Phenylresten ausgewählt sind; wobei diese letztgenannten Heteroaryl- und Phenylreste sowie der Phenylrest der Reste NH-Phenyl und NH (phenylalk) gegebenenfalls selbst durch einen oder mehrere gleiche oder verschiedene Reste substituiert sind, die unter Halogenatomen und Hydroxyl-, NH2-, NHalk-, N(alk)2-, Alkyl-, Hydroxyalkyl- und Alkoxyresten ausgewählt sind;
wobei alle obigen Alkyl-(alk-) und Alkoxyreste linear oder verzweigt sind und höchstens 6 Kohlenstoffatome enthalten;
wobei die Produkte der Formel (I) in allen möglichen tautomeren und isomeren Formen (Racemate, Enantiomere und Diastereoisomere) vorliegen, sowie die Additionssalze der Produkte der Formel (I) mit anorganischen und organischen Säuren oder mit anorganischen und organischen Basen.

8. Produkte der Formel (I) nach einem der anderen Ansprüche, worin:
X-Y für NH-C(S), N=C-NR7R8, N=C-SR, N=C-R oder N=C-OR steht;
R1 für ein Wasserstoffatom oder einen gegebenenfalls substituierten Alkyl- oder Phenylrest steht;
R mit R1 identisch oder davon verschieden ist und unter den Werten von R1 ausgewählt ist;
R2 für ein Wasserstoffatom, ein Halogenatom oder einen Alkylrest steht;
R3 für ein Wasserstoffatom, ein Halogenatom, einen Hydroxylrest oder einen Alkylrest steht;
R4 für ein Wasserstoffatom, ein Halogenatom oder einen Alkylrest steht;
R5 für ein Wasserstoffatom ein Halogenatom einen Hydroxylrest, einen NH2-; NHalk-, N(alk)2-, NR7R8-, NH-Phenyl- oder NH-(phenylalk)rest oder einen Alkyl-, Heterocycloalkyl-, Alkoxy-, Phenyl- oder Heteroarylrest steht; wobei diese letztgenannten Reste sowie der Phenylrest von NH-Phenyl und NH(phenylalk) gegebenenfalls substituiert sind;
R6 für ein wasserstoffatom, ein Halogenatom oder einen NH2-, NHalk-, N(alk)2-, Alkyl- oder Alkoxyrest steht;
R7 und R8 so beschaffen sind, dass:
entweder R7 und R8 gleich oder verschieden sind und so beschaffen sind, dass eine der Gruppen R7 und R8 für ein Wasserstoffatom oder einen Alkylrest steht;
und die andere der Gruppen R7 und R8 für ein Wasserstoffatom oder einen gegebenenfalls substituierten Alkyl- oder Cycloalkylrest steht;
oder R7 und R8 zusammen mit dem Stickstoffatom, an das sie gebunden sind, einen unter Azetidyl, Piperidyl, Morpholinyl, Thiomorpholinyl, Pyrrolidinyl, Imidazolidinyl, Piperazinyl und Homopiperazinyl ausgewählten 4- bis 6-gliedrigen cyclischen Rest bilden, wobei diese Reste gegebenenfalls substituiert sind;
wobei alle Alkyl-, Alkoxy-, Heteroaryl- und Phenylreste sowie der cyclische Rest, den R7 und R8 zusammen mit dem Stickstoffatom, an das sie gebunden sind, bilden können, die als gegebenenfalls substituiert angegeben sind, somit gegebenenfalls durch einen oder mehrere gleiche oder verschiedene Reste substituiert sind, die unter Halogenatomen und Hydroxyl-, NH2-, NH(alk)-, N(alk)2-, NH-Phenyl-, NH(phenylalk)-, Alkyl-, CF3-, Alkoxy-, OCF3-, Cycloalkyl-, Heterocycloalkyl-,
Heteroaryl- und Phenylresten ausgewählt sind; wobei diese letztgenannten Heteroaryl- und Phenylreste sowie der Phenylrest der Reste NH-Phenyl und NH(phenylalk) gegebenenfalls selbst durch einen oder mehrere gleiche oder verschiedene Reste substituiert sind, die unter Halogenatomen und Hydroxyl-, NH2-, NHalk-, N(alk)2-, Alkyl-, Hydroxyalkyl- und Alkoxyresten ausgewählt sind;
wobei alle obigen Alkyl-(alk-) und Alkoxyreste linear oder verzweigt sind und höchstens 6 Kohlenstoffatome enthalten;
wobei die Produkte der Formel (I) in allen möglichen tautomeren und isomeren Formen (Racemate, Enantiomere und Diastereoisomere) vorliegen, sowie die Additionssalze der Produkte der Formel (I) mit anorganischen und organischen Säuren oder mit anorganischen und organischen Basen.

9. Produkte der Formel (I) nach einem der anderen Ansprüche, worin X-Y, R2, R3, R4 und R6 die in einem der anderen Ansprüche angegebenen Bedeutungen besitzen,
R1 für ein Wasserstoffatom oder einen gegebenenfalls substituierten Alkylrest steht;
R5 für ein Wasserstoffatom, ein Halogenatom, einen Hydroxyl-, CF3-, NH2-, NHalk- oder N(alk)2-Rest oder einen Alkyl-, Alkoxy- oder Phenylrest, der gegebenenfalls substituiert ist, steht;
wobei der Alkylrest, der R1 repräsentieren kann, oder der Alkyl-, Alkoxy- oder Phenylrest, der R5 repräsentieren kann, gegebenenfalls durch einen oder mehrere gleiche oder verschiedene Reste substituiert ist, die unter Halogenatomen und Hydroxyl-, NHalk-, N(alk)2-, Alkoxy-, Cycloalkyl-, Heterocycloalkyl-, Heteroaryl- und Phenylresten ausgewählt sind, wobei diese letztgenannten Heteroaryl- und Phenylreste gegebenenfalls durch einen oder mehrere gleiche oder verschiedene Reste substituiert sind, die unter Halogenatomen und Hydroxyl-, NH2-, NHalk-, N(alk)2-, Alkyl-, Hydroxyalkyl- und Alkoxyresten ausgewählt sind;
R7 und R8 so beschaffen sind, dass:
entweder R7 und R8 gleich oder verschieden sind und so beschaffen sind, dass eine der Gruppen R7 und R8 für ein Wasserstoffatom oder einen Alkylrest steht;
und die andere der Gruppen R7 und R8 für ein Wasserstoffatom oder einen Alkylrest, der gegebenenfalls durch einen oder mehrere gleiche oder verschiedene Reste substituiert ist, die unter Halogenatomen, Hydroxyl-, NH2-, NHalk-, N(alk)2-, NH (Phenyl)-, NH(Phenylalkyl)-, Alkoxy-, OCF3- und Cycloalkylresten und Pyrrolidinyl-, Piperazinyl-, Piperidyl-, Morpholinyl- und Phenylresten ausgewählt sind, steht, wobei alle diese letztgenannten cyclischen Reste sowie der Phenylrest des Phenylalkylrests gegebenenfalls selbst durch einen oder mehrere gleiche oder verschiedene Reste substituiert sind, die unter Halogenatomen und Hydroxyl-, NH2-, NHalk-, N(alk)2-, Alkoxy-, Alkyl- und Hydroxyalkylresten ausgewählt sind;
oder R7 und R8 zusammen mit dem Stickstoffatom, an das sie gebunden sind, einen Rest bilden, der vorzugsweise unter Piperidyl- und Morpholinylresten und Pyrrolidinyl-, Piperazinyl- und Homopiperazinylresten, die gegebenenfalls durch einen oder mehrere gleiche oder verschiedene Reste substituiert sind, die unter Alkyl- und Phenylresten, die gegebenenfalls selbst durch einen oder mehrere unter Halogenatomen und Hydroxyl-, NH2-, NHalk-, N(alk)2-, Alkoxy- und Cycloalkylresten ausgewählte Reste substituiert sind, ausgewählt sind, ausgewählt ist;
wobei alle obigen Alkyl-(alk-) und Alkoxyreste linear oder verzweigt sind und höchstens 6 Kohlenstoffatome enthalten;
wobei die Produkte der Formel (I) in allen möglichen tautomeren und isomeren Formen (Racemate, Enantiomere und Diastereoisomere) vorliegen, sowie die Additionssalze der Produkte der Formel (I) mit anorganischen und organischen Säuren oder mit anorganischen und organischen Basen.

10. Produkte der Formel (I) nach einem der anderen Ansprüche, worin
X-Y für ein NH-C(S), N=C-NR7R8 oder N=C-R steht;
wobei R7, R8 und R unter allen in einem der anderen Ansprüche für R7, R8 und R definierten Werten ausgewählt sind und die anderen Substituenten R1, R2, R3, R4, R5 und R6 der Produkte der Formel (I) unter allen in einem der anderen Ansprüche für R1, R2, R3, R4, R5 bzw. R6 definierten Werte ausgewählt sind,
wobei die Produkte der Formel (I) in allen möglichen tautomeren und isomeren Formen (Racemate, Enantiomere und Diastereoisomere) vorliegen, sowie die Additionssalze der Produkte der Formel (I) mit anorganischen und organischen Säuren oder mit anorganischen und organischen Basen.

11. Produkte der Formel (I) nach einem der anderen Ansprüche, worin
X-Y für NH-C(S), N=C-NR7R8 oder N=C-R steht;
R1 für ein Wasserstoffatom oder einen gegebenenfalls durch einen oder mehrere gleiche oder verschiedene Reste, die unter Halogenatomen und N(alk)2- und Alkoxyresten ausgewählt sind, substituierten Alkylrest steht;
R mit R1 identisch oder davon verschieden ist und unter den Werten von R1 ausgewählt ist;
R2 für ein Wasserstoffatom, ein Halogenatom oder einen Alkylrest steht;
R3 für ein Wasserstoffatom oder einen Alkylrest steht; R4 für ein Wasserstoffatom oder ein Halogenatom steht; R5 für ein Wasserstoffatom, ein Halogenatom oder einen Hydroxyl-, NH2-, NHalk-, N(alk)2-, Alkyl-, Alkoxy- oder Phenylrest steht, wobei der Alkylrest gegebenenfalls durch einen Alkoxy-, N(alk)2- oder Heterocycloalkylrest substituiert ist und der Phenylrest gegebenenfalls durch einen oder mehrere gleiche oder verschiedene Reste, die unter Halogenatomen und Hydroxyl-, NH2-, NHalk-, N(alk)2-, Alkyl- und Alkoxyresten ausgewählt sind, substituiert ist;
R6 für ein Wasserstoffatom, ein Halogenatom oder einen Alkylrest steht;
und R7 und R8 so beschaffen sind, dass :
entweder R7 und R8 gleich oder verschieden sind und so beschaffen sind, dass eine der Gruppen R7 und R8 für ein Wasserstoffatom oder einen Alkylrest steht und die andere der Gruppen R7 und R8 für einen gegebenenfalls durch einen Cycloalkylrest substituierten Alkylrest steht;
oder R7 und R8 zusammen mit dem Stickstoffatom, an das sie gebunden sind, einen Pyrrolidinyl-, Morpholin-, Piperidyl- oder Piperazinylrest, der gegebenenfalls durch einen Alkylrest substituiert ist, bilden;
wobei die Produkte der Formel (I) in allen möglichen tautomeren und isomeren Formen (Racemate, Enantiomere und Diastereoisomere) vorliegen, sowie die Additionssalze der Produkte der Formel (I) mit anorganischen und organischen Säuren oder mit anorganischen und organischen Basen.

12. Produkte der Formel (I) nach einem der vorhergehenden Ansprüche, worin
X-Y für NH-C(S) oder N=C-NR7R8 steht;
R1 und R2 gleich oder verschieden sind und für ein Wasserstoffatom oder einen Alkylrest stehen;
R3 für ein Wasserstoffatom steht;
R4, R5 und R6 gleich oder verschieden sind und für ein Wasserstoffatom oder ein Halogenatom stehen;
R7 und R8 für die in einem der Ansprüche 1 bis 4 definierten Werte stehen;
wobei alle obigen Alkylreste (alk-Reste) linear oder verzweigt sind und höchstens 6 Kohlenstoffatome enthalten;
wobei die Produkte der Formel (I) in allen möglichen tautomeren und isomeren Formen (Racemate, Enantiomere und Diastereoisomere) vorliegen, sowie die Additionssalze der Produkte der Formel (I) mit anorganischen und organischen Säuren oder mit anorganischen und organischen Basen.

13. Produkte der Formel (I) nach einem der anderen Ansprüche, mit den folgenden Namen:
- (5Z)-5-(1H-Pyrrolo[2,3-b]pyridin-3-ylmethylen)-2-thioxoimidazolidin-4-on
- (5Z)-5-[(5-Chlor-1H-pyrrolo[2,3-b]pyridin-3-yl)methylen]-2-thioxoimidazolidin-4-on
- (5Z)-2-[(Cyclopropylmethyl)amino]-5-(1H-pyrrolo[2,3-b]pyridin-3-ylmethylen)-3,5-dihydro-4H-imidazol-4-on
- (5Z)-5-[(5-Chlor-1H-yrrolo[2,3-b]pyridin-3-yl)methylen]-2-[(cyclopropylmethyl)amino]-3,5-dihydro-4H-imidazol-4-on
- (5Z)-5-[(6-Chlor-1H-pyrrolo[2,3-b]pyridin-3-yl)methylen]-2-[(cyclopropylmethyl)amino]-3,5-dihydro-4H-imidazol-4-on
- (5Z)-2-[(Cyclopropylmethyl)amino]-3-methyl-5-(1H-pyrrolo[2,3-b]pyridin-3-ylmethylen)-3,5-dihydro-4H-imidazol-4-on
- (5Z)-2-Butyl-5-(1H-pyrrolo[2,3-b]pyridin-3-ylmethylen)-3,5-dihydro-4H-imidazol-4-on
- (5Z)-2-Azepan-1-yl-5-[(5-chlor-1H-pyrrolo[2,3-b]pyridin-3-yl)methyliden]-3-5,-dihydro-4H-imidazol-4-on
- (5Z)-5-[(5-Chlor-1H-pyrrolo[2,3-b]pyridin-3-yl)methyliden]-2-piperidin-1-yl-3,5-dihydro-4H-imidazol-4-on
- (5Z)-5-[(5-Chlor-1H-pyrrolo[2,3-b]pyridin-3-yl)methyliden]-2-[methyl(2-methylpropyl)amino]-3,5-dihydro-4H-imidazol-4-on
- (5Z)-5-[(5-Brom-1H-pyrrolo[2,3-b]pyridin-3-yl)methyliden]-2-[(cyclopropylmethyl)amino]-3-5-dihydro-4H-imidazol-4-on
- (5Z)-2-[(Cyclopropylmethyl)amino]-5-[(5-methoxy-1H-pyrrolo[2,3-b]pyridin-3-yl)methyliden]-3,5-dihydro-4H-imidazol-4-on
- (5Z)-2-[(Cyclopropylmethyl)amino]-5-[(5-Fluor-1H-pyrrolo[2,3-b]pyridin-3-yl)methyliden]-3,5-dihydro-4H-imidazol-4-on
- (5Z)-2-Azepan-1-yl-5-[(5-Fluor-1H-pyrrolo[2,3-b]pyridin-3-yl)methyliden]-3,5-dihydro-4H-imidazol-4-on
- 3-[(Z)-{2-[(Cyclopropylemthyl)amino]-5-oxo-1,5-dihydro-4H-imidazol-4-yliden}methyl]-1H-pyrrolo[2,3-b]pyridin-5-carbonitril
- (5Z)-2-[(Cyclopropylmethyl)amino]-5-[(5-phenyl-1H-pyrrolo[2,3-b]pyridin-3-yl)methyliden]-3,5-dihydro-4H-imidazol-4-on
- (5Z)-2-[(Cyclopropylmethyl)amino]-5-([5-(trifluormethyl)-1H-pyrrolo[2,3-b]pyridin-3-yl]methyliden)-3,5-dihydro-4H-imidazol-4-on
- (5Z)-5-[(5-Chlor-1H-pyrrolo[2,3-b]pyridin-3-yl)methyliden]-2-(methylsulfanyl)-3,5-dihydro-4H-imidazol-4-on
- (5Z)-2-Butyl-5-[(5-chlor-1H-pyrrolo[2,3-b]pyridin-3-yl)methyliden]-3,5-dihydro-4H-imidazol-4-on
- (5Z)-2-(3-Methylbutyl)-5-(1H-pyrrolo[2,3-b]pyridin-3-ylmethyliden)-3,5-dihydro-4H-imidazol-4-on
- (5Z)-2-Cyclohexyl-5-(1H-pyrrolo[2,3-b]pyridin-3-ylmethyliden)-3,5-dihydro-4H-imidazol-4-on
- (5Z)-5-[(5-Chlor-1H-pyrrolo[2,3-b]pyridin-3-yl)methyliden]-3-methyl-2-(tetrahydro-2H-pyran-4-yl)-3,5-dihydro-4H-imidazol-4-on
- (5Z)-5-[(5-Chlor-1H-pyrrolo[2,3-b]pyridin-3-yl)methyliden]-3-methyl-2-(2-methylpropyl)-3,5-dihydro-4H-imidazol-4-on
- (5Z)-2-(Cyclopropylmethyl)-5-(5-chlor-1H-pyrrolo[2,3-b]pyridin-3-ylmethyliden)-3,5-dihydro-4H-imidazol-4-on
- (5Z)-2-(1-Methylethyl)-5-(5-chlor-1H-pyrrolo[2,3-b]pyridin-3-ylmethyliden)-3,5-dihydro-4H-imidazol-4-on
- (5Z)-2-[Benzyl(methyl)amino]-5-[(5-chlor-1H-pyrrolo[2,3-b]pyridin-3-yl)methyliden]-3,5-dihydro-4H-imidazol-4-on
- (5Z)-5-[(5-Chlor-1H-pyrrolo[2,3-b]pyridin-3-yl)methyliden]-2-[(2-methoxyethyl) (methyl)amino]-3,5-dihydro-4H-imidazol-4-on,
wobei die Produkte der Formel (I) in allen möglichen isomeren Formen (Racemate, Enantiomere und Diastereoisomere) vorliegen, sowie die Additionssalze der Produkte der Formel (I) mit anorganischen und organischen Säuren oder mit anorganischen und organischen Basen.

14. Produkte der Formel (I) nach einem der Ansprüche 1 bis 13 sowie die Additionssalze der Produkte der Formel (I) mit anorganischen und organischen Säuren oder mit anorganischen und organischen Basen, die pharmazeutisch unbedenklich sind, als Medikamente.

15. Produkte der Formel (I) nach Anspruch 13 sowie die Additionssalze der Produkte der Formel (I) mit anorganischen und organischen Säuren oder mit anorganischen oder organischen Basen, die pharmazeutisch unbedenklich sind, als Medikamente.

16. Pharmazeutische Zusammensetzungen, die als Wirkstoff mindestens eines der Produkte der Formel (I) nach den Ansprüchen 14 und 15 oder ein pharmazeutisch unbedenkliches Salz dieses Produkts und einen pharmazeutisch unbedenklichen Träger enthalten.

17. Verwendung eines Produkts der Formel (I) nach einem der Ansprüche 1 bis 13 zur Herstellung eines Medikaments zur Behandlung oder Prävention einer Erkrankung aus der folgenden Gruppe: Störungen der Blutgefäßproliferation, fibrotische Störungen, Störungen der Mesangialzellenproliferation, Stoffwechselstörungen, Allergien, Asthma, Thrombosen, Erkrankungen des Nervensystems, Retinopathie, Psoriasis, rheumatoide Arthritis, Diabetes, Muskeldegeneration und Krebserkrankungen.

18. Verwendung eines Produkts der Formel (I) nach einem der Ansprüche 1 bis 13 zur Herstellung eines Medikaments zur Behandlung oder Prävention einer Erkrankung aus der folgenden Gruppe: Störungen der Mesangialzellenproliferation, Psoriasis, rheumatoide Arthritis, Diabetes, Muskeldegeneration und Krebserkrankungen.

19. Verwendung eines Produkts der Formel (I) nach einem der Ansprüche 1 bis 13 zur Herstellung eines Medikaments zur Behandlung von Krebserkrankungen.

20. Verwendung nach Anspruch 19 zur Behandlung von soliden Tumoren.

21. Verwendung nach Anspruch 19 oder 20 zur Behandlung von Krebserkrankungen, die gegenüber Zytotoxika resistent sind.

22. Verwendung nach einem der Ansprüche 19 bis 21 zur Behandlung von Krebserkrankungen der Brust, des Magens, der Eierstöcke, des Kolons, der Lunge, des Gehirns, des Kehlkopfs, des Lymphsystems, des Urogenitaltrakts einschließlich Blase und Prostata und von Krebserkrankungen der Knochen und der Bauchspeicheldrüse.

23. Verwendung nach einem der Ansprüche 19 bis 21 zur Behandlung von Krebserkrankungen der Brust, des Kolons oder der Lunge.

24. Verwendung eines Produkts der Formel (I) nach einem der Ansprüche 1 bis 13 zur Herstellung von Medikamenten für die Krebschemotherapie.

25. Verwendung eines Produkts der Formel (I) nach einem der Ansprüche 1 bis 13 zur Herstellung von Medikamenten für die Krebschemotherapie, die allein oder in Kombination verwendet werden.
